(19) Europäisches Patentamt
European Patent Office
Office européen des brevets

(11) Publication number: 0 017 992 A1

# (12) EUROPEAN PATENT APPLICATION

(21) Application number: 80102076.9

(22) Date of filing: 18.04.80

(51) Int. Cl.³: C 07 D 487/04, C 07 D 205/08, A 61 K 31/40 // C07D498/04, C07D405/12, C07C125/06, C07C69/66, C07F7/08 ,(C07D487/04, 209/00, 205/00)

(30) Priority: 19.04.79 US 31694

(43) Date of publication of application: 29.10.80 Bulletin 80/22

(84) Designated Contracting States: AT BE CH DE FR GB IT LI LU NL SE

(71) Applicant: MERCK & CO. INC., 126, East Lincoln Avenue P.O. Box 2000, Rahway, New Jersey 07065 (US)

(72) Inventor: Christensen, Burton G., 195 Watchung Terrace, Scotch Plains, New Jersey 07060 (US)
Inventor: Johnston, David B.R., 53 Round Top Road, Warren, New Jersey 07060 (US)
Inventor: Schmitt, Susan M., 1949 Mary Ellen Lane, Scotch Plains, New Jersey 07076 (US)

(74) Representative: Abitz, Walter, Dr.-Ing. et al, Abitz, Morf, Gritschneder P.O. Box 86 01 09, D-8000 München 86 (DE)

## (54) 2-Substituted-6-substituted-1-carbadethiapen-2-em-3-carboxylic acids, processes for preparing them, antibiotic pharmaceutical compositions containing same and process for preparing intermediates.

(57) Disclosed is a process for the total synthesis of 1-carbapenem antibiotics (I) from L-aspartic acid via intermediates II und III:

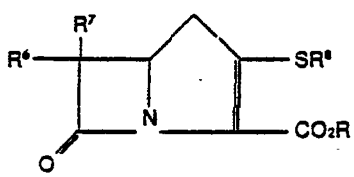
I

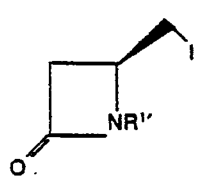
II

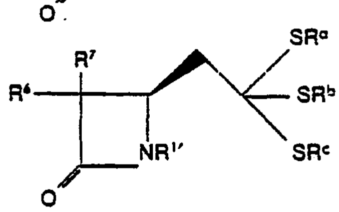
III

wherein R is hydrogen, a pharmaceutically acceptable ester moiety or salt cation, or a readily removable blocking group; $R^6$ and $R^7$ are, inter alia, independently selected from the group consisting of hydrogen, alkyl, alkenyl, aryl and aralkyl; $R^{1'}$ is hydrogen or a protecting group; and $R^a$, $R^b$ and $R^c$ are independently selected from alkyl, aryl and aralkyl.

EP 0 017 992 A1

0017992

TITLE MODIFIED
see front page



2-SUBSTITUTED-6-SUBSTITUTED-1-CARBADETHIAPEN-2-EM-3-CARBOXYLIC ACIDS, PROCESSES FOR PREPARING AND ANTIBIOTIC PHARMACEUTICAL COMPOSITIONS CONTAINING THE SAME

This invention relates to 2- and 6-substituted-1-carbadethiapen-2-em-3-carboxylic acids (I) and the pharmaceutically acceptable salt, ester and amide derivatives thereof which are useful as antibiotics:

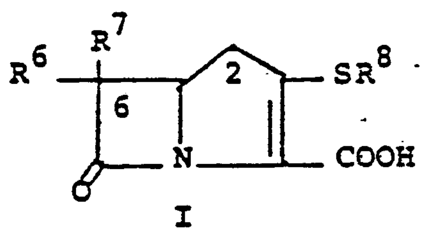

I

wherein $R^6$, $R^7$, and $R^8$ are independently selected from the group consisting of hydrogen, substituted and unsubstituted: alkyl, alkenyl, and alkynyl, having from 1-10 carbon atoms; cycloalkyl, cycloalkylalkyl, and alkylcycloalkyl, having 3-6 carbon atoms in the cycloalkyl ring and 1-6 carbon atoms in the alkyl moieties; aryl, such as phenyl; aralkyl, aralkenyl, and aralkynyl wherein the aryl moiety is phenyl and the aliphatic portion has 1-6 carbon atoms; heteroaryl, heteroaralkyl, heterocyclyl and heterocyclylalkyl; wherein the substituent or substituents relative to the above-named radicals are selected from the group consisting of:

$-X°$ halo (chloro, bromo, fluoro)

$-OH$ hydroxy

$-OR^1$ alkoxy, aryloxy

$-O\overset{O}{\overset{\|}{C}}NR^1R^2$ carbamoyloxy

$-\overset{O}{\overset{\|}{C}}NR^1R^2$ carbamoyl

$-NR^1R^2$ amino

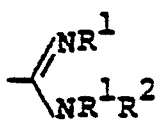 amidino

$-SO_2NR^1R^2$ sulfonamido

$-NH\overset{O}{\overset{\|}{C}}NR^1R^2$ ureido

$R^1\overset{O}{\overset{\|}{C}}NR^2-$ amido

$-CO_2H$ carboxy

$-OSO_2R^1$ sulphate

$-NO_2$ nitro

$-\overset{\oplus}{N}(R^1)_3$ tri-substituted amino ($R^1$ group independently chosen)

$-\overset{R^1}{\overset{|}{C}}=NOR^2$ oximino

$-CO_2R^1$ carboxylate

$-\overset{\overset{O}{\|}}{C}R^1$ acyl

$-O\overset{\overset{O}{\|}}{C}R^1$ acyloxy

$-SH$ mercapto

$-\overset{\overset{O}{\|}}{S}R^1$ alkyl and aryl sulfinyl

$-\underset{\underset{O}{\|}}{\overset{\overset{O}{\|}}{S}}R^1$ alkyl and aryl sulfonyl

$-CN$ cyano

$-N_3$ azido

$-SR^1$ alkyl- and arylthio

wherein, relative to the above listed substituents on $R^6$, $R^7$, and $R^8$, the groups $R^1$ and $R^2$ are independently selected from: hydrogen, alkyl, alkenyl, and alkynyl, having from 1-10 carbon atoms; cycloalkyl,

cycloalkylalkyl, and alkylcycloalkyl, having 3-6 carbon atoms in the cycloalkyl ring and 1-6 carbon atoms in the alkyl moieties; aryl, such as phenyl; aralkyl, aralkenyl, and aralkynyl wherein the aryl moiety is phenyl and the aliphatic portion has 1-6 carbon atoms; heteroalkyl, heteroaralkyl, heterocyclyl and heterocyclylalkyl and wherein the hetero atom or atoms in the above-named heterocyclic moieties are selected from the group consisting of 1-4 oxygen, nitrogen or sulphur atoms and wherein the alkyl moieties associated with said heterocyclic moieties have 1-6 carbon atoms.

Further, relative to $R^8$ radicals which carry an amino group ($-NH_2$) or an N-substituted amino group ($-NR^1H$), and which can be represented conveniently as: $-R^8-NH_2$, and $-R^8-NR^1H$, respectively, there exists the following groups classed under previously defined $R^8$:

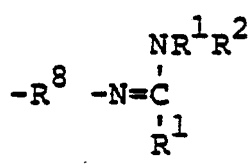 amidino

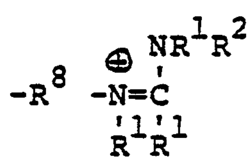 amidino

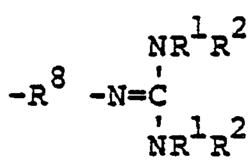 guanidino

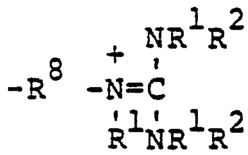 guanidino

wherein: $R^1$ and $R^2$ are as defined above.

This invention also relates to the carboxyl derivatives of I which are antibiotics and which may be represented by the following generic structure (I):

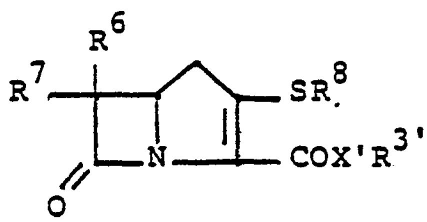

I

wherein X' is oxygen, sulphur or NR' (R' = H or lower alkyl having 1-6 carbon atoms); and $R^{3'}$ is, <u>inter alia</u>, representatively selected from the group consisting of hydrogen, conventional blocking groups such as trialkylsilyl, acyl and the pharmaceutically acceptable salt, ester and amide moieties known in bicyclic β-lactam antibiotic art; the definition of $R^{3'}$ is given in greater detail below.

This invention also relates to processes for the preparation of such compounds (I); pharmaceutical compositions comprising such compounds; and to methods of treatment comprising administering such compounds and compositions when an antibiotic effect is indicated.

There is a continuing need for new antibiotics. For unfortunately, there is no static effectiveness of any given antibiotic because continued wide scale usage selectively gives rise to resistant strains of pathogens. In addition, the known antibiotics suffer from the disadvantage of being effective only against certain types of microorganisms. Accordingly, the search for new antibiotics continues.

Thus, it is an object of the present invention to provide a novel class of antibiotics which are useful in animal and human therapy and in inaminate systems. These antibiotics are active against a broad range of pathogens which representatively include both gram positive bacteria such as S. aureus, Strep. pyogenes, and B. subtilis, and gram negative bacteria such as E. coli, Pseudomonas, Proteus morganii, Serratia, and Klebsiella. Further objects of this invention are to provide chemical processes forthe preparation of such antibiotics and their nontoxic pharmaceutically acceptable salts; pharmaceutical compositions comprising such antibiotics; and to provide methods of treatment comprising administering such antibiotics and compositions when an antibiotic effect is indicated.

## DETAILED DESCRIPTION OF THE INVENTION

The compounds of the present invention (I, above) are conveniently prepared by the following scheme:

## DIAGRAM I

$H_2C=CH-CH=CHOR^1$ + $O=C=N-SO_2Cl$

1 2

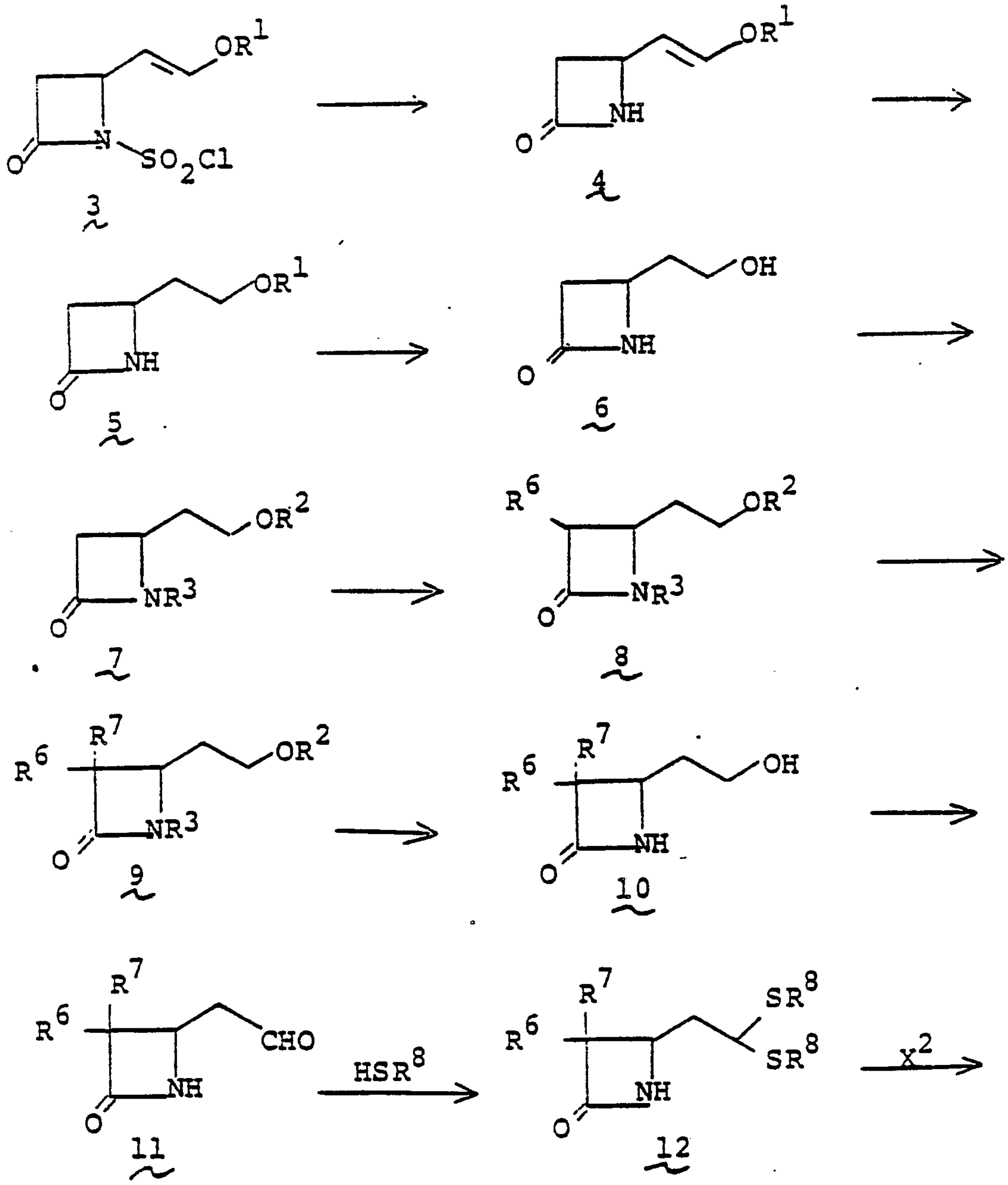


3 4

5 6

7 8

9 10

11 12

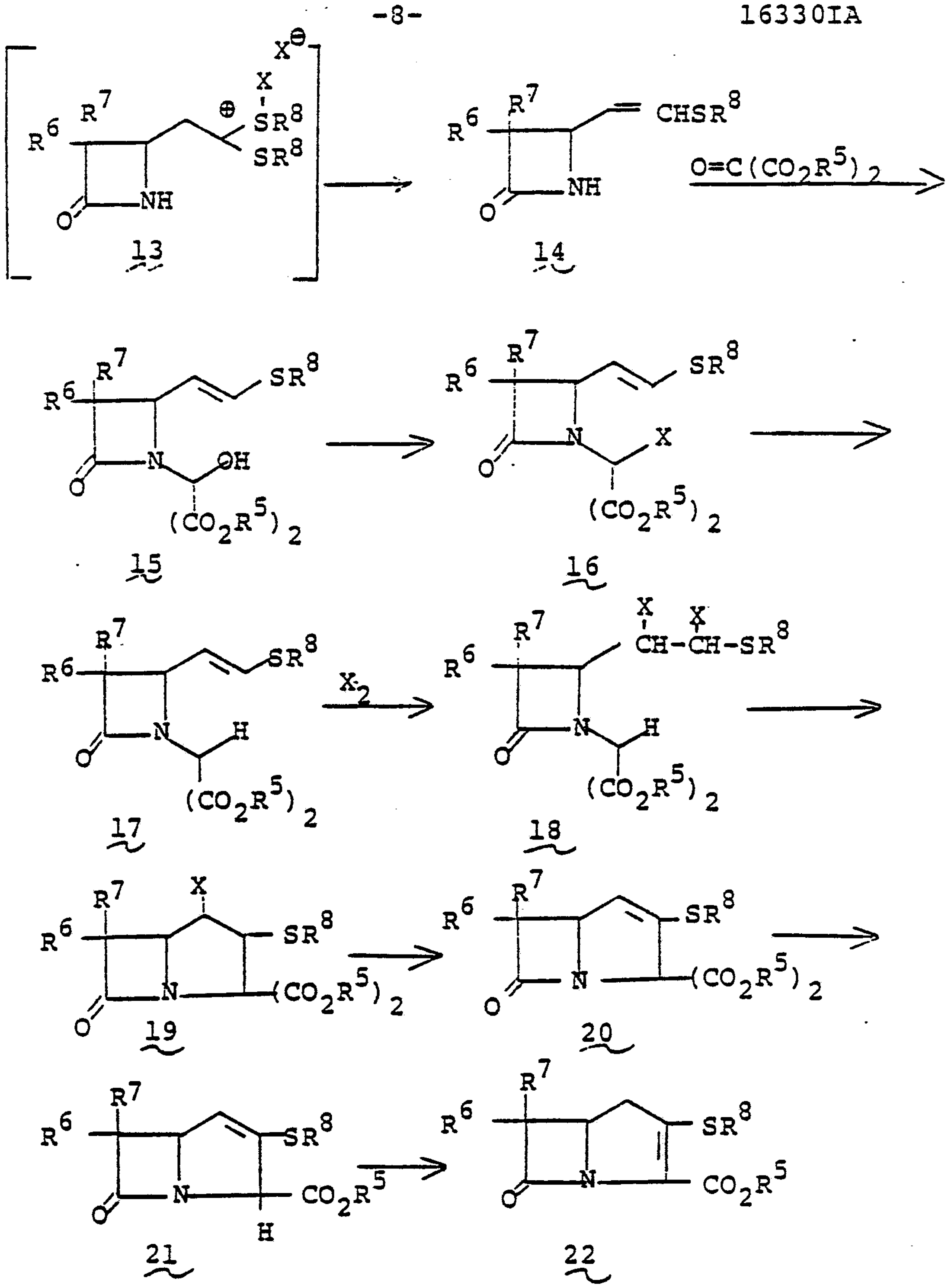


13 14 15 16 17 18 19 20 21 22

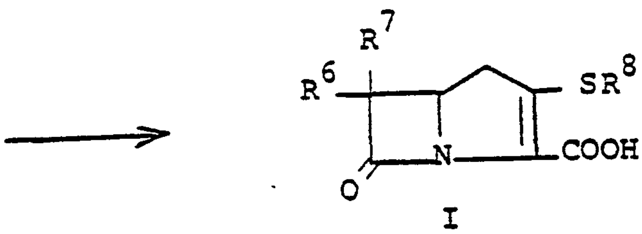

I

In words relative to the above diagram, the 4-(2-substituted-vinyl)azetidine-2-one, 4, starting material is prepared by reacting an $R^1$-oxybutadiene, 1, with chlorosulfonylisocyanate 2. The reaction is conducted without solvent or may be run in solvent such as diethyl ether, ethyl acetate, chloroform, methylene chloride, or the like, at a temperature of from -78°C to 25°C for from a few minutes to 1 hour to provide 3. The radical $R^1$ is an easily removable acyl blocking group such as alkanoyl or aralkanoyl which bears no functional group or groups which might interfere with the desired course of reaction (1 + 2 $\rightarrow$ 3 $\rightarrow$ 4). Intermediate species 3 is converted to the sulfinamide by reduction which is then hydrolyzed to 4 at pH 6-8. Typically the reaction solution comprising 3 is contacted (5-30 minutes) with an aqueous solution (at 0-25°C) of a reducing agent such as sodium sulfite, thiophenol, or the like, at pH 6-8 to provide 4.

The reaction 4 $\rightarrow$ 5 is a reduction, and is preferably achieved by hydrogenation in a solvent such as ethyl acetate, ether, dioxane, tetrahydrofuran (THF), ethanol or the like at 0 to 25°C for from 5 minutes to 2 hours under 1 to 10 atmospheres of hydrogen in the presence of a hydrogenation catalyst such as a platinum metal or oxide thereof such as 10% Pd/C or the like.

The de-blocking reaction 5 $\rightarrow$ 6 is usually desirable when $R^1$ is acyl to permit the later alkylation, 7 $\rightarrow$ 8. The preferred de-blocking procedure is by alcoholysis

wherein the solvent is a lower alkanol such as methanol, ethanol or the like in the presence of the corresponding alkali metal alkoxide, such as sodium methoxide. Typically, the reaction is conducted for from 5 minutes to 1 hour at a temperature of from -10° to 25°C.

Blocking groups $R^3$ and $R^2$ are established (6→7) to provide a suitably protected species for alkylation (7→8→9). There is no criticality in the choice of blocking groups, provided only that they do not interfere with the intended alkylation. $R^3$ may be hydrogen, a triorganosilyl group such as trimethylsilyl or the like, or a cyclic ether such as 2-tetrahydropyranyl. $R^2$ may also be a cyclic ether such as 2-tetrahydropyranyl; alternatively $R^3$ and $R^2$ may be joined together to form protected species such as 7a:

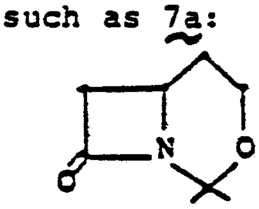

7a

For example, species such as 7a are conveniently prepared by treating 6 with 2,2-dimethoxypropane in the presence of a catalyst such as boron trifluoride etherate, toluene sulphonic acid, or the like in a solvent such as methylene chloride, ether, chloroform, dioxane or the like at a temperature of from -10°C to 35°C for from a few minutes to 1 hour. Species 7 can be mono- or dialkylated at ring position 6. Alkylation of 7 provides 8. Typically, 7 is treated with a strong base such as lithium diisopropyl amide, sodium hydride, phenyl lithium or butyl lithium and the like in a solvent such as tetrahydrofuran (THF), ether, dimethoxyethane and the like at a temperature of from -80°C to 0°C., whereupon the alkylating agent of choice, $R^6X$, is added ($R^6$ is as described above and X is chloro,

iodo or bromo; alternatively the alkylating agent may be $R^6$-tosylate, $R^6$-mesylate or an aldehyde or ketone such as acetaldehyde and the like) to provide monoalkylated species 8. When desired, dialkylated species 9 may be obtained from 8 by repeating the alkylating procedure, 7→8.

The de-blocking reaction 9→10 is typically conducted by acid hydrolysis such as aqueous acetic acid at a temperature of from 25°C to 75°C for from 5 minutes to 3 hours.

The aldehyde intermediate 11 is prepared by treating 10 with an oxidizing agent such as $CrO_3$·2 (pyridine) in $CH_3CN$, 1:1 mixture of dimethylsulfoxide and acetic anhydride, cyclohexylcarbodiimide in DMSO or the like at a temperature of from 0-25°C for from 5 minutes to 1 hour. The resulting species 11 in a solvent such acetonitrile, methylene chloride, chloroform or the like at a temperature of from -10 to 25°C is treated with an excess of the reagent $HSR^8$ in the presence of an acid catalyst such as boron trifluoride etherate, toluene sulphonic acid or the like to provide 12. Typically, the reaction requires from 1 to 60 minutes.

The vinyl sulphide 14 is obtained *via* intermediate 13 by treating 12 with a halogen such as chlorine or bromine(X=Cl or Br) in a solvent such as ether, methylene chloride, tetrahydrofuran, glyme or the like at a temperature of from -78° to 30°C for from 1 to 30 minutes, followed immediately by treating with an olefin such as cyclohexene, isobutylene, or the like in the presence of base such as triethylamine, DBU, sodium hydride, or the like in a solvent such as DMF, glyme, THF, HMPA. The solution is held at -20° to 25°C for from 1 to 8 hours to yield 14.

The vinyl sulphide species 14 is reacted with a

diester of oxomalonic acid (or its monohydrate) to provide 15. There is no criticality as to the identity of the ester moiety, $R^5$, of the oxomalonic acid. $R^5$ may be a conventional, easily removable blocking group or it may be a pharmaceutically acceptable ester moiety. Suitable ester radicals $R^5$ are p-nitrobenzyl, benzyl, o-nitrobenzyl, t-butyl, 2,2,2-trichloroethyl. The reaction 14→15 is typically conducted in a high boiling organic solvent such as benzene, toluene, cyclohexane, halo aromatic or the like at a temperature of from about 50°C to reflux for from 0.5 to 6 hours.

The halogenation reaction 15→16 is typically conducted in a solvent such as THF, glyme, ether, methylene chloride, chloroform or the like in the presence of a halogenating agent such as thionyl chloride, phosphorous pentachloride or the like in the presence of base such as pyridine at a temperature of from -20° to 25°C for from 5 minutes to 3 hours. The selective reduction of 15→17 via 16 is completed by treating 16 with tributylphosphine, triphenylphosphine or the like in aqueous DMF or similar aqueous systems involving dioxane, THF, glyme, DMSO, or acetone at a temperature of from about 0-50°C for from 10 minutes to 5 hours.

Species 17 is halogenated by the previous procedure (12→13), but omitting the addition of the cyclohexene or other olefin, to provide the dihalo species 18. Species 18 is treated with a base such as triethylamine, sodium hydride or potassium hydride in a solvent such as DMF, acetonitrile, methylene chloride, chloroform, glyme or the like at a temperature of from about -78° to 25°C for 1 to 5 hours to provide 19. Species 19 is converted to 20 on treatment with a strong base such as 1,5-diazabicyclo

[5·4·0]-undec-5-ene(DBU),1,5-diazabicyclo[3·4·0]non-5-ene(DBN), or the like in a solvent such as DMSO, acetone, chloroform, DMF, THF, glyme or the like or on treatment with AgF in pyridine at a temperature of from 0-40°C for from 1/4 to 24 hours. The reaction 20→21 is conducted by treating 20 with an aromatic base such as pyridine, aqueous dimethylsulfoxide, s-collidine or lutidine, in the presence of a displacing agent such as lithium iodide, sodium chloride, lithium bromide, sodium bromide, or the like at a temperature of from about 80-150°C for from 15 minutes to 2 hours. An aqueous work up of the resulting reaction mixture provides 21. Isomerization of the double bond 21→22 is accomplished by treating 21 in a solvent such as DMF, DMSO, ethyl ether, THF, glyme, methylene chloride with a strong base such as diisopropylamine, DBU, DBN, or the like at a temperature of from 0° to about 25°C for from a few minutes to 2 hours or until equilibrium has been established as determined by examination of sample aliquots by ultraviolet absorption or by thin layer chromatography. The final reaction 22→I (hydrogenolysis of the blocking group) is accomplished by treating 22 in a solvent such as dioxane, ethanol, THF or the like or an aqueous mixture thereof in the presence of a Platinum metal catalyst such as Pd/C under a hydrogen pressure of from 1-4 atmospheres for from 0.5 to 8 hours at a temperature of from about 0-25°C.

The above-described total synthesis may also advantageously start with 4-vinyl azetidinone[(23), below; E.J. Moriconi, W.C. Meyer, J. Org. Chem., 36, 2841(1971)] rather than the enol acylate azetidinone (4, above). This variation in the total synthesis has the advantage of conveniently imparting stereoselectivity to the process at an early stage. The

following scheme illustrates this 4-vinyl azetidinone embodiment of the present invention; notice that it ties into the above scheme at species 14.

DIAGRAM II

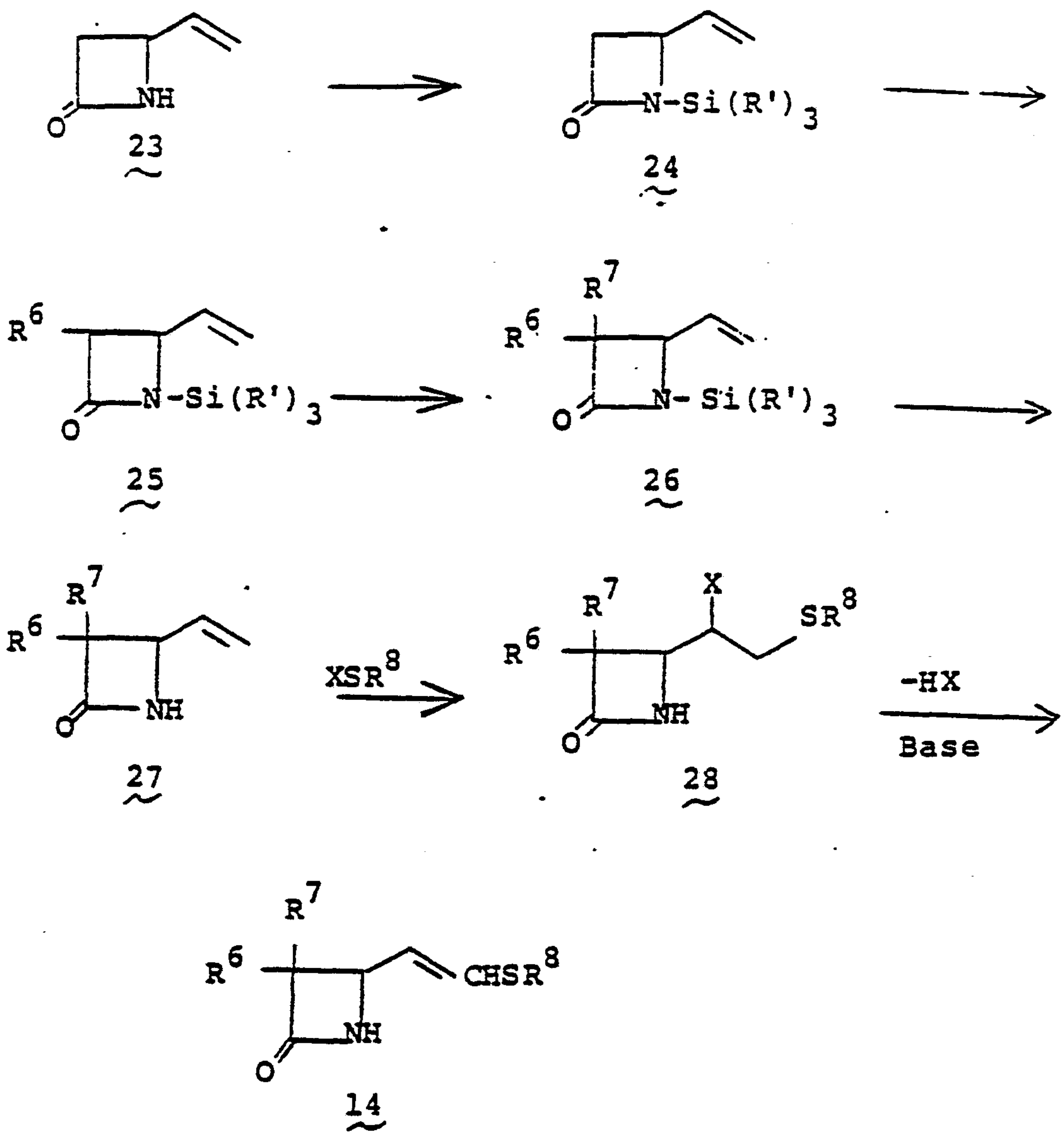


23

24

25

26

27

28

14

In words relative to the above reaction diagram, 4-vinyl azetidinone 23 is silylated to provide the N-silyl species 24. The groups R' on the silyl radical are lower alkyl having from 1-6 carbon atoms especially preferred triorganosilyl groups are trimethylsilyl and t-butyl-dimethylsilyl. Typically, the silylation (23→24) is achieved by treating 23 in a solvent such as DMF, DMSO, HMPA or the like with the silylating agent of choice, dimethyl t-butylsilyl chloride, and a base such as $Et_3N$, pyridine, N,N-dimethylaniline and the like at a temperature of from -10° to 30°C for from 1 to 8 hours. Species 24 is alkylated to form 25 or 26 and this alkylation is conducted exactly as described above for the alkylation 7→8→9. It should be noted here that the reactions (24→25) and (25→26) represent convenient opportunities to separate species 25 and 26 into their racemic diastereoisomers if desired. The removal of the N-triorganosilyl group is accomplished in reaction 26→27 by mild acid catalyzed solvolysis. The halo sulfide species 28 is obtained from 27 by treating 27 in a solvent such as methylene chloride, THF, glyme, or the like with the reagent $XSR^8$ wherein $R^8$ has previously been defined and X is halogen such as chloro or bromo at a temperature of from -50° to 50°C for from 1 to 16 hours. The vinyl sulfide intermediate 14, which is common to the above illustrated scheme of total synthesis is obtained from 28 by elimination of HX on treatment of 28 with a base such as 1,5-diazabicyclo[5·4·0]undec-5-ene (DBU), 1,5-diazabicyclo[4·3·0]non-5-ene, (DBN), 1,4-diazabicyclo[2·2·2]octane, (DABCO), or silver fluoride in a solvent such as DMSO, pyridine,DMF, HMPA or the like at a temperature of from -20° to 50°C for from 1/4 to 16 hours.

HSR$^8$ and XSR$^8$ REAGENTS

Relative to the foregoing description of the invention, suitable reagents HSR$^8$ utilized in the transformation (11→12) and XSR$^8$ (27→28) are listed below. The list is arranged according to structural and functional characteristics of the thia side chain -SR$^8$; annotation is provided where necessary. [It should be noted that only HSR$^8$ reagents are expressly shown. The reagents XSR$^8$ (X = Cl or Br) are shown implicitly for each entry on permissible substitution of Cl or Br for "H" in HSR$^8$.] The thia side chain of choice is derived from the corresponding mercaptan reagent HSR$^8$. When the mercaptan contains a functional group which might interfere with the intended course of reaction, the offending group is covered. For example, when a basic nitrogen group is encountered (-NHR or $-NH_2$, for example) it is usually protected by acylation (e.g., $-CO_2$PNB) and when a carboxyl group ($-CO_2H$) is present, it is usually protected by esterification (e.g., PNB ester). Such protection also facilitates in the purification of products by chromatographic means. (PNB is p-nitrobenzyl). It should be noted that the processes incorporated by reference below [1.) Process for the Preparation of 1-Carbapenems and Intermediates via 4-Allylazetidinone; 2.) Process for the Preparation of 1-Carbapenems and Intermediates via Trithioorthoacetates; and 3.) Process for the Preparation of 1-Carbapenems and Intermediates via Silyl-Substituted Dithioacetals] are preferred when R$^8$ is unsaturated.

1.) <u>Aliphatic Mercaptans</u>: $HSR^8$ wherein $R^8$ is 1-10 carbon alkyl, cycloalkyl, alkenyl, cycloalkenyl, or alkynyl; $R^8$ may be branched or unbranched,

<u>Examples</u>

$HSCH_3$

$HSCH_2CH_3$

$HSCH_2CH_2CH_3$

$HSCH(CH_3)_2$

$HS(CH_2)_3CH_3$

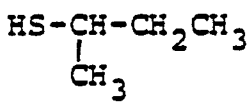

$HSCH_2CH(CH_3)_2$

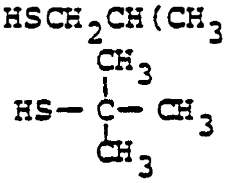

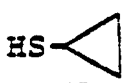

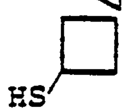

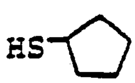

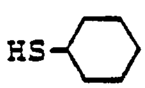

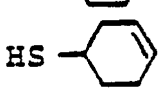

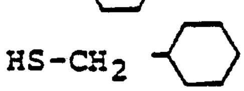

$HS-CH_2-CH=CH_2$
$HS-CH_2-CH=C(CH_3)_2$
$HS-CH_2-C\equiv CH$
$HS-CH_2-C\equiv C-CH_3$

2.) <u>Substituted Aliphatic Mercaptans</u>: $HSR^8$ wherein $R^8$ is a 1-10 carbon branched or unbranched alkyl, cycloalkyl, alkenyl, cycloalkenyl, or alkynyl group substituted by one or more halo, $OH$, $OR^1$, $O\overset{O}{C}R^1$, $O\overset{O}{C}NH_2$, $O\overset{O}{C}NHR^1R^2$, $NH_2$, $NHR^1$, $NR^1R^2$, $\overset{O}{C}R^1$, $CO_2H$, $CO_2R^1$, $CONH_2$, $CONHR^1$, $CONHR^1R^2$, $CN$, $SR^1$, $\overset{O}{S}R^1$, $SO_2R^1$, $SO_2NH_2$, $SO_2NHR^1$, $SO_2NR^1R^2$, $NH\overset{O}{C}R^1$, $NH\overset{O}{C}NH_2$, $NH\overset{O}{C}NHR^1$, $NH\overset{O}{C}NR^1R^2$, $NH\overset{O}{C}OR^1$, $\overset{NH}{C}NH_2$, $\overset{NR^1}{C}NHR^2$, wherein $R^1$ and $R^2$ are as previously defined relative to substituents on $R^8$. Preferred substituents are basic nitrogen-containing groups.

<u>EXAMPLES</u>

| | |
|---|---|
| $HS(CH_2)_nOR^1$ | $n = 2-4$, $R^1 = H$, $\overset{O}{C}CH_3$, $CH_3$ |
| $HS(CH_2)_n\overset{O}{C}XR$ | $n=1-3$, $X=O$, $NH$, $NR^1$; $R$, $R^1=H$, $CH_3$ |
| $HS(CH_2)_nNH_2$ | $n=2-4$ |
| $HS(CH_2)_nNHR^1$ | $n=2-4$, $R^1=CH_3$, $CH_2CH_3$, $CH_2CH_2CH_3$, $\overset{O}{C}CH_3$ |
| $HS(CH_2)_nNR^1R^2$ | $n=2-4$, $R^1/R^2=CH_3$, $CH_2CH_3$ |
| $HS-\overset{CH_3}{\overset{\vert}{C}}H-CH_2NH_2$ | |

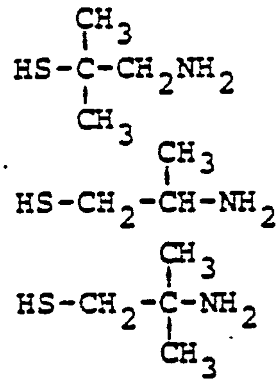

$HS-CH_2CH_2SCH_3$

$HS-CH_2CH_2NHC(CH_3)_3$

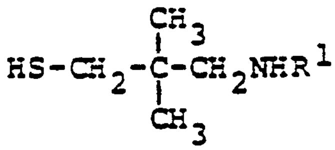

$R^1 = H,\ CH_3,\ \overset{O}{\ddot{C}}CH_3$

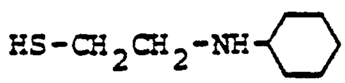

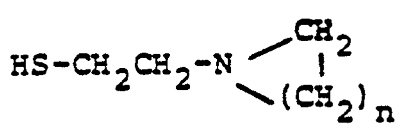

n=3-5

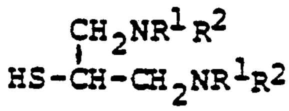

$R^1$=H, $CH_3$ ;    $R^2$ = H, $CH_3$

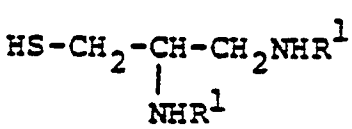

$R^1$=H, $CH_3$

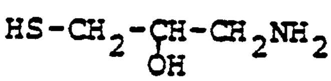

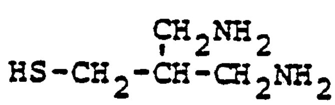

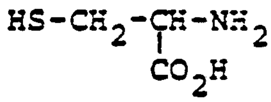

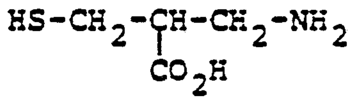

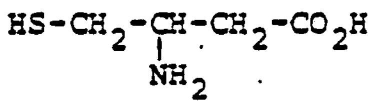

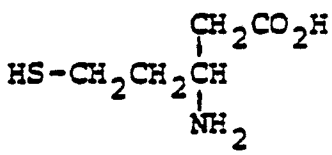

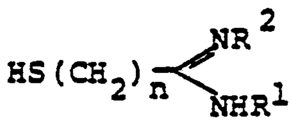

$n=1-3, R^2=H, CH_3, R^1=H, CH_3$

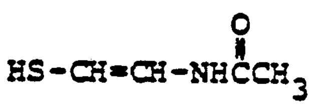

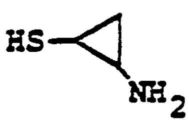

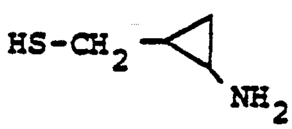

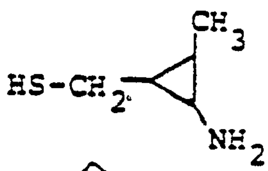

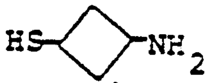

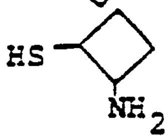

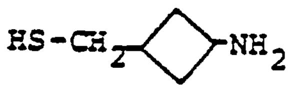

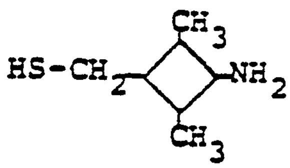

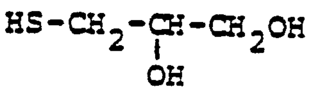

5-thio-D-glucose

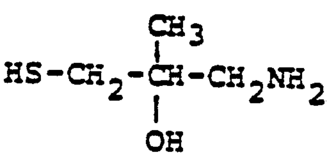

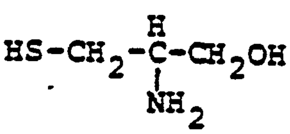

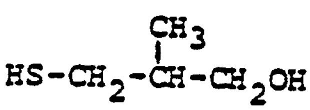

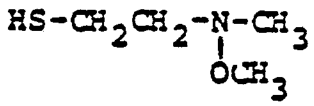

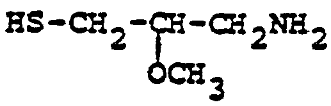

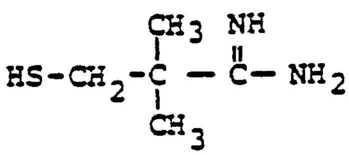

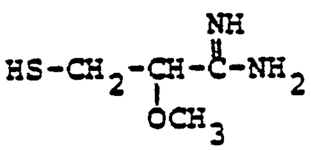

$HS-CH_2-CH=CH-CH_2NH_2$

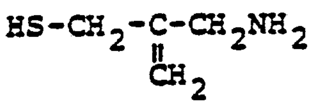

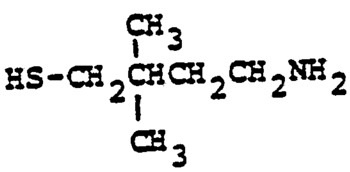

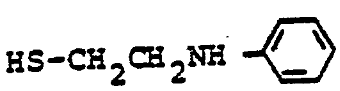

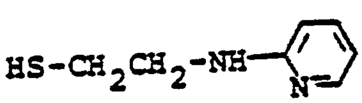

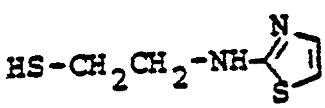

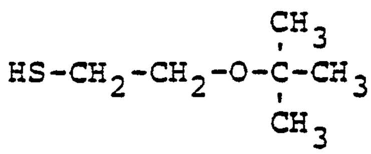

3.) Aryl Mercaptans: $HSR^8$ wherein $R^8$ is phenyl or substituted phenyl. The substituents are independently selected from those previously defined for $R^8$. Especially preferred substituents include alkyl, halo, hydroxy, alkoxy, acyloxy, acyl, carboxy, mercapto, sulfinyl, sulfonyl, amino, substituted amino, aminoalkyl, substituted aminoalkyl, amido, and ureido.

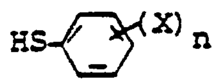

n= 1, 2 or 3,
X= F, Cl, Br, OH, OR, $O\overset{O}{\overset{\|}{C}}R^1$, $NH_2$, $NHR^1$, $NR^1R^2$, $CH_2NH_2$, $CH_2NR^1R^2$, $CO_2H$, $CO_2R^1$, $COR^1$, $CONH_2$, $CONR^1R^2$, $R^1CONH$, $R^1NHCONH$, $SR^1$, $\overset{O}{\overset{\|}{S}}R^1$, $SO_2R^1$, $CH_3$, $CF_3$;
$R^1$ and $R^2$ are as previously defined under $R^8$.

Examples

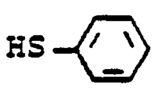

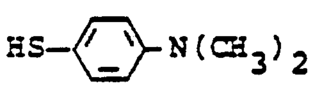

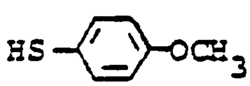

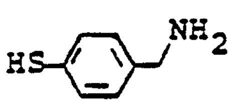

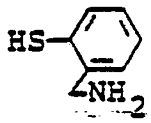

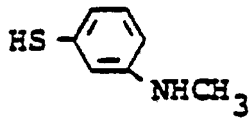

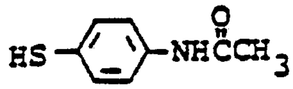

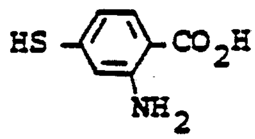

4.) Heteroaryl Mercaptans: $HSR^8$ wherein $R^8$ is a substituted or unsubstituted heteroaryl group containing 1-4 O, N or S atoms. Typical substituents include those mentioned above under "Aryl Mercaptans".

Examples

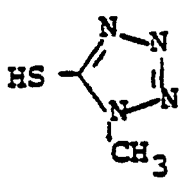

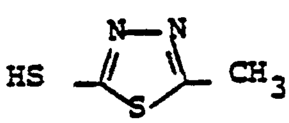

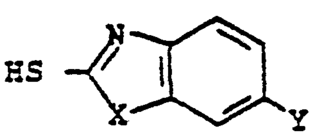

X=N,O Y=H
X=S Y=H, Cl, $OCH_2CH_3$

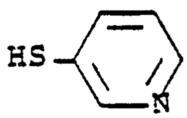

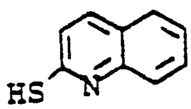

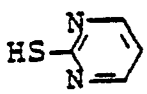

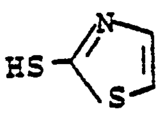

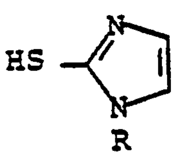

R=H, $CH_3$

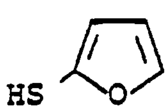

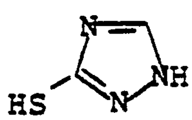

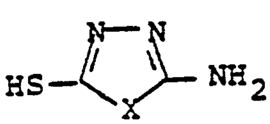

X=NH, S

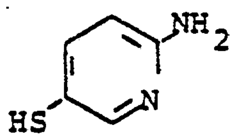

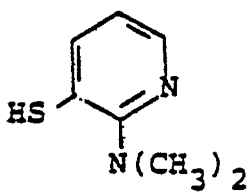

5.) <u>Arylaliphatic Mercaptans</u>: $HSR^8$ where $R^8$ is a 1-6 carbon branched or unbranched alkyl, cycloalkyl, alkenyl, or alkynyl group substituted by a phenyl or substituted phenyl group. Typical phenyl substituents include those mentioned under "Aryl Mercaptans".

<u>Examples</u>

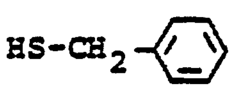

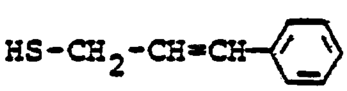

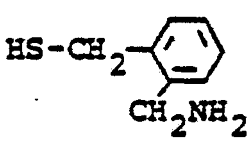

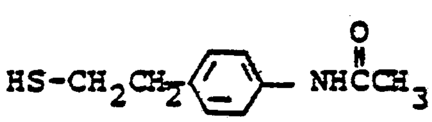

6.) Heteroarylaliphatic and Heterocyclylaliphatic Mercaptans

$HSR^8$ wherein $R^8$ is a 1-6 carbon branched or unbranched alkyl, cycloalkyl, alkenyl, or alkynyl group substituted by a heteroaryl or heterocyclyl group containing 1-4, O, N, or S atoms. The heteroaryl or heterocyclic group is unsubstituted or substituted by those substituents mentioned under "Aryl Mercaptans", (No. 3 above).

Examples

$HS-(CH_2)_n$ 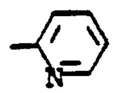

$HS-(CH_2)_n$ 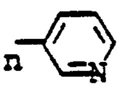

$HS-(CH_2)_n$ 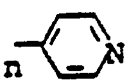 $n=1,2$

$HS-(CH_2)_n$ 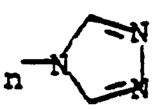

$HS-(CH_2)_n$ 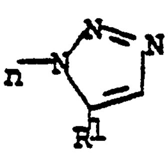 $R^1 = OCH_2CH_3$

$HS-(CH_2)_n$ 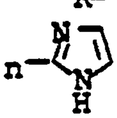

$HS-(CH_2)_n$ 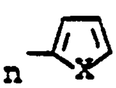

$HS-(CH_2)_n$ 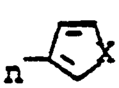 X= O, S, NH

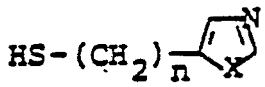

X = O, S, NH

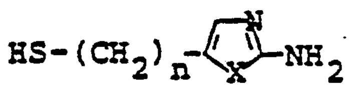

X = O, S, NH

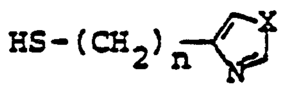

X = O, S, NH

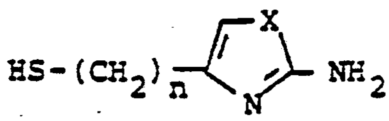

X = O, S, NH

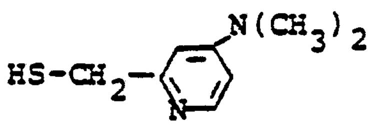

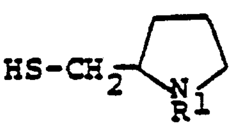

$R^1$ = H, $CH_3$

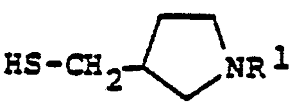

$R^1$=H, $CH_3$

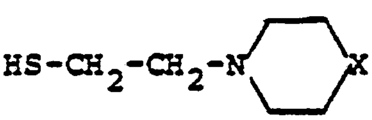

X = O, NH, $NCH_3$

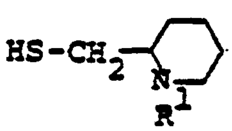

$R^1$ = H, $CH_3$

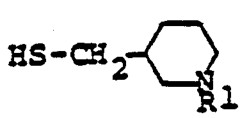

$R^1$ = H, $CH_3$

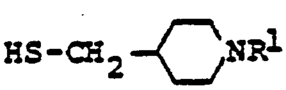

$R^1$=H, $CH_3$

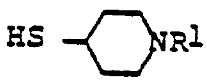

$R^1 = H, CH_3$

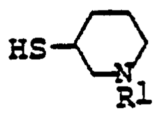

$R^1 = H, CH_3$

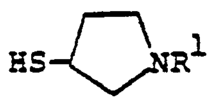

$R^1 = H, CH_3$

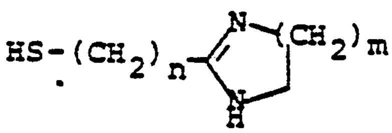

$n = 1-3$, $m = 1-3$

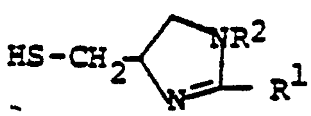

$R^2 = H, CH_3$, $R^1 = H, CH_3, NH_2$

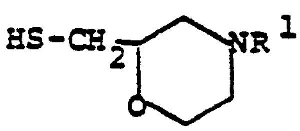

$R^1 = H, CH_3$

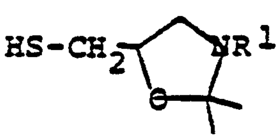

$R^1 = H, CH_3$

7.) <u>Alkyl-Heteroatom-Alkyl Mercaptans, $HSR^8$</u>

Wherein $R^8$ is

$-(CH_2)_nX(CH_2)_mR^9$

wherein n = 2 to 4, m = 2 to 4; X is $NR^{\circ}$, O or S; and wherein $R^{\circ}$ is H, $CH_3$, $CH_2CH_3$, $CH_2CH_2OH$, or $CH_2CH_2NH_2$ and $R^9$ is OH, $NH_2$, $NHCH_3$, $N(CH_3)_2$, $O\underset{O}{\underset{\|}{C}}CH_3$, $NH\underset{O}{\underset{\|}{C}}CH_3$.

Note, in the above representation, the methylene carbons may be branched; for example: $-\underset{CH_3}{\underset{|}{CH}}-$ ,

$$-\overset{CH_3}{\overset{|}{\underset{CH_3}{\underset{|}{C}}}}-$$

, and the like.

The following $HSR^8$ are representative of this class:

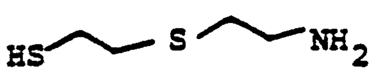

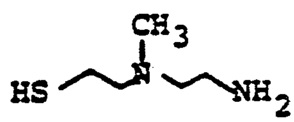

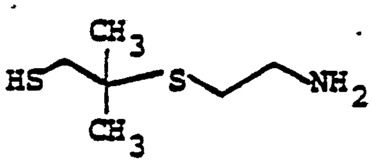

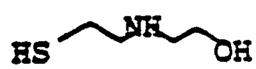

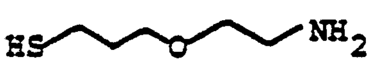

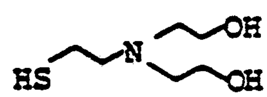

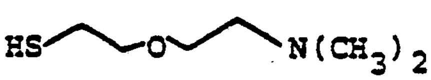

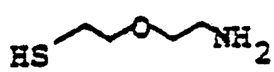

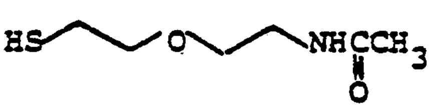

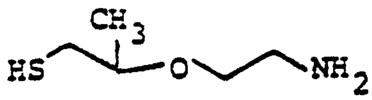

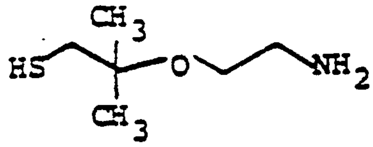

## ALKYLATING AND ACYLATING REAGENTS FOR ESTABLISHING $R^6$ AND $R^7$

Relative to Diagrams I and II above, the establishment of $R^6$ and $R^7$ by alkylation has been shown (7 ⟶ 8 ⟶ 9, Diagram I; and analogously 24 → 25 ⟶ 26, Diagram II). There is yet a third scheme for establishing $R^6$ and $R^7$. It involves direct acylation followed by reduction. The schemes are conveniently compared below (Diagram III) and, there following, is a representative list of suitable alkylating and acylating reagents for establishing $R^6$ and $R^7$.

### DIAGRAM III
(Scheme I)

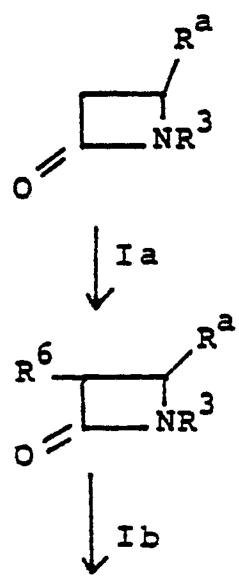

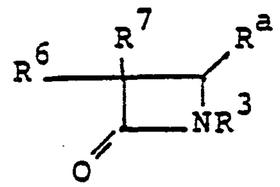

Ic

wherein: $R^a$ is $CH_2CH_2OR^2$ or $CH=CH_2$ ;
$R^3$ and $R^2$ are as defined above.

In words relative to the above reaction diagram, and as described above, starting material Ia can be mono-, or dialkylated at ring position 3. Alkylation of Ia provides Ic. Typically, Ia is treated with a strong base such as lithium diisopropylamide, lithium 2,2,6,6-tetramethylpiperidide, potassium hydride, lithium hexamethyldisilazane, phenyllithium or the like in a solvent such as tetrahydrofuran (THF), hexamethylphosphoramide, ether, dimethoxyethane, and the like at a temperature of from -80°C to 0°C whereupon the alkylating agent of choice, $R^6X°$ is added (X° is chloro, iodo or bromo); alternatively the alkylating agent may be $R^6$-tosylate, $R^6$-mesylate or an aldehyde or ketone such as acetaldehyde to provide monoalkylated species Ib. When desired, dialkylated species Ic may be obtained from Ib by repeating the alkylating procedures Ia $\longrightarrow$ Ib.

The eventual 6-substituents (nomenclature relative to final, bicyclic structure) can also be established by direct acylation using an acylating agent such as N-acyl imidazole or the like. Such N-acyl imidazole acylating reagents are listed below.

Also given below is a detailed description of this second approach for establishing, $R^6$ and $R^7$.

The following list is representative of useful <u>alkylating</u> agents for establishing $R^6$ and $R^7$, according to the above scheme 7 → → 9; and 24 → → 26 (this will be referred to as Scheme I, to be distinguished from Scheme II, below, which involves <u>acylation</u>):

Alkylating Agents

$CH_3CHO$

$\text{Ø}CH_2CHO$ Ø = phenyl

$\text{Ø}CH_2CH_2CHO$

$CH_2O$

$CH_3I$

$\text{Ø}CH_2Br$

$CH_3COCH_3$

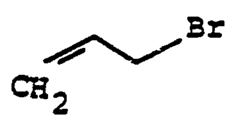

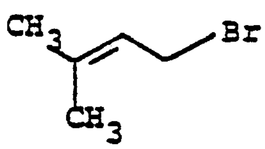

$CH_3OCH_2CHO$

$CH_3CH_2I$

$(CH_3)_2CHI$

$N_3CH_2CHO$

$(CH_3)_2NCH_2CHO$

$RO_2CCH_2Br$ R = $CH_3$, benzyl, p-nitrobenzyl

$CF_3CF_2CHO$

$RO_2CCH_2CHO$ R = $CH_3$, benzyl, p-nitrobenzyl

$CH_3CH(CH_3)CHO$,

$CH_3(CH_3)CHCH_2CHO$,

$CH_3CH_2CHO$ ,

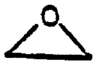 ,

$CF_3CHO$,

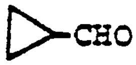

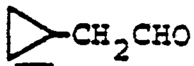

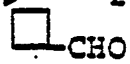

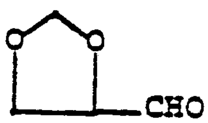

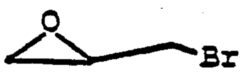

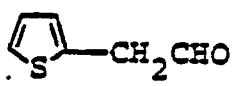 ,

$[(CH_3)_3C](CH_3)_2SiOCH_2\overset{O}{\overset{\|}{C}}H$

$F_2CH\overset{O}{\overset{\|}{C}}H$

$FCH_2\overset{O}{\overset{\|}{C}}H$

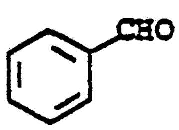

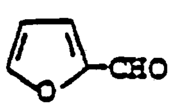

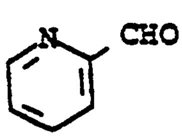

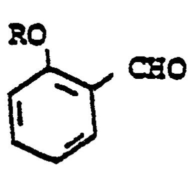

R = protecting group

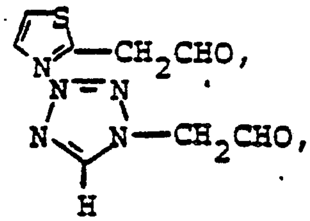

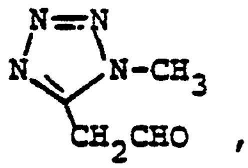

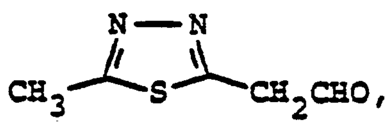

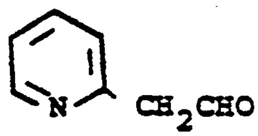

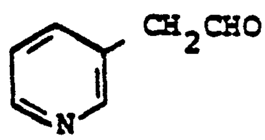

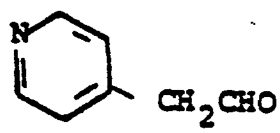

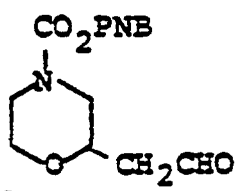

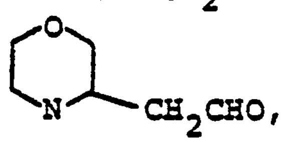

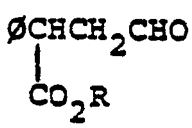

R is removable carboxyl protecting group, such as benzyl.

As mentioned above, the 6-substituents may also be established by acylation. Utilization of such acylating agents may be demonstrated in the following manner with regard to a preferred starting material Ib or Ic:

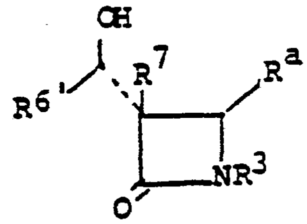

Ib/Ic

wherein $R^7$, $R^a$ and $R^3$ are as defined above. $R^{6'}$ is defined relative to the definition of $R^6$ and in that sense is the balance of the previously identified group $R^6$. In other words, for purposes of this definition $R^{6'}CH(OH)- = R^6$. An especially preferred material Ib is when $R^7$ is hydrogen and $R^{6'}$ is methyl. Basically, such 1'-hydroxy $R6'$ species Ib are prepared according to the following scheme:

<u>SCHEME II</u>

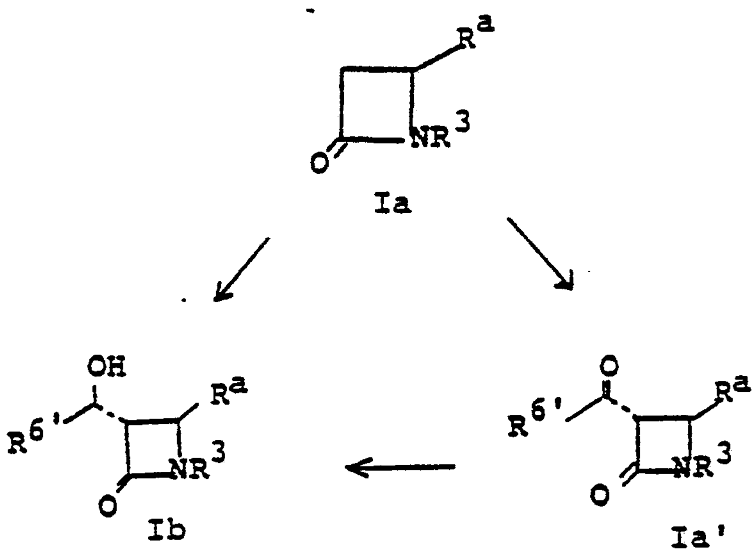

Ia

Ib

Ia'

The alkylation Ia ⟶ Ib, Scheme II, is accomplished as previously described, by treating Ia in a solvent such as tetrahydrofuran, dimethoxyethane, diethylether, hexamethylphosphoramide, at a temperature of from -100° to -20°C with a strong base such as lithium diisopropylamide, lithium hexamethyldisilazide, lithium 2,2,6,6-tetramethylpiperidide, potassium hydride or the like followed by the addition of an equivalent to 10 fold excess of an aldehyde. This reaction gives a mixture of isomers from which the desired <u>trans</u>-R form Ib can be conveniently separated by chromatography or crystallization.

Intermediate Ia may proceed directly to Ib as indicated above, or it may take the circuitous path <u>via</u> Ia'. The direct acylation, to Ia' is accomplished by treating Ia with two or more equivalents of a base such as lithium diisopropylamide, lithium hexamethyldisilazide, lithium 2,2,6,6-tetramethylpiperidide, in a solvent such as tetrahydrofuran, diethylether, or dimethoxyethane, for example, at a temperature of from -100 to -20°C with an acylating agent such as N-acyl imidazole or the like. Addition of the Ia plus base mixture to the acylating agent is preferred.

Representative acylating agents for this scheme Ia → Ia' → Ib are listed below.

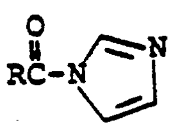 ; R=$CH_3$, $ClCH_2$, $CH_3CH_2$, $N_3CH_2$, $CH_3OCH_2$,

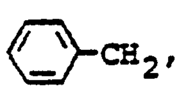, 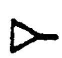, 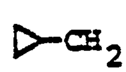, 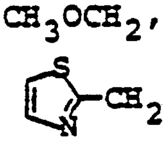,

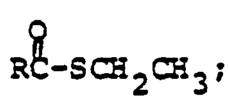; R=$CF_3$, $CF_2H$, $CH_2$=CH, 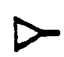,

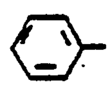, 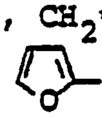

Further with respect to Scheme II, the reduction, Ia' → Ib is accomplished by contacting the ketone with a reducing agent such as potassium tri(sec-butyl)borohydride, lithium tri(sec-butyl)borohydride, sodium borohydride, sodium tris(methoxyethoxy)aluminum hydride, lithium aluminum hydride or the like in a solvent such as diethylether, tetrahydrofuran, toluene, i-propanol or the like at a temperature of from -78° to 25°C. The reaction can conveniently be conducted in the presence of an added complexing salt such as potassium iodide, magnesium bromide or the like.

In a similar manner, unresolved Ib (<u>cis</u> and <u>trans</u>) may be oxidized to Ia' for reduction to Ib as indicated above:

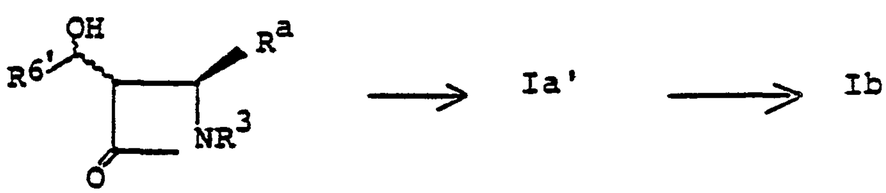


The oxidation is accomplished with an oxidizing agent such as dipyridine chromium (VI) oxide, trifluoroacetic anhydride-dimethylsulfoxide-triethylamine, pyridinium dichromate, acetic anhydride-dimethylsulfoxide in a solvent such as methylene chloride, acetonitrile, or the like at a temperature of from -78 to 25°C for from 5 minutes to 5 hours.

As noted above, the compounds of the present invention may also generally be represented by the following structural formula:

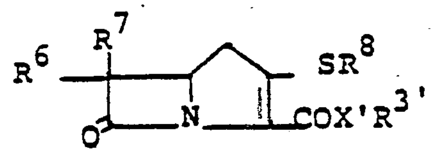

I

wherein X' is oxygen, sulfur or NR' (R' is hydrogen or loweralkyl having from 1 to 6 carbon atoms); and $R^{3'}$ is hydrogen, or, <u>inter alia</u>, is representatively selected to provide the pharmaceutically acceptable salt, ester, anhydride ($R^{3'}$ is acyl), and amide moieties known in the bicyclic β-lactam antibiotic art; $R^{3'}$ may also be a readily removable blocking group.

Identification of the Radical $-COX'R^{3'}$

In the generic representation of the compounds of the present invention (I, above), the radical represented by $-COX'R^{3'}$ is, *inter alia*, -COOH (X' is oxygen and $R^{3'}$ is hydrogen) and all radicals known to be effective as pharmaceutically acceptable ester, anhydride ($R^{3'}$ is acyl) and amide radicals in the bicyclic β-lactam antibiotic art, such as the cephalosporins and penicillins and nuclear analogues thereof.

Suitable blocking esters ($R^{3'}$, X' = O) include those selected from the following list which is representative:

(i) $R^{3'}$ = $CR^aR^bR^c$ wherein at least one of $R^a$, $R^b$ and $R^c$ is an electron-donor, e.g., p-methoxyphenyl. The remaining $R^a$, $R^b$ and $R^c$ groups may be hydrogen or organic substituting groups. Suitable ester groups of this type include p-methoxybenzyloyxcarbonyl.

(ii) $R^{3'}$ = $CR^aR^bR^c$ wherein at least one of $R^a$, $R^b$ and $R^c$ is an electron-attracting group, e.g., p-nitrophenyl, trichloromethyl, and o-nitrophenyl. Suitable esters of this type include p-nitrobenzyloxycarbonyl, and 2,2,2-trichloroethoxycarbonyl.

(iii) $R^{3'}$ = $CR^aR^bR^c$ wherein at least two of $R^a$, $R^b$ and $R^c$ are hydrocarbon such as alkyl, e.g., methyl or ethyl, or aryl, e.g., phenyl and the remaining $R^a$, $R^b$ and $R^c$ group, if there is one, is hydrogen. Suitable esters of this type include t-butyloxycarbonyl, diphenylmethoxycarbonyl and triphenylmethoxycarbonyl.

Silyl esters, under this category of blocking groups, may conveniently be prepared from a halosilane of the formula: $R^4{}_3SiX'$
wherein X' is a halogen such as chloro or bromo and $R^4$ is alkyl, e.g., methyl, ethyl, t-butyl.

Pharmaceutically acceptable carboxyl derivatives of the present invention are those derived by reacting I with alcohols, acylating reagents

and the like. For example, esters and amides of interest are the above-listed starting materials and final products having the $-COX'R^{3'}$ group at the 3-position; wherein X' is oxygen, sulfur or NR' (R' is H or $R^{3'}$), and $R^{3'}$ is alkyl having 1-6 carbon atoms, straight or branched, such as methyl, ethyl, t-butyl, and the like; carbonylmethyl, including phenacyl; aminoalkyl including 2-methylaminoethyl, 2-diethylaminoethyl; alkanoyloxyalkyl wherein the alkanoyloxy portion is straight or branched and has 1-6 carbon atoms and the alkylportion has 1-6 carbon atoms, such as pivaloyloxymethyl; haloalkyl wherein halo is chloro, and the alkyl portion is straight or branched having 1-6 carbon atoms, e.g., 2,2,2-trichloroethyl; alkenyl having 1-4 carbon atoms such, as 2-propenyl, 3-butenyl, and 4-butenyl; aralkyl and lower alkoxyl- and nitro- substituted aralkyl such as benzyl, benzhydryl, o-nitrobenzyl, p-methoxybenzyl, and p-nitrobenzyl; phthalidyl; benzyloxyalkyl having 8-10 carbon atoms such as benzyloxymethyl, and (4-nitro) benzyloxymethyl.

In addition to the esters (and thio esters) listed above, amides are also embraced by the present invention, i.e., wherein X' is the $-\overset{R'}{N}-$ group. Representative of such amides are those wherein R' is selected from the group consisting of hydrogen and lower alkyl such as methyl and ethyl.

The most preferred $-COX'R^{3'}$ radicals of the present invention are those wherein (relative to Structure I above), X' is oxygen and $R^{3'}$ is hydrogen; loweralkyl having 1-4 carbon atoms; lower alkenyl such as 3-methylbutenyl, 4-butenyl and the like; benzyl and substituted benzyl such as p-nitrobenzyl; pivaloyloxymethyl, 3-phthalidyl; and phenacyl.

The compounds of the present invention (I) are valuable antibiotics active against various gram-positive and gram-negative bacteria and accordingly find utility in human and veterinary medicine. Representative pathogens which are sensitive to antibiotics I include: Staphyloccus aureus, Escherichia coli, Klebsiella pneumoniae, Bacillus subtilis, Salmonella typhosa, Psuedomonas and Bacterium proteus. The antibacterials of the invention are not limited to utility as medicaments; they may be used in all manner of industry, for example: additives to animal feed, preservation of food, disinfectants, and in other industrial systems where control of bacterial growth is desired. For example, they may be employed in aqueous compositions in concentrations ranging from 0.1 to 100 parts of antibiotic per million parts of solution in order to destroy and inhibit the growth of harmful bacteria on medical and dental equipment and as bactericides in industrial applications, for example in waterbased paints and in the white water of paper mills to inhibit the growth of harmful bacteria.

The products of this invention may be used in any of a variety of pharmaceutical preparations. They may be employed in capsule, powder form, in liquid solution, or in suspension. They may be administered by a variety of means; those of principal interest include: orally, topically or parenterally by injection (intravenously or intramuscularly).

Such tablets and capsules, designed for oral administration, may be in unit dosage form, and may contain conventional excipients, such as binding agents, for example, syrup, acacia, gelatin, sorbitol, tragacanth, or polyvinylpyrrolidone; fillers, for example, lactose, sugar, cornstarch, calcium phosphate, sorbitol, or glycerine; lubricants, for example, magnesium stearate, talc, polyethylene glycol, silica; disintegrants, for example, potato starch; or acceptable wetting agents such as sodium lauryl sulphate. The tablets may be coated according to methods well known in the art. Oral liquid preparations may be in the form of aqueous or oily suspensions, or solutions, or they may be presented as a dry product for reconstitution with water or other suitable vehicle before use. Such liquid preparations may contain conventional additives such as suspending agents, for example, sorbitol, methyl cellulose, glucose/sugar syrup, gelatin, hydroxyethylcellulose, or carboxymethyl cellulose. Suppositories will contain conventional suppository bases, such as cocoa butter or other glycerides.

Compositions for injection, the preferred route of delivery, may be prepared in unit dosage form in ampules, or in multidose containers. The compositions may take such forms as suspensions, solutions, or emulsions in oily or aqueous vehicles, and may contain formulatory agents such as suspending, stabilizing and/or dispersing agents. Alternatively, the active ingredient may be in powder form for reconstitution, at the time of delivery, with a suitable vehicle, such as sterile water.

-The compositions may also be prepared in suitable forms for absorption through the mucous membranes of the nose and throat or bronchial tissues and may conveniently take the form of liquid sprays or inhalants, lozenges, or throat paints. For medication of the eyes or ears, the preparation may be presented in liquid or semi-solid form. Topical applications may be formulated in hydrophobic or hydrophilic bases as ointments, creams, lotions, paints, or powders.

The dosage to be administered depends to a large extent upon the condition and size of the subject being treated as well as the route and frequency of administration -- the parenteral route by injection being preferred for generalized infections. Such matters, however, are left to the routine discretion of the therapist according to principles of treatment well known in the antibiotic art. In general, a daily dosage consists of from about 5 to about 600 mg of active ingredient per kg. of body weight of the subject in one or more treatments per day. A preferred daily dosage for adult humans lies in the range of from about 10 to 240 mg. of active ingredient per kg. of body weight. Another factor influencing the precise dosage regimen, apart from the nature of the infection and peculiar identity of the individual being treated, is the molecular weight of the chosen species of this invention (I).

The compositions for human delivery per unit dosage, whether liquid or solid, may contain from 0.1% to 99% of active material, the preferred range being from about 10-60%. The composition will generally contain from about 15 mg. to about 1500 mg. of the active ingredient; however, in general, it is preferable to employ a dosage amount in the range of from about 250 mg to 1000 mg. In parenteral administration, the unit dosage is usually the pure compound I in sterile water solution or in the form of a soluble powder intended for solution. For zwitterionic species described under Structure I, the pH of such solutions typically will correspond to the zwitterionic point; however, consideration of individual properties of solubility and stability may require such aqueous solutions to have a pH other than that of the zwitterionic point, for example in the range of 5.5 to 8.2.

In the foregoing word description of the above schematic reaction diagram for the total synthesis of the defined carbapenem antibiotics, it is to be understood that there is considerable latitude in selection of precise reaction parameters. Suggestion of this latitude and its breadth is generally indicated by the enumeration of equivalent solvent systems, temperature ranges, protecting groups, and range of identities of involved reagents. Further, it is to be understood that the presentation of the synthetic scheme as comprising distinct steps in a given sequence is more in the nature of a descriptive convenience than as a necessary requirement; for one will recognize that the mechanically dissected scheme represents a unified scheme of synthesis and that certain steps, in actual practice, are capable of being merged, conducted simultaneously, or effected in a reverse sequence without materially altering the progress of synthesis.

The following examples recite a precise scheme of total synthesis. It is to be understood that the purpose of this recitation is to further illustrate the total synthesis and not to impose any limitation.

Incorporation by Reference

The compounds of the present invention may also be prepared by the processes disclosed and claimed in the three (3) following, co-pending, commonly assigned, concurrently filed U.S. Patent Applications of Christensen, Ratcliffe and Salzmann. To the extent that these applications define $R^6$, $R^7$, and $R^8$ of Structure I and to the extent that they describe processes for the synthesis of I, they are hereby incorporated by reference.

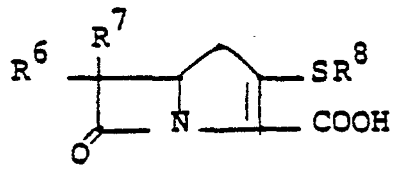

I

1.) Process for the Preparation of 1-Carbapenems and Intermediates via 4-Allylazetidinone; U.S. Patent Application Serial Number _______, filed_____ [Merck & Co., Inc. Attorney's Docket Case 16479].

2.) Process for the Preparation of 1-Carbapenems and Intermediates via Trithioorthoacetates; U.S. Patent Application Serial Number______, filed_______ [Merck & Co., Inc. Attorney's Docket Case 16485].

3.) Process for the Preparation of 1-Carbapenems and Intermediates via Silyl-Substituted Dithioacetals; U.S. Patent Application Serial Number _________, filed _______ [Merck & Co., Inc. Attorney's Docket Case 16478].

Also incorporated by reference is Belgian Patent 848,545 (which corresponds to co-pending, commonly assigned U.S. Serial Number 852,425 filed 11-17-77, now U.S. Patent 4,194,047 issued 3-18-80). This patent discloses and claims processes for converting the natural product thienamycin to certain amino derivatives:

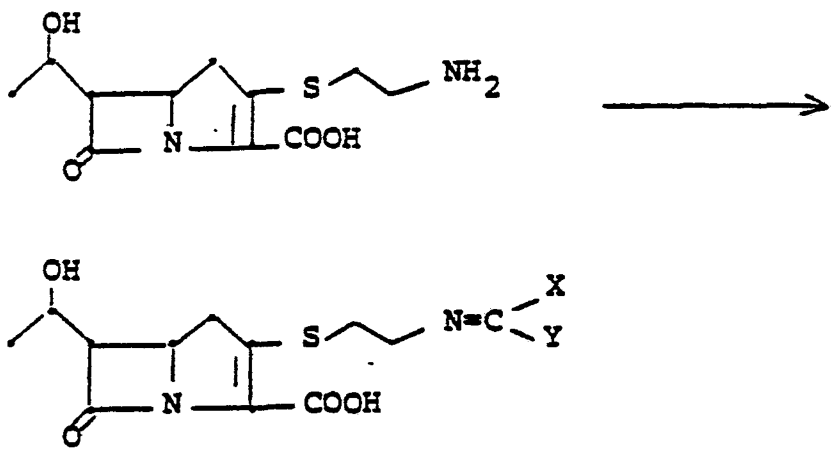

The process disclosed in the cited Belgian Patent is also suitable to prepare preferred, antibiotic embodiments of the present invention (Structure I', see below). The applicability of the process arises from the presence of an amino group on previously defined side chain $-SR^8$ of the compounds of the present invention, Structure I, thus:

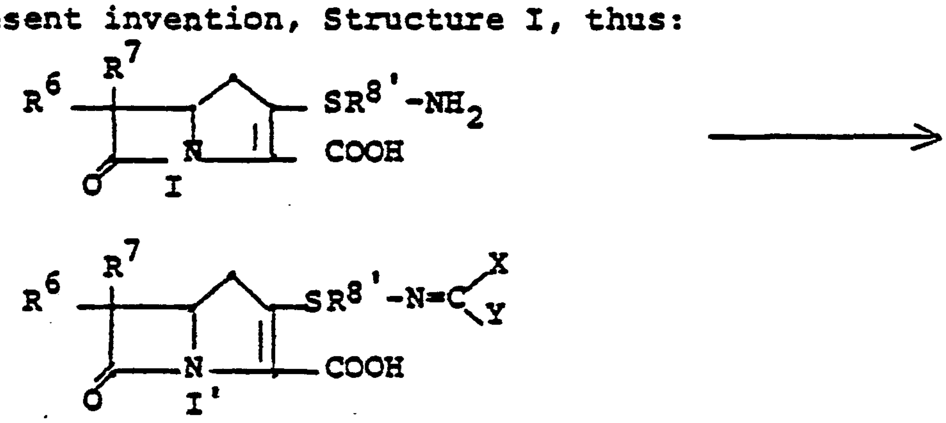

I

I'

wherein: $-SR^{8'}-NH_2 = -SR^8$; that is, the symbol $-SR^{8'}-NH_2$ indicates, and is specific to $-SR^8$, above-defined, bearing an amino substituent; preferred values for X and Y include: $X=NH_2$; $Y=H$, $CH_3$, $NH_2$.

Thus, to the extent that the Belgian Patent describes the amino derivatization process, the generic definition of X and Y and the preferred, above-indicated, amidine and guanidine embodiments, it is hereby incorporated by reference.

Also incorporated by reference is published European Patent Application 0007614 (Application Number 79102615.6, filed 24 July 1979). This application discloses certain dipeptidase inhibitors which, on co-administration to mammalian subjects, enhance the efficacy of certain 1-carbadethiapenem antibiotics. Thus, to the extent that the cited application: 1.) defines the manner by which susceptible carbadethiapenems substrates of the present invention may be identified; and 2.) discloses suitable inhibitors, compositions, and methods of treatment, it is incorporated herein by reference. A particularly preferred inhibitor is 6-(L-2-Amino-2-carboxyethylthio)-2-(2,2-DCC)-2-hexenoic acid.

EXAMPLE 1

Preparation of 4-(2-acetoxyvinyl)azetidinone-2-one

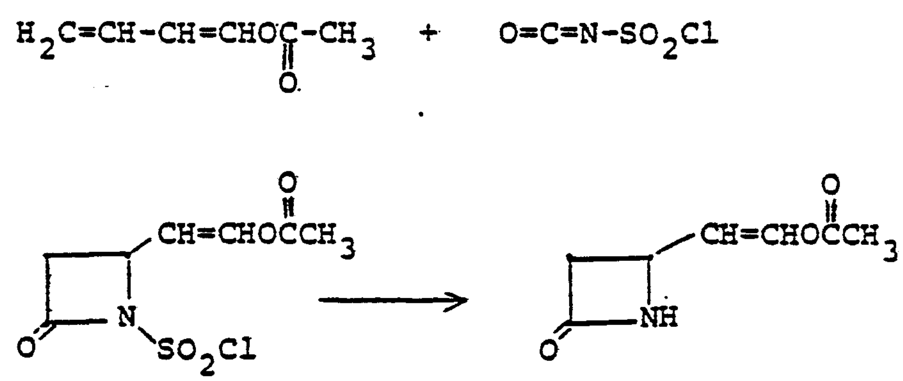

A solution of 1.0 ml distilled chlorosulfonylisocyanate (1.65 g; 11.7 mmoles) in 2.5 ml anhydrous diethyl ether is cooled under $N_2$ in a -20°C bath.

A solution of 2.5 g 1-acetoxybutadiene (22 mmoles) in 2.5 ml anhydrous ether is similarly cooled under $N_2$ in a -20°C bath.

The chlorosulfonylisocyanate solution is added dropwise to the acetoxybutadiene solution by means of a Teflon tube immersed in the CSI solution and pressurized with $N_2$. The addition takes 10 minutes. Little or no color is seen and the reaction is stirred at -20°C for 0.5 hour. The solution is clear and has a light yellow color.

A solution of 2 g sodium sulfite and 5 g $K_2HPO_4$ in 20 ml $H_2O$ is prepared during the above 0.5 hour reaction time and is cooled in an ice bath; 20 ml of ether is added and the mixture is vigorously stirred

in an ice bath. At the end of the 30 minute reaction time, the reaction mixture is transferred, again using $N_2$ pressure and the Teflon tube, from the reaction flask which is maintained in the -20°C bath, to the vigorously stirred hydrolysis mixture. Rapid dropwise addition is completed in 5 minutes. The hydrolysis is allowed to continue for 5 additional minutes. The hydrolysis mix has a pH of 6-8, preferably pH 8.

The phases are separated, leaving a yellowish-orange gum with the aqueous phase. The ether phase is dried directly with $MgSO_4$. The aqueous/gum phase is extracted three more times with 50 ml portions of ether, each being added to the initial ether/$MgSO_4$.

The dried extracts are filtered and concentrated under a $N_2$ stream to 5 ml; a portion of the product is crystalline at this stage.

A column of 10 g Baker silica gel, packed in ether is prepared, and the ether concentrate is applied to the top and run in. The flask/solids are rinsed three times with 2 ml ether, each being pipetted off and run into the column. Elution is then begun with ether. The first 25 ml is primarily void volume. The next five 10 ml fractions are collected followed by three 50 ml fractions, and all are reduced in volume under a $N_2$ stream. The product crystallizes from fractions 4-6, with traces in 3 and 7. Fractions 1-3 contain a yellowish sharp-smelling material which resinifies on standing. Yield: 100 mg as a mixture of the cis and trans isomers.

EXAMPLE 2

Preparation of 4- (2-Acetoxyethyl)-2-Azetidinone

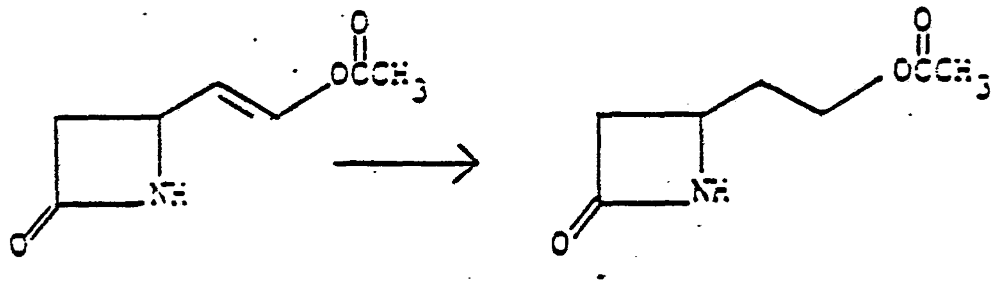

A solution of 4- (2-acetoxyvinyl)-2-azetidinone (10.0 g, 0.065 mole) in 200 ml ethyl acetate containing 100 mg of 10% Pd/C is hydrogenated on a Parr shaker at 25°C under 40 psi hydrogen for 15 minutes. The mixture is filtered through a bed of Supercel and washed with additional ethyl acetate. The combined filtrate is evaporated in vacuo to give 4- (2-acetoxyethyl)-2-azetidinone (10.0 g) as a crystalline solid. Recrystallization from ether affords white crystals: M.P. 44-7°; ir $(CHCl_3)_{\mu}$ 5.66, 5.74; nmr $(CDCl_3)\tau$ 3.44 (broad s, 1, NH), 5.82 (m, 2, $C\underline{H}_2OCOCH_3$), 6.29 (m, 1, C-4H), 6.87 (1/2 AB pattern further split in four by C-4H and NH, 1, $J_{gem}$ = 12.8Hz, J=4.5 H $J_{NH}$ = 1.9 Hz, 7.38 (1/2 AB pattern further split in four by C-4H and NH, 1, $J_{gem}$ = 12.8Hz, J = 2.3Hz, $J_{NH}$ = 1.0Hz), 7.93 and 8.02 (s, on m, total 5, $OCOC\underline{H}_3$ and $C\underline{H}_2CH_2OCOCH_3$, respectively).

EXAMPLE 3

Preparation of 4-(2-Hydroxyethyl)-2-Azetidinone

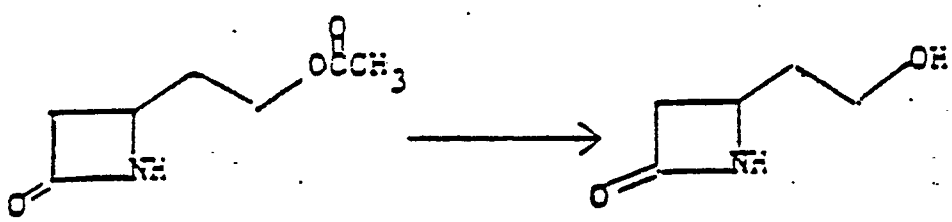

Under nitrogen at 0°, a solution of 4-(2-acetoxyethyl)-2-azetidinone (2.24 g, .014 mole) in 25 ml anhydrous methanol is treated with a solution of sodium methoxide (77 mg, 1.4 mmoles) in 5 ml anhydrous methanol. After stirring for 1 hour, the solution is neutralized with glacial acetic acid. Removal of the methanol *in vacuo* gives crude 4-(2-hydroxyethyl)-2-azetidinone as an oil. The product is purified by chromatography on silica gel eluting with 10% $MeOH/CHCl_3$ to give 1.55 g of the alcohol: m.p. 50°; ir ($CHCl_3$) μ 5.67; nmr ($CDCl_3$) $\tau$ 3.20 (broad s, 1, NH), 6.24 and 6.28 (m on t, total 3, C-4H and $C\underline{H}_2OH$ respectively), 6.90 (broad s on 1/2 AB pattern further split in four by C-4H and NH, total 2, OH and C-3H respectively, $J_{gem}$ = 13.0Hz, $J_{vic}$ = 4.2Hz, $J_{NH}$ = 1.6Hz), 7.42 (1/2 AB pattern further split in four by C-4H and NH, 1, C-3H, $J_{gem}$ = 13.0Hz, $J_{vic}$ = 2.2Hz, $J_{NH}$ = 1.1Hz), 8.16 (m, 2, $C\underline{H}_2CH_2OH$).

## EXAMPLE 4

Preparation of 8-Oxo-2,2-dimethyl-3-oxa-1-azabicyclo /4.2.0/octane

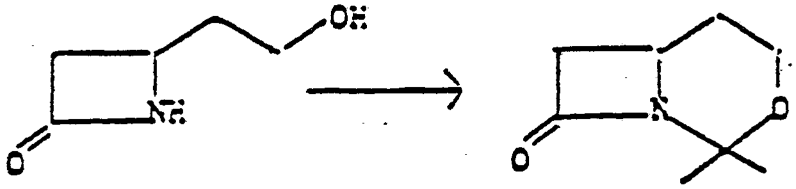

A solution of 4-(2-hydroxyethyl)-2-azetidinone (1.87 g, .016 mole) and 2,2-dimethoxypropane (1.69 g, .016 mole) in 25 ml anhydrous methylene chloride is treated with boron trifluoride etherate (.201 ml, .002 mole) at 25°C. The resulting solution is stirred for ten minutes. Removal of the solvent under reduced pressure gives an oil (2.5 g). Chromatography of the crude product on silica gel using 2:1 ethyl acetate/benzene as eluting solvent gives 8-oxo-2,2-dimethyl-3-oxa-1-azabicyclo/4.2.0/-octane (1.59 g) as a crystalline solid. Recrystallization from ether/hexane gives product of m.p. 60-1°.

ir ($CHCl_3$)$\mu$ : 5.73 ($\beta$-lactam)

nmr ($CDCl_3$)$\tau$: 6.02 - 6.28, m, 2H, C-4 methylene

6.22 - 6.62, m, 1H, C-6 methine

6.90, dd, 1H, $J_{7,7}$ = 14Hz, $J_{6,7}$ = 4.5Hz

C-7 proton <u>cis</u> to C-6H

7.47, dd, 1H, $J_{7,7}$ = 14Hz, $J_{6,7}$ = 2Hz

C-7 proton <u>trans</u> to C-6H

7.82 - 8.68, m, 2H, C-5 methylene

8.23, s, 3H } C-2 methyls
8.57, s, 3H }

## EXAMPLE 4a

Preparation of 8-oxo-2,2-dimethyl-7α-isopropyl-3-oxa-1-azabicyclo[4·2·0]octane

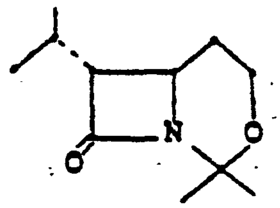

THF, 20 ml is placed under $N_2$, treated with 1.54 ml diisopropylamine and cooled to -78°C. A solution of n-butyl lithium 1.97M in hexane 5.6 ml is added dropwise over 5 min. The reaction mixture is stirred at -78°C for 10 min. and then treated with 8-oxo-2,2-dimethyl-3-oxa-1-azabicyclo[4·2·0]octane 1.55 g in 15 ml THF added dropwise over 5 min. After another 10 min. hexamethylphosphoramide 1.97 ml is added. The mixture is stirred another 10 min., then treated with 2 ml of isopropyl iodide. The reaction mixture is stirred at -78°C for 15 min. and allowed to warm to 25°C and stirred for 15 min. The residue mixture is diluted with EtOAc, washed once with pH 7 phosphate buffer then dried and evaporated. The residue is chromatographed on silica gel using 25% EtOAc/$C_6H_6$ as eluant to give 8-oxo-2,2-dimethyl-7α-isopropyl-3-oxa-1-azabicyclo[4·2·0]-octane. μ i.r. : 5.7 (β-lactam). n.m.r. δ: 0.96d, 1.06d ($CH_3$-C-H); 1.4S, 1.76S (gem dimethyl);
$CH_3$

1.9m (C-5 H); 2.59d of d (C-7 H);
3.33m (C-6 H); 3.83 d of d (C-4 H).

EXAMPLE 4b

Preparation of 8-oxo-2,2,7-trimethyl-3-oxa-1-azabicyclo [4·2·0]octane

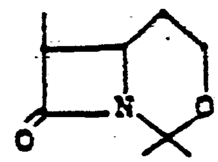

Following the procedure of Example 4a, except substituting an equivalent amount of methyl iodide for the isopropyl iodide, the title compound is obtained.

EXAMPLE 5

Preparation of 8-oxo-2,2,7-trimethyl-7-(hydroxymethyl)-3-oxa-1-azabicyclo[4·2·0]octane

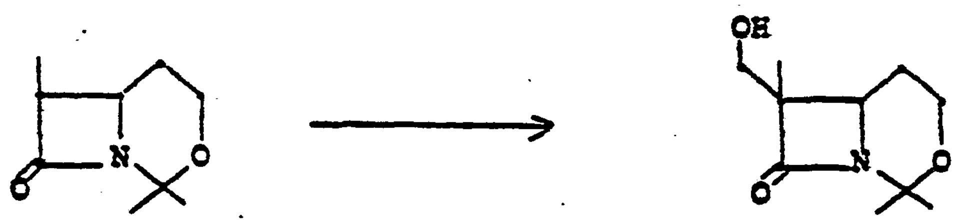

To a solution of 1.1 equivalents of freshly prepared lithium diisopropylamide in anhydrous tetrahydrofuran under a nitrogen atmosphere at -78° is added a solution of 8-oxo-2,2,7-trimethyl-3-oxa-1-azabicyclo-[4·2·0]octane in anhydrous tetrahydrofuran which has been cooled to -78°C. After two minutes, the resulting lithium enolate is treated with excess formaldehyde, introduced as a gas just above the surface of the stirred solution. The solution is stirred for 30 minutes at -78° and then poured into water. The aqueous phase is saturated with sodium

chloride and extracted with ethyl acetate. The combined ethyl acetate solutions are dried over magnesium sulfate and filtered. The filtrate is evaporated under reduced pressure to give the crude product. Purification by chromatography on silica gel using ethyl acetate/benzene gives 8-oxo-2,2,7-trimethyl-7(hydroxymethyl)-3-oxa-1-azabicyclo[4·2·0] octane.

## EXAMPLE 6

Preparation of 8-oxo-2,2,7-trimethyl-7-(p-nitrobenzyl-carbonyldioxymethyl)-3-oxa-1-azabicyclo[4·2·0] octane

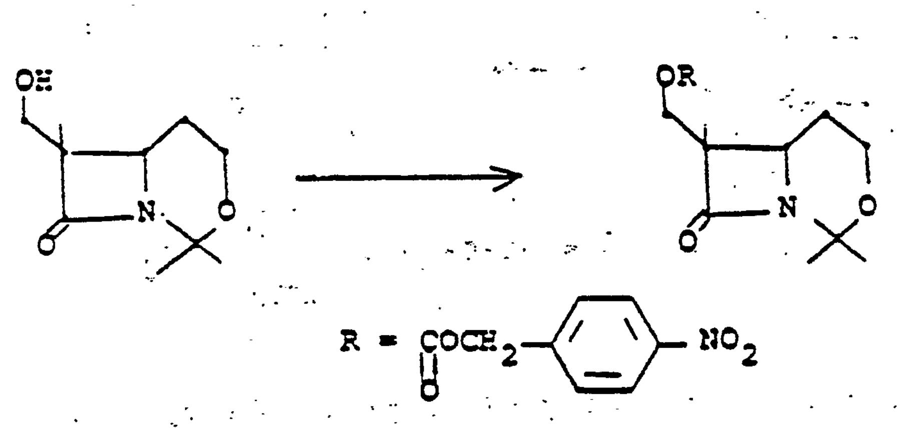

Under anhydrous conditions at 0°C. a solution of 8-oxo-2,2,7-trimethyl-7-(hydroxymethyl)-3-oxa-1-azabicyclo[4·2·0]octane (60 mg., .302 mmole) in 0.6 ml ether is treated with powdered potassium hydroxide (19 mg, .332 mmole). After a period of 15 minutes, p-nitrobenzyl chloroformate (65 mg, .302 mmole) is added to the reaction mixture. Stirring is continued at 25°C for an additional 15 hours. The mixture is partitioned between 1M pH 7 phosphate buffer and more ether. The ether phase is washed with water and brine, dried over magnesium sulfate and filtered. Evaporation of the

filtrate under reduced pressure gives 67 mg of a colorless oil. Purification by preparative thick-layer chromatography on silica gel developing with 1:9 ethyl acetate/benzene gives 8-oxo-2,2,7-trimethyl-7-(p-nitrobenzylcarbonyldioxymethyl)-3-oxa-1-azabicyclo[4·2·0]octane (40 mg) as a mixture of diastereomers.

## EXAMPLE 7

Preparation of 3-methyl-3-(p-nitrobenzylcarbonyldioxymethyl)-4-(2-hydoxyethyl)-2-azetidinone

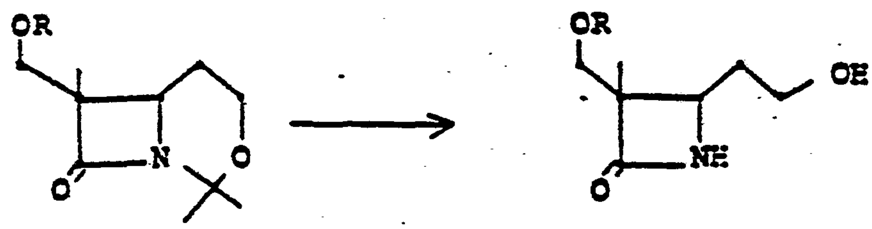

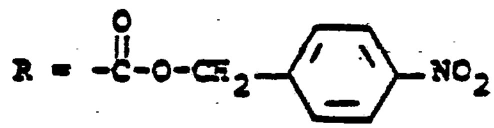

8-Oxo-3-oxa-2,2,7-trimethyl-7-(1-p-nitrobenzylcarbonyldioxymethyl)-1-azabicyclo[4·2·0]octane (1.0 g) is dissolved in 8 ml acetic acid and 2 ml water and heated at 65°C for 1.25 hours. The acetic acid and water are removed under reduced pressure and the residue is taken up in benzene and evaporated to give 3-methyl-3-(p-nitrobenzylcarbonyldioxymethyl)-4-(2-hydroxyethyl)-2-azetidinone as a mixture of diastereoisomers.

EXAMPLE 8-11

Examples 8, 9, 10 and 11 as alternative to Examples 4, 5, 6 and 7 for the preparation of 3-methyl-3-(p-nitrobenzylcarbonyldioxymethyl)-4-(2-hydroxyethyl)-2-azetidinone

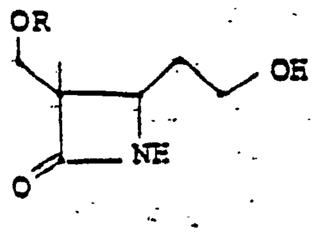

R = 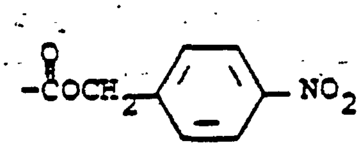

EXAMPLE 8

Preparation of 1-(2-Tetrahydropyranyl)-4-[2-(2-tetrahydropyranyl)oxyethyl]-2-azetidinone

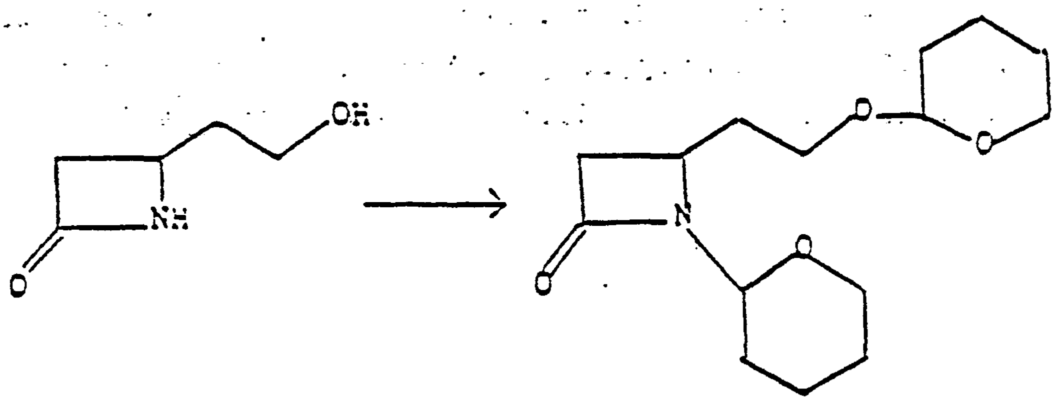

Under nitrogen and at 25°C, a solution of 4-(2-hydroxyethyl)-2-azetidinone (62 mg, .539 mmole) in .5 ml of anhydrous p-dioxane is treated with 2,3-dihydro-

pyran (.98 ml, 1.08 mmoles) and p-toluenesulfonic acid monohydrate (19 mg, .10 mmole). The resulting solution is stirred for a period of 60 minutes and then partitioned between 10 ml of .5M pH7 phosphate buffer and 10 ml of ethyl acetate. The aqueous phase is extracted a second time with ethyl acetate. The combined ethyl acetate solutions are washed with brine, dried over magnesium sulfate and filtered. The filtrate is evaporated under reduced pressure to give 216 mg of crude product. Purification by preparative thick-layer chromatography developing with ethyl acetate gives 80 mg of 1-(2-tetrahydropyranyl)-4-[2-(2-tetrahydropyranyl)oxyethyl]-2-azetidinone as an oil.

nmr ($CDCl_3$) $\tau$: 5.13-5.60, m, OC<u>H</u>

5.83-6.85, m, C-4H + OC<u>H</u>$_2$

6.95, dd, J = 5Hz and 15 Hz } C-3 methylene

7.35, dd, J = 3Hz and 15 Hz }

7.62-8.95, m, CHC<u>H</u>$_2$C<u>H</u>$_2$C<u>H</u>$_2$CH$_2$ + CHC<u>H</u>$_2$CH$_2$O

The corresponding 3-methyl-1-(2-tetrahydropyranyl)-4-[2-(2-tetrahydropyranyl)oxyethyl]-2-azetidinone is obtained from the product of Example 4b *via* Examples 7.

EXAMPLE 9

Preparation of 1-(2-tetrahydropyranyl)-3-methyl-3-(1-hydroxymethyl)-4-[2-(2-tetrahydropyranyl)oxyethyl]-2-azetidinone

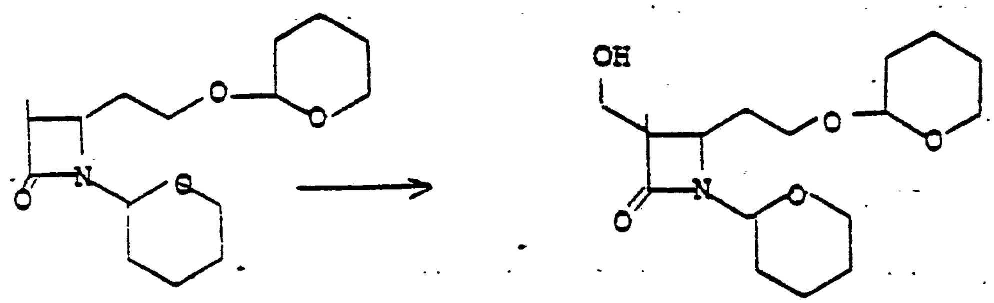

Following the procedure described for the preparation of 8-oxo-2,2,7-trimethyl-7-(hydroxymethyl)-3-oxa-1-azabicyclo[4·2·0]octane from 8-oxo-2,2,7-trimethyl-3-oxa-1-azabicyclo[4·2·0]octane (Example 5, above) and using 3-methyl-1-(2-tetrahydropyranyl)-4-[2-(2-tetrahydropyranyl)-oxyethyl]-2-azetidinone one obtains a diastereomeric mixture of 1-(2-tetrahydropyranyl)-3-methyl-3-(hydroxymethyl)-4-[2-(2-tetrahydropyranyl) oxyethyl]-2-azetidinone.

EXAMPLE 10

Preparation of 3-Methyl-1-(2-tetrahydropyranyl)-3-(1-p-nitrobenzylcarbonyldioxymethyl)-4-[2-(2-tetrahydropyranyl)oxyethyl]-2-azetidinone

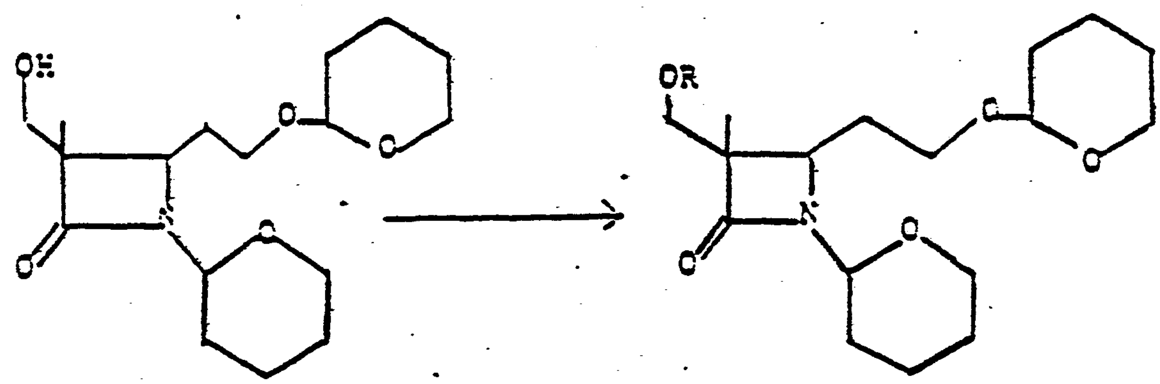

R = 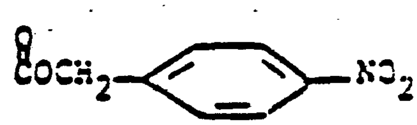

Following the procedure described for the preparation of 8-oxo-2,2,7-trimethyl-7-(1-p-nitrobenzylcarbonyldioxymethyl)-3-oxa-1-azabicyclo[4·2·0]octane from 8-oxo-2,2,7-trimethyl-7-(1-hydroxymethyl)-3-oxa-1-azabicyclo[4·2·0]octane and using 3-methyl-1-(2-tetrahydropyranyl)-3-(hydroxymethyl)-4-[2-(2-tetrahydropyranyl)oxyethyl]-2-azetidinone there is obtained 3-methyl-1-(2-tetrahydropyranyl)-3-(p-nitrobenzylcarbonyldioxymethyl)-4-([2-(2-tetrahydropyranyl)-oxyethyl]-2-azetidinone.

EXAMPLE 11

Preparation of 3-Methyl-3-(p-nitrobenzylcarbonyldioxyethyl)-4-(2-hydroxyethyl)-2-azetidinone

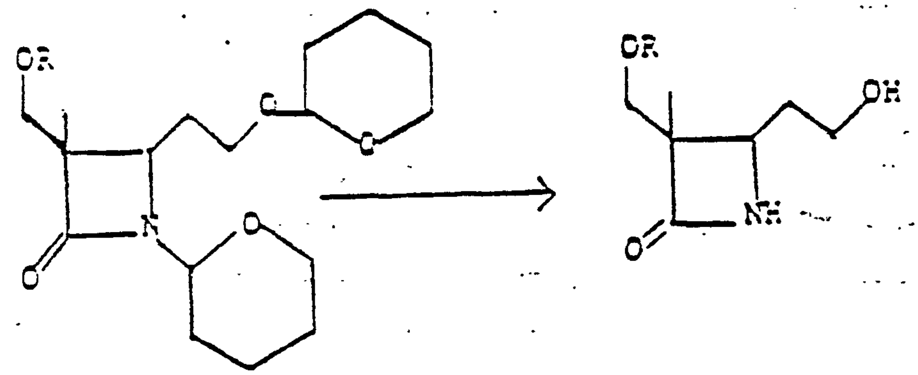

R = 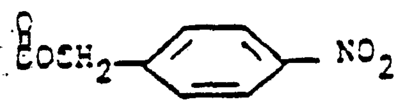

A solution of 3-methyl-1-(2-tetrahydropyranyl)-3-(p-nitrobenzylcarbonyldioxymethyl)-4-[2-(2-tetrahydropyranyl)-oxyethyl]-2-azetidinone in methanol at 25°C. is treated with .1 molar equivalent of p-toluenesulfonic acid monohydrate. The solution is stirred for a period of 2 hours and then neutralized with 1M pH 7 phosphate buffer. The product is extracted into ethyl acetate. The ethyl acetate solution is washed with brine, dried over magnesium sulfate and filtered. The filtrate is evaporated under reduced pressure to give 3-methyl-3-(p-nitrobenzylcarbonyldioxymethyl)-4-(2-hydroxyethyl)-2-azetidinone.

EXAMPLE 12

Preparation of 3-(2-aminoethylthio)-6-methyl-6-(hydroxymethyl)-7-oxo-1-azabicyclo[3·2·0]hept-2-ene-2-carboxylic acid

Step A

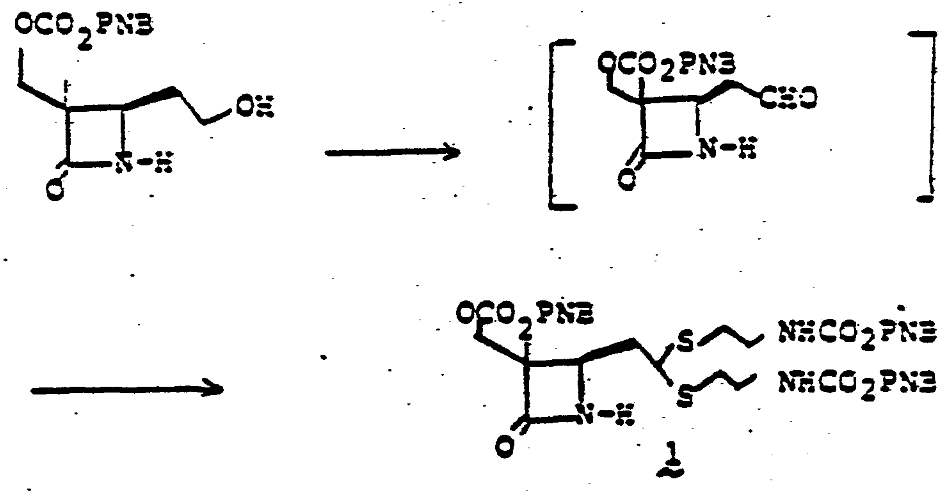

1

PNB =

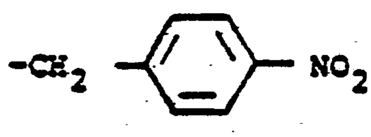

To 6.75 ml anhydrous pyridine (mw=79; $\rho$=0.982; 83.9 mmole) in 350 ml anhydrous acetonitrile is added 4.05 g anhydrous powdered chromium trioxide (mw=100; 40.5 mmole). After stirring at room temperature (25°C) for 30 minutes, 9.6g dried Supercell is added and stirring is continued for 5 additional minutes. A solution of 3.21g 3-methyl-3-(p-nitrobenzylcarbonyldioxymethyl)-4-(2-hydroxyethyl)-2-azetidinone (mw = 338; 9.5 mmole) in 30 ml anhydrous acetonitrile is added all at once. The reaction mixture is stirred under anhydrous conditions at room temperature (25°C) for one hour. Addition of 9.6g $NaHSO_3$ is followed by 5 minutes of stirring after which the reaction mixture is filtered through a mixed packed bed of 40g silica

gel and 40g anhydrous magnesium sulfate. The bed is washed repeatedly with acetonitrile (total volume of filtrate∽600 ml). The filtrate is concentrated under a $N_2$ stream to 130 ml total volume. To this solution containing crude aldehyde at 0°C under $N_2$ is added 9.64g p-nitrobenzyloxycarbonylaminoethanethiol (mw = 256; 37.7 mmole) as prepared below (Example 12, Step B). To the stirred reaction mixture is added 8.0 ml boron trifluoride etherate (mw = 142; $\rho$ =1.125; 63.4 mmole). After 1.5 hours at 0°C, the reaction mixture is poured into a stirred ice-cold mixture of 69g $K_2HPO_4$ - 500 ml $H_2O$ and 700 ml ethyl acetate (EA). The layers are separated, and the aqueous one is saturated with NaCl and re-extracted with additional EA. The combined organic layers are washed twice with brine, dried over anhydrous $MgSO_4$ and filtered. The filtrate is concentrated under a $N_2$ stream and then pumped on high vacuum to give crude **1**.

The material is chromatographed on 450g silica gel (column height = 48 cm; diameter = 5.5 cm) packed and applied in $CHCl_3$ and eluted with increasing percentages of MeOH in $CHCl_3$ (0-4% $MeOH/CHCl_3$). Those fractions containing the desired product are combined, concentrated under a $N_2$ stream; and pumped on high vacuum to give **1**.

Step B

Preparation of p-Nitrobenzyloxycarbonylaminoethanethiol

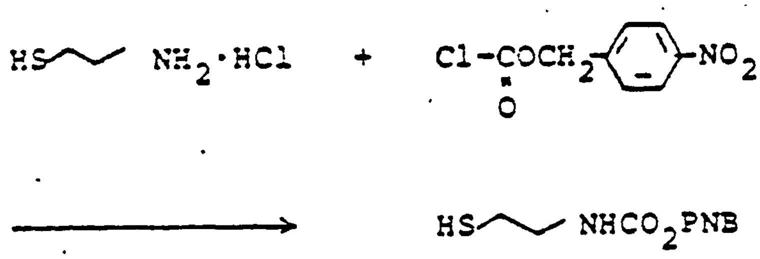

To 600 ml diethyl ether ($Et_2O$) - 75 ml $H_2O$ in an ice bath with stirring is added 3.2g cysteamine hydrochloride (mw = 114; 28.1 mmole). A solution of 7.14g $NaHCO_3$ (mw = 84; 85 mmole) in 75 ml $H_2O$ is added. The ice bath is removed, and at room temperature a solution of 6.75g p-nitrobenzylchloroformate (mw = 216; 31.3 mmole) in 270 ml $Et_2O$ is added dropwise over a period of one hour. After 10 additional minutes, the layers are separated. The ether layer is extracted with 150 ml 0.25 N HCl, and then with 200 ml brine. Each aqueous layer is then backwashed successively with 100 ml $Et_2O$. The combined $Et_2O$ layers are dried over anhydrous $MgSO_4$, filtered, and concentrated under a $N_2$ stream. The crystalline residue is slurried in a small amount of ether, filtered, and the pale yellow crystals are dried under high vacuum to give 4.7g p-nitrobenzyloxycarbonylaminoethanethiol (65% yield).

NMR ($CDCl_3$) 8.18 (d, J=8Hz, aromatic protons ortho to nitro), 7.47 (d, J=8Hz, aromatic protons meta to nitro), 5.27 (-NH-), 5.20 (s, $-CH_2-$Ø-$pNO_2$), 3.40 (m, $-CH_2-NH-$), 2.67 (m, $-CH_2-SH$), 1.35 (t, J=8.5Hz, -SH) in ppm downfield from TMS.

IR ($CHCl_3$ solution) carbonyl- ~ 1725 $cm^{-1}$

M.S. - molecular ion-256, (M-47) at 209, (M-136) at 120, $^{+}CH_2$Ø$pNO_2$ at 136.

STEP C

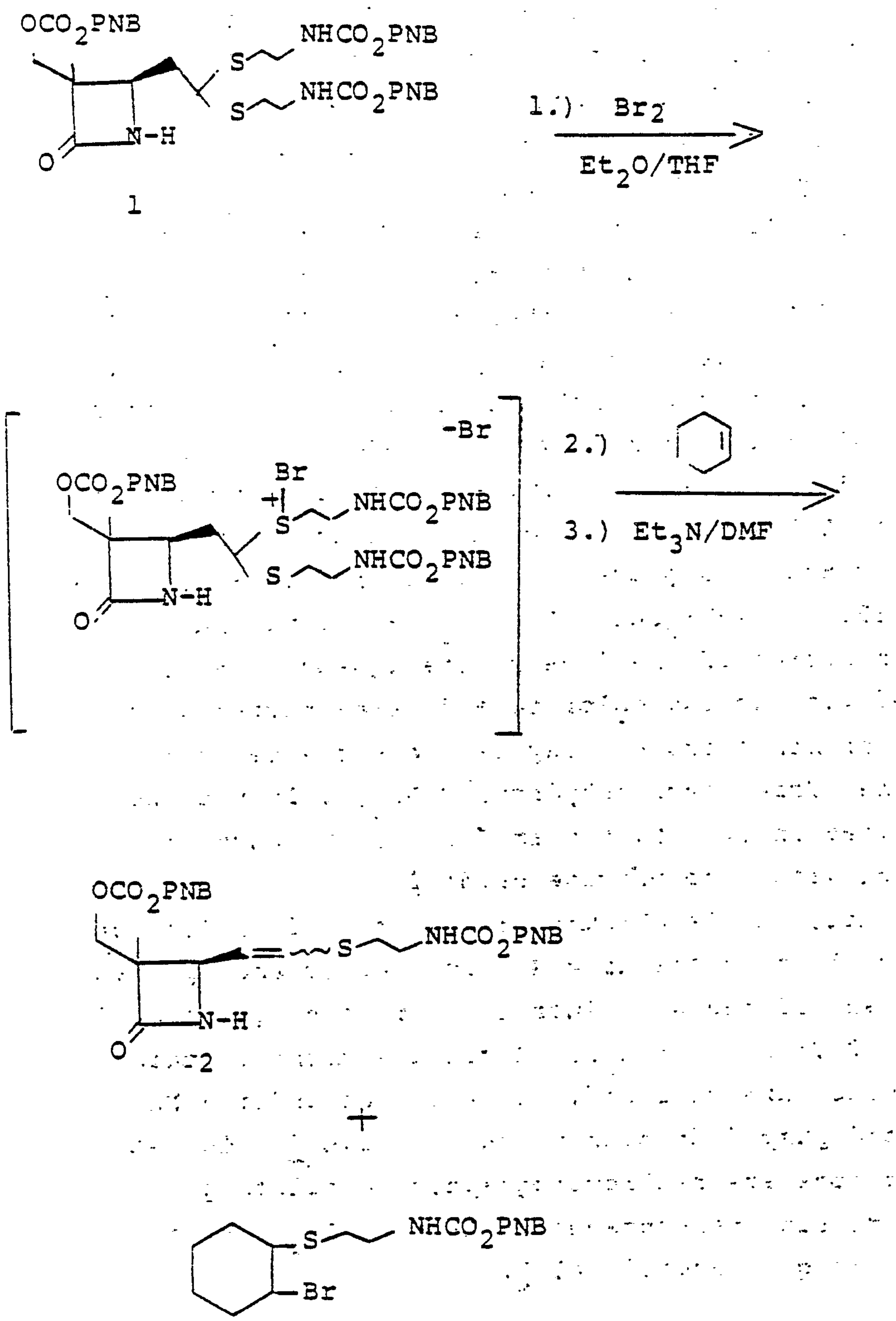


1

2

To 14.2 pentane (dried over 4A Linde molecular sieves) is added 0.5 ml $Br_2$ (mw = 160; 9.75 mmole). To 5g of 1 (mw = 830; 6.02 mmole) in 58 ml tetrahydrofuran (THF) (freshly distilled from lithium aluminum hydride) (LAH) and 65 ml $Et_2O$ (dried over 3A 1/16" Linde molecular sieves) at 0°C under $N_2$ with stirring is added dropwise 10 ml of the above 0.66M $Br_2$ solution (6.6 mmole). After 10 minutes at 0°C, 0.67 ml cyclohexene (mw = 82; $\rho$=0.81; 6.6 mmole) is added. After 5 minutes at 0°C, 1.7 ml triethylamine (mw=101; $\rho$=0.729; 12.3 mmole) is added immediately followed by 40 ml ice-cold dimethylformamide (DMF) (distilled from anhydrous $CaSO_4$ at 40 mm and stored over 4A Linde molecular sieves). The ice bath is removed, and stirring is continued for 2 1/4 hours at room temperature. The reaction mixture is poured into a stirred ice-cold mixture of 12.6 ml 1M$KH_2PO_4$ 160 ml $H_2O$ - 500 ml (EA). After separation of the layers, the aqueous one is saturated with sodium chloride and re-extracted with EA. The combined organic layers are extracted once with brine, dried over anhydrous $MgSO_4$, filtered and concentrated under a $N_2$ stream followed by pumping under high vacuum to provide crude 2.

The material is chromatographed on 250g silica gel (height = 45 cm; diameter = 4.5 cm) packed and applied in $CHCl_3$ and eluted with increasing percentages of MeOH in $CHCl_3$ (0-3% MeOH/$CHCl_3$). Those fractions containing clean product are combined, concentrated under a $N_2$ stream, and pumped on high vacuum to give 2. Contaminated fractions are rechromatographed on silica gel using increasing percentages of EA in $CHCl_3$ (0-25% EA/$CHCl_3$) to give additional 2.

STEP D

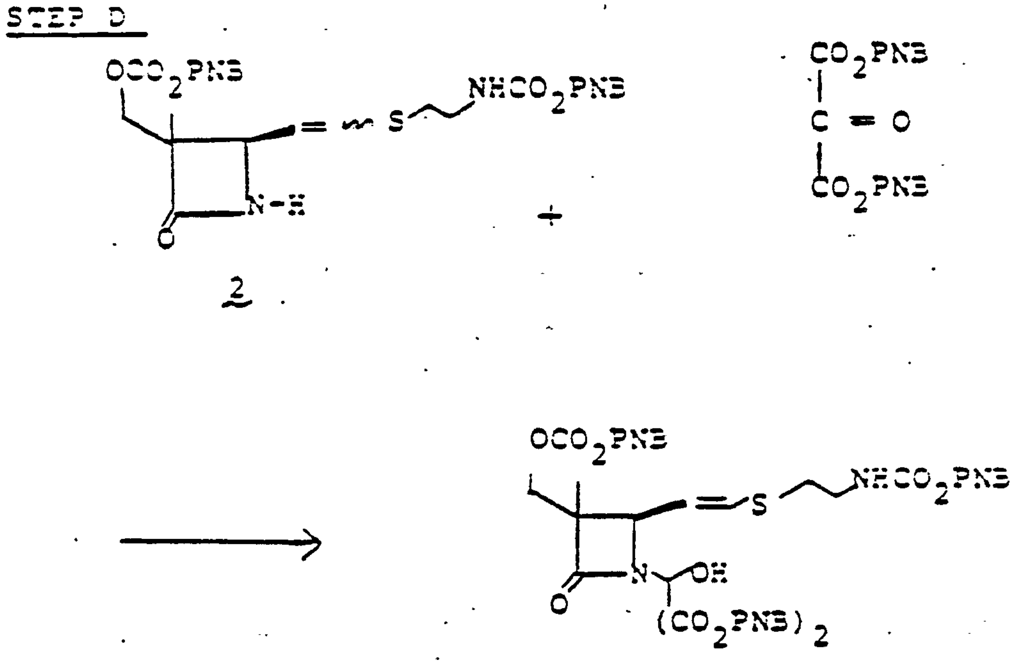

2

3

To a stirred solution of 2.48g di(p-nitrobenzyl) ketomalonate (from Example 12, Step E) (mw = 388; 6.39 mmole) in 400 ml hot anhydrous toluene is added a solution of 2.52g of 2 (mw = 574; 4.39 mmole) in 20 ml THF (distilled from LAH) and 40 ml anhydrous toluene. After some of the solvent is boiled off, additional anhydrous toluene is added, and the azeodrying process is repeated three times. The solution is then refluxed under $N_2$ for 30 minutes. Additional toluene is then allowed to boil off yet the volume is not allowed to diminish so much that precipitation occurs. Total heating time is approximately 2 1/2 hours. The clear yellow reaction mixture is removed from the oil bath and placed under a stream of $N_2$ which instantaneously causes clouding. After concentration to a yellow oil, the residue is dissolved in $CH_2Cl_2$, dried over anhydrous $MgSO_4$, filtered, and concentrated under a $N_2$ stream to

give crude 3.

The material is chromatographed on 250g silica gel packed and applied in $CHCl_3$ (height = 43 cm; diameter = 4.5 cm). Elution with 500 ml 0.5% MeOH/$CHCl_3$ is followed by continued elution with 1% MeOH/$CHCl_3$ for the remainder of the chromatography. After the emergence of excess reagent, those fractions containing pure 3 are combined, concentrated under a $N_2$ stream and then on high vacuum to give 3.

Later fractions containing 3 and the corresponding cis thioenol ether are re-chromatographed on silica gel to give additional 3.

Step E

Preparation of di-p-Nitrobenzyl Ketomalonate

$$CH_2(CO_2H)_2 \quad + \quad 2\ BrCH_2\text{Ø}\underline{p}NO_2 \quad \xrightarrow[EtOH]{KOH}$$

$$\underset{\mathbf{1'}}{CH_2(CO_2CH_2\text{Ø}\underline{p}NO_2)_2} \quad \xrightarrow[\text{ØH},\ \Delta]{SeO_2} \quad \underset{\mathbf{2'}}{O{=}C(CO_2CH_2\text{Ø}\underline{p}NO_2)}$$

ØH = benzene

A mixture of 100 g p-nitrobenzyl bromide (0.46 mole), 28.6g malonic acid (0.275 mole) and 750 ml ethanol (EtOH) is stirred and warmed on the steam bath until solution is achieved. A solution of 33g KOH (>85% purity; ~0.6 mole) in 200 ml of water is added carefully with swirling. An additional 200 ml of water is added, and the two-phase system is refluxed for 1.8 hours. The lighter color homogeneous solution is cooled in ice for 1 hour and the crude product isolated by filtration, washed twice with a minimum of cold EtOH, and dried by pulling dry $N_2$ through the cake;

33.7g of solid is obtained. If, during the refluxing stage the reaction mixture is slowly concentrating to ca. half volume by allowing refluxing solvent to distill off, the crude product yield rises to 77g. The material is recrystallized from methanol to give pure di-p-nitrobenzyl malonate 1'.

A mixture of 23.4 of 1', 10g $SeO_2$, and 30-40 ml of xylene is stirred in a flask immersed in an oil bath. The bath temperature is raised over 1 hour to 130-135°. A gradual darkening of the reaction mixture is noted, and after a total of 4 hours at 130-135°, most of the insoluble residue is black Se°. The mixture is cooled, $MgSO_4$ is added to remove the water, and Celite is added to aid in filtration. The mixture is filtered through Celite and the cake washed with xylene and a small portion of EtOAc. Final volume: 60 ml. A 100g column of Baker Silica Gel is prepared in benzene and 10 ml of filtrate applied, then eluted with increasing amounts of EtOAc in benzene, 500 ml fractions being collected. After one 2% ethyl acetate (EtOAc)/ØH, and two 10% EtOAc/ØH fractions, the third 10% and first 20% EtOAc/ØH provide the bulk of the product (~1.6g from 10 ml filtrate) as judged by tlc (20% EtOAc/$CHCl_3$; silica gel GF). Recrystallization from benzene, (1g in ca. 50 ml concentrated to ~1/3 volume and "spiked" with 1 ml of $H_2O$ saturated benzene): provides 0.24g 2'; mp(117) 121-122°.

STEP F

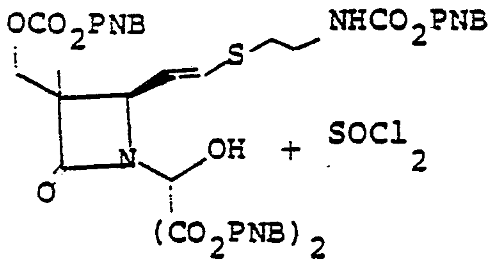


Py.
THF

3

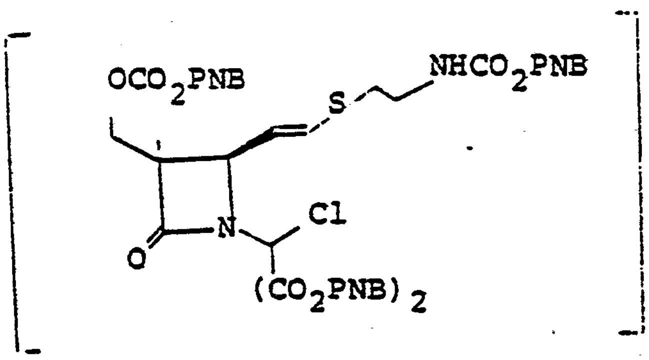

+ $Bu_3P$

aq. DMF

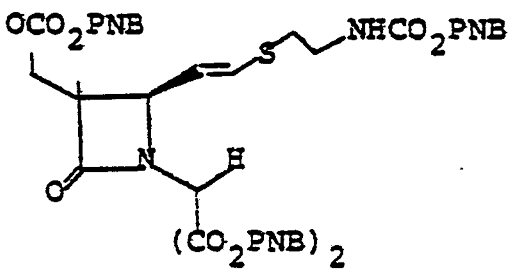

4

A solution of 1.468g of 3 (mw = 962; 1.53 mmole) in $CH_2Cl_2$ is dried over anhydrous $MgSO_4$, filtered, concentrated under a $N_2$ stream, and dried further under high vacuum just prior to the following reaction. To a solution of 3 in 24 ml THF (freshly distilled from LAH) at -20°C is added 0.206 ml anhydrous pyridine (mw = 79; $\rho$ = .982; 2.56 mmole). With stirring under $N_2$, 294 mg of freshly distilled thionyl chloride (mw = 119; 2.47 mmole) in 5 ml THF is added dropwise. The reaction mixture is stirred for 10 minutes at -20°C., then 1/2 hour at 0°C and finally 1 hour at 25°C. The pyridine hydrochloride is filtered under $N_2$ and washed with 20 ml THF. The filtrate is concentrated under a $N_2$ stream followed by pumping on high vacuum. The resulting yellow foam is swirled in 25 ml anhydrous THF, and a small amount of orange-red insoluble material is filtered off under $N_2$. The filtrate is reconcentrated as above to a yellow foam.

To this freshly prepared chloro compound is added with stirring a freshly shaken suspension of 678 mg tributylphosphine (mw = 202; 3.36 mmole) in 36.5 ml 9:1 DMF - $H_2O$ followed by 294 mg $K_2HPO_4$ (mw = 174; 1.69 mmole). The reaction mixture is stirred at 25°C., for 35 minutes. After dilution with 120 ml EA and 60 ml brine, the layers are separated, and the aqueous one is extracted two times with EA. The combined organic layers are washed one time with brine, dried over anhydrous $MgSO_4$, filtered and concentrated under a $N_2$ stream followed by pumping on high vacuum to give crude 4.

The material is chromatographed on 100g silica gel (height = 28.5 cm; d = 4 cm) packed and applied in $CHCl_3$ and eluted with 0.5% MeOH in $CHCl_3$. Those fractions containing clean product are combined, concentrated under a $N_2$ stream and then on high vacuum to

give 4. Contaminated fractions are re-chromatographed on silica gel thin layer plates (eluant = 50% acetone/hexane; extraction of desired u.v. band with $CHCl_3$ and EA to provide additional 4.

Step G

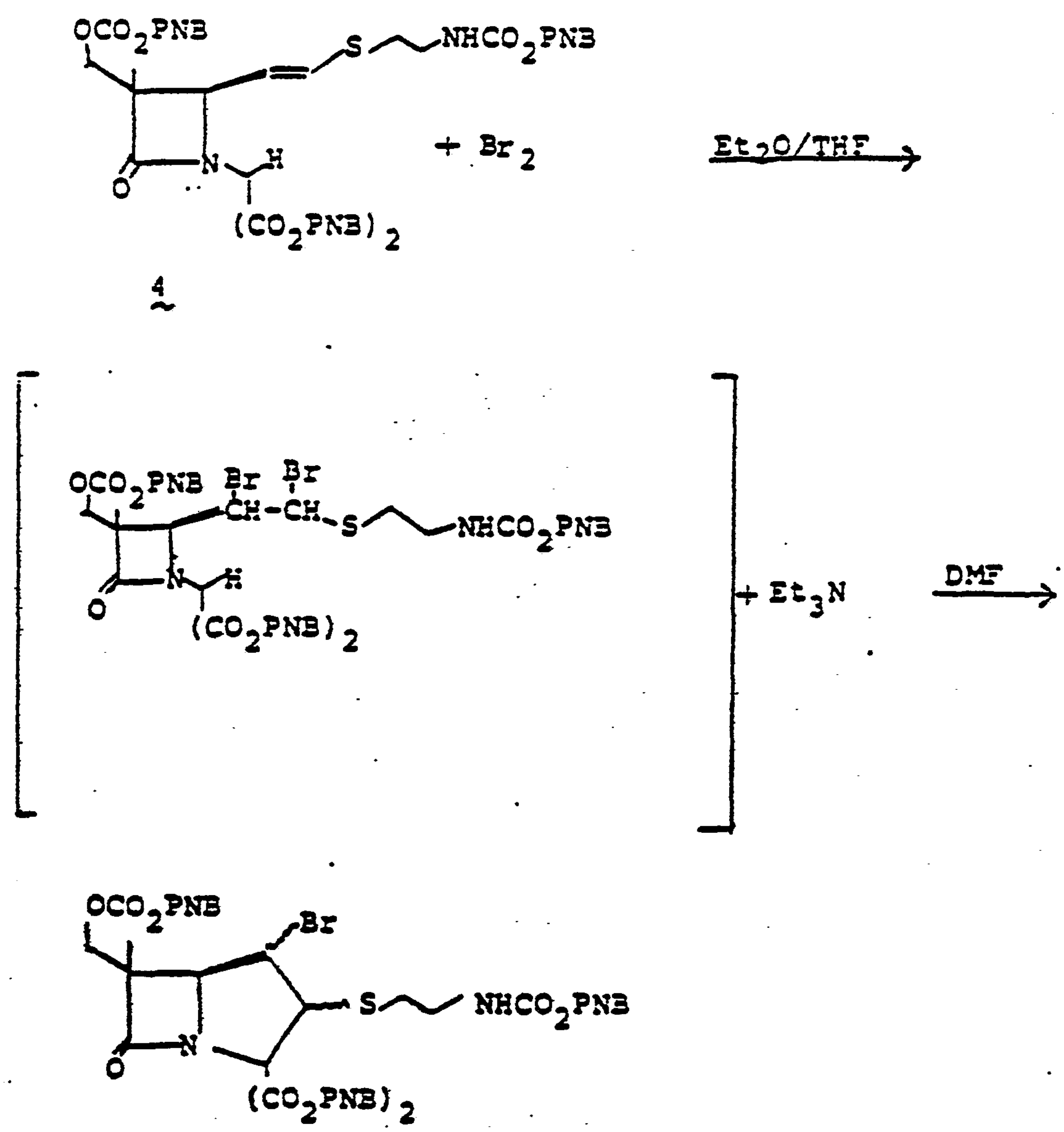


4

5

To 8.5 ml pentane (dried over 4A Linde molecular sieves) is added 0.2 ml $Br_2$ (mw = 160; 3.9 mmole). To 0.706g of 4 (mw = 946; 0.746 mmole) in 18 ml THF (freshly distilled from LAH) and 5.7 ml $Et_2O$ (dried over 3A 1/16" Linde molecular sieves) at 0°C under $N_2$ with stirring is added dropwise 1.8 ml of the above 0.45M $Br_2$ solution (0.81 mmole). After 15 minutes at 0°C., 0.42 ml triethyl amine (mw = 101; $\rho$ = 0.729; 3.03 mmole) is added immediately followed by 10.5 ml ice-cold DMF (distilled from $CaSO_4$ at 40 mm and stored over 4A Linde molecular sieves). The ice-bath is removed, and stirring at room temperature is continued for 2 hours. The reaction mixture is poured into a stirred ice-cold mixture of 3.1 ml 1M $KH_2PO_4$ - 70 ml $H_2O$ - 100 ml EA. The layers are separated, and the aqueous one is saturated with NaCl and re-extracted with EA. The combined organic layers are washed once with brine, dried over anhydrous $MgSO_4$, and filtered. The filtrate is concentrated under a $N_2$ stream and then pumped on high vacuum to give crude 5.

The material is chromatographed on 60g silica gel (diameter = 2.8 cm) packed and applied in $CHCl_3$ and is eluted with 100 ml-2% EA/$CHCl_3$; 100 ml-4% Ea/$CHCl_3$ and then 5% EA/$CHCl_3$ for the remainder of the chromatography. The fractions containing pure 5 are combined, concentrated under a $N_2$ stream, and pumped on high vacuum to give 5.

Step H

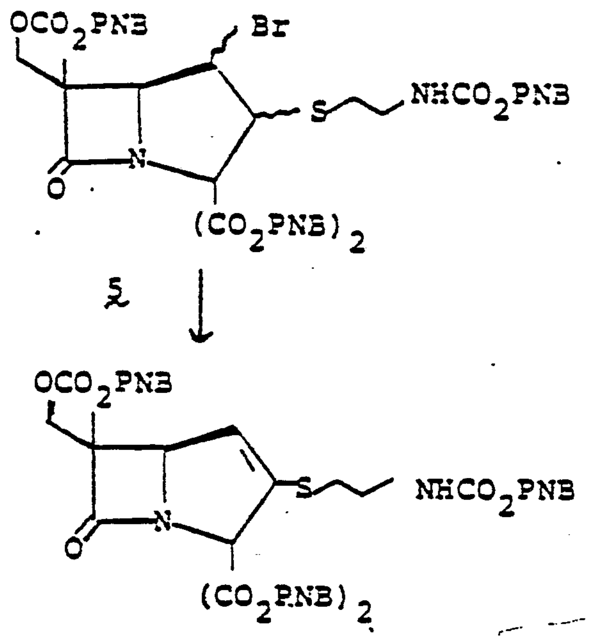

5

6

To 29 mg anhydrous silver fluoride (mw = 127; 0.23 mmole) is added a solution of 146 mg of 5 (mw = 1024; 0.14 mmole) in 3.5 ml anhydrous pyridine. The stoppered reaction mixture is stirred at room temperature in the dark for one hour and then poured into 20 ml cold water - 30 ml EA. After separation of the layers, the aqueous one is extracted two times with EA and one time with $CHCl_3$. Each organic layer is extracted one time with $H_2O$ and one time with brine. The combined organic layers are dried over anhydrous $MgSO_4$, filtered, and concentrated under a $N_2$ stream followed by pumping on high vacuum to give crude 6.

Preparative thin layer chromatography (eluant = 40% acetone/hexane; repeated extraction of desired u.v. band with a large volume of $CHCl_3$) yields

slightly contaminated 6. Re-chromatographing on silica using EA in $CHCl_3$ as an eluting system gives 6.

Step I

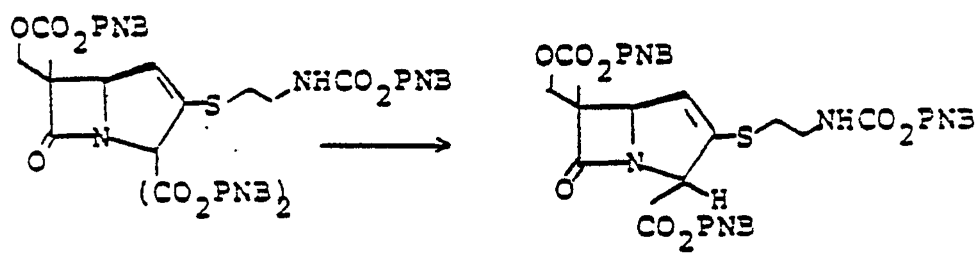

6 7

A solution of 77 mg of 6 (mw = 944; 0.082 mmole) in 0.9 ml S-collidine (distilled from powdered KOH~ 30 mm pressure) is added to 13.4 mg anhydrous LiI (dried for few hours at 100°C over $P_2O_5$ under vacuum) (mw = 134; 0.1 mmole). With stirring under $N_2$, the reaction mixture is heated in an oil bath at 120°C. After a total of 30 minutes, the reaction mixture is cooled to 25°C., diluted with $CH_2Cl_2$, and transferred to a round bottom flask for concentration under a $N_2$ stream and then on high vacuum. Partitioning the residue between EA-$H_2O$ and 1 ml 1M $KH_2PO_4$ is followed by extraction of the aqueous layer two additional times with EA and one time with $CHCl_3$. Each organic layer is then backwashed with brine. The combined organic layers are dried over anhydrous $MgSO_4$, filtered, concentrated under a $N_2$ stream and then on high vacuum to give crude 7.

Preparative thin layer chromatography on silica gel (plate is eluted two times with 40% acetone/ hexane; repeated extraction of the appropriate u.v.

bands with large volume of $CHCl_3$) yields recovered starting material and 7.

Step J

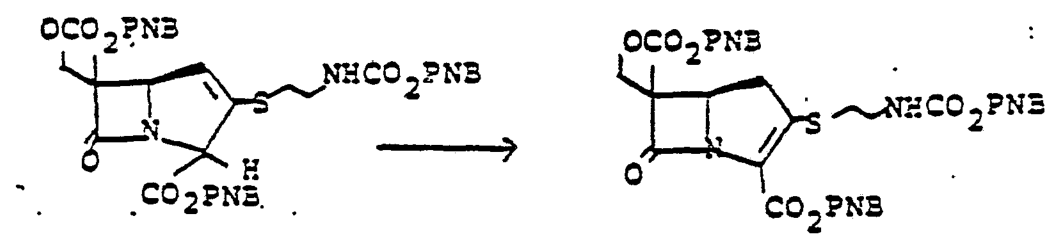

7 8

To 49 mg of 7 (mw = 765; 0.064 mmole) in 0.7 ml DMSO (distilled from $CaH_2$ at 8mm and stored over 4A Linde molecular sieves) is added 100 µl diisopropylamine (distilled from NaH under $N_2$ and stored over 4A Linde molecular sieves) (mw = 101; $\rho$ =0.722; 0.71 mmole). The stoppered reaction mixture is stirred for a few minutes and then allowed to stand for 2 hours. The amine and most of the DMSO are then concentrated off under high vacuum with no external heating. The residue is passed quickly through a column of silica gel (packed, applied, and eluted with EA) to remove residual DMSO. After concentration under a $N_2$ stream of all fractions having u.v. absorbance, the material is chromatographed on a thin layer silica gel plate (eluant = 50% EA/$CHCl_3$; repeated extraction of desired u.v. bands with a large volume of chloroform). Recovered starting material is re-submitted to the reaction conditions and isolation procedure two more times to yield additional 8.

Step K

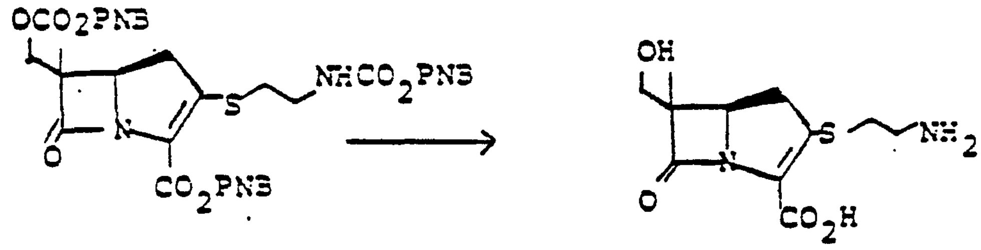

8 9

To 5.2 mg 8 is added 0.60 ml dioxane, 0.05 ml ethanol, 0.35 ml deionized water and 0.01 ml of 1.0M $K_2HPO_4$. To the resultant clear solution is added 5 mg of 10% Pd/C. The suspension is flushed with $N_2$, then 5-6 times alternately with 50 psi $H_2$ and vacuum. Finally, it is shaken under a 50 psi $H_2$ atmosphere for 30-40 min. After centrifugation, the Pd/C is washed and centrigufed 2-3X with 0.5 ml portions of deionized water. The combined centrifugates are extracted 5x1 - 2 ml ether. Residual ether is removed under vacuum and the aqueous solution applied to an XAD-2 column (20 x 140 mm). Fractions of 100 drops (6-7 ml) are collected, with continuous UV monitoring, by elution with deionized water. Emergence of strongly UV absorbing material begins around fractions 3-5 and is usually complete by fractions 25-30. Early fractions are examined by UV to exclude those few deemed too strongly absorbing in the 270-280 mμ region. The remaining fractions are combined and lyophilized. The residue is evaluated by dissolving in 10.0 ml of deionized water and measuring the UV absorption at 298 mμ indicating a 10-30% yield of desired product.

The following Example specifically illustrates a preferred stereo-selective process embodiment of the present invention. As described above in detail, the starting material is a pure optical isomer of 4-vinyl-2-azetidinone (23, above).

EXAMPLE 13

Step A

Preparation of [1-(t-butyldimethylsilyl)-4-vinyl-2-azetidinone]

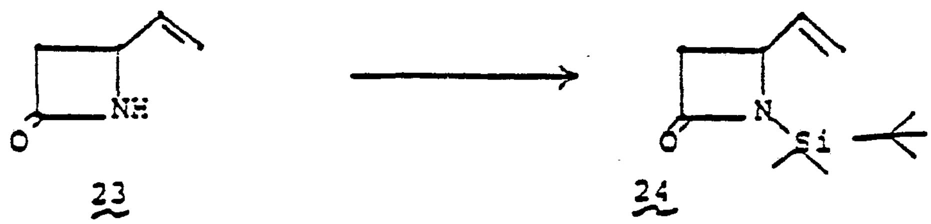

23 24

A solution of 23 [4-vinyl-2-azetidinone] (1.153 g, 11.89 mmoles) and triethylamine (1.82 ml, 13.08 mmoles) in anhydrous N,N-dimethylformamide is placed under a nitrogen atmosphere, cooled to 0°C and treated with t-butyldimethylchlorosilane (1.885 g., 12.48 mmoles) resulting in the immediate appearance of a heavy white precipitate. This mixture is stirred for one hour while gradually warming to room temperature. The mixture is partitioned between 30 ml methylene chloride and 90 ml cold 1M potassium dihydrogen phosphate. The aqueous phase is extracted with 20 ml methylene chloride. The combined organic phases are washed four times with 30 ml portions of water and finally with 50 ml brine. The methylene chloride solution is dried over anhydrous magnesium sulfate and filtered. The filtrate is evaporated under reduced pressure to give 2.25 g of 24 [1-(t-butyldimethylsilyl)-4-vinyl-2-azetidinone] as a colorless liquid.

NMR ($CDCl_3$) δ : 6.23 - 5.10, m, C<u>H</u>=C$\underline{H}_2$

4.07, 7-line m, J=8,6 and 3Hz, C-4<u>H</u>

3.35, dd, J=15 and 6Hz, C-3<u>H</u> <u>cis</u> to C-4<u>H</u>

2.73, dd, J=15 and 3Hz, C-3<u>H</u> <u>trans</u> to C-4 H

.98, s, $(C\underline{H}_3)_3$ C Si

.23, s
.18, s $(C\underline{H}_3)_2$ Si

Following the above procedure, but making the indicated substitution, the other isomer is obtained.

Step A'

Preparation of 3-methyl-(t-butyldimethylsilyl)-4-vinyl-2-azetidinone

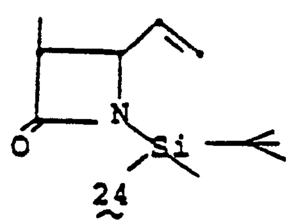

24

Following the procedure of the preparation of 8-oxo-2,2,7-trimethyl-3-oxa-1-azabicyclo[4·2·0]octane (Example 4b, above), except that an equivalent amount of 1-(t-butyldimethylsilyl)-4-vinyl-2-azetidinone is substituted for the 8-oxo-2,2-dimethyl-3-oxa-1-azabicyclo[4·2·0] octane of Example 4b, the title compound is obtained.

Step B

Preparation of 3-methyl-[1-(t-butyldimethylsilyl)-3-(hydroxymethyl)-4-vinyl-2-azetidinone]

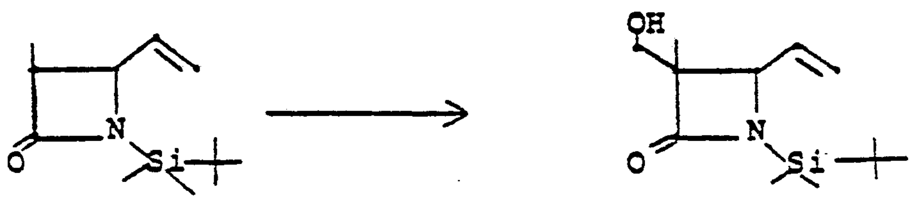

24 25

To a solution of freshly prepared lithium diisopropylamide (7.82 mmoles) in 36 ml anhydrous tetrahydrofuran under a nitrogen atmosphere at -75°C is added a solution of 3-methyl-[1-(t-butyldimethylsilyl)-4-vinyl-2-azetidinone, 24, (1.60 g, 7.11 mmoles) in 10 ml anhydrous THF. The resulting yellow solution of the lithium enolate is, after 16 minutes, treated with excess formaldehyde (see Example 15, below). In 10 minutes, the reaction is quenched by adding 30 ml of a saturated aqueous ammonium chloride solution. This mixture is extracted with 30 ml and 25 ml portions of ethyl acetate. The combined ethyl acetate solutions are washed with 50 ml of brine and dried over anhydrous magnesium sulfate. The drying agent is removed by filtration and the filtrate is evaporated *in vacuo* to give the crude product as a yellow oil. Purification by chromatography on silica gel eluting with 10% ethyl acetate/chloroform gives 3-methyl-[1-(t-butyldimethylsilyl)-3-hydroxymethyl)-4-vinyl-2-azetidinone, 25.

Step C

Preparation of 3-methyl-1-(t-butyldimethylsilyl)-3-(1-p-nitrobenzylcarbonyldioxymethyl)-4-vinyl-2-azetidinone

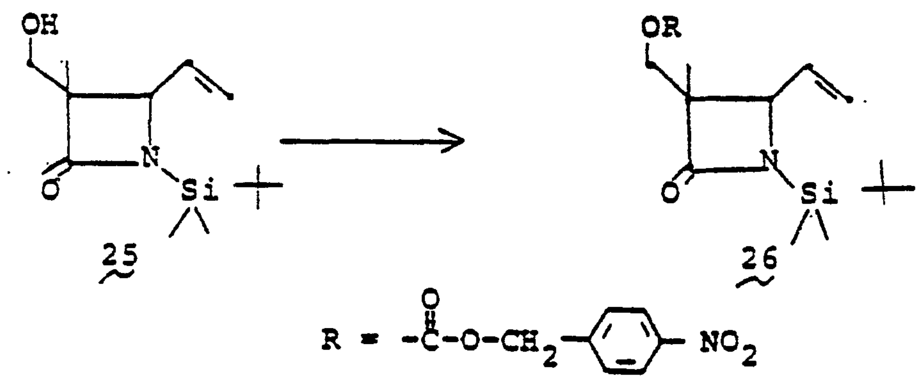

25

26

R =

Under nitrogen at -78°C a solution of 25 (56 mg, .220 mmole) in 1 ml of anhydrous tetrahydrofuran is treated with 2.4M n-butyllithium in hexane (101 µl, .242 mmole). To this solution is added, in five minutes, a solution of p-nitrobenzyl chloroformate (52 mg, .242 mmole) in anhydrous tetrahydrofuran. After stirring at -78°C for a period of 55 minutes, 10 ml of a saturated aqueous ammonium chloride solution is added and the product extracted into ethyl acetate. The combined ethyl acetate solutions are washed with brine and dried over anhydrous magnesium sulfate. The drying agent is removed by filtration, and the filtrate is evaporated *in vacuo* to give crude 26. Purification by preparative thick-layer chromatography on silica gel developing with 5% ethyl acetate/chloroform gives 26.

Step D

Desilylation of 26 to provide 27 [3-methyl-3-(p-nitrobenzylcarbonyldioxymethyl)-4-vinyl-2-azetidinone]

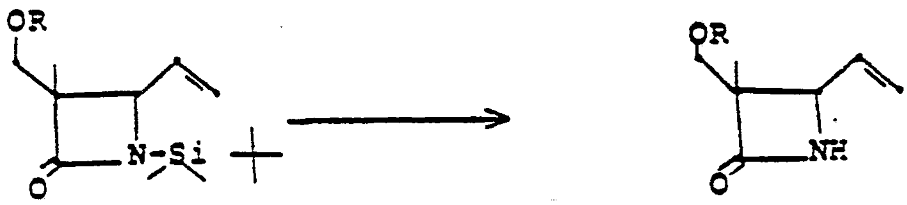

26 27

R = 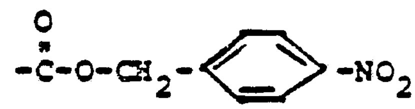

A solution of 26 [1-(t-butyldimethylsilyl)-3-methyl-3-p-nitrobenzylcarbonyldioxymethyl)-4-vinyl-2-azetidinone] (61 mg, .141 mmole) in 2 ml of .5N HCl/MeOH is stirred at room temperature (25°C) for a period of 3 hours. The solution is then cooled to 0°C and neutralized by the addition of 5 ml of 5% aqueous sodium bicarbonate. The product is extracted into ethyl acetate (10 ml, 2x5 ml). The combined ethyl acetate solutions are washed with water (2 x 5 ml) and 10 ml brine and then dried over anhydrous magnesium sulfate. The drying agent is removed by filtration, and the filtrate is evaporated *in vacuo* to give an oil. Preparative thick-layer chromatography of this material on silica gel developing with 10% ethyl acetate/chloroform gives 3-methyl-3-(p-nitrobenzylcarbonyldioxymethyl)-4-vinyl-2-azetidinone, 27.

Step E

Preparation of 14 via 28 by sulfenyl halide addition and dehydrohalogenation

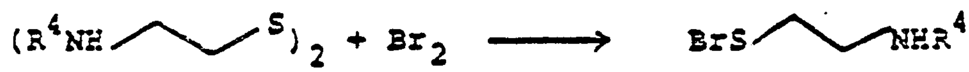

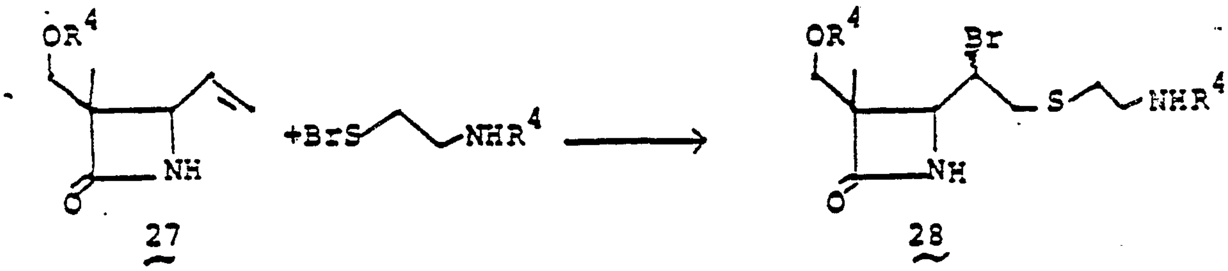

27

28

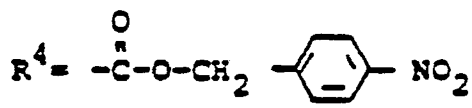

A solution of the N-p-nitroCBZ cysteamine disulfide, 96 mg (0.19 mmoles) in 1.5 ml THF (freshly distilled from $LiAlH_4$) is cooled to -25°C and treated dropwise with stirring with 0.5 ml of a solution of 135 mg $Br_2$ in sieve dried $CCl_4$ (2.2 ml final volume; portion added is equivalent to 0.19 mmoles of $Br_2$). The resultant orange solution is stirred at -20°C for 5 min. then treated with 54.0 mg of the vinyl azetidinone, 27, in 0.5 ml sieve dried $CH_2Cl_2$. The color lightens to yellow. The mixture is allowed to come to 0°C over 5-10 minutes. Examination by tlc (silica 5% MeOH in $CH_2Cl_2$ or 20% EtOAc in $CH_2Cl_2$) shows a main spot with $R_f$ and $Ce^{IV+}/H^+$/heat characteristics different from either disulfide or starting 4-vinyl-2-azetidinone. The reaction mixture is concentrated to 0.5 ml under $N_2$, streaked directly on two 8" x 8" 1000μ silica GF plates, and developed with 20% EtOAc in $CH_2Cl_2$. The main band under U.V., is scraped off, and extracted with EtOAc to give 28.

Step F

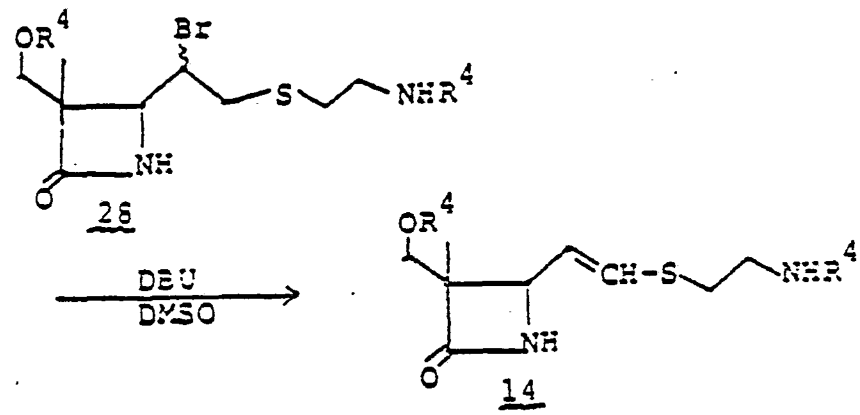


28

14

The bromosulfide, 28, 77.0 mg (0.162 mmole) is dissolved in 1.0 ml sieve stored DMSO (distilled from $CaH_2$), and stirred under nitrogen while 25λ DBU (0.19 mmole) is added. After 3 hours, the mixture is poured into water/$KH_2PO_4$ and extracted repeatedly with EtOAc. The combined extracts are washed twice with water, dried with anhydrous $MgSO_4$ and evaporated under nitrogen. The crude product, is streaked on an 8 x 8" 1000µ silica GF plate and developed with 20% EtOAc in $CH_2Cl_2$ to give 14.

## EXAMPLE 14

Preparation of Bis (p-Nitrobenzyloxycarbonylaminoethyl)-disulfide

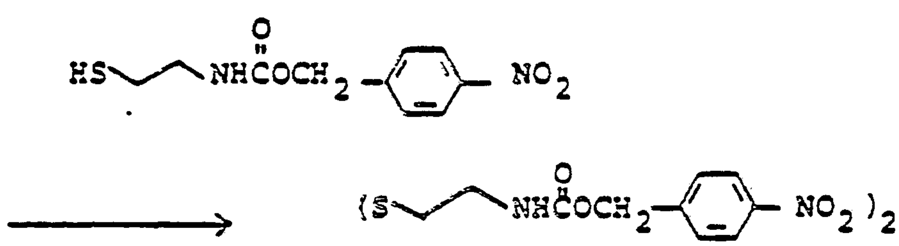

Under nitrogen at -20°C, bromine (1.21 ml, .022 mmole) is added to a solution of p-nitrobenzyloxycarbonyl-aminoethanethiol (11.28g, .044 mole) in 100 ml of anhydrous tetrahydrofuran. The cooling bath is removed, and the cold solution is stirred for 15 minutes. The solution is then diluted with 400 ml ethyl acetate and washed with 200 ml 1M pH 7 phosphate buffer, 200 ml 1M dibasic potassium phosphate, water (2 x 200 ml, 100 ml) and 200 ml brine. It is dried over anhydrous magnesium sulfate and filtered. The filtrate is evaporated *in vacuo* giving a yellow solid residue. This material is chromatographed on silica gel eluting with 5% ethyl acetate/chloroform to give 10.5 g of crystalline bis (p-nitrobenzyloxycarbonylaminoethyl)disulfide:

IR ($CH_2Cl_2$) μ : 3.04NH
5.96 carbonyl
6.22, 6.61 nitro

NMR ($CDCl_3$)δ : 8.24, 7.54 } d, J=8.5Hz, ArH
5.37, broad s, NH
5.26, s, $ArCH_2O$
3.60, q, J=6Hz and 6Hz, $NHCH_2CH_2$
2.86, t, J=6Hz, $NHCH_2CH_2S$

## EXAMPLE 15

Preparation of 8-oxo-2,2-dimethyl-7α and β-hydroxymethyl-3-oxa-1-azabicyclo[4·2·0]octane

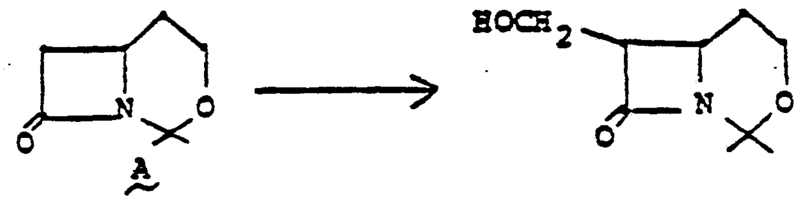

A

The procedure of Example 5, substituting A for 8-oxo-2,2,7-trimethyl-3-oxa-1-azabicyclo[4·2·0]octane, is carried out. Upon purification by silica gel chromatography, the title compound is obtained.

## EXAMPLE 16

Preparation of 3-(2-aminoethylthio)-6-hydroxymethyl-7-oxo-1-azabicyclo[3·2·0]hept-2-ene-2-carboxylic acid

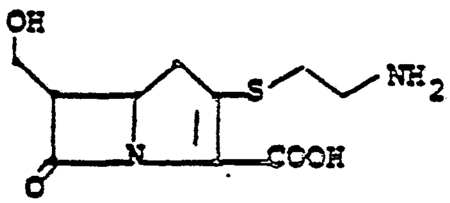

Following the procedure described above for the preparation of 3-(2-aminoethylthio)-6-methyl-6-hydroxymethyl-7-oxo-1-azabicyclo [3·2·0]hept-2-ene-2-carboxylic acid, except that in Example 6 an equivalent amount of 8-oxo-2,2-dimethyl-7-hydroxymethyl-3-oxa-1-azabicyclo[4·2·0]octane rather than the 2,2,7-trimethyl species is taken; the title compound is obtained when the procedures of Examples 6,7 and 12 are followed.

## EXAMPLE 17

Preparation of 8-oxo-2,2-dimethyl-7α and β-(1-hydroxy-2-phenylethyl)-3-oxa-1-azabicyclo[4·2·0]octane

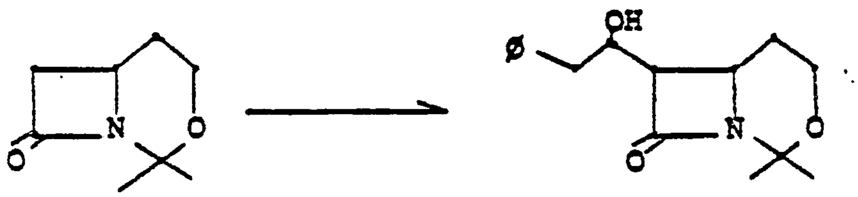

Following the procedure of Example 15, except that instead of formaldehyde, an equivalent amount of phenylacetaldehyde is used, upon purification by silica gel chromatography, the title compound is obtained.

## EXAMPLE 18

Preparation of 3-(2-aminoethylthio)-6-(1-hydroxy-2-phenylethyl)-7-oxo-1-azabicyclo[3·2·0]hept-2-ene-2-carboxylic acid

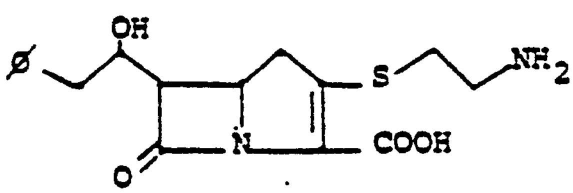

Following the procedure of Example 16 except that 8-oxo-2,2-dimethyl-7(1-hydroxy-2-phenylethyl)-3-oxa-1-azabicyclo[4·2·0]octane is substituted in equivalent amount for its analogous substrate, the title compound is obtained.

EXAMPLE 19

Preparation of 8-oxo-2,2-dimethyl-7α-benzyl-3-oxa-1-azabicyclo [4·2·0]octane

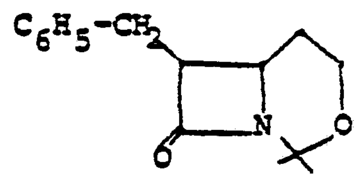

Following the procedure described for the preparation of 8-oxo-3-oxa-2,2-dimethyl-7α-isopropyl-1-azabicyclo [4·2·0]-octane from 8-oxo-3-oxa-2,2-dimethyl-1-azabicyclo[4·2·0]-octane (Example 4a, above) and using benzyl bromide instead of isopropyl iodide there is obtained 8-oxo-2,2-dimethyl-7α-benzyl-3-oxa-1-azabicyclo[4·2·0]octane.

i.r. μ : 5.73 (β-lactam)

n.m.r. δ : 1.33S, 1.75S (gem dimethyl); 1.74m (C-5 H)
3.0 d of d ($C_6H_5$-$C\underline{H}_2$); 3.73 d of d (C-2 H)
7.25S ($C_6H_5$).

EXAMPLE 20

Preparation of 8-oxo-2,2-dimethyl-7α and β-benzyl-7β and α-(1-hydroxyethyl)-3-oxa-1-azabicyclo[4·2·0]octane

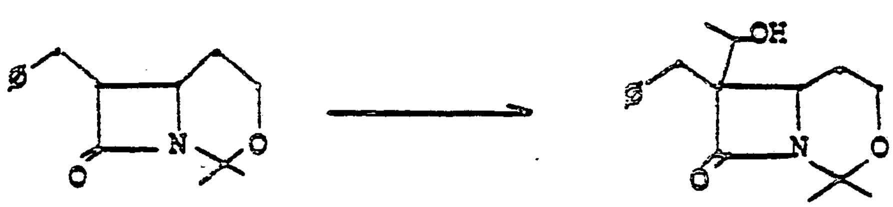

Using the procedure of Example 5 but substituting an

equivalent amount of 8-oxo-2,2-dimethyl-7α-benzyl-3-oxa-1-azabicyclo[4·2·0]octane (Example 19) for 8-oxo-2,2,7-trimethyl-3-oxa-1-azabicyclo[4·2·0]octane, and an equivalent amount of acetaldehyde for formaldehyde, there is obtained, upon purification by silica gel chromatography, the title compounds.

## EXAMPLE 21

Preparation of 8-oxo2,2-dimethyl-7α(1-mesyloxyethyl)-3-oxa-1-azabicyclo[4·2·0]octane

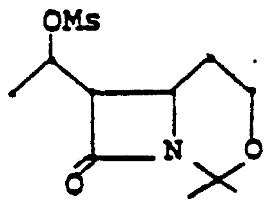

Under nitrogen at 0° a solution of rel-(6S,7R)-8-oxo-2,2-dimethyl-7,(1R-hydroxyethyl)-3-oxa-1-azabicyclo [4·2·0]octane and rel(6S,7R) 8-oxo-2,2-dimethyl-7(1S-hydroxyethyl)-3-oxa-1-azabicyclo-[4·2·0]octane as an approximately 1:2 diastereomeric mixture (128 mg, 0.643 mm) and triethylamine (134 μl or .965 mm) in 5 ml of sieve dried methylene chloride is treated with redistilled methane sulfonyl chloride (55 μl or .707 mm). The resulting solution is stirred for 30 minutes. It is then washed with 10 ml each of cold water, 1 molar pH 3 phosphate buffer, 5% sodium bicarbonate, water and finally brine. The organic phase is dried over magnesium sulfate and filtered. The filtrate is evaporated in vacuo to give 156 mg of a pale yellow oil. This material is chromatographed on preparative thick layer silica gel plates developed four times with 1:9 acetone/hexane to give a separated diastereomeric mesylate in 68% overall yield.

Diastereomer of $R_f$ .13 (25%) -

i.r. $CH_2Cl_2$ μ, 5.73 β-lactam.

n.m.r. $C_6D_6$ δ 5.06-4.61, 8 line multiplet, 1H, J=6.5 and 7.5 Hz, $CH_3C\underline{H}$

3.58-3.26 multiplet 3H, $C_4$-methylene, $C_6$-methine

2.61, dd, 1H, J=1.8 and 7.5 Hz, $C_7$-methine.

2.40, s, 3H, $C\underline{H}_3SO_2$

1.83, s, 3H } C-2 methyls
1.09, s, 3H }

1.30, d, 3H, J=6.5 Hz, $C\underline{H}_3CH$

Diastereomer of $R_f$ .08 (43%)

i.r. $CH_2Cl_2$ μ β-lactam 5.71

n.m.r. $C_6D_6$ δ 5.04 - 4.64, 8 line multiplet 1H, J=4.5 and 6.5 Hz, $CH_3C\underline{H}$

3.63 - 3.10 multiplet, 3H, $C_4$-methylene and $C_6$ methine.

2.67 dd, 1H, J=1.8 and 4.5 Hz, $C_7$-methine

2.46 s, 3H, $C\underline{H}_3SO_2$

1.81, s, 3H } C-2 methyls
1.16, s, 3H }

1.34 d, 3H, J=6.5 Hz, $C\underline{H}_3CH$.

EXAMPLE 21a

Preparation of 8-oxo-2,2-dimethyl-7α-(1-azidoethyl)-3-oxa-1-azabicyclo[4·2·0]octane

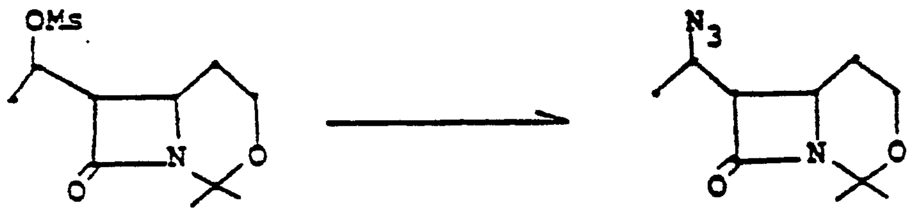

A solution of lithium azide, prepared by stirring over night a mixture of 170 mg sodium azide and 100 mg lithium chloride in 3 ml of anhydrous dimethyl sulfoxide, followed by filtration, is added to 160 mg of 8-oxo-2,2-dimethyl-7α(1-mesyloxyethyl)-3-oxa-1-azabicyclo-[4·2·0]octane and stirred under nitrogen at 25°C for eight hours or until the reaction is judged complete by tlc. The mixture is then poured into 10 ml ice water and extracted with ethyl acetate, the combined extracts washed once with water, once with saturated sodium chloride solution, dried ($MgSO_4$) and evaporated under a nitrogen stream. The crude product is purified by preparative tlc to afford the title compound.

EXAMPLE 22

Preparation of 8-oxo-2,2-dimethyl-7α-(1-p-nitrobenzyloxycarbonylaminoethyl)-3-oxa-1-azabicyclo[4·2·0]octane

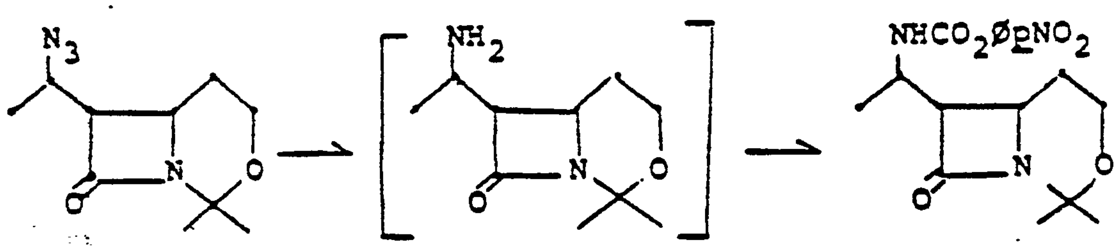

A mixture of 107 mg of 8-oxo-2,2-dimethyl-7α-(1-azidoethyl)-3-oxa-1-azabicyclo[4·2·0]octane and 50 mg of 10% Pd/C in 2 ml of anhydrous dioxane is shaken under 3 atmospheres of hydrogen for 1.5 hours. The mixture is filtered and concentrated to 1 ml under a nitrogen stream, then treated with 1 ml of 1M $K_2HPO_4$ and 1 ml methylene chloride. The mixture is stirred vigorously in an ice bath under nitrogen while a solution of 120 mg of p-nitrobenzylchloroformate in 0.5 ml methylene chloride is added dropwise over one minute. The solution is stirred for another 15 minutes, then treated with 0.1 ml pyridine in 2 ml water and stirred vigorously for another 15 minutes. The organic phase is removed and the aqueous phase is extracted several more times with methylene chloride. The combined organic phases are washed twice with water, once with saturated NaCl, dried ($MgSO_4$) and evaporated under reduced pressure. Purification by preparative tlc affords the title compound.

## EXAMPLE 23

Preparation of 8-oxo-2,2-dimethyl-7[(1-o-nitrobenzylthioethyl)-3-oxa-1-azabicyclo[4·2·0]octane

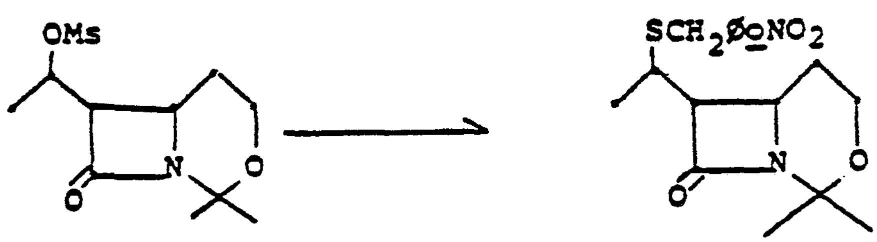

When in Example 21a an equivalent solution of o-nitrobenzyl mercaptan in DMF is substituted for the lithium azide solution, the title compound is obtained.

EXAMPLE 24

Preparation of 4-(2,2-(bisbenzylthio)ethyl)-3-methyl-3-(p-nitrobenzylcarbonyldioxymethyl)-2-azetidinone

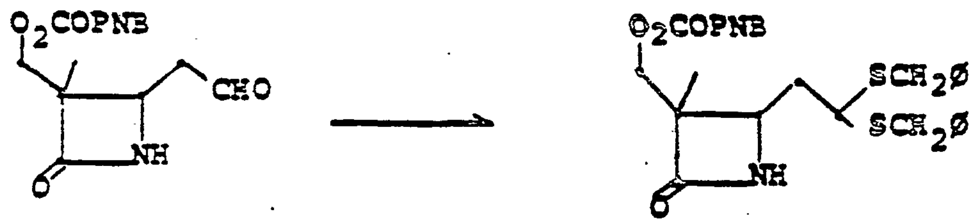

When in Example 12, Step A, an equivalent amount of benzyl mercaptan is substituted for 2-(p-nitrobenzyloxycarbonylamino)ethane thiol, the title compound is obtained.

EXAMPLE 25

Preparation of 3-methyl-4-(2,2-(bis-o-nitrobenzylthio)ethyl)-3-(nitrobenzylcarbonyldioxymethyl)-2-azetidinone

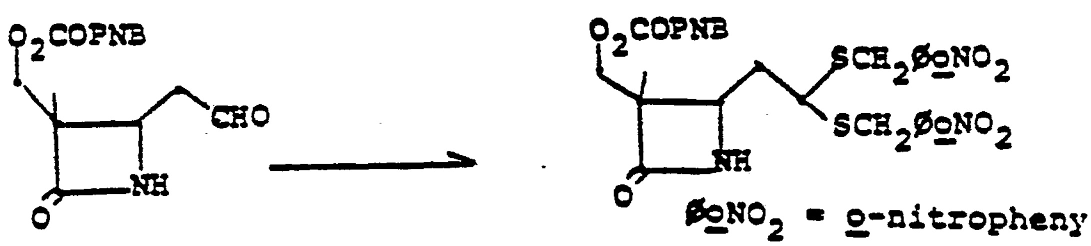

ØoNO₂ = o-nitrophenyl

When in Example 12, Step A, an equivalent amount of o-nitrobenzylthiol is substituted for 2-(p-nitrobenzyloxycarbonylamino)ethane thiol, the title compound is obtained.

EXAMPLE 26

Preparation of 3-methyl-3-(p-nitrobenzylcarbonyldioxymethyl)-4β-[1-bromo-2-(2-p-nitrobenzyloxycarbonylamino) 1,1-dimethylethylthio)ethyl]-2-azetidinone

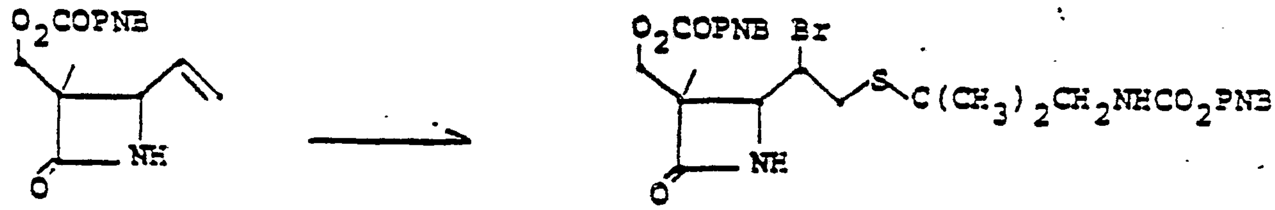

If in Example 13, Step E, an equivalent solution of 2-(p-nitrobenzyloxycarbonylamino)-1,1-dimethylethylsulfenyl bromide, prepared by cleavage of bis(2-(p-nitrobenzyloxycarbonylamino)-1,1-dimethylethylthio)mercury with bromine in THF/ether at 0°C., is substituted for the solution of 2-(p-nitrobenzyloxycarbonylamino)ethylsulfenyl bromide, the title compound is obtained.

EXAMPLE 27

Preparation of 3-benzylthio-6-methyl-6-hydroxymethyl-7-oxo-1-azabicyclo[3·2·0]hept-2-ene-2-carboxylic acid

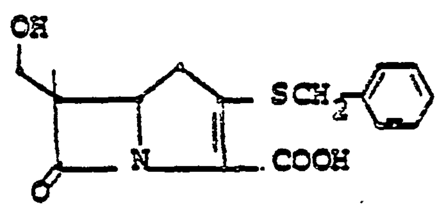

Following the procedure of Example 12, Steps A-K except substituting for the indicated azetidinone the azetidinone of Example 24, the title compound is obtained.

## EXAMPLE 28

Preparation of 3-(2-amino-1,1-dimethylethylthio)-6-methyl-6-(1-hydroxymethyl)-7-oxo-1-azabicyclo[3·2·0]hept-2-ene-2-carboxylic acid

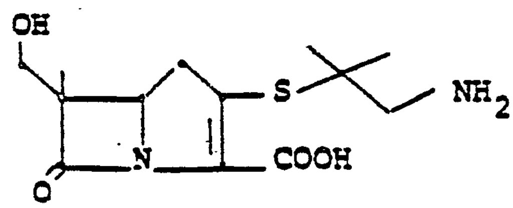

Following the procedure of Example 13, Step F, except substituting for the indicated azetidinone the azetidinone of Example 26, followed by the reactions corresponding to those of Example 12 D-K, the title compound is obtained.

## EXAMPLE 29

Preparation of 3-mercapto-6-methyl-6-(p-nitrobenzylcarbonyldioxymethyl)-7-oxo-1-azabicyclo-[3·2·0]hept-2-ene-2-carboxylic acid, p-nitrobenzyl ester

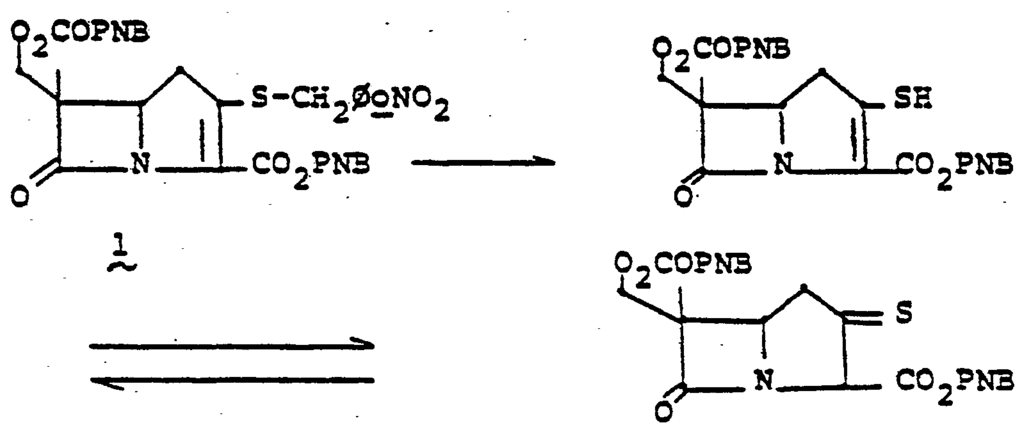

1

A solution of 5 mg of 1 (prepared from the azetidinones

of Example 25 and the procedure of Example 12) in 0.6 ml of dioxane is irradiated for one hour in a quartz vessel under nitrogen with nitrogen being slowly bubbled through (1 bubble per 5 sec.) using 300 nm sources in a Rayonet apparatus, to give the title compound as a mixture of thiol-thione tautomers.

## EXAMPLE 30

Preparation of 6-Methyl-6-(hydroxymethyl)-3-mercapto-7-oxo-1-azabicyclo[3·2·0]hept-2-ene-2-carboxylic acid

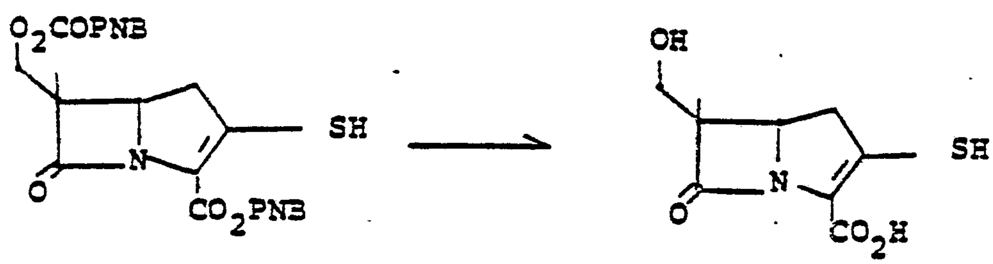

If the solution obtained after irradiation in Example 29 is immediately treated with 0.05 ml of ethanol, 0.35 ml deionized water, 0.01 ml of 1.0M $K_2HPO_4$, and 5 mg of 10% Pd/C and then treated as in Example 12, Step K, except that instead of purification on the XAD-2 column the ether extracted aqueous solution is cooled in ice, carefully acidified to pH 2 and extracted with ethyl acetate, and the combined extracts then washed once with saturated NaCl solution, dried with $MgSO_4$ and concentrated under a stream of $N_2$, the title compound is obtained.

## EXAMPLE 31

Preparation of (±)-9,9-dimethylthienamycin

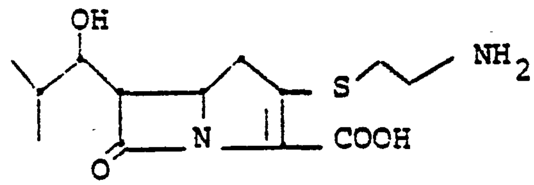

### Step A

Preparation of 1-t-butyldimethylsilyl-3α-(1(R*)-hydroxy-2-methylpropyl)-4β-vinyl-2-azetidinone

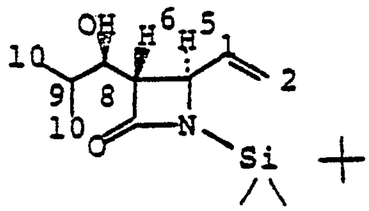

A solution of 1.226 g (5.8 m moles) of 1-t-butyldimethylsilyl-4-vinyl-2-azetidinone in 6 ml of anhydrous THF containing ~1 mg of 2,2'-dipyridyl and 0.075 ml (0.53 m moles) of diisopropyl amine is cooled under nitrogen to -78°C and treated with 2.5 ml of 2.3 M n-butyl lithium (5.75 m moles) with stirring over 5 minutes.

To the resultant orange solution is added 0.8 ml (0.63 g; 8.2 m moles) of freshly distilled isobutyraldehyde over one minute to give a pale yellow solution. The cooling bath is removed and the mixture is allowed to stir for 15 minutes, and is then worked up by pouring into 60 ml of ice/water plus 10 ml 1 M $KH_2PO_4$, and extracting 4 times with 15 ml portions of ethyl acetate. The combined extracts are washed once with 0.2 M $KH_2PO_4$, once with saturated NaCl, dried with $MgSO_4$, and evaporated evaporated under a $N_2$ stream to give 1.74 g (1.67 g = 100%) of pale yellow oil. Examination by 300MHz PMR shows three main components, identified as the 3β-(1R*), 3α-(1S*),

and the desired 3α-(1R*) isomers in ca. 20, 60, and 20% yield respectively. If desired the product may be obtained at this point by chromatography on silica-gel using $CH_2Cl_2$ with low percentages of EtoAc. Because the product is crystalline, it can be isolated from enriched fractions even though not completely resolved. The 300 MHz PMR ($CDCl_3$) shows (see numbering system above) 5.91 ($H_1$, ddd, $J_{1,2\ cis}$=10Hz; $J_{1,2\ trans}$=17Hz; $J_{1,5}$= 9Hz), 5.21 ($H_{2\ cis}$, d, $J_{1,2\ cis}$= 10Hz), 5.33 ($H_{2\ trans}$, d, $J_{1,2\ trans}$= 17Hz), 4.17 ($H_5$, dd, $J_{1,5}$=9Hz; $J_{5,6}$=3Hz), 3.86-3.79 ($H_8$, m), 3.17 ($H_6$, dd, $J_{5,6}$=3 Hz; $J_{6,8}$=3 Hz), 1.86 - 1.74 ($H_9$, m, $J_5 \sim$ 6 Hz), 0.96 (Si-t-butyl,s), 0.81 ($H_{10}$,d,$J_{9,10}$=6 Hz), 0.25 and 0.17 (Si-$CH_3$,s) δ.

A preferred procedure entails oxidizing the initial aldol mix to the ketone and reducing with borohydride to give a mixture much enriched in the desired product.

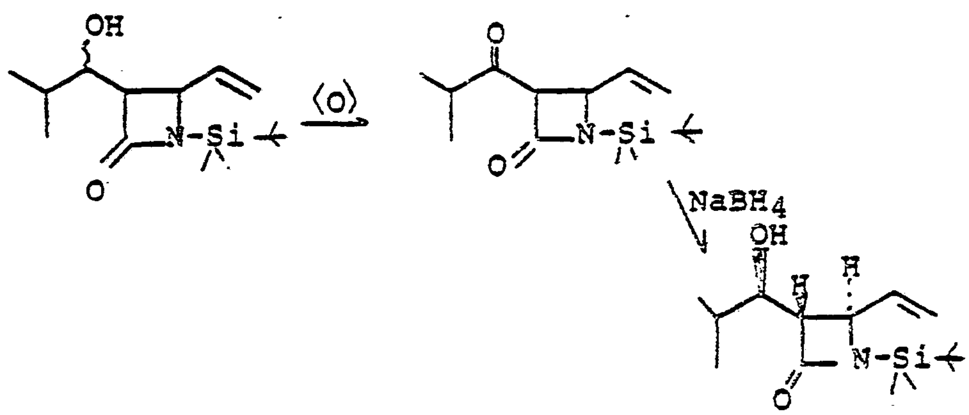

Thus the crude product above is taken up in 10 ml dry $CH_2Cl_2$ and cooled under $N_2$ in a -78°C bath. To 10 ml dry $CH_2Cl_2$ is added 0.86 ml (945 mg; 12.1 m mole) DMSO and the mix cooled to -65°C under $N_2$. After 10 minutes, 1.30 ml (1.94 g, 9.2 m moles) of trifluoroacetic anhydride is added and the mixture stirred for 15 minutes at -65°C. To this clear solution is added the chilled aldol solution by siphoning. The mixture is stirred for 1 hour at -65°C, treated with 2.7 ml (1.98 g; 19.5 m moles) of $Et_3N$, the cooling bath is removed and stirring is continued for 1 hour. The mix is then washed four times with water, once with saturated NaCl, and dried with $MgSO_4$; upon removing the solvent under a $N_2$ stream, 1.2 g of an orange oil is obtained. Examination by tlc (silica-gel; $CH_2Cl_2$) shows the material to be overwhelmingly one component. If desired, further purification can be effected by chromatography on silica-gel, eluting with $CH_2Cl_2$. A 300MHz PMR spectrum ($CDCl_3$) shows 5.89 ($H_1$, ddd $J_{1,2cis}$=10Hz; $J_{1,2trans}$=17Hz; $J_{1,5}$=9Hz), 5.26 ($H_{2cis}$, d, $J_{1,2cis}$=10Hz), 5.39 ($H_{2trans}$, d, $J_{1,2}$= 17Hz), 4.41 ($H_5$, dd, $J_{1,5}$=9Hz; $J_{5,6}$=2Hz), 4.18 ($H_6$, d, $J_{5,6}$=3Hz), 2.75-2.90 ($H_9$, m, $J_{9,10}$=7Hz), 1.16 ($H_{10}$, d, $J_{9,10}$=7Hz) 0.97 (Si-*t*-butyl, s), 0.17 and 0.25 (Si-$CH_3$, s) $\delta$ .

More commonly, the reduction is carried out on crude ketone which has been freed of the most contaminants by filtration through silica-gel with $CH_2Cl_2$.

Thus, 63.85 g crude ketone is passed through 76 g Baker silica-gel and washed on through with a total of 2 liters of $CH_2Cl_2$ to give, upon evaporation 61.64 g (219 m mole) material which is taken up in 480 ml isopropyl alcohol and cooled with stirring in an ice/saturated NaCl bath for 30 minutes. To this is added 10.2 g $NaBH_4$ (268 m moles) and the cloudy suspension stirred for 2 hours with continued cooling. It is then (cautiously) poured, with continued swirling, into 2 liters of ice/water containing 150 g $KH_2PO_4$ (1.08 moles). The mix is extracted five times with 100 ml $CH_2Cl_2$, the combined extracts are washed once with water, once with saturated NaCl, dried with $MgSO_4$ and evaporated to an oil. Examination by PMR shows no significant 3β-isomer; the 3α-(1S*) and 3α-(1R*) isomers are present in 45-40% and 55-60% yield respectively. By concentrating in petroleum ether to ~ 200 ml and seeding, 13 g of crystalline product is obtained directly; a second crop of 10 g is obtained upon further concentration. The mother liquors may be further processed using a preparative HPLC apparatus using 6% $EtOAc/CH_2Cl_2$.

When product can no longer be isolated, the combined mother liquors may be recycled through the oxidation-reduction sequence to generate more.

Step B

Preparation of 1-t-butyldimethylsilyl-3α-(1(R*)-o-nitrobenzylcarbonyldioxy-2-methylpropyl)-4β-vinyl-2-azetidinone

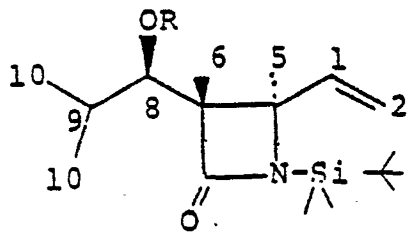

R= 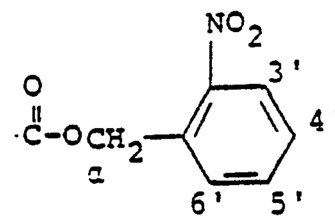

To a mixture of 1.7 g of 1-t-butyldimethylsilyl-3α-(1(R*)-hydroxy-2-methylpropyl)-4β-vinyl-2-azetidinone (6.0 mmoles) and 1.6 g of 4-dimethylaminopyridine (13.1 mmole) in 20 ml of sieve dried $CH_2Cl_2$, stirring in an ice bath under a nitrogen atmosphere, is added 2.9 g of o-nitrobenzyloxycarbonyl chloride (13.4 mmole) in 5 ml of sieve dried $CH_2Cl_2$. The initially slightly milky solution turns within minutes to a thick paste. The bath is removed and the mix allowed to stir over night. The mix is then stirred with 20 ml ice/water for several minutes affording two clear phases. The organic phase is removed and washed twice with water, dried with $MgSO_4$, concentrated to ~5 ml and passed through 5 g of Baker Silica followed by 200 ml of $CH_2Cl_2$.

The entire filtrate is blown down with nitrogen to yield 3.1 g gum. Chromatography was carried out with a Waters Prep 500 Preparative HPLC apparatus, using one 500 ml pack and eluting with 3% EtOAc in $CH_2Cl_2$. After two column volumes, product emerges in ca. three column volumes; after another five column volumes, starting aldol begins to emerge and is more quickly eluted by changing to 7.5% EtOAc in $CH_2Cl_2$ and finally straight EtOAc. One obtains 0.97 g recovered starting material and 1.5 g of product which exhibits a 300 MHz PMR($CDCl_3$) with peaks at 8.34($H_3$, d, $\underline{J}_{3',4'}$=8Hz), 7.74-7.62 ($H_{5',6'}$,M) 7.52($H_{4'}$, dd $\underline{J}_{3',4'}$=8Hz, $\underline{J}_{4',5'}$=8Hz), 5.88 ($H_1$, ddd, $\underline{J}_{1,2\underline{cis}}$=9Hz; $\underline{J}_{1,2\underline{trans}}$=17Hz; $\underline{J}_{1,5}$=9Hz), 5.62($H_\alpha$,s), 5.29 ($H_{2\underline{trans}}$,d,$\underline{J}_{1,2\underline{trans}}$=17Hz), 5.21($H_{2\underline{cis}}$,d,$\underline{J}_{1,2\underline{cis}}$=9Hz), 5.07($H_8$,dd,$\underline{J}_{6,8}$=5Hz; $\underline{J}_{8,9}$=5Hz), 4.13($H_5$,dd,$\underline{J}_{1,5}$=9Hz; $\underline{J}_{5,6}$=3Hz), 3.28($H_6$,dd,$\underline{J}_{5,6}$=3Hz;$\underline{J}_{6,8}$=5Hz), 2.18-2.02($H_9$, m), 0.95-0.85 ($H_{10}$ and Si-t-butyl), 0.24 and 0.15(Si-$CH_3$,s)$\delta$.

On tlc(silica gel GF, 3%EtOAc/$CH_2Cl_2$) product $R_f$ was ~0.4, starting material ~ 0.15.

Step C

Preparation of 3α-(1(R*)-o-nitrobenzylcarbonyl-dioxy-2-methylpropyl)-4β-vinyl-2-azetidinone

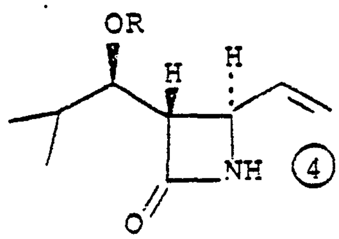 (4)

To 50.3 g of 1-t-butyldimethylsilyl-3α-(1(R*)-o-nitrobenzylcarbonyldioxy-2-methylpropyl)-4β-vinyl-2-azetidinone (109 mmoles) in 480 ml of methanol is added 4 ml of concentrated HCl (48 mmoles) and the mix is stirred for four hours, poured into 4 liters of water plus 50 ml of 1M $K_2HPO_4$ and extracted with EtOAc several times. An insoluble interface solid, isolated by filtration, proves to be 9.8 g of product. The combined extracts are washed once with water, once with saturated NaCl solution, dried and evaporated to give 24.7 g of solids. This is combined with the interface solid and recrystallized from acetone/ether to give 29.7 g product in three crops. Pure material has mp. 137.5-139°C. The 60 MHz PMR ($CDCl_3$) shows

8.15($H_3$,d,$J_{3',4'}$=7Hz), 7.75-7.35($H_{4'-6'}$,m), 6.25-5.00($H_{1-2,8}$,complex), 6.18($H_4$,bs), 5.30($H_c$,s), 4.21 ($H_5$,dd,$J_{1,5}$=7Hz; $J_{5,6}$=2Hz), 3.22($H_6$,dd,$J_{5,6}$=2Hz;$J_{6,8}$=6Hz), 1.7-2.5($H_9$,m),0.9 & 1.0 ($H_{10}$,s), δ.

Step D

Preparation of 3α-(1(R*)-o-nitrobenzylcarbonyldioxy-3-methylpropyl)-4β-[1-bromo-2-(2-p-nitrobenzyloxycarbonylaminoethylthio)ethyl]-2-azetidinone

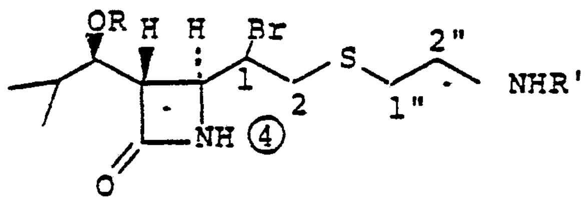

R'=$CO_2CH_2$ 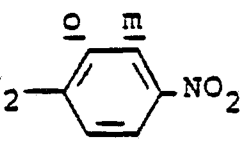

A solution of 254 mg of p-nitrobenzyloxycarbamidoethyldisulfide (0.5 mmole) in 3 ml of THF (freshly distilled from $LiAlH_4$) is cooled under a nitrogen atmosphere to -40°C. and treated dropwise with 80 mg of $Br_2$(0.5 mmole) in 0.5 ml of $CCl_4$. After stirring for ca. 10 minutes, a solution of 349 mg (1.0 mmole) of 3α-(1(R*)-o-nitrobenzylcarbonyldioxy-2-methylpropyl)-4β-vinyl-2-azetidinone in ~3 ml of methanol free $CHCl_3$ (incompletely soluble) is added and rinsed in with additional dry THF. The color of the solution lightens considerably, and it is allowed to stir 2 hours without maintaining the cooling bath. At this point the mix has lightened further and the temperature is ca. -10°C. It is poured into ~50 ml ice/water, shaken vigorously and the phases separated. The aqueous phase is then extracted three more times with $CHCl_3$ the combined extracts washed once with water, once with saturated NaCl solution, dried with $MgSO_4$, and evaporated under a nitrogen stream to give 0.71 g

of gum. Examination by tlc (silica; 20%EtOAc/ $CH_2Cl_2$) shows some unreacted starting materials plus two slower major product spots. The material is separated using the Waters Autoprep 500 with one 500 ml pack and eluting with 20% EtOAc in $CH_2Cl_2$. The starting materials emerge with the 3rd and 4th column volumes, the faster bromo-product (105 mg) emerges in the 5th and 6th column volumes and the slower bromo-product (282 mg) emerges in the 8th and 9th column volumes.

The faster bromo isomer has a 300 MHz PMR spectrum ($CDCl_3$) showing 8.25($H_{\underline{m}}$,d,$\underline{J}_{\underline{o},\underline{m}}$=8Hz), 8.18($H_{3'}$, d, $\underline{J}_{3',4'}$=8Hz), 7.8-7.5($H_{4'-6'}$,m), 7.55($H_2$,d,$\underline{J}_{\underline{o},\underline{m}}$=8Hz), 6.26($H_4$,s), 5.70&5.62($H_\alpha$, pair of doublets, $\underline{J}_{\alpha\alpha}$=14 Hz), 5.23($H_{\alpha'}$,s), 5.06($H_8$,dd,$\underline{J}_{8,9}$=4Hz; $\underline{J}_{6,8}$=8Hz), 4.17($H_1$, ddd, $\underline{J}_{1,2x}$=7Hz; $\underline{J}_{1,2y}$=7Hz; $\underline{J}_{1,5}$=7Hz), 3.54-3.30($H_{2''}$,m), 3.10-2.94($H_2$,m), 2.76($H_{1''}$,t, $J_{1'',2''}$=6Hz), 2.32-2.14($H_9$, m), 1.0 and 0.98($H_{10}$, pair of doublets, $\underline{J}_{9,10}$=6Hz), $\delta$.

The slower bromo isomer has a 300 MHz PMR spectrum ($CDCl_3$) showing 8.25($H_m$,d,$J_{o,m}$ = 8Hz), 8.17($H_{3'}$,d,$J_{3',4'}$=8Hz), 7.8-7.5($H_{4'-6'}$,m), 7.55($H_o$,d,$J_{o,m}$=8Hz) 6.58($H_4$,s), 5.57($H_\alpha$,s), 5.19($H_{\alpha'}$, s), 5.05($H_8$,dd,$J_{8,9}$=5Hz; $J_{6,8}$=8.5Hz), 4.12($H_1$,ddd,$J_{1,2}$ =5Hz; $J_{1,2y}$=7.5Hz; $J_{1,5}$=7Hz), 3.54-3.34($J_{2"}$,m), 3.18($H_2$, dd,$J_{2,2y}$=14Hz; $J_{1,2}$ =5Hz), 3.04($H_{2y}$,dd,$J_{1,2y}$=7.5Hz; $J_{2,2y}$=14Hz). 2.74($H_{1"}$,t,$J_{1",2"}$=6Hz) 2.32-2.18($H_9$,m), 1.4 & 0.98($H_{10}$, bs) $\delta$.

In most cases the reaction is more complete than in this example. The ratios of fast to slow isomer appear variable and may even change on handling or standing; however, in the following reaction, the yields and ratios of cis to trans olefins obtained are substantially the same, regardless of which isomer is used.

Step F

Preparation of 3α-(1(R*)-o-nitrobenzylcarbonyldioxy-2-methylpropyl)-4β-[2-(2-p-nitrobenzyloxycarbonylaminoethylthio)vinyl]-2-azetidinone

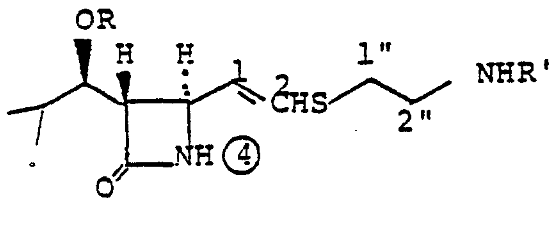

The fast bromo isomer from Step D, 105 mg. (0.154 mmole) in 1 ml. KOH dried pyridine was treated with 49 mg. AgF (0.39 mmole) and stirred for 2 hours under $N_2$, the mix poured into 10 ml. ice/water plus 1-2 ml. EtOAc, shaken vigorously and centrifuged. The organic phase is removed and the aqueous phase extracted three more times with 1-2 ml. EtOAc. The combined extracts are washed twice with water, once with saturated NaCl solution, dried with $MgSO_4$ and evaporated under a nitrogen stream. The residue is flushed twice by evaporating from $CHCl_3$ solution under high vacuum to give 58 mg. dark gum. Preparative tlc on silica with 20% EtOAc/$CH_2Cl_2$ gives 27 mg. of trans olefin and 14 mg. of cis olefin. (In larger runs, using somewhat lower ratios of AgF to substrate, the ratio of trans to cis appears somewhat higher.) The pure trans isomer has a 300 MHz PMR spectrum ($CDCl_3$) which exhibits peaks at 8.22 ($H_m$, d, $J_{o,m}$ = 8Hz), 8.19 ($H_3$, d, $J_{3',4'}$ = 8Hz), 7.80-7.77 ($H_{3',5'}$,m) 7.56 ($J_{o,m}$ = 8Hz), 6.31 ($H_2$, d,$J_{1,2}$=14Hz), 5.94 ($H_4$, s), 5.71 ($H_1$, dd, $J_{1,2}$ = 14 Hz,

$\underline{J}_{1,5}$ = 8Hz), 5.58 and 5.66 ($H_\alpha$, two doublets, $J_{\alpha\alpha}$ = 14), 5.23 ($H_\alpha$', s), 5.10 ($H_8$, dd, $\underline{J}_{6,8}$=6Hz; $\underline{J}_{8,9}$ = 5Hz), 4.30 ($H_5$, dd, $\underline{J}_{1,5}$ = 8Hz; $\underline{J}_{5,6}$ = 2Hz), 3.47 ($H_{2"}$, dd, $\underline{J}_{1",2"}$ = 6Hz; $\underline{J}_{2",NH}$ = 14Hz), 3.28 ($H_6$, dd, $\underline{J}_{5,6}$ = 2Hz; $\underline{J}_{6,8}$ = 6Hz), 2.90 ($H_{1"}$, t, $\underline{J}_{1",2"}$ = 6Hz), 2.21 - 2.03 ($H_9$,m), 0.97 and 0.95 ($H_{10}$, two doublets, $\underline{J}_{9,10}$ = 6Hz)$\delta$. The cis isomer has a 300 MHz PMR spectrum ($CDCl_3$) with peaks at 8.24 ($H_m$, d, $\underline{J}_{o,m}$ = 8Hz), 8.17 ($H_{3'}$, d, $\underline{J}_{3',4'}$ = 8Hz), 7.76 - 7.49 ($H_{4'-6'}$,m), 7.53 ($H_o$, d,$J_{o,m}$ = 8Hz), 6.19 ($H_2$, d, $\underline{J}_{1,2}$ = 10Hz), 5.95 ($H_4$, s), 5.74 - 5.54 ($H_1$ and $H_\alpha$, complex), 5.20 ($H_\alpha$, s), 5.11 ($H_8$, dd, $\underline{J}_{6,8}$ = 6Hz; $\underline{J}_{8,9}$=6Hz), 4.57 ($H_5$,dd, $\underline{J}_{1,5}$= 9Hz, $\underline{J}_{5,6}$ = 2Hz), 3.49 - 3.37 ($H_{2"}$,m), 3.33 ($H_6$, dd, $\underline{J}_{5,6}$ = 2Hz; $\underline{J}_{6,8}$ = 6Hz), 2.89 - 2.81 ($H_{1"}$,m), 2.3 - 2.1 ($H_9$, m), ~1.0 ($H_{10}$,bs)$\delta$.

Step F

Preparation of 1-bis carbo-p-nitrobenzyloxy-hydroxymethyl-3α(1-(R*)-o-nitrobenzylcarbonyldioxy-2-methylpropyl)-4β-[2-(2-p-nitrobenzyloxycarbonylaminoethylthio)vinyl]-2-azetidinone

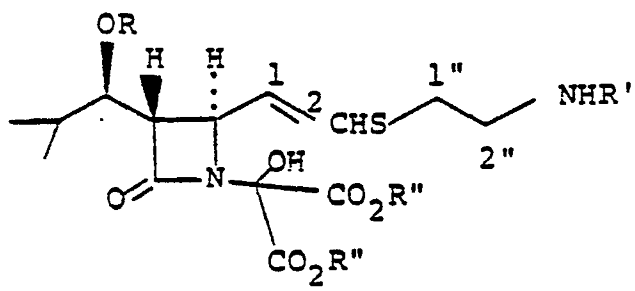

R" = PNB= 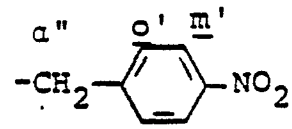

The procedure used in Example 31a, Step F, is followed except that 588 mg. (1.31 mmole) of ketomalonate, 25 ml. toluene, 605 mg. (1.0 mmoles) of 3α-(1-(R*)-o-nitrobenzylcarbonyldioxy-2-methylpropyl)-4β-[2-(2-p-nitrobenzyloxycarbonylaminoethylthio)vinyl]-2-azetidinone in 4 ml. THF, distillation of 18 ml. toluene and 1.5 hours of refluxing are employed. The analytical tlc's are developed three times in 1:2 acetone/hexane for optimum resolution. Chromatography on 30 g. silica gel packed in 1:2 acetone/hexane and collecting 25 ml. fractions after a 150 ml. forerun fraction gives 265 mg. ketomalonic acid (fractions 4-8), 177 mg. starting azetidinone (fractions 9-11), and 706 mg. of product (fractions 12-20). The

60 MHz PMR ($CDCl_3$) shows peaks at 8.24 - 7.95 ($H_{3',\underline{m},m',}$m), 7.68 - 7.38 ($H_{\underline{o},\underline{o}',4'-6',}$m), 6.26 ($H_1$, d, $\underline{J}_{1,2}$ = 15 Hz), 5.65 ($H_2$, dd, $\underline{J}_{1,2}$ = 15 Hz; $\underline{J}_{1,5}$ =8Hz), 5.48 ($H_{\alpha,}$s), 5.30 ($H_{\alpha'',}$s), 5.12 ($H_{\alpha'}$, s), ~5.12 - 4.9 ($H_8$, partially buried under α'), 4.7 ($H_5$, dd, $\underline{J}_{1,5}$ = 8Hz, $\underline{J}_{5,6}$ = 2Hz), 3.58 - 3.12 ($H_{2'',6,}$m), 3.0 - 2.55 ($H_{1'',}$ m), 2.3 - 1.7 ($H_9$, m), 0.95 and 0.83 ($H_{10,}$ two bs) δ.

Step G

Preparation of 1-biscarbo-p-nitrobenzyloxymethyl-3α-(1-(R*)-o-nitrobenzylcarbonyldioxy-2-methylpropyl)-4β-[2-(2-p-nitrobenzyloxycarbonylaminoethylthio)vinyl]-2-azetidinone

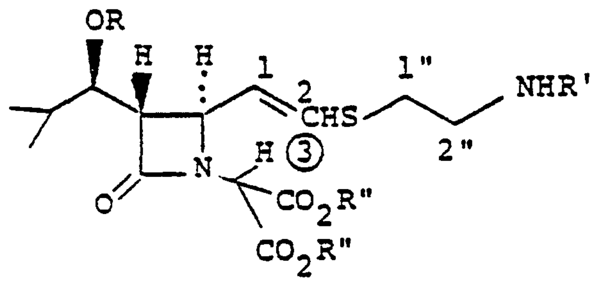

If 706 mg. (0.71 mmole) of 1-biscarbo-p-nitrobenzyloxyhydroxymethyl-3α-(1-(R*)-o-nitrobenzylcarbonyldioxy-2-methylpropyl)-4β-[2-(2-p-nitrobenzyloxycarbonylaminoethylthio)vinyl]-2-azetidinone in 7 ml. THF containing 100λ pyridine (98 mg.; 1.24 mmole) is treated substantially as in Example 32, Step G, with 86λ $SOCl_2$ (144 mg.; 1.21 mmole) are subsequently with 353λ $(n\text{-but})_3P$ (1.42 mmole) in 7.2 ml. sieve dried DMF with 167 mg. $K_2HPO_4$ (0.96 mmole) and worked up as above, the crude title compound is obtained. Chromatography on 50 g. silica gel packed in $CH_2Cl_2$ and developed with 5% $EtOAc/CH_2Cl_2$, collecting a liter fraction after discarding a 125 ml. forerun; elution with 10% $EtOAc/CH_2Cl_2$, collecting 25 ml. fractions, gives product in fractions 10-30. Further purification on preparative tlc with 10% $EtOAc/CH_2Cl_2$ gives a 40-50% yield of product which displays a 60 MHz PMR($CDCl_3$) with peaks at 8.2 ($H_{3,m,m'}$, bd, $J$ = 7-8Hz), 7.75 - 7.35 ($H_{4'-6',o,o''}$, m), 6.3 ($H_2$, d, $J_{1,2}$ = 15Hz),

5.6($H_1$, dd, $\underline{J}_{1,2}$ = 15 Hz; $\underline{J}_{1,5}$ = 9 Hz), 5.58 ($H_a$, s), 5.44-5.18 ($H_{a,a'',3}$, complex), 5.35 - 5.0 ($H_8$, m), 4.60 ($H_5$, dd, $\underline{J}_{5,6}$ = 2Hz; $\underline{J}_{1,3}$ = 9 Hz), 3.6-3.2 ($H_{2'',6'}$, m), 2.65-3.05 ($H_{1''}$, m), 2.4 - 1.8 ($H_9$, m), 1.02 and 0.95 ($H_{10}$, two singlets )$\delta$ .

Step H

Preparation of 2,2-biscarbo-p-nitrobenzyloxy-3-(2-p-nitrobenzyloxycarbonylaminoethylthio)-4-bromo-6-exo-(1-(R*)-o-nitrobenzylcarbonyldioxy-2-methylpropyl)—1-azabicyclo[3.2.0]heptan-7-one

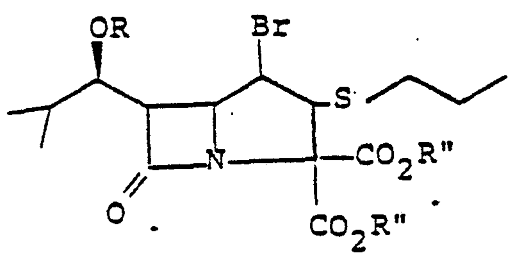

[All numbering and symbolism is the same as before.]

Following the procedure of Example 31a, Step H, and adding 0.20 ml of 132 mg Br/1.3 ml $CCl_4$ (0.125 mmoles) to 98 mg (0.10 mmoles) of 1-biscarbo-p-nitrobenzyloxymethyl-3α-(1(R*)-o-nitrobenzylcarbonyldioxy-2-methylpropyl)-4β-[2-(2-p-nitrobenzyloxycarbonylaminoethylthio)-vinyl]-2-azetidinone in 2 ml THF, and then using 40λ $Et_3N$-(0.54 mmoles) and 1.5 ml DMF as in Example 32, Step H, there is obtained, upon work-up, 111 mg crude product. Preparative tlc using 77% EtOAc/$CH_2Cl_2$ affords the product, 48 mg, the slowest of the 3-4 major bands observed. The 60 MHz PMR ($CDCl_3$) shows peaks at 8.15 - 8.0 and 7.7 - 7.4 ($H_{aromatics}$; clusters of multiplets), 5.60($H_c$, s), 5.34($H_{c''}$,s), 5.20($H_{c'}$, s), 4.8 - 5.1 ($H_8$, complex), 4.45 - 4.15 ($H_5$, m), 4.70($H_1$, dd,$J_{1,2}$=6; $J_{1,5}$=6), 3.7 - 3.25 ($H_{2,2''}$,m), 2.65 - 3.00($H_{2''}$, m), 2.35 - 1.6 ($H_9$,bm), 0.98 and 1.1 ($H_{10}$, two singlets) δ.

Step I

Preparation of 2,2-biscarbo-p-nitrobenzyloxy-3-(2-p-nitrobenzyloxycarbonylaminoethylthio)-6-exo-(1-(R*)-o-nitrobenzylcarbonyldioxy-2-methylpropyl)-1-azabicyclo[3.2.0]hept-3-en-7-one.

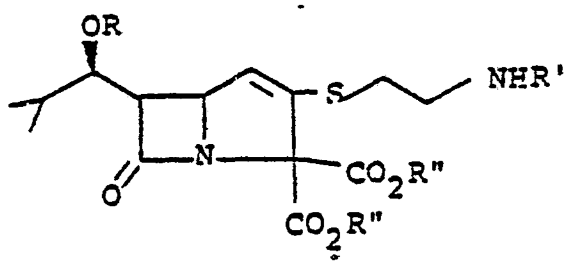

Following the procedure of Example 32, Step I, using 48 mg (0.046 mmole) of 2-biscarbo-p-nitrobenzyloxy-3-(2-p-nitrobenzyloxycarbonylaminoethylthio)-4-bromo-6-exo-(1-(R*)-o-nitrobenzylcarbonyldioxy-2-methylpropyl)-1-azabicyclo[3.2.0]heptan-7-one in 10 ml pyridine with 15 mg AgF (0.12 mmole) but working up by pouring into $H_2O$/NaCl and extracting only with EtOAc, crude title compound is obtained. Preparative tlc with 8% EtOAc/$CH_2Cl_2$ affords 38 mg clean product with a 60 MHzPMR($CDCl_3$) spectrum showing peaks at 8.25-8.02($H_{3'}$, m,m', multiplet), 7.7-7.4($H_{4'-6'}$, o,o', m), 6.23($H_1$, d, $J_{1,5}$=1Hz) 5.80 and 5.45($H_\alpha$, two doublets, $J_{\alpha\alpha}$=14Hz), 5.35($H_{\alpha''}$, s), 5.20($H_{\alpha'}$, s), 5.3-5.0($H_8$, m, partially buried under $H_{\alpha'}$), 4.8($H_5$, bm), 3.6-3.25($H_{2'',6}$, m) 3.2-2.9($H_{1''}$, m), 2.4-1.9($H_9$, bm), 1.05 and 0.95-($H_{10}$, two singlets) $\delta$.

Step J

Preparation of 2-carbo-p-nitrobenzyloxy-3-(2-p-nitrobenzyloxycarbonylaminoethylthio)-6-exo-(1(R*)-o-nitrobenzylcarbonyldioxy-2-methylpropyl)-1-azabicyclo[3.2.0]hept-3-en-7-one

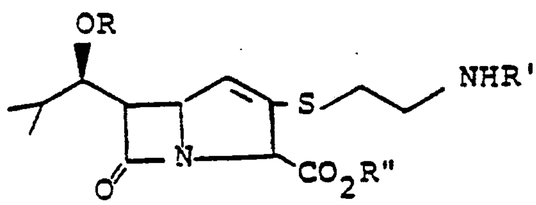

The procedure of Example 31a, Step J, is followed except: 491 mg (0.51 mmoles) of 2-bis carbo-p-nitrobenzyloxy-3-(2-p-nitrobenzyloxycarbonylaminoethylthio)-6-exo-(1-(R*)-o-nitrobenzylcarbonyldioxy-2-methylpropyl)-1-azabicyclo[3.2.0]-hept-3-en-7-one, 6 ml collidine, 87 mg (0.65 mmole) LiI and the crude product is purified by tlc with 40% acetone/hexane, the title compound is obtained which gives a 300 MHz PMR spectrum ($CDCl_3$) with peaks at 8.15 ($H_{3',m,m'}$, d, $J$=8Hz), 7.66 - 7.22 ($H_{4'-6',2,2'}$, m), 5.86($H_1$, s), 5.68 and 5.55($H_{\alpha}$, two doublets, $J_{\alpha\alpha}$=14Hz), 5.13($H_{\alpha''}$, s), 5.05($H_{\alpha',3}$, bs), 4.96($H_8$, dd, $J_{8,9}$=4Hz; $J_{6,8}$=7Hz), 4.54($H_5$, 6s), 3.3-3.6($H_{2''}$, m), 3.38($H_6$, dd, $J_{5,6}$=2Hz; $J_{6,8}$=7Hz), 2.90-3.11($H_{1''}$, m), 1.86-2.04($H_9$, bm), ~1.0($H_{10}$, off scale) $\delta$ .

Step K

Preparation of 2-carbo-p-nitrobenzyloxy-3-(2-p-nitrobenzyloxycarbonylaminoethylthio)-6-exo-(1-(R*)-hydroxy-2-methylpropyl)-1-azabicyclo-[3,2,0]hept-3-en-7one

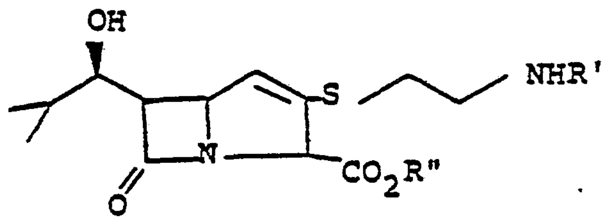

A solution of ca. 130 gm of 2-carbo-p-nitrobenzyloxy-3-(2-p-nitrobenzyloxycarbonylaminoethylthio)-6-exo-(1-(R*)-o-nitrobenzylcarbonyldioxy-2-methylpropyl)-1-azabicyclo[3,2,0]hept-3-en-7-one in 10 ml of $CHCl_3$ is placed in a quartz vessel (~1x1x10cm) equipped with a 14/20 standard taper mouth and jacketed by a larger quartz messel through which cold water may be run. A fine stream of nitrogen is blown through the solution for 10 minutes and the system is then closed with a nitrogen line fitted with a pressure-releasing bubbler. The water flow through the jacket is turned on and the vessel placed in a Rayonet Photolysis apparatus equipped with 3500Å sources. The photolyzer is turned on and the sample is irradiated for 3 hours. Upon evaporation of the solvent and preparative tlc, with an EtOAc/$CH_2Cl_2$Stytem, a 5-10% yield of the title compound is obtained along with a 75-80% recovery of starting material which is resubjected to the photolysis. Overall, a 30-40% conversion

is effected. The product has a 300 MHz PMR spectrum ($CDCl_3$) with peaks at 8.23 ($H_{\underline{m},m'}$, d, $\underline{J}_{\underline{o},\underline{m}}$ = 8Hz), 7.53 and 7.51($H_{\underline{o}}$ and $H_{\underline{o}'}$, doublets, J=8Hz), 6.01($H_1$,s), 5.20($H_{\alpha''}$,s), 5.17($H_{\alpha',3}$,bs), 4.67($H_5$,bs), 3.92($H_8$, dd, $\underline{J}_{6,8}$=6Hz; $\underline{J}_{8,9}$=6Hz), 3.53-3.46 ($H_{2''}$, bm), 3.25($H_6$, dd, $\underline{J}_{5,6}$=3Hz; $\underline{J}_{6,8}$=6Hz), 3.12-2.87($H_{1''}$, bm), 1.94 - 1.78 ($H_9$, bm), ~0.97 ($H_{10}$, bs) $\delta$.

Step L

Preparation of 2-carbo-p-nitrobenzyloxy-3-(2-p-nitrobenzyloxycarbonylaminoethylthio)-6-exo-(1-(R*)-hydroxy-2-methylpropyl)-1-azabicyclo[3.2.0]-hept-2-en -7-one

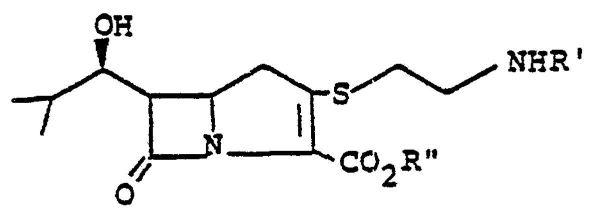

A solution of 9 mg(0.014 mmoles) of 2-carbo-p-nitrobenzyloxy-3-(2-p-nitrobenzyloxycarbonylaminoethylthio)-6-exo-(1-(R*)-hydroxy-2-methyl-propyl)-1-azabicyclo-[3.2.0]hept-3-en—7-one in 0.1 ml DMSO is treated with 0.2-0.3 λ(0.002 mmole) DBU. After standing for 1-2 hours, the solution is diluted with 1 ml of water and extracted several times with EtOAc. The extracts are combined , washed with water, 1M $KH_2PO_4$, dried with $MgSO_4$ and concentrated. The concentrate is processed by preparative tlc using 1:1 $CH_2Cl_2$/EtOAc to give the starting compound and the title compound. The former may be recycled through

Step M

Preparation of (±)9,9-dimethylthienamycin

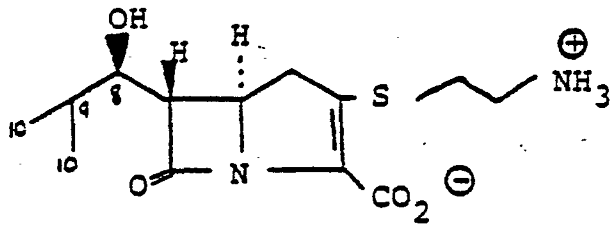

A 5-6 mg sample of 2-carbo-p-nitrobenzyloxy-3-(2-p-nitrobenzyloxycarbonylaminoethylthio)-6-exo-(1-(R*)-hydroxy-2-methylpropyl)-1-azabicyclo-[3,2,0]hept-2-en-7-one in a 13x100 mm culture tube is deblocked under precisely the conditions given in Example 12 Step K . The aqueous extract, however, is not applied to an XAD-2 column; instead it is injected in portions onto a Waters Associates

Microbondapak $C_{18}$ column ( 3ml void volume) and eluted with 5% THF in deionized water. The desired material appears as a major peak (254 photocell) at approximately 6 minutes with a flow rate of 0.7 ml/minute. Collected peaks from multiple injections are combined and carefully concentrated on a rotary evaporator equipped with a Dry Ice/acetone charged cold finger condenser and attached to a high vacuum oil pump to 0.5-1.0 ml. The concentrate is shell frozen and placed in a shelf lyophilizer with T≤ -10°C overnight. The web-like residue exhibits a 300 mHz PMR Spectrum ($D_2O$) with peaks at 4.27($H_5$,bt,$J_{1,5}$=8-10Hz) 3.83($H_8$,dd,

$J_{8,9}$≅5Hz; $J_{6,8}$≅5Hz), 3.62($H_6$,dd,$J_{5,6}$=3Hz; $J_{6,8}$=4Hz),

3.31-2.95($H_{1'',2''}$,bm) 1.87-1.71($H_9$,bm), 0.94 & 0.88

the isomerization process to increase the overall conversion to the latter. The product gives a 300 MHz PMR spectrum ($CDCl_3$) with peaks at 8.24 ($H_{m,m'}$, d, J=8Hz), 7.68 ($H_{o'}$, d, $J_{o'm'}$=8Hz), 7.52 ($H_o$, d, $J_{o,m}$=8Hz), 5.53 and 5.25 ($H_{\alpha''}$, pair of doublets $J_{\alpha\alpha}$=14Hz), 5.20 ($H_{\alpha'}$, s), 4.38-4.28-($H_5$m), 3.94-3.88 ($H_8$, bm), 3.51-3.42 ($H_{2''}$, m), 3.41-3.32 ($H_6$, bdd), 3.20-2.94 ($H_{1'',1}$, m), 1.95-1.85 ($H_9$, bm), ~ 0.97 ($H_{10}$, not resolved ) $\delta$. ($H_{10}$, two doublets, $J_{9,10}$=6Hz) $\delta$ . There is no significant absorption below the HDO peak at 4.84 $\delta$ . The PMR sample has a U.V. spectrum with $\lambda_{max.}$ 299 mu, quenchable with hydroxylamine.

EXAMPLE 31 a

Preparation of (±)-9-benzylthienamycin

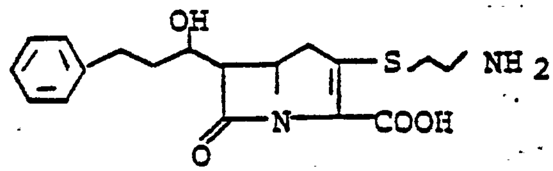

Step A

Preparation of 1-t-butyldimethylsilyl-3α-[(1(R*)-hydroxy-3-phenyl)propyl]-4β-vinyl-2-azetidinone- and 1-t-butyldimethylsilyl-3α-[(1(S*)-hydroxy-3-phenyl)propyl]-4β-vinyl-2-azetidinone(1 and 2 respectively)

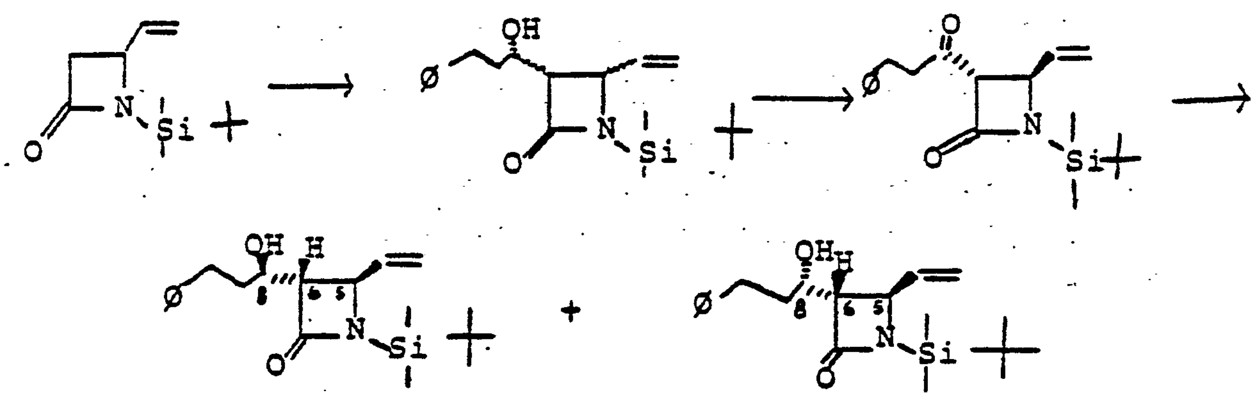

1 2

Generation of the anion of 17.8 g of 1-t-butyldimethylsilyl-4-vinyl-2-azetidinone (mw=211; 84.4 mmole), as in Example 31, Step A, is followed by condensation with 12.6 g of hydrocinnamaldehyde (mw=134; 94 mmole) rather than with isobutyraldehyde. Upon work-up, the crude aldol product is quickly chromatographed on silica gel (100 g). Elution with 5% EA/$CH_2Cl_2$ provides 30 g of 1-t-butyldimethylsilyl-3-[(1-hydroxy-3-phenyl)propyl]-4-vinyl-2-azetidinone as a mixture of diastereomers.

NMR (300 MHz, d6-acetone)δ 0.14-0.22 (series of s, $Si(CH_3)_2$'s), 0.94 (s's, $-Si-C(CH_3)_3$'s), 1.76-2.10 (m, $ØCH_2CH_2-$), 2.59-2.89 (m, $ØCH_2CH_2-$), 3.00-3.06 (2dd, $H_6$ of (8R* & 8S*)-trans-β-lactams) 3.46 (overlapping dd appearing as a t, $J_{5,6}=J_{6,8}=5Hz$, $H_6$ of cis-β-lactam), 3.77-3.97 (m's, $H_8$ 's), 4.06-4.23 (m's, $H_5$'s), 5.13-5.38 & 5.92-6.29 (m's, $-CH=CH_2$), 7.14-7.30(m, Ø).

A preferred procedure for isolation of the title compounds in quantity involves oxidation of the mixture of diastereomers to the all <u>trans</u>-ketone followed by reducing with $NaBH_4$ to a mixture from which the desired (1<u>R</u>* -hydroxy)diastereomer is more readily isolated.

Using essentially the same oxidation procedure as in Example 31, Step A, 30g of diastereomeric 1-<u>t</u>-butyldimethylsilyl-3-[(1-hydroxy-3-phenyl)propyl]-4-vinyl-2-azetidinone is oxidized to the corresponding <u>trans</u>-ketone (30 g crude material). Recrystallization from a small volume of $Et_2O$ gives 10.9 of crystalline product in two crops. The mother liquor is chromatographed on silica-gel (100 g; eluting with $CH_2Cl_2$). The fractions containing product are combined and washed with cold petroleum ether to give 2.73g additional crystalline ketone. A second recrystallization from $Et_2O$ gives an analytical sample:

mp= 79.5-82.5°C; IR($CHCl_3$)μ 5.76, 5.86; MS m/e 343 (M+), 328, 286;
NMR (300MHz, $CDCl_3$) δ 0.16 & 0.24 (2s, >Si$(CH_3)_2$), 0.93 (s, -Si-$C(CH_3)_3$), 2.74-3.14 (m, Ø$CH_2CH_2$), 3.97 (d, $J_{5,6}$=3Hz, $H_6$), 4.44 (dd, J=3Hz & 8Hz, H5), 5.21-5.39 & 5.77-5.89 (m's, CH=$CH_2$), 7.19-7.31(m,Ø).
Anal ($C_{20}H_{29}NO_2Si$)C, H, N.

With stirring, 3.4g of crystalline ketone (mw=343, 9.9 mmole) dissolved in 70 ml i-propanol is treated with 471mg $NaBH_4$ (mw=38; 12.4 mmole). After stirring under $N_2$ at room temperature for 0.5 to 2 hours the reaction mixture is poured (cautiously) into a mixture of 60 ml. 1M$KH_2PO_4$-ice water-$CH_2Cl_2$. Continuing the work-up as in Example 31, Step A, 3.5g crude product is isolated. Chromatography on silica-gel (100g, eluting with 5% EA/$CH_2Cl_2$) followed by chromatography on a Waters Associates Prep LC/System 500 instrument (10% acetone/hexane recycle mode) gives 1.0g of 1-t-butyldimethylsilyl-3α-[(1(S*)-hydroxy-3-phenyl)propyl]-4β-vinyl-2-azetidinone and 1.1g of the desired 1-t-butyldimethylsilyl-3α-[(1(R*)-hydroxy-3-phenyl)propyl]-4β-vinyl-2-azetidinone diastereomer as a crystalline solid. Recrystallization from petroleum ether gives an analytical sample: mp=63-65°C; IR($CHCl_3$) μ 5.79 br; MS m/e 345 (M+), 288, 105, 91;

NMR(300MHz,$d_6$-acetone)$\delta$0.14 & 0.21 (2s, $\rangle$Si-$(CH_3)_2$), 0.93 (s,-$\rangle$Si-$C(CH_3)_3$), 1.73-1.94(m,$\emptyset CH_2$-$C\underline{H}_2$), 2.62-2.88(m,$\emptyset CH_2C\underline{H}_2$), 3.02 (dd, $J_{5,6}$=3Hz, $J_{6,8}$=5Hz,H6), 3.92-3.98 (m,$H_8$), 4.21 (dd,J=3Hz & 9Hz, $H_5$), 5.14-5.39 & 5.96-6.08 (m's, $C\underline{H}$=$C\underline{H}_2$), 7.14-7.30 (m,$\emptyset$).

Anal ($C_{20}H_{31}NO_2Si$) C,H,N.

Data for 1(S*)-hydroxy diastereomer: IR($CHCl_3$)$\mu$ 5.80 br; MS m/e 345(M+), 288, 105, 91;

NMR(300MHz, $d_6$-acetone) $\delta$ 0.13 & 0.20 (2s, -Si-$(CH_3)_2$), 0.93(s, -Si-$C(CH_3)_3$), 1.80-2.10 (m,$\emptyset$-$CH_2C\underline{H}_2$), 2.62-2.88 (m,$\emptyset C\underline{H}_2CH_2$), 3.04 (dd, $J_{5,6}$= 3 Hz, $J_{6,8}$=4.5Hz,$H_6$), 3.83-3.90 (m,$H_8$), 4.08 (dd, J=3Hz and 6Hz, $H_5$), 5.13-5.35 & 5.92-6.06 (m's, $C\underline{H}$=$C\underline{H}_2$), 7.14-7.30 (m,$\emptyset$).

**Step B**

Preparation of 1-t-butyldimethylsilyl-3α-[(1(R*)-o-nitrobenzylcarbonyldioxy-3-phenyl)propyl]-4β-vinyl-2-azetidinone (3)

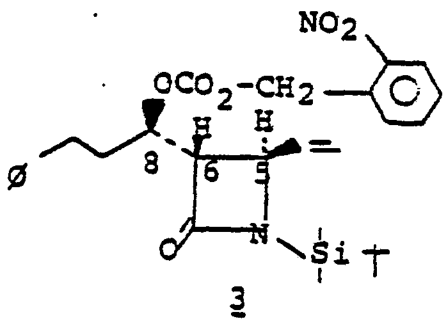

3

As in Example 31, Step B, 5.7 g of 1-t-butyldimethylsilyl-3α-[(1(R*)-hydroxy-3-phenyl)propyl]-4β-vinyl-2-azetidinone (mw=345; 16.5 mmole) and 4.24 g 4-dimethylaminopyridine (mw=122, 34.8 mmole) in 100 ml $CH_2Cl_2$ is treated with 7.56 g o-nitrobenzyloxycarbonylchloride (mw=217; 34.8 mmole) in 13 ml $CH_2Cl_2$. The cooling bath is removed, and stirring is continued under $N_2$ for 4 hours at room temperature. The reaction mixture is poured into a mixture of 18 ml 1M $K_2HPO_4$-water. Continuing the work-up as in Example 31, Step B, 11.7 g crude material is obtained. Chromatography on silica gel (350 g, eluting with 0→2% EA/$CH_2Cl_2$) gives 5.76 g of the title compound. Chromatography on silica gel (100 g, 0→2% EA/$CH_2Cl_2$) of contaminated column fractions gives 1.36 g. additional product (82% total yield). IR ($CHCl_3$): 5.75br; NMR(300MHz,$CDCl_3$) δ 0.15 & 0.24(2s Si-$(CH_3)_2$), 0.93(s, -Si-$C(CH_3)_3$), 2.06-2.14(m,Ø$CH_2CH_2$), 2.60-2.78(m,Ø$CH_2CH_2$), 3.22 (dd, $J_{5,6}$=3Hz,$J_{6,8}$=7Hz, $H_6$), 4.12(dd,J=3Hz & J=9Hz, $H_5$), 5.17-5.32 & 5.81-5.93 (m 's, $H_8$ & $CH=CH_2$), 5.60(mid-point of 2d, J=14Hz, nonequivalent-$OCO_2CH_2$-arom.) 7.16-8.2(m,aromatics).

Step C

Preparation of 3α-[(1(R*)-o-nitrobenzylcarbonyldioxy-3-phenyl)propyl]-4β-vinyl-2-azetidinone (4)

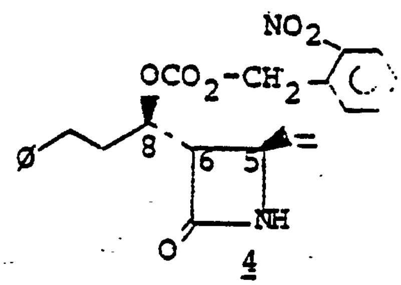

4

Using essentially the same procedure as in Examples 31, Step C, 150 mg of 1-t-butyldimethylsilyl-3α-[(1(R)-o-nitrobenzylcarbonyldioxy-3-phenyl)propyl]-4β-vinyl-2-azetidinone is converted to 116 mg crude product. Preparative thin layer chromatography on silica gel (5% EA/$CH_2Cl_2$) provides 104 mg of the title compound (87%).

IR($CHCl_3$)μ 5.69 br.

NMR(300MHz,$CDCl_3$)δ 2.06-2.23(m,Ø$CH_2$$C\underline{H}_2$), 2.62-2.81 (m,Ø$CH_2$$C\underline{H}_2$), 3.20(dd, $J_{5,6}$=2Hz, $J_{6,8}$=7Hz, $H_6$), 4.23 (dd, J=2Hz & 7Hz, $H_5$), 5.18-5.34 & 5.85-5.97(m's,$H_8$ & $C\underline{H}$=$C\underline{H}_2$). 5.59(s,-$OCO_2$$C\underline{H}_2$-arom.), 7.17-8.19 (m, aromatics).

Larger runs can be purified by chromatography on silica gel eluting with 0 ⟶ 10% EA/$CH_2Cl_2$ or 0 ⟶ 4% EA/$CHCl_3$.

Step D

Preparation of 4β-[[1-bromo-2-(2-p-nitrobenzyloxycarbonylamino)ethylthio]ethyl]-3α-[(1(R*)-o-nitrobenzylcarbonyldioxy-3-phenyl)propyl]-2-azetidinone (5)

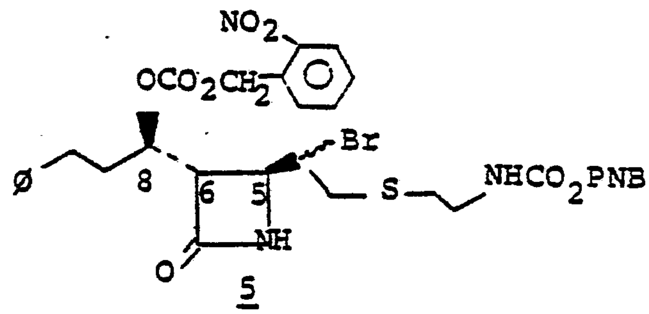

5

Following the procedure of Example 31, Step D, except that the reaction is allowed to stir for 1.5 hours during which time the temperature is not allowed to rise above + 10°C, 100 mg of 3α-[(1(R*)-o-nitrobenzylcarbonyldioxy-3-phenyl)propyl]-4β-vinyl-2-azetidinone is converted to 171 mg of crude product. Chromatography on silica gel (~10 g, eluting with 0 ⟶ 20% EA/$CH_2Cl_2$) provides 139 mg of the more polar product as a mixture of diastereomeric bromides (77%).

IR($CHCl_3$) 5.66, 5.74 sh, 5.82

NMR(300MHz, $CDCl_3$)δ 2.06-2.34(m,Ø$CH_2$$\underline{CH_2}$), 2.64-2.85 (m,S$\underline{CH_2}$$CH_2$N and Ø$\underline{CH_2}$$CH_2$), 2.95-3.09 & 3.16-3.23 (m'S,CHBr$\underline{CH_2}$S),3.32-3.50(m,S$CH_2$$\underline{CH_2}$N and $H_6$'s), 3.95 (dd,$J_{5,6}$=2Hz,$J_{CH_5-CHBr}$=7Hz, $H_5$ of minor diastereomer), 4.01(dd,$J_{5,6}$=2Hz, $J_{CH_5-CHBr}$=7.5Hz, $H_5$ of major diastereomer), 4.04-4.16(m,-CHBr-'s), 5.16(Midpt. of m,$H_8$), 5.18(s,$NHCO_2$$\underline{CH_2}$arom.), 5.52-5.66(m,-$OCO_2$$\underline{CH_2}$arom.), 6.30, 6.46 & 6.65(3br s, NH'S), 7.16-8.25(m,aromatics).

Step E

Preparation of 3α-[(1(R*)-o-nitrobenzylcarbonyldioxy-3-phenyl)propyl]-4β-[2-(2-p-nitrobenzyloxycarbonylamino)ethylthio]vinyl]-2-azetidinone (6)

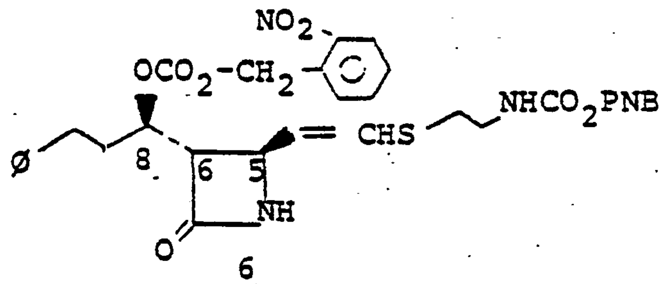

6

Treatment with 1.01 g. AgF(mw=127; 7.95 mmole) of 3.1 g. crude 4β-[[1-bromo-2-(2-p-nitrobenzyloxycarbonylamino)ethylthio]ethyl]-3α-[(1(R*)-o-nitrobenzylcarbonyldioxy-3-phenyl)propyl]-2-azetidinone (mw=744; 4.2 mmole) in 34 ml. pyridine at room temperature in the dark under $N_2$ for 1 hr. is followed by concentration of the entire reaction mixture under high vacuum and with a water bath at 25-30°C. to a brown-black residue. After chasing a few times with $CHCl_3$, the residue is slurried in $CH_2Cl_2$ and run through a short column of silica gel (eluting with 2% $MeOH/CH_2Cl_2$) thus removing most of the silver salts. The fractions containing product are combined. Two additional runs carried out as above give a total of 3.26 g. product (from a total of 9.84 mmole starting material) still containing less polar impurities. Chromatography on silica gel (200 g., 1% $MeOH/CH_2Cl_2$) provides 0.63 g. of the desired product. Preparative thin layer chromatography on silica gel of 2.3 g. from column fractions which still contained less polar impurities (2.5% $MeOH/CH_2Cl_2$), provides 1.29 g. additional product total yield = 30%). Although the product is used as is for completion of the synthesis of (±)-9-benzylthienamycin, for complete characterization, preparative thin layer chromatography (100% $Et_2O$) of 35 mg. of product provides 30 mg. of the faster running trans-olefin and 5 mg. of the cis-olefin.

Data for trans-olefin: IR($CHCl_3$)µ 5.66, 5.77sh

NMR (300 MHz, $CDCl_3$)δ 2.02-2.22 (m, ø$CH_2$$\underline{CH}_2$), 2.61-2.82 (m, ø$\underline{CH}_2$$CH_2$), 2.85 (t, J=6.5Hz, S$\underline{CH}_2$$CH_2$N), 3.18 (dd, $J_{5,6}$ = 2Hz, $J_{6,8}$ = 8Hz, $H_6$) 3.40-3.47 (m, $SCH_2$$\underline{CH}_2$N), 4.25 (dd, J = 2Hz + 7.5 Hz, $H_5$), 5.18 (s & m, $H_8$ & $NHCO_2$$\underline{CH}_2$-arom.), 5.57 (midpt. of 2d, J = 15Hz, nonequivalent-$OCO_2$-$\underline{CH}_2$-arom.), 5.66 (dd, J = 15 Hz & 7.5 Hz,-$\underline{CH}$=CHS), 5.92 (s, NH), 6.27 (d, J = 15 Hz, CH=C$\underline{H}$S), 7.15 - 8.24 (m, aromatics).

Data for cis-olefin: IR($CHCl_3$)µ 5.68, 5.79sh;

NMR (300 MHz, $CDCl_3$)δ 2.06-2.20 (m, ø$CH_2$$C\underline{H}_2$), 2.60-2.86 (m, ø$C\underline{H}_2$$CH_2$ &S$C\underline{H}_2$ $CH_2$N), 3.23 (dd, $J_{5,6}$ = 2Hz, $J_{6,8}$ = 7 Hz, $H_6$), 3.32 - 3.43 (m, $SCH_2$$C\underline{H}_2$N), 4.54 (dd, J=2Hz & 9.5 Hz, $H_5$), 5.18 (s & m, $H_8$ & -$NHCO_2$$C\underline{H}_2$-arom, 5.52 - 5.69 (m, nonequivalent-$OCO_2$-$C\underline{H}_2$-arom. and -$C\underline{H}$=CHS), 6.00 (s, NH), 6.16 (d, J=9Hz, CH=C$\underline{H}$S), 7.14 - 8.22 (m, aromatics).

Step F

Preparation of α-hydroxy 3α-[(1-(R*)-o-nitrobenzylcarbonyldioxy-3-phenyl)propyl]-4β-[2-[2-(p-nitrobenzyloxycarbonylamino)ethylthio]vinyl]-2-oxo-1-azetidinemalonic acid, di-p-nitrobenzyl ester (7).

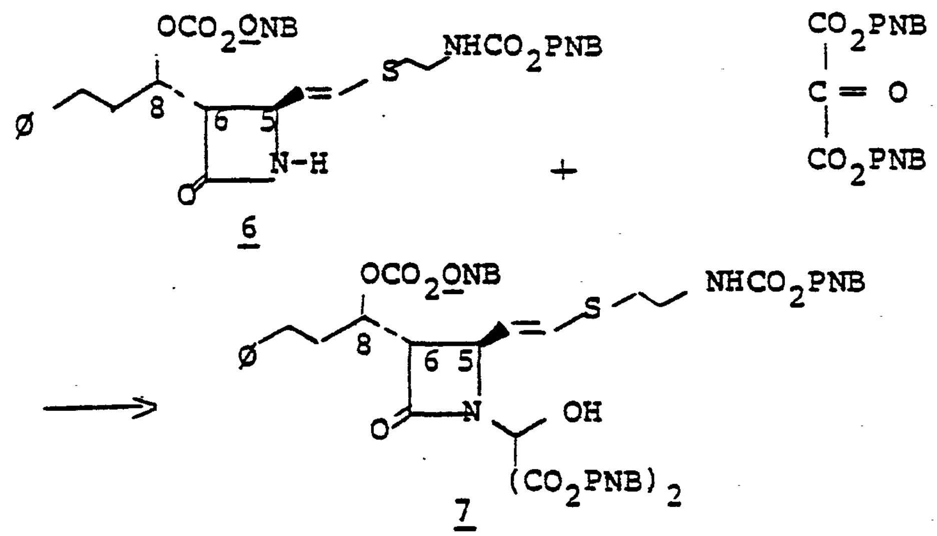

6

7

To a stirred solution of 473 mg. di(p-nitrobenzyl) ketomalonate (from Example 12, Step E) (mw = 388; 1.22 mmole) in 40 ml. hot anhydrous toluene is added a solution of 516 mg. of mainly trans 6 (mw = 664; 0.78 mmole) in 5 ml. THF (distilled from LAH) and 5 ml. anhydrous toluene. After some of the solvent is boiled off, additional anhydrous toluene is added, and the azeodrying process is repeated three times. The solution is then refluxed under $N_2$ for 30 minutes. Additional toluene is then allowed to boil off yet the volume is not allowed to diminish so much that precipitation occurs. Total heating time is approximately 2 1/2 hours. The clear yellow reaction mixture is removed from the oil bath and placed under a stream of $N_2$ which instantaneously causes clouding. After concentration to a yellow oil, the residue is dissolved in $CH_2Cl_2$, dried over anhydrous $MgSO_4$, filtered, and

concentrated under a $N_2$ stream to give crude 7.

The material is chromatographed on 120 g. silica gel packed and applied in $CHCl_3$. The column is eluted with $CHCl_3$ until the excess ketomalonate reagent emerges. Continued elution (1→2% MeOH/$CHCl_3$) provides 563 mg. of slightly impure 7. Preparative thin layer chromatography on silica gel (30% EA/$CH_2Cl_2$) provides 450 mg. of pure 7 (55%).

IR ($CHCl_3$)μ 5.67, 5.80sh

NMR (300 MHz, $CDCl_3$)δ 2.02 - 2.14 (m, $ØCH_2C\underline{H}_2$), 2.58 - 2.82 (m, $ØC\underline{H}_2$ $CH_2$ & $SC\underline{H}_2CH_2N$), 3.22 (dd, $J_{5,6}$ = 2Hz, $J_{6,8}$ = 7Hz, $H_6$), 3.26 - 3.51 (m, $SCH_2C\underline{H}_2N$), 4.66 (dd, J = 2Hz & 9Hz, $H_5$), 5.15 (s & m, $H_8$ & $NHCO_2C\underline{H}_2$-arom.), 5.29 (s,-COH($CO_2C\underline{H}_2$-arom.)$_2$), 5.52 (midpt. of 2 d, J = 14Hz, nonequivalent -$OCO_2C\underline{H}_2$-arom), 5.63 (dd, J=9Hz & 15 Hz, C$\underline{H}$=CHS), 6.21 (d, J=15 Hz, CH=C$\underline{H}$S), 7.13 - 8.22 (m, aromatics).

Cis olefin or mixtures of cis and trans olefin may also be carried through the following steps to yield more (±)-9-benzylthienamycin.

Step G

Preparation of 3α-[(1-(R*)-o-nitrobenzylcarbonyldioxy-3-phenyl)propyl]-4β-[2-(2-p-nitrobenzyloxycarbonylamino)ethylthio]vinyl]-2-oxo-1-azetidinemalonic acid, di-p-nitrobenzylester (8).

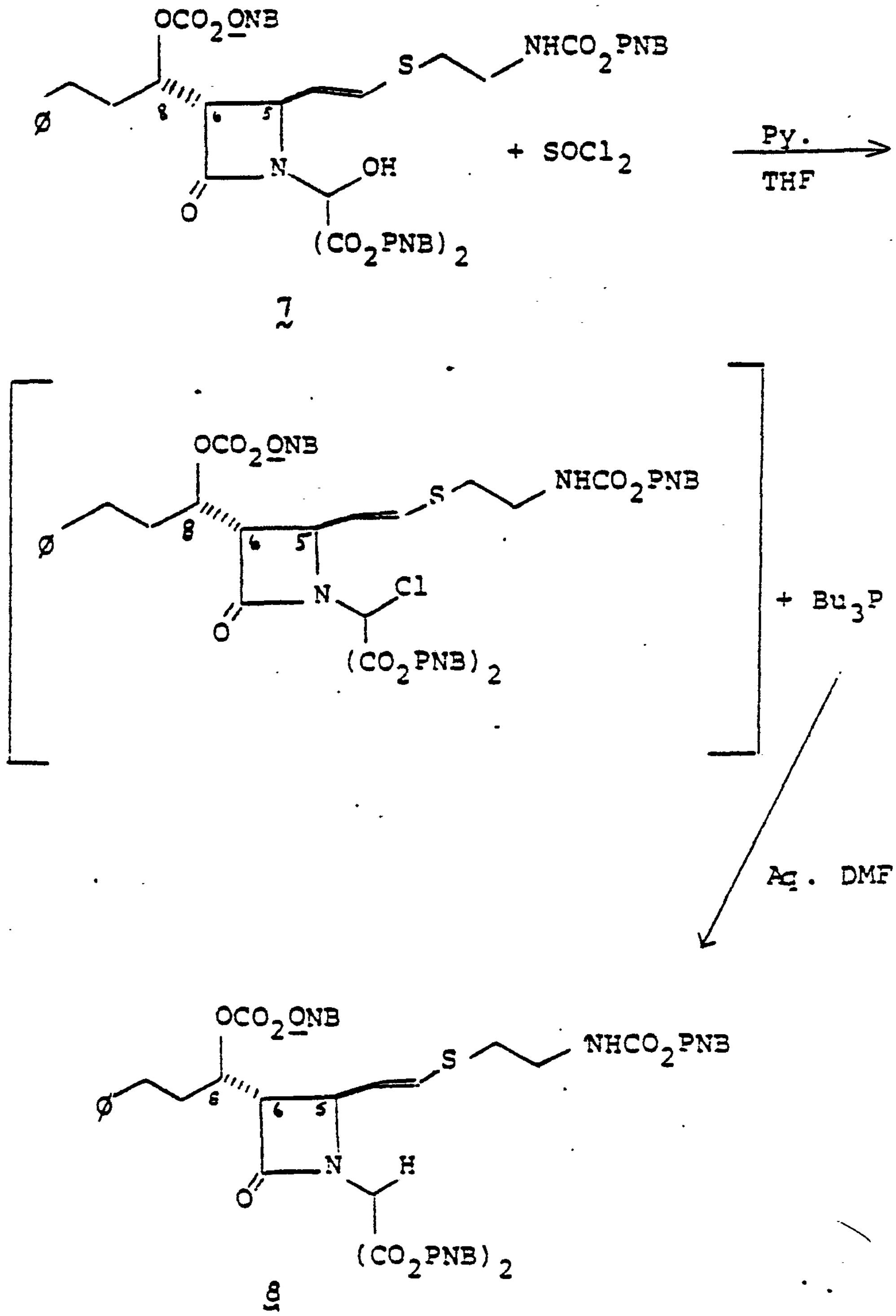


7

8

A solution of 431 mg. of 7 (mw = 1052; 0.41 mmole) in $CH_2Cl_2$ is dried over anhydrous $MgSO_4$, filtered, concentrated under a $N_2$ stream, and dried further under high vacuum just prior to the following reaction. To a solution of 7 in 77 ml. THF (freshly distilled from LAH) at -20°C. is added 56 μl anhydrous pyridine (mw = 79; ρ = .982; 0.70 mmole). With stirring under $N_2$, 80 mg. of freshly distilled thionyl chloride (mw = 119; 0.67 mmole) in 0.7 ml. THF is added. The reaction mixture is stirred for 10 minutes at -20°C., then 1/2 hour at 0°C. and finally 1 hour at 25°C. The pyridine hydrochloride is filtered under $N_2$ and washed with a few ml. THF. The filtrate and washings are concentrated under a $N_2$ stream followed by pumping on high vacuum. The resulting yellow foam is swirled in 7 ml. anhydrous THF, and a small amount of orange-red insoluble material is filtered off under $N_2$. The filtrate is reconcentrated as above to a yellow foam.

To this freshly prepared chloro compound is added with stirring a freshly shaken suspension of 181 mg. tributylphosphine (mw = 202; 0.90 mmole) in 10.4 ml. 9:1 DMF - $H_2O$ followed by 83 mg. $K_2HPO_4$ (mw = 174; 0.48 mmole). The reaction mixture is stirred at 25°C., for 35 minutes. After dilution with EA and brine, the layers are separated, and the aqueous one is extracted two times with EA. The combined organic layers are washed one time with brine, dried over anhydrous $MgSO_4$, filtered and concentrated under a $N_2$ stream followed by pumping on high vacuum to give crude 8. The residue is slurried with a small volume of petroleum ether.

The supernatant is removed, and the process repeated a few times. The petroleum ether supernatants containing some of the excess n-$Bu_3P$ and n-$Bu_3P(O)$ are discarded.

The residue is chromatographed on 40 g. silica gel packed, applied in, and eluted with $CHCl_3$. Those fractions containing product are combined, concentrated under a $N_2$ stream and then on high vacuum to give 286 mg. of slightly impure 8. Preparative thin layer chromatography on silica gel (40% acetone/hexane) then provides 251 mg. of pure 8 (59%).

IR($CHCl_3$)μ 5.64 sh, 5.71, 5.81 sh

NMR (300 MHz, $CDCl_3$) δ 1.99 - 2.18 (m, $ØCH_2C\underline{H}_2$), 2.58 - 2.82 (m, $ØC\underline{H}_2CH_2$ and $SC\underline{H}_2CH_2N$), 3.27 (dd, $J_{5,6}$ = 2.5Hz, $J_{6,8}$ = 7.5Hz, $H_6$), 3.30 - 3.42 (m, $SCH_2C\underline{H}_2N$), 4.54 (dd, J = 2.5Hz and 9Hz, $H_5$), 5.13-5.36 (m, $NHCO_2C\underline{H}_2$-arom. and $C\underline{H}(CO_2C\underline{H}_2\text{-arom})_2$), 5.44 - 5.61 (m, $-OCO_2C\underline{H}_2$-arom. and $C\underline{H}$=CHS), 6.23 (d, J = 15Hz, CH=$C\underline{H}$S), 7.13 - 8.22 (m, aromatics).

Step H

Preparation of 2,2-bis(carbo-p-nitrobenzyloxy)-4-bromo-6-exo-[(1-(R*)-o-nitrobenzylcarbonyldioxy-3-phenyl)propyl]-3-[(2-p-nitrobenzyloxycarbonylamino)ethylthio]-1-azabicyclo[3.2.0]heptan-7-one (9).

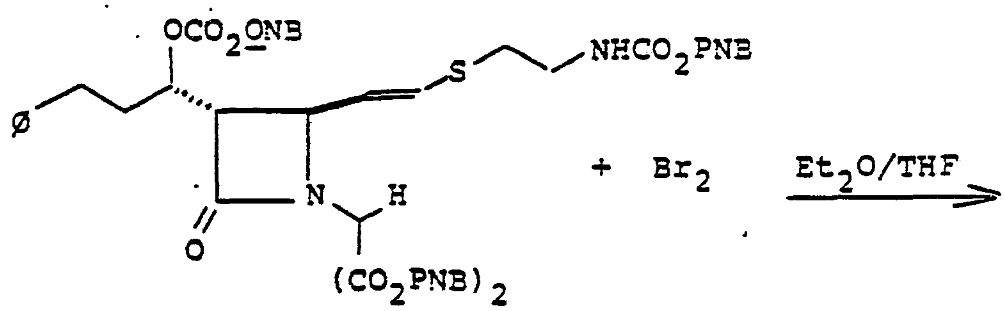


8

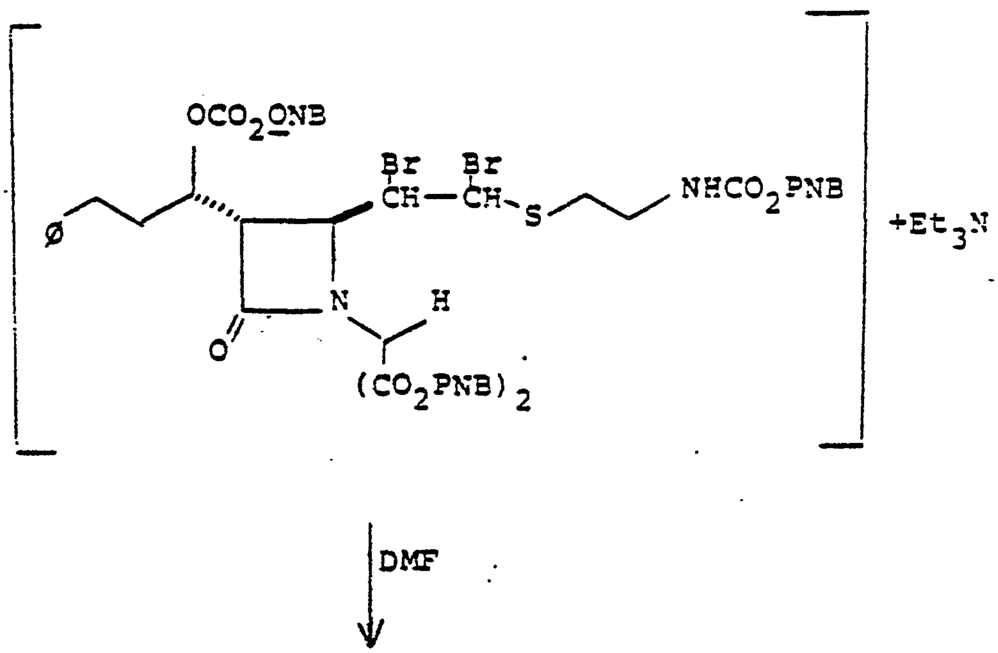


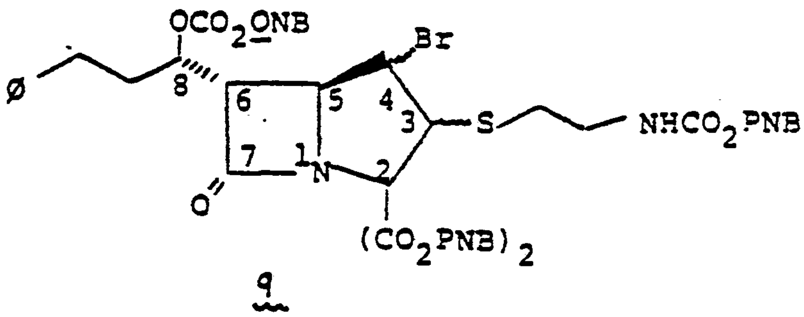

9

To 8.5 ml. $CCl_4$ (dried over 4A Linde molecular sieves) is added 0.2 ml. $Br_2$ (mw = 160; 3.9 mmole). To 0.244 g. of 8 (mw = 1036; 0.236 mmole) in 6 ml. THF (freshly distilled from LAH) and 1.8 ml. $Et_2O$ (dried over 3A 1/16" Linde molecular sieves) at 0°C. under $N_2$ with stirring is added dropwise 0.6 ml. of the above 0.45M $Br_2$ solution (0.27 mmole). After 15 minutes at 0°C., 143μl triethyl amine (mw = 101; $\rho$ = 0.729; 1.03 mmole) is added immediately followed by 3.4 ml. ice-cold DMF (distilled from $CaSO_4$ at 40 mm and stored over 4A Linde molecular sieves). The ice-bath is removed, and stirring at room temperature is continued for 2 hours. The reaction mixture is poured into a stirred ice-cold mixture of 1.1 ml. 1M $KH_2PO_4$ - $H_2O$-EA. The layers are separated, and the aqueous one is saturated with NaCl and re-extracted with EA. The combined organic layers are washed once with brine, dried over anhydrous $MgSO_4$, and filtered. The filtrate is concentrated under a $N_2$ stream and then pumped on high vacuum to give crude 9.

Preparative thin layer chromatography on silica gel (40% acetone/hexane) provides 250 mg. of 9 (93%). IR ($CHCl_3$): 5.61, 5.74, 5.81 sh; NMR (300 MHz, $d_6$-acetone) $\delta$ peaks related to major diasteriomer: 2.10 (m buried under $d_6$-acetone, $\text{Ø}CH_2CH_2$), 2.71 - 3.16 (m, $\text{Ø}CH_2CH_2$ and $SCH_2CH_2N$), 3.37 - 3.53 (m, $SCH_2CH_2N$), 3.94 (dd, $J_{5,6}$ = 3Hz, $J_{6,8}$ = 5.5Hz, $H_6$), 4.41 (dd, $J_{5,6}$ = 3Hz, $J_{4,5}$ = 6Hz, $H_5$), 4.52 (d, $J_{3,4}$ = 6Hz, >CHS), 5.11 (dd appearing as a t, $J_{4,5} = J_{3,4}$ = 6Hz, >CHBr), 5.21 - 5.65 (m, all $CH_2$-arom. and $H_8$), 6.72 (br, NH), 7.2 - 8.27 (m, aromatics); peaks unique to two minor diastereomers:

4.13 (m, $H_6$), 4.24 (m, $H_5$), 4.36 (m, >CHS and >CHBr of one minor diastereomer), 5.02 (d, $J_{3,4}$ = 5Hz, >CHS of 2nd minor diastereomer), 5.14 (m, >CHBr of 2nd minor diastereomer.

Step I

Preparation of 2,2-bis(carbo-*p*-nitrobenzyloxy)-6-*exo*-[(1-(*R**)-*o*-nitrobenzylcarbonyldioxy-3-phenyl)propyl]-3-[(2-*p*-nitrobenzyloxycarbonylamino)ethylthio]-1-azabicyclo[3.2.0]hept-3-en-7-one (10).

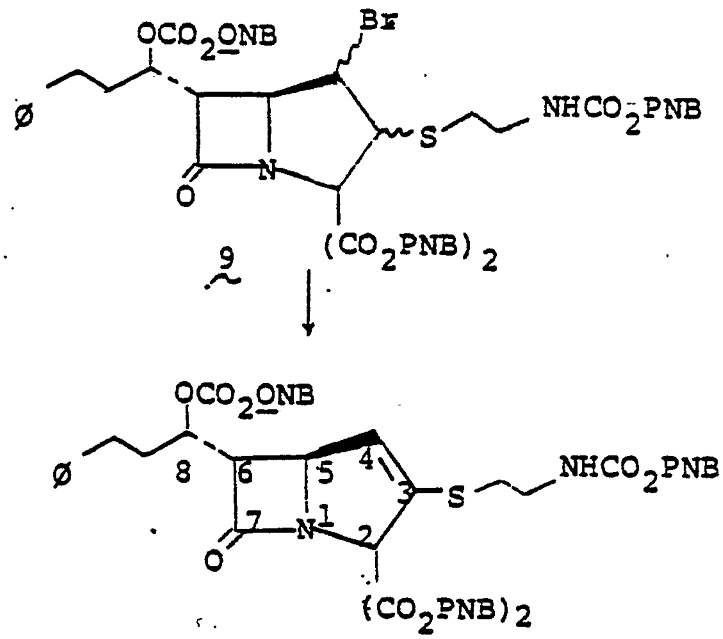

9

10

To 80 mg anhydrous silver fluoride (mw=127; 0.63 mmole) is added a solution of 503 mg of 9 (mw=1114; 0.45 mmole) in 13 ml anhydrous pyridine. Under $N_2$, the reaction mixture is stirred at room temperature in the dark for one hour and then poured into cold water and EA. After separation of the layers, the aqueous one is extracted two times with EA and one time with $CHCl_3$. Each organic layer is extracted one time with $H_2O$ and one time with brine. The combined organic layers are dried over anhydrous $MgSO_4$, filtered, and concentrated under a $N_2$ stream followed by pumping on high vacuum to give crude 10.

Preparative thin layer chromatography on silica gel (40% acetone / hexane) provides 398 mg of 10 which by thin layer chromatography (3% $Et_2O$/$CH_2Cl_2$) still contained a slightly faster impurity. Therefore, further preparative thin layer chromatography (3%$Et_2O$/$CH_2Cl_2$) provides 255 mg of pure 10 (55%).

IR($CHCl_3$): 5.61, 5.73, 5.81 sh

NMR (300MHz, $CDCl_3$)$\delta$ 2.04-2.14(m,Ø$CH_2$$\underline{CH_2}$) 2.61-2.83 (m,Ø$\underline{CH_2}$$CH_2$), 2.91-3.08(m,S$\underline{CH_2}$$CH_2$N), 3.36-3.46 (m,$H_6$ & S$CH_2$$\underline{CH_2}$N), 4.70(brs*,$H_5$), 5.16(s &m,NH$CO_2$-$\underline{CH_2}$-aromatics & $H_8$), 5.29 & 5.31(2s, >C+$CO_2$$\underline{CH_2}$-arom.)$_2$), 5.58(midpt. of 2d,J=15Hz, nonequivalent -$OCO_2$-$\underline{CH_2}$-arom.), 6.12(br s*, vinyl $H_4$), 7.14-8.22(m,aromatics).

* 60 MHz shows additional couplings not observable with 300MHz: 4.70(dd, $J_{5,6}$=3Hz;$J_{4,5}$=1.5Hz,$H_5$), 6.12(d,$J_{4,5}$=1.5Hz,vinyl$H_4$).

Step J

Preparation of 2-carbo-p-nitrobenzyloxy-6-exo-[(1-(R*)-o-nitrobenzylcarbonyldioxy-3-phenyl)propyl]-3-[(2-p-nitrobenzyloxycarbonylamino)ethylthio]-1-azabicyclo [3.2.0]hept-3-en-7-one (11).

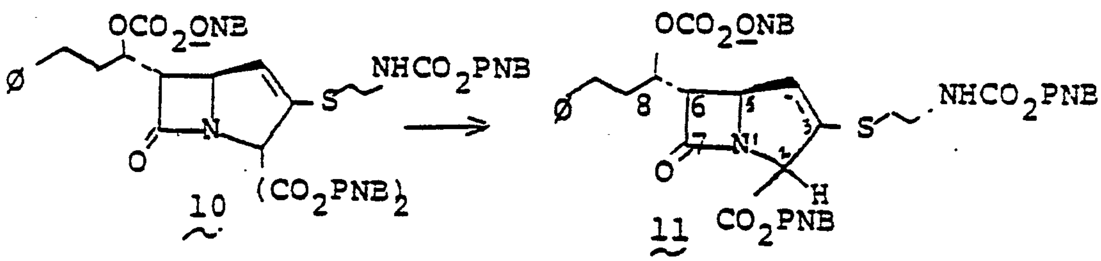

10 11

A solution of 144 mg of 10 (mw = 1034; 0. 014 mmole) in 1.5 ml S-collidine (distilled from powdered KOH ~ 30 mm pressure) is added to 25.1 mg anhydrous LiI (dried for few hours at 100°C over $P_2O_5$ under vacuum) (mw = 134; 0.19 mmole). With stirring under $N_2$, the reaction mixture is heated in an oil bath at 120° - 130°C. After a total of 30 minutes, the reaction mixture is cooled to 25°C., diluted with $CH_2Cl_2$, and transferred to a round bottom flask for concentration under a $N_2$ stream and then on high vacuum. Partitioning the residue between EA-$H_2O$ and 2.3 ml 1M $KH_2PO_4$ is followed by extraction of the aqueous layer two additional times with EA and one time with $CHCl_3$. Each organic layer is then backwashed with brine. The combined organic layers are dried over anhydrous $MgSO_4$, filtered, concentrated under a $N_2$ stream and then on high vacuum to give crude 11.

Preparative thin layer chromatography on silica gel (40% acetone/hexane) provides 57 mg of slightly impure 11 and 48 mg of slightly impure starting material, travelling just slightly slower.

Two subsequent decarbalkoxylations (starting with 146 mg and 234 mg of 10) followed by preparative thin layer chromatography as above yields a total (for the three runs) of 200 mg slightly impure 11 and 111 mg slightly impure starting material. Further preparative thin layer chromatography on silica gel (5% $Et_2O/CH_2Cl_2$) provides 164 mg of pure 11 (38%). Similar chromatography of the recovered starting material provides 78 mg of pure 10 (15%) which may be recycled.

Data for 11:
IR($CHCl_3$): 5.63, 5.72, 5.76
NMR(300MHz, $CDCl_3$) δ 2.07-2.15(m,$ØCH_2C\underline{H}_2$) 2.63-2.85(m,$ØC\underline{H}_2CH_2$), 2.89-3.09(m,$SC\underline{H}_2CH_2N$), 3.33 (dd,$J_{5,6}$=2.5Hz, $J_{6,8}$=7.5Hz, $H_6$), 3.40-3.49 (m,$SCH_2C\underline{H}_2N$), 4.66(brs,*$H_5$), 5.19(s & m, $NHCO_2C\underline{H}_2$-arom., $H_8$ and $H_2$), 5.27(midpt. of 2d, J=15Hz, nonequivalent $CO_2C\underline{H}_2$-arom.), 5.60(midpt. of 2d, J=14Hz, nonequivalent-$OCO_2C\underline{H}_2$-arom.), 5.94(brs,* vinyl $H_4$), 7.15-8.24(m,aromatics).

*60 MHz shows additional couplings not observable with 300 MHz: 4.57-4.70(m,coupling to $H_6$,vinyl $H_4$, and homoallylic coupling to $H_2$ evident, $H_5$), 5.94 overlapping dd appearing as a t, $J_{2,4}$=$J_{4,5}$= 1.5Hz, vinyl $H_4$).

Step K

Preparation of 2-carbo-p-nitrobenzyloxy-6-exo-[(1-(R*)-o-nitrobenzylcarbonyldioxy-3-phenyl)propyl]-3-[(2-p-nitrobenzyloxycarbonylamino)ethylthio]-1-azabicyclo[3.2.0]hept-2-en-7-one (12).

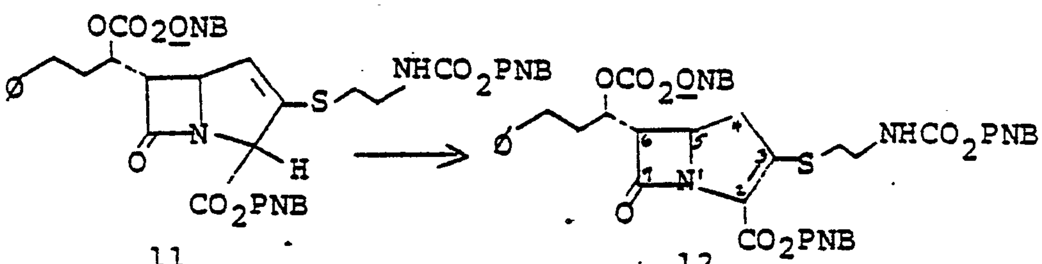

11 12

To 52 mg of 11 (mw=855; 0.061 mmole) in 0.75 ml DMSO (distilled from $CaH_2$ at 8 mm and stored over 4A Linde Molecular Sieves) is added 102 µl diisopropylamine (distilled from NaH under $N_2$ and stored over 4A Linde molecular sieves) (mw=101; ρ=0.722; 0.73 mmole). The stoppered reaction mixture is stirred for a few minutes and then allowed to stand for 2.5 hours. The amine and most of the DMSO are then concentrated off under high vacuum with no external heating. The residue is chased two times with EA and two times with $CHCl_3$ to give a yellow foam essentially free of DMSO. Preparative thin layer chromatography on silica gel (15% EA/$CHCl_3$) provides 10 mg of the more polar product 12. Extraction of the starting material band yields 39 mg recovered 11. Resubmitting 11 to the reaction conditions and isolation procedure three more times gives a total of 24 mg of 12. A final preparative thin layer chromatography of 12 on silica gel (50% EA/hexane) removes two very minor less polar impurities and provides 22 mg of pure 12 (42%). A similar final purification of recovered starting material (40% EA/hexane) yields 11 mg of recovered 11 (21%).

Data for 12:

IR($CHCl_3$)μ 5.63, 5.74 sh, 5.81

NMR(300MHz, $CDCl_3$) δ 2.10-2.22(m,Ø$CH_2C\underline{H}_2$), 2.64-2.88(m,Ø$C\underline{H}_2CH_2$), 2.92-3.12(m,$SC\underline{H}_2CH_2N$,- & dd, $J_{4,5}$=8.5Hz & $J_{4,4'}$=18Hz,$H_4$), 3.36(dd,$J_{4',5}$= 10Hz,$J_{4',4}$=18Hz,$H_{4'}$), 3.40-3.50(m,$SCH_2C\underline{H}_2N$ and $H_6$), 4.25-4.32(m,$H_5$), 5.18(midpt. of m,$H_8$), 5.19(s,-$NHCO_2C\underline{H}_2$-arom.), 5.36(midpt. of 2 widely spaced d, J=14Hz, nonequivalent -$CO_2C\underline{H}_2$-arom.), 5.38(midpt. of 2 widely spaced d,J=14Hz, nonequivalent, -$OCO_2C\underline{H}_2$-arom.), 7.18-8.24(m,aromatics).

When spectrum is run in $d_6$-acetone, $H_6$ is no longer buried under $SCH_2C\underline{H}_2N$: NMR(300MHz,$d_6$-acetone) δ 3.82(dd,$J_{5,6}$=3Hz, $J_{6,8}$=6Hz,$H_6$).

Step L

Preparation of (±)-9-benzylthienamycin (13).

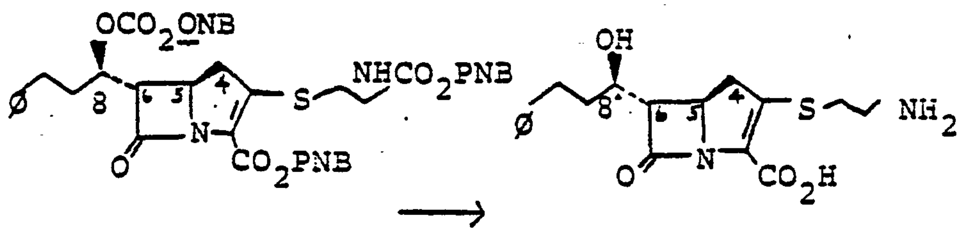

12 13

To 5.3 mg 12(mw=855; 0.0062 mmole) is added 350 µl dioxane, 350 µl ethanol, 175 µl deionized water, and 11 µl 1M $K_2HPO_4$. To the resultant clear solution is added 5.3 mg of 10% Pd/C and a glass bead for efficient mixing. The suspension is flushed with $N_2$, then 5-6 times alternately with 50 psi $H_2$ and vacuum. Finally, it is shaken under a 50 psi $H_2$-atmosphere for 50 minutes. After cooling in an ice bath followed by centrifugation, the supernatant is removed and filtered through a small plug of cotton. The Pd/C is washed and centrifuged 5x with small volumes of cold, deionized water, and the supernatants are filtered as above. The combined, cold filtrates are extracted 3x0.5ml $Et_2O$, 2x0.5 ml EA, and then 2x0.5 ml $Et_2O$. The aqueous layer is then pumped briefly on a water aspirator to remove any residual organic solvents. The remaining solution (ca0.7 ml in volume) is chromatographed (portionwise) by reverse phase HPLC on a Waters analytical $\mu C_{18}$-column (eluting with 10% THF in water). The peak having a $UV_{Max.}$ at 299m_ is collected and is estimated to contain 280-300 ug of 13 (13% yield based upon hydroxylamine quenchable UV). The solution is concentrated on a rotary evaporator under high vacuum and with a water bath no warmer than 25-30°C to a final volume of ca 1 ml.

The concentrate is then shell-frozen in a lyophilizing vial and lyophilized in a shelf lyophilizer wherein the shelf may be held at -20°C. The product exhibits a 300MHz NMR spectrum in $D_2O$ with peaks at: $\delta$ (no internal standard is used) 1.92-2.04 (m, $\emptyset CH_2\underline{CH}_2$), 2.70-3.28 (m, $\emptyset\underline{CH}_2$ $\underline{CH}_2$, $S\underline{CH}_2\underline{CH}_2N$, and $H_4$'s), 3.66 (dd, $J_{5,6}$=3HZ, $J_{6,8}$=6Hz,$H_6$), 4.10-4.17 and 4.24-4.30 (m's, $H_5$&$H_8$), 4.90 (HDO), 7.36-7.50 (m,$\emptyset$).

## EXAMPLE 32

Following the procedure of the foregoing Examples and text, the following representative compounds of the present invention (Table I) are obtained by analogy.

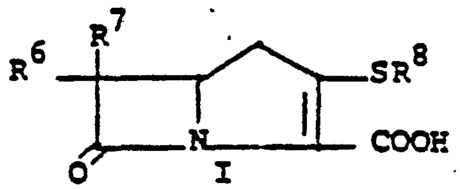

I

TABLE I

| Compound | $R^8$ | $R^6$ | $R^7$ | Remarks |
|---|---|---|---|---|
| 1.) | $-(CH_2)_3NH_2$ | H | $CH_2OH$ | From $BrS(CH_2)_3NHCO_2PNB$, Example 13, Step E; or $HS(CH_2)_3NHCO_2PNB$, Example 12, Step A. |
| 2.) | 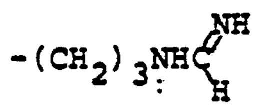 | H | $CH_2OH$ | From Compound 1. of Example 32 in reaction with methyl formimidate hydrochloride in water at pH 8.5. |
| 3.) | 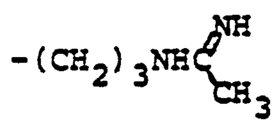 | H | $CH_2OH$ | From Compound 1. of Example 32 in reaction with ethyl acetimidate hydrochoride in water at pH 8.5. |
| 4.) | 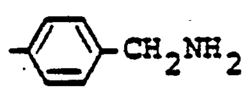 | H | $CH_2OH$ | From HS 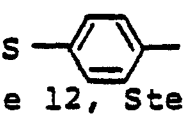 $CH_2NHCO_2PNB$, Example 12, Step A. |
| 5.) | 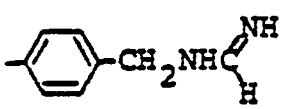 | H | $CH_2OH$ | As in 2., above. |

| Compound | $R^8$ | $R^6$ | $R^7$ | Remarks |
|---|---|---|---|---|
| 6.) | 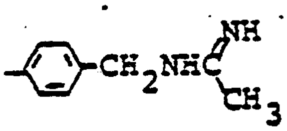 | H | $CH_2OH$ | As in 3., above. |
| 7.) | 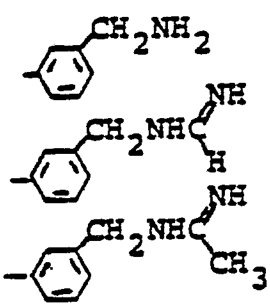 | H | $CH_2OH$ | From 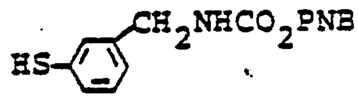 Example 12, Step A. |
| 8.) | | H | $CH_2OH$ | As in 2., above. |
| 9.) | | H | $CH_2OH$ | As in 3., above. |
| 10.) | 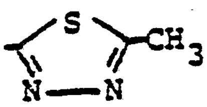 | H | $CH_2OH$ | From 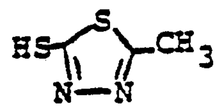 Example 12, Step A. |
| 11.) | 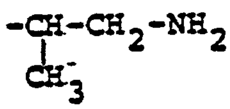 | H | $CH_2OH$ | From $HSCH(CH_3)CH_2NHCO_2PNB$, Example 12, Step A; or $BrSCH(CH_3)CH_2NHCO_2PNB$, Example 13, Step E. |
| 12.) | $-CH_3$ | H | $CH_2OH$ | From $HSCH_3$, Example 12, Step A; or $BrSCH_3$, Example 13, Step E. |
| 13.) | 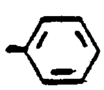 | H | $CH_2OH$ | From HSØ, Example 12, Step A; or BrSØ, Example 13, Step E. |
| 14.) | 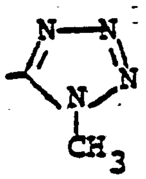 | H | $CH_2OH$ | From: 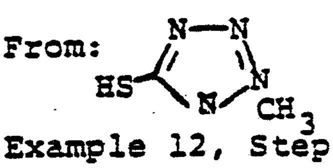 Example 12, Step A. |
| 15.) | 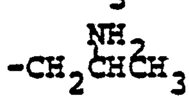 | H | $CH_2OH$ | From $HSCH_2CH(CH_3)NHCO_2PNB$, Example 12, Step A; or $BrSCH_2CH(CH_3)NHCO_2PNB$, Example 13, Step E. |
| 16.) | 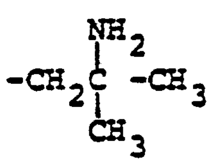 | H | $CH_2OH$ | From $HSCH_2C(CH_3)_2NHCO_2PNB$, Example 12, Step A; or $BrSCH_2C(CH_3)_2NHCO_2PNB$, Example 13, Step E. |
| 17.) | 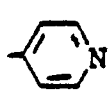 | H | $CH_2OH$ | From 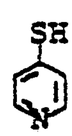, Example 12, Step A. |

| Compound | $R^8$ | $R^6$ | $R^7$ |
|---|---|---|---|
| 18.) | $-CH_2CH_2NH_2$ | H | $CH_3\overset{NH_2}{C}H-$ |
| 19.) | - Ø | $CH_3$ | $CH_3\overset{NH_2}{C}H-$ |
| 20.) | $-CH_2CH_2CH_2NH_2$ | $CH_3$ | $CH_3\overset{OH}{C}H-$ |
| 21.) | - Ø | $CH_3$ | $CH_2OH$ |
| 22.) | $CH_2CH_2NH_2$ | H | $(CH_3)_2CHCH_2\overset{OH}{C}H-$ |
| 23.) | $CH_2CH_2NH-\overset{NH}{\overset{\|}{C}}-H$ | H | $(CH_3)_2CHCH_2\overset{OH}{C}H-$ |
| 24.) | $CH_2CH_2NH-\overset{NH}{\overset{\|}{C}}-CH_3$ | H | $(CH_3)_2CHCH_2\overset{OH}{C}H-$ |
| 25.) | $CH_2CH_2NH_2$ | $CH_3$ | $(CH_3)_2CHCH_2\overset{OH}{C}H-$ |
| 26.) | $CH_2CH_2NH-\overset{NH}{\overset{\|}{C}}-H$ | $CH_3$ | $(CH_3)_2CHCH_2\overset{OH}{C}H-$ |
| 27.) | $CH_2CH_2NH-\overset{NH}{\overset{\|}{C}}-CH_3$ | $CH_3$ | " |

| Compound | $R^5$ | $R^6$ | $R^7$ |
|---|---|---|---|
| 28.) | $CH_2\overset{CH_3}{CH}\ NH_2$ | $CH_3$ | " |
| 29.) | $CH_2CH_2NH_2$ | H | 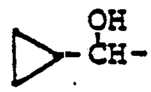 |
| 30.) | " | $CH_3$ | " |
| 31.) | $CH_2CH_2NH.\overset{NH}{C}-H$ | H | " |
| 32.) | $CH_2CH_2NH-\overset{NH}{C}-CH_3$ | H | " |
| 33.) | $CH_2CH_2NH\overset{NH}{C}-H$ | H | $\phi CH_2CH_2\overset{OH}{C}-$ |
| 34.) | $CH_2CH_2NH\overset{NH}{C}CH_3$ | H | " |
| 35.) | $CH(CH_3)_2CH_2NH_2$ | H | " |
| 36 ) | ø | H | $CF_3\overset{OH}{CH}-$ |
| 37.) | 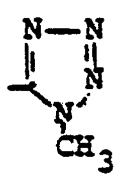 | H | 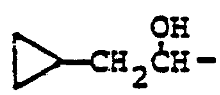 |
| 38) | $CH_2CH_2NH_2$ | H | $CH_3CH_2\overset{OH}{CH}-$ |
| 39.) | $CF_3$ | H | " |
| 40.) | $CH_2CH_2$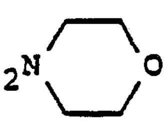 | H | 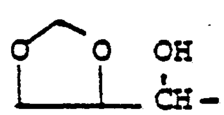 |

| Compound | $R^8$ | $R^6$ | $R^7$ |
|---|---|---|---|
| 41.) | $CH_2CH_2NH_2$ | H | HO<br>$CH_2-CH_2-$ |
| 42.) | $CH_2CO_2CH_3$ | H | OH<br>$CH_3CH-$ |
| 43.) | 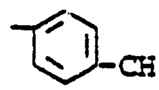$-CH_2NH_2$ | H | OH<br>$CH_3CH_2CH-$ |
| 44.) | 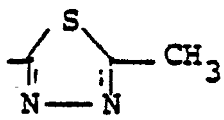 | H | 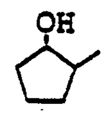 |
| 45.) | 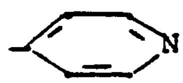 | H | HO<br>$CH_2CH_2-$ |
| 46.) | 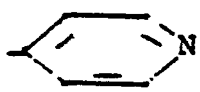 | H | N-OMe<br>″<br>$HC.CH_2-$ |
| 47.) | 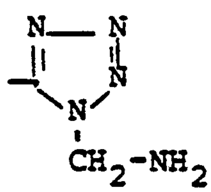 | H | HO<br>$CH_2-CH_2-$ |
| 48.) | 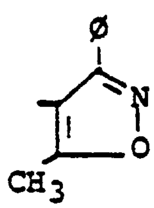 | H | OH<br>$(CH_3)_2CHCH_2CH-$ |
| 49.) | 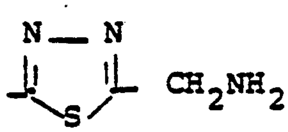 | $CH_3$ | " |
| 50.) | $CH_2CH_2OH$ | H | OH<br>$ØCH_2CH_2C-$ |
| 51.) | $CH_2CH_2NH_2$ | H | OH<br>$ØCH_2CH_2CH-$<br>$CO_2H$ |

| Compound | $R^5$ | $R^6$ | $R^7$ |
| --- | --- | --- | --- |
| 52.) | $CH_2CH_2NH_2$ | H | 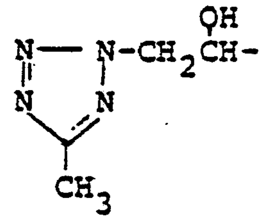 |
| 53.) | $CH_2CH_2NH_2$ | H | 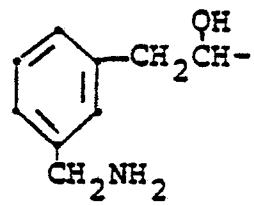 |
| 54.) | 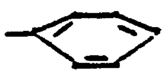 | H | " |
| 55.) | 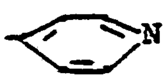 | H | " |
| 56.) | $CH_2CH_2NH\overset{NH}{\overset{\|}{C}}-CH_3$ | H | 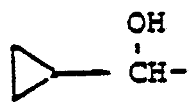 |
| 57.) | $CH_2CH_2NH\overset{NH}{\overset{\|}{C}}CH_3$ | H | 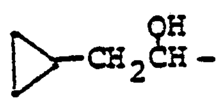 |

| Compound | $R^8$ | $R^6$ | $R^7$ |
|---|---|---|---|
| 58.) | $CH_2CH_2NH\overset{\overset{NH}{\|}}{C}CH$ | H | 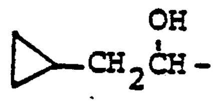 |
| 59.) | $CH_2CH_2NH_2$ | H | " |
| 60.) | " | $CH_3$ | " |
| 61.) | $CH_2CH_2NH\overset{\overset{NH}{\|}}{C}H$ | $CH_3$ | " |
| 62.) | $CH_2CH_2NH\overset{\overset{NH}{\|}}{C}CH_3$ | $CH_3$ | " |
| 63.) | $CH_2CH_2NH\overset{\overset{NH}{\|}}{C}H$ | H | $CH_3CH_2\overset{\overset{OH}{\vert}}{C}H-$ |
| 64.) | $CH_2CH_2NH\overset{\overset{NH}{\|}}{C}CH_3$ | H | " |
| 65.) | $CH_2CH_2NH_2$ | $CH_3$ | " |
| 66.) | $CH_2CH_2NH\overset{\overset{NH}{\|}}{C}CH_3$ | $CH_3$ | " |
| 67.) | $CH_2CH_2NH\overset{\overset{NH}{\|}}{C}H$ | $CH_3$ | " |
| 68.) | $CH_2CH_2NH\overset{\overset{NH}{\|}}{C}CH_3$ | H | $ØCH_2CH_2\overset{\overset{OH}{\vert}}{C}H-$ |
| 69.) | $CH_2CH_2NH\overset{\overset{NH}{\|}}{C}H$ | H | " |
| 70.) | " | H | $Ø\underset{\underset{CO_2H}{\vert}}{C}HCH_2\overset{\overset{OH}{\vert}}{C}H-$ |

| Compound | $R^8$ | $R^6$ | $R^7$ |
|---|---|---|---|
| 71 .) | 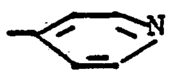 | $CH_3$ | OH<br>ØCHCH$_2$CH<br>CO$_2$H |
| 72 .) | " | " | OH<br>ØCH$_2$CH$_2$CH- |
| 73 .) | " | " | OH<br>(CH$_3$)$_2$CHCH- |
| 74 .) | " | " | OH<br>(CH$_3$)$_2$CH-CH$_2$CH- |
| 75 .) | " | " | OH<br>CH$_3$CH$_2$CH- |
| 76 .) | $CF_3$ | " | HOCH$_2$CH$_2$- |
| 77 .) | " | " | OH<br>(CH$_3$)$_2$CHCH$_2$CH - |
| 78 .) | " | " | OH<br>(CH$_3$)$_2$CHCH - |
| 79 .) | " | " | OH<br>ØCH$_2$CH$_2$CH - |
| 80 .) | " | " | CO$_2$H OH<br>ØCHCH$_2$CH - |
| 81 .) | " | " | 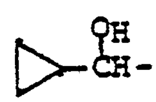 |
| 82 .) | " | " | 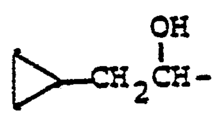 |
| 83 .) | " | " | OH<br>CF$_3$CH - |
| 84 .) | " | H | " |
| 85 .) | " | " | HOCH$_2$CH$_2$- |

| Compound | $R^8$ | $R^6$ | $R^7$ |
|---|---|---|---|
| 86.) | $CF_3$ | H | 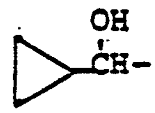 |
| 87.) | " | " | $Ø-CH(CO_2H)CH_2CH(OH)-$ |
| 88.) | " | " | $ØCH_2CH_2CH(OH)-$ |
| 89.) | " | " | $(CH_3)_2CHCH(OH)-$ |
| 90.) | " | " | $(CH_3)_2CHCH_2CH(OH)-$ |
| 91.) | 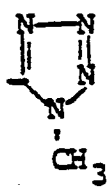 | " | " |
| 92.) | " | " | $CH_3CH_2CH(OH)-$ |
| 93.) | " | " | $(CH_3)_2CHCH(OH)-$ |
| 94.) | " | " | $ØCH_2CH_2CH(OH)-$ |
| 95.) | " | " | $ØCH(CO_2H)CH_2CH(OH)-$ |
| 96.) | " | " | 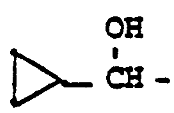 |
| 97.) | " | " | $CF_3CH(OH)-$ |
| 98.) | " | " | $HOCH_2CH_2-$ |

| Compound | $R^8$ | $R^6$ | $R^7$ |
|---|---|---|---|
| 99.) | 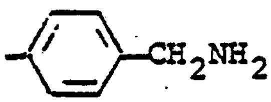 | H | OH<br>$CF_3CH$ |
| 100.) | " | $CH_3$ | " |
| 101.) | 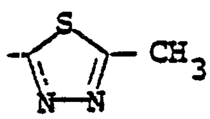 | H | " |
| 102.) | " | " | 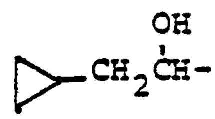 |
| 103.) | " | " | 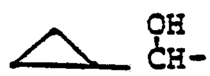 |
| 104.) | " | " | OH<br>$(CH_3)_2CH-CH-$ |
| 105.) | " | " | $HOCH_2CH_2-$ |
| 106.) | " | $CH_3$ | OH<br>$(CH_3)_2CH-CH-$ |
| 107.) | " | " | $HOCH_2CH_2-$ |
| 108.) | " | " | 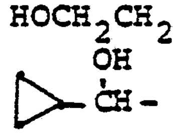 |
| 109.) | " | " | 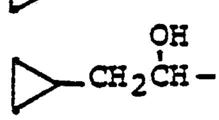 |
| 110.) | " | " | OH<br>ØCHCH$_2$CH-<br>CO$_2$H |
| 111.) | " | " | OH<br>ØCH$_2$CH$_2$CH- |
| 112.) | " | " | OH<br>$(CH_3)_2CHCH-$ |
| 113.) | " | " | OH<br>$(CH_3)_2CHCH_2CH-$ |
| 114.) | 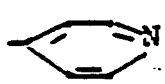 | H | " |

| Compound | $R^8$ | $R^6$ | $R^7$ |
|---|---|---|---|
| 115.) | 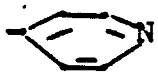 | H | $(CH_3)_2CHCH(OH)-$ |
| 116.) | " | " | $ØCH_2CH_2CH(OH)-$ |
| 117.) | " | " | $ØCH(CO_2H)CH_2CH(OH)-$ |
| 118.) | " | " | 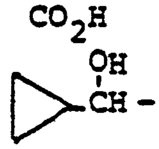 |
| 119.) | " | " | 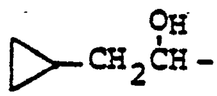 |
| 120.) | " | " | $CH_3CH(OH)-$ |
| 121.) | " | " | $CH_3CH_2CH(OH)-$ |
| 122.) | " | $CH_3$ | $CH_3CH(OH)-$ |
| 123.) | " | " | 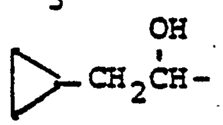 |
| 124.) | " | " | 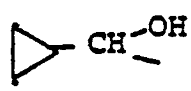 |
| 125.) | $CH_2CH_2NHC(=NH)CH_3$ | " | " |
| 126.) | " | $CH_3$ | " |
| 127.) | $CH_2CH_2NHC(=NH)-H$ | " | " |
| 128.) | $CH_2CH_2NH_2$ | " | " |

| Compound | $R^8$ | $R^6$ | $R^7$ |
|---|---|---|---|
| 129.) | $CH_2CH_2NH_2$ | H | $CF_3\overset{OH}{C}H-$ |
| 130.) | $CH_2CH_2NH-\overset{NH}{C}CH_3$ | " | " |
| 131.) | $CH_2CH_2NH\overset{NH}{C}H$ | " | " |
| 132.) | " | $CH_3$ | " |
| 133.) | $CH_2CH_2NH\overset{NH}{C}CH_3$ | " | " |
| 134.) | $CH_2CH_2NH_2$ | " | " |
| 135.) | " | " | $HO\text{-}CHCH_2-$ |
| 136.) | $CH_2CH_2NH\overset{NH}{C}CH_3$ | " | " |
| 137.) | $CH_2CH_2NH\overset{NH}{C}H$ | " | " |
| 138.) | " | H | " |
| 139.) | $CH_2CH_2NH\overset{NH}{C}CH_3$ | " | " |
| 140.) | 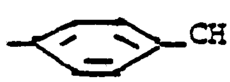 $CH_2NH_2$ | " | " |
| 141.) | " | $CH_3$ | " |

| Compound | $R^8$ | $R^6$ | $R^7$ |
|---|---|---|---|
| 142.) | 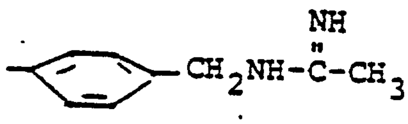 | $CH_3$ | $HO-CH_2CH_2-$ |
| 143.) | 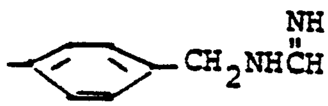 | " | " |
| 144.) | 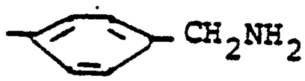 | H | $CF_3\overset{OH}{C}H-$ |
| 145.) | 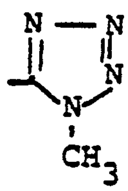 | $CH_3$ | $CH_3CH\overset{OH}{C}H-$ |
| 146.) | " | " | 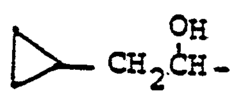 |
| 147.) | " | " | 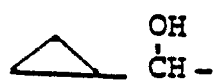 |
| 148.) | " | " | $CF_3\overset{OH}{C}H-$ |
| 149.) | " | " | $ØCH_2CH_2\overset{OH}{C}H-$ |
| 150.) | " | " | $Ø\underset{CO_2H}{C}HCH_2\overset{OH}{C}H-$ |
| 151.) | " | " | $(CH_3)_2CH\overset{OH}{C}H-$ |
| 152.) | " | " | $(CH_3)_2CHCH_2\overset{OH}{C}H-$ |

| Compound | $R^8$ | $R^6$ | $R^7$ |
|---|---|---|---|
| 153.) | 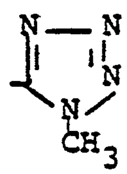 | $CH_3$ | $HOCH_2CH_2-$ |
| 154.) | 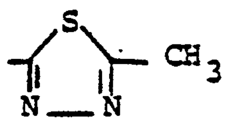 | H | $CH_3CH_2\overset{OH}{CH}-$ |
| 155.) | " | " | $ØCH_2CH_2\overset{OH}{CH}-$ |
| 156.) | " | " | $Ø\underset{CO_2H}{CH}\ CH_2\overset{OH}{CH}-$ |

| Compound | |
| --- | --- |
| 157-313 | Compounds 157-313 correspond sequentially to compounds 1-156, above, except that the value for $R^7$ is taken as $CH_3CH(OH)$ rather than the values shown for compounds 1-156. |
| 314-470 | Compounds 314-470 correspond sequentially to compounds 1-156, above, except that the value for $R^7$ is taken as $CH_3CH_2$ rather than the values shown for compounds 1-156. |
| 471-627 | Compounds 471-627 correspond sequentially to compounds 1-156, above, except that the value for $R^7$ is taken as $Cl_2CHCH(OH)$ rather than the values shown for compounds 1-156. |

| Compound | |
| --- | --- |
| 628-784 | Compounds 628-784 correspond sequentially to compounds 1-156, above, except that the value for $R^7$ is taken as $CF_3CH(OH)$ rather than the values shown for compounds 1-156. |
| 785-941 | Compounds 785-941 correspond sequentially to compounds 1-156, above, except that the value for $R^7$ is taken as $HOCH_2CH(OH)$ rather than the values shown for compounds 1-156. |
| 942-1098 | Compounds 942-1098 correspond sequentially to compounds 1-156, above, except that the value for $R^7$ is taken as $ClCH_2CH(OH)$ rather than the values shown for compounds 1-156. |

| Compound | |
|---|---|
| 1099-1255 | Compounds 1099-1255 correspond sequentially to compounds 1-156, above, except that the value for $R^7$ is taken as $CH_3CH_2CH(OH)$ rather than the values shown for compounds 1-156. |
| 1256-1412 | Compounds 1256-1412 correspond sequentially to compounds 1-156, above, except that the value for $R^7$ is taken as 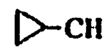-CH(OH) rather than the values shown for compounds 1-156. |
| 1413-1569 | Compounds 1413-1569 correspond sequentially to compounds 1-156, above, except that the value for $R^7$ is taken as $H_2NCH_2CH(OH)$ rather than the values shown for compounds 1-156. |
| 1570-1726 | Compounds 1570-1726 correspond sequentially to compounds 1-156, above, except that the value for $R^7$ is taken as $CF_2HCH(OH)$ rather than the values shown for compounds 1-156. |

| Compound | |
|---|---|
| 1727-1883 | Compounds 1727-1883 correspond sequentially to compounds 1-156, above, except that the value for $R^7$ is taken as $HOCH_2$ rather than the value shown for compounds 1-156. |
| 1884-2040 | Compounds 1884-2040 correspond sequentially to compounds 1-156, above, except that the values for $R^7$ is taken as $CH_3OCH_2CH(OH)$ rather than the values shown for compounds 1-156. |
| 2041-2197 | Compounds 2041-2197 correspond sequentially to compounds 1-156, above, except that the values for $R^7$ is taken as $(CH_3)_3CCH_2CH(OH)$ rather than the values shown for compounds 1-156. |
| 2198-2354 | Compounds 2198-2354 correspond sequentially to compounds 1-156, above, except that the values for $R^7$ is taken as $HO_2CCH_2$ rather than the values shown for compounds 1-156. |

NOTES FOR TABLE I EXAMPLE 32, COMPOUNDS 18-156

18.) Same as Example 7 substituting the produce from Example 22 and then carrying on following procedures of Example 12.

19.) Same as Examples 21-22, except substituting the product from Example 5 in which acetaldehyde has been substituted for formaldehyde. The resultant product is exposed to the conditions of Examples 7 and 12 except that in Step 12a, thiophenol is substituted for the blocked cysteamine.

20.) Same as in Example 5 except substituting acetaldehyde for formaldehyde. The product is treated as in Examples 6, 7 and 12, except that in 12a the blocked homocysteamine is substituted for the blocked cysteamine.

21.) From Example 12 by substituting in Step A thiophenol for the blocked cysteamine.

22.) Following Example 31 except substituting isovaleraldehyde in Step A for isobutyraldehyde.

23.) By treating compound 22, Example 32 with methylformimidate hydrochloride in water at pH 8.5

24.) By treating compound 22 Example 32 with methyl acetimidate hydrochloride in water at pH 8.5

25.) By following the procedure of Example 31, except substituting the product from Example 13, Step A' for the simple blocked vinylazetidinone.

26.) By treating compound 25 Example 32 with methylformimidate hydrochloride in water at pH 8.5.

27.) By treating compound 25 Example 32 with methylacetimidate hydrochloride in water at pH 8.5.

28.) As in preparation of compound 25 Example 32 except that 2-amino-mercaptopropane is substituted for cysteamine.

29.) As in Example 31 except substituting cyclopropane carboxaldehyde for isobutyraldehyde in Step A.

30.) As in preparing compound 29 Example 32, except substituting the product from Example 13 Step A' for the simple blocked vinyl azetidinone.

31.) From compound 29 Example 32 by treatment with methylformimidate hydrochloride in water at pH 8.5

32.) From compound 29 Example 32 by treatment with methylacetimidate hydrochloride in water at pH 8.5.

33.) From the product of Example 31a by treatment with methylformimidate hydrochloride in water at pH 8.5

34.) From the product of Example 31a by treatment methylacetimidate hydrochloride in water at pH 8.5.

35.) As in Example 31a except substituting 1-aminopropane-2-thiol for cysteamine.

36.) If in Example 15, trifluoroacetaldehyde is substituted for acetaldehyde and the product is carried through the steps of Example 16 except that thiophenol is substituted for the p-nitroCbz blocked cysteamine, the indicated compound is obtained.

37.) If the procedure for the preparation of compound 57, Example 32 is carried out except that 5-mercapto-1-methyltetrazole is substituted for the p-nitroCbz blocked cysteamine, the indicated compound is obtained.

38.) If the procedure in Example 31a is followed except that propionaldehyde is substituted for dihydrocinnamaldehyde, the indicated compound is obtained.

39.) If the procedure for preparing compound 38 Example 32 is followed except that trifluoromethylmercaptan is substituted for the p-nitroCbz blocked cysteamine, the indicated compound is obtained.

40.) If the procedure used in Example 15 is followed substituting the acetonide of dihydroxypropionaldehyde for formaldehyde, and the product is exposed to the conditions of Example 16, except substituting N-2-mercapto ethylmorpholine for the p-nitroCbz blocked cysteamine, the indicated product is obtained.

41.) Following the procedure of Examples 15 and 16 except substituting ethylene oxide for the formaldehyde in the former, the indicated product is obtained.

42.) Following the procedure of Examples 15 and 16 except substituting acetaldehyde for formaldehyde in the former and substituting methylmercaptoacetate for p-nitroCbz blocked cysteamine, the indicated compounds are obtained.

43.) Following the procedure for the preparation of compound 38 Example 32, except substituting 4-(4-nitrobenzyloxycarbonylaminomethyl)-thiophenol for the p-nitroCbz blocked cysteamine in Step D, the indicated compound is obtained.

44.) Following the procedure for the preparation of compound 41 example 32, except substituting cyclopentene oxide for ethylene oxide and 2-mercapto-5-methyl-1,3,4-thiadiazole for the p-nitroCbz blocked cysteamine, the indicated compound is obtained.

45.) Following the procedure for the preparation of compound 41, Example 32 except substituting 4-mercaptopyridine for the p-nitroCbz blocked cysteamine the indicated compound is obtained.

46.) If Compound 45 Example 32 is converted to the o-nitrobenzyl ester, isomerized to the $\Delta^1$ compound oxidized with Moffats reagent to the aldehyde, treated with methoxyamine to form the methoxime, re-isomerized to the $\Delta^2$ compound and photolytically deblocked, the indicated compound is obtained.

47.) Following the procedure for Compound 45, Example 32, except substituting 5-mercapto-4-(4-nitrobenzyloxycarbonylaminomethyl)tetrazole for the 4-mercaptopyridine, the indicated compound is obtained.

48.) Following the procedure for Compound 22, Example 32, except substituting 3-phenyl-5-methyl-4-mercapto-1,2-oxazole for the p-nitroCbz blocked cysteamine, the indicated compound is obtained.

49.) Following the procedure for Compound 25 Example 32 except substituting 5-mercapto-2-(4-nitrobenzyloxycarbonylaminomethyl)-1,3,4-thiadiazole for the p-nitroCbz blocked cysteamine, the indicated compound is obtained.

50.) Following the procedure of Example 31a, if the p-nitroCbz blocked cysteamine is replaced with 2-(4-nitrobenzylcarbonyldioxy)ethyl mercaptan, the indicated compound is obtained.

51.) Following the procedure of Example 31a, but replacing the dihydrocinnamaldehyde with 4-nitrobenzyl 2-phenyl-4-oxobutyrate, the indicated compound is obtained.

52.) Following the procedure of Example 31 but substituting 2-(5-methyl-2-tetrazolyl)-acetaldehyde for the isobutyraldehyde, the indicated compound is obtained.

53.) Following the procedure of Example 31, but substituting 3-(4-nitrobenzyloxycarbonylaminomethyl)phenylacetaldehyde for the isobutyraldehyde the indicated compound is obtained.

54.) Following the procedure for Compound 53, Example 32, except substituting thiophenol for the p-nitroCbz blocked cysteamine, the indicated compound is obtained.

55.) Following the procedure for Compound 53, Example 32 except substituting 4-mercapto-pyridine for the p-nitroCbz blocked cysteamine, the indicated compound is obtained.

56.) If compound 29 Example 32 is treated with methylacetimidate hydrochloride in water at pH 8.5 the indicated compound is obtained.

57.) If Compound 59, Example 32 is treated with methylacetimidate hydrochloride in water at pH 8.5 the indicated compound is obtained.

58.) If Compound 59, Example 32 is treated with methylformimidate hydrochloride in water at pH 8.5 the indicated compound is formed.

59.) If the procedure of Example 31 is used except that cyclopropylacetaldehyde is substituted for isobutyraldehyde, the indicated compound is obtained.

60.) If the procedure used to prepare Compound 59 Example 32 is carried out, with the substitution of the product from Example 13, Step $A^1$ for the simple blocked vinyl azetidinone.

61.) By treating compound 60, Example 32 with methylformimidate hydrochloride in water at pH 8.5.

62.) By treating product 60 Example 32 with methyl-acetimidate hydrochloride in water at pH 8.5.

63.) By treating product 38 Example 32 with methylformimidate hydrochloride in water at pH 8.5

64.) By treating product 38 Example 32 with methylacetimidate hydrochloride in water at pH 8.5.

65.) If the compound obtained from Example 13, Step $A^1$, is used in place of the simple blocked vinyl azetidinone in the procedure for preparing compound 38 Example 32, the indicated compound is obtained.

66.) By treating product 65 Example 32 with methylacetimidate hydrochloride in water at pH 8.5.

67.) By treating product 65, Example 32 with methylformimidate hydrochloride in water at pH 8.5.

68.) By treating the product from Example 31a with methylacetimidate hydrochloride in water at pH 8.5.

69.) By treating the product from Example 31a with methylformimidate hydrochloride in water at pH 8.5.

70.) By treating product 51 Example 32 with methylformimidate hydrochloride in water at pH 8.5.

71.) If the procedure for preparing compound 55, Example 32 is followed except that 4-nitrobenzyl 2-phenyl-4-oxobutyrate is substituted for isovaleraldehyde and 4-mercaptopyridine is substituted for thiophenol, the indicated compound is formed.

72.) As in 71 except substituting dihydrocinnamaldehyde for isovaleraldehyde.

73.) As in 71 except substituting isobutyraldehyde for the isovaleraldehyde.

74.) As in 71 except that isovaleraldehyde is kept and only the 4-mercaptopyridine for thiophenol change is made.

75.) As in 71 except substituting propionaldehyde for the isovaleraldehyde.

76.) The procedure. for Compound 39 Example 32, is followed substituting the product from procedure 13 Step A[1] for the simple blocked vinyl azetidinone and ethylene oxide for propionaldehyde.

77.) As in 76 except isovaleraldehyde is substituted for propionaldehyde.

78.) As in 76 except isobutyraldehyde is substituted for propionaldehyde.

79.) As in 76 except dihydrocinnamaldehyde is substituted for propionaldehyde.

80.) As in 76 except 4-nitrobenzyl 2-phenyl-4-oxobutyrate is substituted for propionaldehyde.

81.) As in 76 except cyclopropylcarboxaldehyde is substituted for propionaldehyde.

82.) As in 76 except cyclopropylacetaldehyde is substituted for propionaldehyde.

83.) As in 76 except trifluoroacetaldehyde is substituted for propionaldehyde.

84.) The procedure for compound 39 Example 32 is followed substituting trifluoroacetaldehyde for propionaldehyde.

85.) As in 84 except ethylene oxide is substituted for propionaldehyde.

86.) As in 84 except cyclopropylcarboxaldehyde is substituted for propionaldehyde.

87.) As in 84 except 4-nitrobenzyl 2-phenyl-4-oxobutyrate is substituted for propionaldehyde.

88.) As in 84 except dihydrocinnamaldehyde is substituted for propionaldehyde.

89.) As in 84 except isobutyraldehyde is substituted for propionaldehyde.

90.) As in 84 except isovaleraldehyde is substituted for propionaldehyde.

91.) The procedure for Compound 37 Example 32 is followed except that isovaleraldehyde is substituted for cyclopropylacetaldehyde.

92.) As in 91 except propionaldehyde is substituted for cyclopropylacetaldehyde.

93.) As in 91 except isobutyraldehyde is substituted for cyclopropylacetaldehyde.

94.) As in 91 except dihydrocinnamaldehyde is substituted for cyclorpopylacetaldehyde.

95.) As in 91 except 4-nitro 2-phenyl-4-oxobutyrate is substituted for cyclopropylacetaldehyde.

96.) As in 91 except cyclopropylcarboxaldehyde is substituted for cyclopropylacetaldehyde.

97.) As in 91 except trifluoroacetaldehyde is substituted for cyclopropylacetaldehyde.

98.) As in 91 except ethylene oxide is substituted for cyclopropylacetaldehyde.

99.) If the procedure for Compound 43 Example 32 is followed, substituting trifluoroacetaldehyde for propionaldehyde, the indicated compound is obtained.

100.) If the procedure for Compound 99 Example 32 is followed, substituting the product from Example 13 Step A1 for the simple blocked vinyl azetidinone, the indicated product is obtained.

101.) If the procedure for Compound 44 Example 32 is followed, substituting trifluoroacetaldehyde for cyclopentene oxide, the indicated compound is obtained.

102.) As in 101 except cyclopropylacetaldehyde is substituted for cyclopentene oxide.

103.) As in 101 except cyclopropylcarboxaldehyde is substituted for cyclopentene oxide.

104.) As in 101 except isobutyraldehyde is substituted for cyclopentene oxide.

105.) As in 101 except ethylene oxide is substituted for cyclopentene oxide.

106.) If the procedure of compound 104 is followed, substituting the product from Example 13, Step A' for the simple blocked vinyl azetidinone, the indicated product is obtained.

107.) As in 106 except ethylene oxide is substituted for cyclopentene oxide.

108.) As in 106 except cyclopropylcarboxaldehyde is substituted for cyclopentene oxide.

109.) As in 106 except cyclopropylacetaldehyde is substituted for cyclopentene oxide.

110.) As in 106 except 4-nitrobenzyl 2-phenyl-4-oxobutyrate is substituted for cyclopentene oxide.

111.) As in 106 except dihydrocinnamaldehyde is substituted for cyclopentene oxide.

112.) As in 106 except isobutyraldehyde is substituted for cyclopentene oxide.

113.) As in 106 except isovaleraldehyde is substituted for cyclopentene oxide.

114.) If the procedure for Compound 45 Example 32 is followed, using isovaleraldehyde in place of ethylene oxide, the indicated compound is obtained.

115.) As in 114 except isobutyraldehyde is substituted for ethylene oxide.

116.) As in 114 except dihydrocinnamaldehyde is substituted for ethylene oxide.

117.) As in 114 except 4-nitrobenzyl 2-phenyl-4-oxobutyrate is substituted for ethylene oxide.

118.) As in 114 except cyclopropylcarboxaldehyde is substituted for ethylene oxide.

119.) As in 114 except cyclorpropylacetaldehyde is substituted for ethylene oxide.

120.) As in 114 except acetaldehyde is substituted for ethylene oxide.

121.) As in 114 except propionaldehyde is substituted for ethylene oxide.

122.) As in 75 except acetaldehyde is substituted for propionaldehyde.

123.) As in 75 except cyclopropylcarboxaldehyde is substituted for propionaldehyde.

124.) As in 75 except cyclopropylacetaldehyde is substituted for propionaldehyde.

125.) Use methyl acetimidate hydrochloride in water at pH 8.5.

126.) Use methyl acetimidate hydrochloride in water at pH 8.5.

127.) Use methyl formimidate hydrochloride in water at pH 8.5.

128.) As in compound 124, using $HSCH_2CH_2NHCO_2PNB$.

129.) Following the procedures of Example 15 and 16, except substituting trifluoroacetaldehyde for formaldehyde in the former, gives the indicated product.

130.) By treating product 129 Example 32 with methyl acetimidatè hydrochloride in water at pH 8.5

131.) By treated product 129 Example 32 with methyl formimidate hydrochloride in water at pH 8.5

132.) By treating product 134 Example 32 with methyl formimidate hydrochloride in water at pH 8.5.

133.) By treating product 134 Example 32 with methyl acetimidate hydrochloride in water at pH 8.5.

134.) If the reactions of Examples 5, 6, 7 and 12 are carried out substituted trifluoroacetaldehyde for formaldehyde in Example 5, the indicated product is obtained.

135.) If the reactions of Examples 5,6,7 and 12 are carred out substituted ethylene oxide for formaldehyde in Example 5, the indicated product is obtained.

136.) By treating product 135 Example 32 with methyl acetimidate hydrochloride in water at pH 8.5.

137.) By treating product 135 Example 32 with methyl formimidate hydrochloride in water at pH 8.5.

138.) By treating product 41 Example 32 with methyl formimidate hydrochloride in water at pH 8.5.

139.) By treating product 41 Example 32 with methyl acetimidate hydrochloride in water at pH 8.5.

140.) Following the procedure for compound 43 Example 32 but substituting ethylene oxide for propionaldehyde, the indicated compound is obtained.

141.) As in 140 but using the product from Example 13 Step A[1] in place of the simple blocked vinyl azetidinone.

142.) By treating the product from 141 with methyl acetimidate hydrochloride in water at pH 8.5.

143.) By treating the product from 141 with methyl formimidate hydrochloride in water at pH 8.5.

144.) Following the procedure for compound 43 Example 32 but substituting trifluoroacetaldehyde for propionaldehyde, the indicated compound is obtained.

145.) If in the preparation of compound 92 Example 32, the product from Example 13 Step A' is substituted for the simple blocked vinyl azetidinone, the indicated compound is obtained.

146.) As in compound 145 Example 32, substituting cyclopropylacetaldehyde for propionaldehyde.

147.) As in compound 145 Example 32, substituting cyclopropylcarboxaldehyde for propionaldehyde.

148.) As in compound 145 Example 32, substituting trifluoroacetaldehyde for propionaldehyde.

149.) As in compound 145 Example 32, substituting dihydrocinnamaldehyde for propionaldehyde.

150.) As in compound 145 Example 32, substituting 4-nitrobenzyl 2-phenyl-4-oxobutyrate for propionaldehyde.

151.) As in compound 145 Example 32, substituting isobutyraldehyde for propionaldehyde.

152.) As in compound 145 Example 32, substituting isovalerylaldehyde for propionaldehyde.

153.) As in compound 145 Example 32, substituting ethylene oxide for propionaldehyde.

154.) As in compound 101 except propionaldehyde is substituted for cyclopentene oxide.

155.) As in 101 except dihydrocinnamaldehyde is substituted for cyclopentene oxide.

156.) As in 101 except 4-nitrobenzyl 2-phenyl-4-oxobutyrate is substituted for cyclopene oxide.

EXAMPLE 33

Preparation of the N-Formimidoyl derivative of 3-(2-aminoethylthio)-6-methyl-6-(hydroxymethyl)-7-oxo-1-azabicyclo[3.2.0]hept-2-ene-2-carboxylic acid

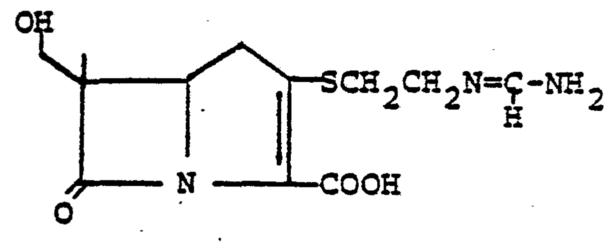

Compound 9 from Example 12 - Step K (517 mg) is dissolved in pH 7 0.1N phosphate buffer (25 ml) and cooled in an ice bath with magnetic stirring. The solution is adjusted to pH 8.5 using 2.5N sodium hydroxide solution dispensed from an automatic burette. While maintaining a pH of 8.5, methyl formimidate hydrochloride (711 mg) is added portionwise over 2-3 minutes. After an additional 10 minutes, the pH of the solution is brought to 7.0 using 2.5N hydrochloric acid. The solution is chromatographed on a column of XAD-2 resin (150 cc) which is eluted with water. The N-formimidoyl derivative is eluted and lyophilized.

Following the procedure of Example 33, enhanced product isolation is achieved when the XAD-2 column is replaced by an otherwise equivalent column of Dowex 50-X4 ($Na^+$ cycle, 200-400 mesh).

Amidine embodiments of the present invention, such as that illustrated in Example 33, represent a preferred class. With reference to the generic representation of the compounds of the present invention (Structure I, above), such embodiments are possible when the radical $-SR^8$ bears an amino functional group. The preparation of amidine and amidine-like species is fully described in co-pending, commonly assigned U.S. Patent Application

Serial Number 852,425 (filed 11-17-77), which application is incorporated herein by reference to the extent that it describes the preparation of amidine and amidine-like derivatives from species of the present invention which carry an amino group on $-SR^8$.

## EXAMPLE 34

Preparation of the N-Acetimidoyl derivative of 3-(2-aminoethylthio)-6-methyl-6-(hydroxymethyl)-7-oxo-1-azabicyclo[3.2.0]hept-2-ene-2-carboxylic acid

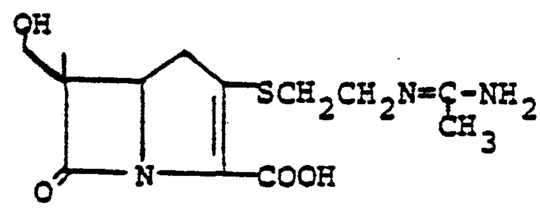

Compound 9 from Example 12, Step K (190 mg) is dissolved in pH 7 0.1N phosphate buffer (13 ml) and cooled in an ice bath with magnetic stirring. The solution is adjusted to pH 8.5 using 2.5N sodium hydroxide solution dispensed from an automatic burette. While maintaining a pH of 8.5, ethyl acetimidate hydrochloride (400 mg) is added portionwise over a few minutes. After an additional 40 minutes the solution is adjusted to pH 7.0 with 2.5N hydrochloric acid. The solution is then chromatographed on Dowex 50-X8 resin (250 cc, $Na^+$ cycle, 100-200 mesh) and is eluted with water. The N-acetimidoyl derivative is eluted and lyophilized.

EXAMPLE 35

Preparation of Silylated 3-(2-aminoethylthio)-6-methyl-6-(hydroxymethyl)-7-oxo-1-azabicyclo[3.2.0]hept-2-ene-2-carboxylic acid

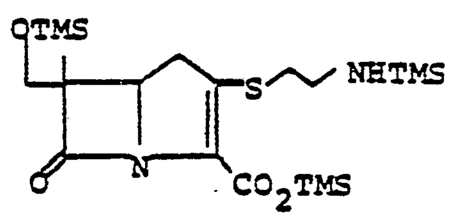

Compound 9 from Example 12, Step K (80.0 mg) is suspended in 40 ml tetrahydrofuran (THF) under a $N_2$ atmosphere and is concentrated to 10 ml; hexamethyldisilazane (1.0 ml) and trimethylchlorosilane (300 μl) is added. The mixture is reacted for 20 mins. at 25°C. with vigorous stirring. The suspension is then centrifuged to remove ammonium chloride. The supernatant is evaporated to provide the title compound under a nitrogen stream for future reaction.

EXAMPLE 36

Preparation of the N-Piperidin-1-yl Methylene Derivative of 3-(2-aminoethylthio)-6-methyl-6-(hydroxymethyl)-7-oxo-1-azabicyclo[3.2.0]hept-2-ene-2-carboxylic acid

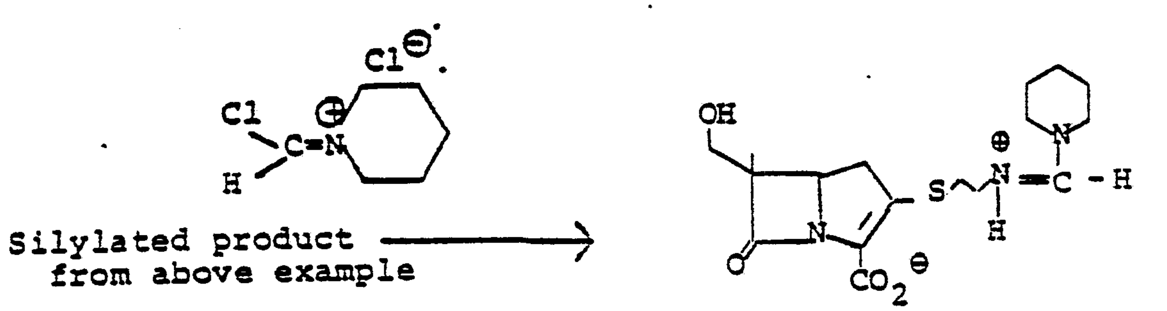

Compound 9 from Example 12, Step K (57 mg., 162 µmol) is silylated according to the procedure previously described. The silylated antibiotic is dissolved in methylene chloride (6 cc) in a septum stoppered flask under positive nitrogen pressure and cooled in a dry iceacetone bath. To the magnetically stirred solution is added a solution (180 µl) of triethylamine (644 µmol) in methylene chloride. This is followed by the addition of a solution of chloropiperidinomethylium chloride (67 mg, 405 µmol) in methylene chloride (465 µl). After 1 hour in the dry ice bath, the reaction solution is rapidly added to a tetrahydrofuran -pH 7, 0.1N phosphate buffer (1:1) solution (50 ml). The mixture is then concentrated under vacuum to 10 ml to give a homogeneous solution. The solution is washed twice with ethyl acetate (2 x 5 ml) and ether (2 x 5 ml) and briefly pumped under vacuum. This aqueous solution is then chromatographed on an XAD-2 resin column (60 ml bed). The product is eluted in 10% aqueous tetrahydrofuran (following water elution) to give the captioned product.

## EXAMPLE 37

Preparation of the N'-Tert-Butyl-N-Formimidoyl derivative of 3-(2-aminoethylthio)-6-methyl-6-(hydroxymethyl)-7-oxo-1-azabicyclo[3.2.0]hept-2-ene-2-carboxylic acid

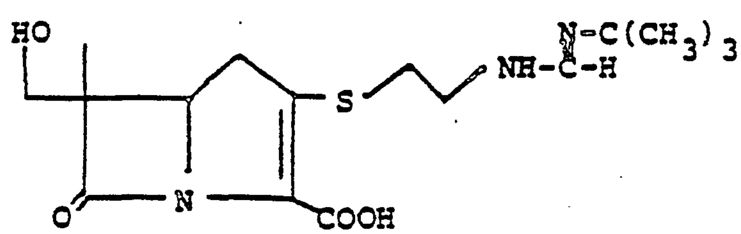

Compound 9 from Example 12, Step K (105 mg) is dissolved in pH 7 0.1N phosphate buffer (5 ml) and to

this is added a solution of ethyl N-*tert*-butyl formimidate (290 mg) in tetrahydrofuran (1 ml). The pH of the solution is adjusted to and maintained at 8.5 using an autoburette dispensing 1N NaOH. After 30 minutes, the pH is adjusted to 7.0 with 2.5N HCl. The solution is chromatographed on an ice water jacketed column of Dowex 50-X4 resin (53 cc, $Na^+$ cycle, 200-400 mesh) and eluted with deionized water. The fractions containing the title product are combined and lyophilized.

## EXAMPLE 38

Preparation of 8-oxo-2,2-dimethyl-7-ethyl-3-oxa-1-azabicyclo[4.2.0]octane

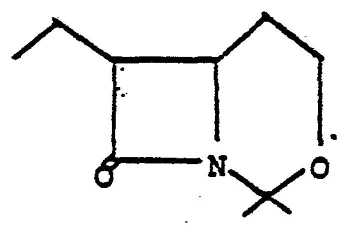

Following the procedure described for the preparation of 8-oxo-3-oxa-2,2-dimethyl-7α-isopropyl-1-azabicyclo[4.2.0] octane from 8-oxo-3-oxa-2,2-dimethyl-1-azabicyclo[4.2.0] octane (Example 4a, above) and using ethyl iodide instead of isopropyl iodide there is obtained 8-oxo-2,2-dimethyl-7-ethyl-3-oxa-1-azabicyclo[4.2.0]octane

## EXAMPLE 39

Preparation of 3-(2-aminoethylthio)-6-ethyl-7-oxo-1-azabicyclo[3.2.0]hept-2-ene-2-carboxylic acid

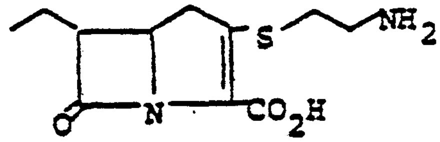

Following the procedures described in Example 7 followed by Example 12 - Steps A-K except that in Example 7 an equivalent amount of 8-oxo-2,2-dimethyl-7-ethyl-3-oxa-1-azabicyclo[4.2.0]octane rather than the 2,2,7-trimethyl species is taken, the title compound is obtained.

EXAMPLE 40

Preparation of the N-formimidoyl derivative of 3-(2-aminoethylthio)-6-ethyl-7-oxo-1-azabicyclo[3.2.0]hept-2-ene-2-carboxylic acid

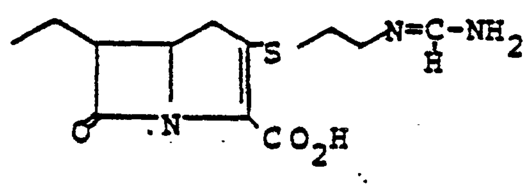

Following the procedure described in Example 33 except that the equivalent amount of 3-(2-aminoethylthio)-6-ethyl-7-oxo-1-azabicyclo[3.2.0]hept-2-ene-2-carboxylic acid rather than Compound 9 is taken, the title compound is obtained.

EXAMPLE 41

Preparation of 3-ethyl-1-(t-butyldimethylsilyl)-4-vinyl-2-azetidinone

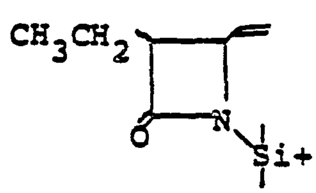

Following the procedure for the preparation of 8-oxo-2,2-dimethyl-7c-isopropyl-3-oxa-1-azabicyclo[4.2.0]octane (Example 4a, above), except that an equivalent amount of 1-(t-butyl-dimethylsilyl)-4-vinyl-2-azetidinone is substituted for the 8-oxo-2,2-dimethyl-3-oxa-1-azabicyclo [4.2.0]octane of Example 4a and using ethyl iodide instead of isopropyl iodide, the title compound is obtained.

## EXAMPLE 42

Preparation of 3-ethyl-4-[2-(2-p-nitrobenzyloxycarbonyl-amino)ethylthio)vinyl]-2-azetidinone

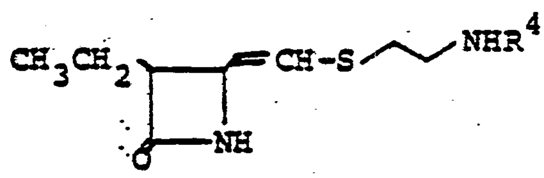

Following the procedures in Example 13 - Steps D-F except that an equivalent amount of 3-ethyl-1-(t-butyl-dimethylsilyl)-4-vinyl-2-azetidinone is used in place of Compound 26 of Example 13 - Step D, the title compound is obtained.

EXAMPLE 43

Following the foregoing Examples and text, the following additionally preferred antibiotic compounds of the present invention are prepared in further representative demonstration of the disclosed process (Table II). The column labelled "Remarks and Reagents" annotates the established procedure where necessary to obtain the indicated compound. In most instances the compounds are deblocked according to the previously described procedure. However, when the $SR^8$ side chain does not contain a basic function, the final product I is more conveniently isolated as the sodium salt (M=Na); which result is facilitated by conducting the deblocking in a slight excess of $NaHCO_3$. In any event, when either $R^6$ or $R^7$ bears a basic group, the final product I is most conveniently isolated as the free acid (M=H), rather than the sodium salt. It should be noted that compounds designated as "free acids" in reality are isolated as inner salts as a consequence of their zwitterionic nature.

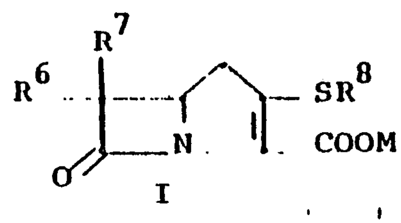

I

M = H, Na

Also entered in Table II, under the 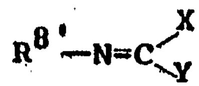 column, are certain preferred amidine and guanidine derivatives of I. Such derivatives are formed from compounds of Table II which carry an $R^8$ side chain bearing an amino group ($-NH_2$). As mentioned earlier such side chains may be symbolized by $R^{8'}-NH_2$ and thus such amidine and guanidine derivatives may be depicted generically by the structure:

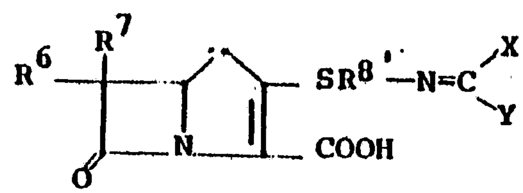

TABLE II

| Compound | $R^6$ | $R^7$ | $R^8$ | $R^{8'}N{=}C{<}^{X}_{Y}$ | Remarks, Reagents |
|---|---|---|---|---|---|
| 1.) | $(CH_3)_2CH$ | H | Ø | | As in Example 12, but substitute HSØ for $HSCH_2CH_2NHCO_2PNB$. Deblock as described in Example 12 and isolate product as Na salt. M=Na. |
| 2.) | $CH_3$ | H | $CH_2$Ø | | $HSCH_2$Ø; M=Na |
| 3a-d.) | $HOCH_2$ | $CH_3$ | $CH_2CH_2CH_2NH_2$ | $X=NH_2$<br>$Y=H, CH_3, NH_2$ | $HSCH_2CH_2CH_2NHCO_2PNB$; M=H. |
| 4a-d.) | Ø$CH_2CH(OH)$ | H<br>Ø=phenyl | $CH_2C(CH_3)_2NH_2$ | $X=NH_2$<br>$Y=H, CH_3, NH_2$ | $HSCH_2C(CH_3)_2NHCO_2PNB$; M=H. |

| Compound | $R^6$ | $R^7$ | $R^8$ | $R^{8'}N{=}C{<}^X_Y$ | Remarks, Reagents |
|---|---|---|---|---|---|
| 5a-d.) | $CH_3CH(OH)$ | $CH_3$ | $CH_2CH_2NH_2$ | $X{=}NH_2$<br>$Y{=}H$, $CH_3$, $NH_2$ | M = H |
| 6a-d.) | $CH_3CH(OH)$ | $CH_3CH(OH)$ | $CH_2CH_2NH_2$ | $X{=}NH_2$<br>$Y{=}H$, $CH_3$, $NH_2$ | M = H |
| 7.) | $CH_3CH(OH)$ | $\phi CH_2$ | $CH_2CH_2N(CH_3)_2$ | | $HSCH_2CH_2N(CH_3)_2$; M = H |
| 8a-d.) | $CH_3CH(NH_2)$ | H | $CH_2CH_2NH_2$ | $X{=}NH_2$<br>$Y{=}H$, $CH_3$, $NH_2$ | M = H |
| 9.) | $(CH_3)_2CHCH(OH)$ | H | $CH_2$ 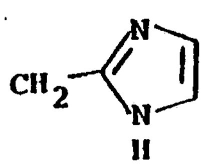 | | $HSCH_2$ 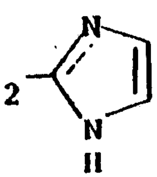 ; M = H |

| Compound | $R^6$ | $R^7$ | $R^8$ | $R^{8'}$ $N=C{<}^{X}_{Y}$ | Remarks, Reagents |
|---|---|---|---|---|---|
| 10.) | $(CH_3)_2CHCH_2CH_2\overset{OH}{CH}$ | H | 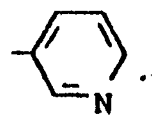 | | HS 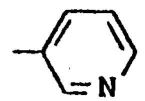 ; M = H |
| 11.) | OH<br>-CH | H | $CH_2$ 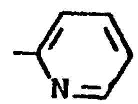 | | $HSCH_2$ 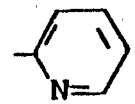 ; M = H |
| 12a-d.) | $CF_3\overset{OH}{CH}$ | H | $CH_2CH_2NH_2$ | $X=NH_2$<br>$Y=H,\ CH_3,\ NH_2$ | M = H |
| 13a-d.) | $HOCH_2\overset{OH}{CH}$ | H | $CH_2CH_2NH_2$ | $X=NH_2$<br>$Y=H,\ CH_3, NH_2$ | M = H |
| 14.) | $HOCH_2CH_2$ | H | $CH_2CH_2$- 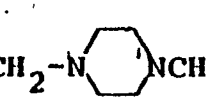 $CH_3$ | | $HSCH_2CH_2$- 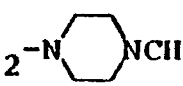 $CH_3$; M = H |

| Compound | $R^6$ | $R^7$ | $R^8$ | $R^{8'}N{=}C\begin{smallmatrix}X\\Y\end{smallmatrix}$ | Remarks, Reagents |
|---|---|---|---|---|---|
| 15.) | 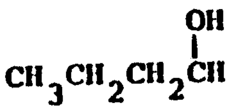 | H | 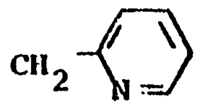 | | 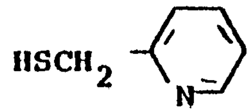 ; M = H |
| 16a-d.) | 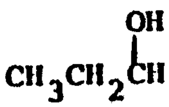 | H | 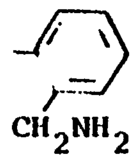 | X=$NH_2$<br>Y=H, $CH_3$, $NH_2$ | 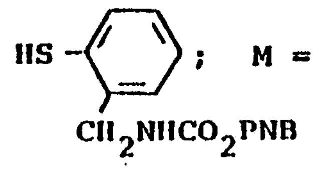 ; M = H |
| 17a-d.) | 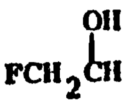 | H | $CH_2CH_2NH_2$ | X=$NH_2$<br>Y=H, $CH_3$, $NH_2$ | M = H |
| 18.) | 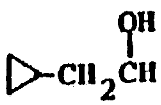 | H | $CH_2CH_2CO_2H$ | | $HSCH_2CH_2CO_2PNB$; Product isolated as disodium salt. |
| 19a-d.) | $CH_3CH_2$ | H | $CH_2CH_2NH_2$ | X=$NH_2$<br>Y=H, $CH_3$, $NH_2$ | M = H |

| Compound | $R^6$ | $R^7$ | $R^8$ | 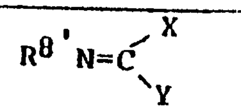 | Remarks, Reagents |
|---|---|---|---|---|---|
| 20.) | $CH_3$ | $CH_3$ | 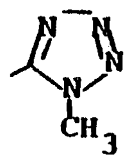 | | 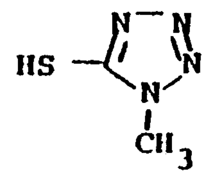 ; M = Na |
| 21.) | 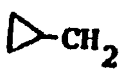 | H | $CH_2CH_2OH$ | | $HSCH_2CH_2OCO_2PNB$<br>M = Na |
| 22a-d.) | $HOCH_2CH_2$ | $CH_3$ | $CH_2CH_2CH_2CH_2NH_2$ | X=$NH_2$<br>Y=H, $CH_3$, $NH_2$ | $HSCH_2CH_2CH_2CH_2CO_2PNB$;<br>M = H |
| 23a-d.) | 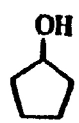 | H | $CH_2C(CH_3)_2CH_2NH_2$ | X=$NH_2$<br>Y=H, $CH_3$, $NH_2$ | $HSCH_2C(CH_3)_2CH_2NHCO_2PNB$;<br>M = H |

| Compound | $R^6$ | $R^7$ | $R^8$ | $R^{8'}N{=}C\genfrac{}{}{0pt}{}{X}{Y}$ | Remarks, Reagents |
|---|---|---|---|---|---|
| 24a-d.) | 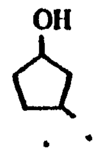 | H | $CH_2CH_2NH_2$ | $X{=}NH_2$<br>$Y{=}H,\ CH_3,\ NH_2$ | M = H |
| 25a-d.) | 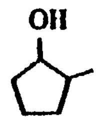 | H | $CH_2CH_2NH_2$ | $X{=}NH_2$<br>$Y{=}H,\ CH_3,\ NH_2$ | M = H |
| 26.) | $CH_3\overset{OH}{\overset{\vert}{C}}H$ | H | 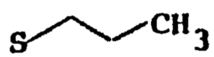 | | 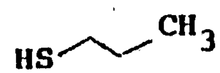 ; M=Na |
| 27.) | " | H | 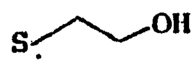 | | 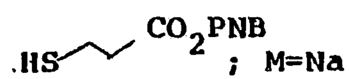 ; M=Na |
| 28a-d.) | " | H | 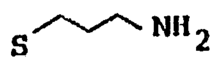 | $X{=}NH_2$<br>$Y{=}H,\ CH_3,\ NH_2$ | 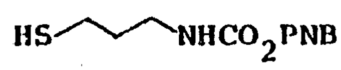 ; M=H |
| 29.) | " | H | 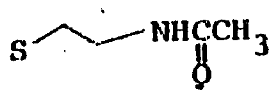 | | 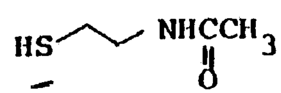 ; M=Na |

| Compound | $R^6$ | $R^7$ | $R^8$ | 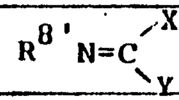 | Remarks, Reagents |
|---|---|---|---|---|---|
| 30a-d.) | $CH_3\overset{OH}{CH}$ | H | 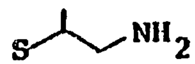 | $X=NH_2$<br>$Y=H, CH_3, NH_2$ | 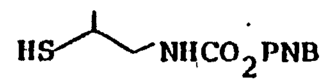; M=H |
| 31a-d.) | " | H | 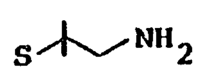 | $X=NH_2$<br>$Y=H, CH_3, NH_2$ | 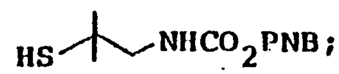; M=H |
| 32a-d.) | " | H | 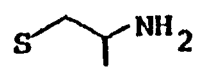 | $X=NH_2$<br>$Y=H, CH_3, NH_2$ | 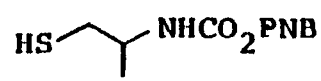; M=H |
| 33a-d.) | " | H | 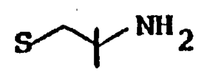 | $X=NH_2$<br>$Y=H, CH_3, NH_2$ | 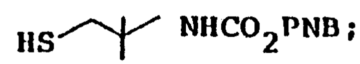; M=H |
| 34a-d.) | " | H | 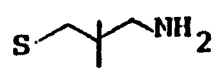 | $X=NH_2$<br>$Y=H, CH_3, NH_2$ | 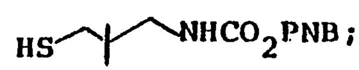; M=H |
| 35.) | " | H | 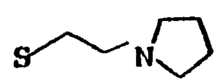 | | 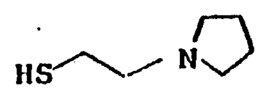; M=H |

| Compound | $R^6$ | $R^7$ | $R^8$ | $R^{8'}N{=}C\genfrac{}{}{0pt}{}{X}{Y}$ | Remarks, Reagents |
|---|---|---|---|---|---|
| 36.) | $CH_3\overset{OH}{CH}$ | H | 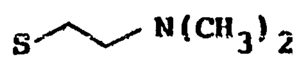 | | 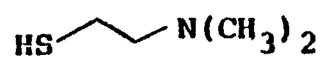 ; M=H |
| 37a-d.) | " | H | 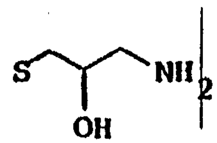 | X=$NH_2$<br>Y=H, $CH_3$, $NH_2$ | 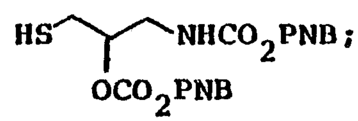 ; M=H |
| 38a-d.) | " | H | 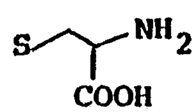 | X=$NH_2$<br>Y=H, $CH_3$, $NH_2$ | 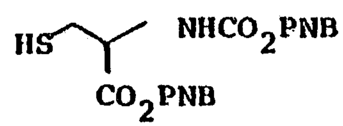 ; M=H |
| 39a-d.) | " | H | 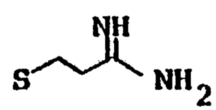 | X=$NH_2$<br>Y=H, $CH_3$, $NH_2$ | 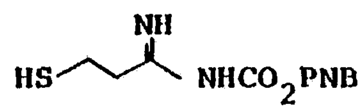 ; M=H |
| 40a-d.) | " | H | 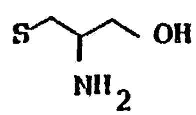 | X=$NH_2$<br>Y=H, $CH_3$, $NH_2$ | 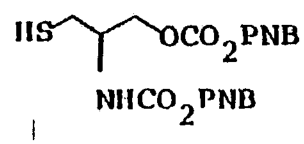 M = H |

| Compound | $R^6$ | $R^7$ | $R^8$ | $R^{8'}N=C{<}^{X}_{Y}$ | Remarks, Reagents |
|---|---|---|---|---|---|
| 41a-d.) | $CH_3CH(OH)$ | H | 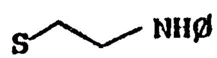 | $X=NH_2$<br>$Y=H,\ CH_3,\ NH_2$ | M=H |
| 42a-d.) | " | H | 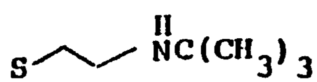 | $X=NH_2$<br>$Y=H,\ CH_3,\ NH_2$ | 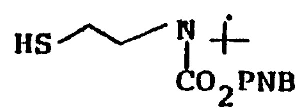 M=H. |
| 43.) | " | H | SØ | | M=Na |
| 44a-d.) | " | H | 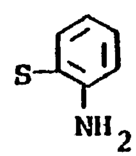 | $X=NH_2$<br>$Y=H,\ CH_3,\ NH_2$ | 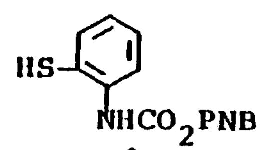 M=H |
| 45.) | " | H | 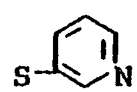 | | 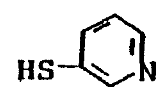 ; M=H |

| Compound | $R^6$ | $R^7$ | $R^8$ | $R^{8'}$ $N=C\genfrac{}{}{0pt}{}{X}{Y}$ | Remarks, Reagents |
|---|---|---|---|---|---|
| 46.) | 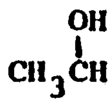 | H | 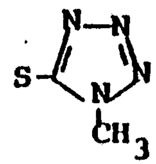 | | M=Na |
| 47.) | " | H | 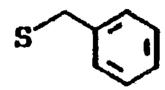 | | M=Na |
| 48.) | " | H | 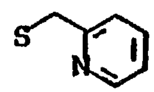 | | M=H |
| 49.) | " | H | 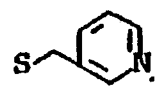 | | M=H |
| 50a-d.) | " | H | 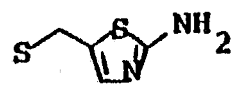 | X=$NH_2$<br>Y=H, $CH_3$, $NH_2$ | 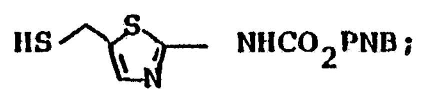 ; M=H |
| 51.) | " | H | 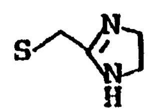 | | M=H |
| 52a-d.) | " | H | 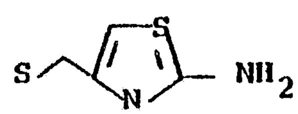 | X=$NH_2$<br>Y=H, $CH_3$, $NH_2$ | 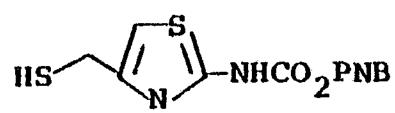<br>M = H |

| Compound | $R^6$ | $R^7$ | $R^8$ | $R^{8'}N=C\genfrac{}{}{0pt}{}{X}{Y}$ | Remarks, Reagents |
|---|---|---|---|---|---|
| 53.) | $CH_3\overset{OH}{CH}$ | H | 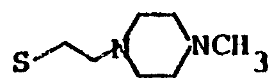 | | M=H |
| 54.) | " | H | 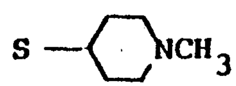 | | M=H |
| 55.) | " | H | 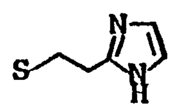 | | 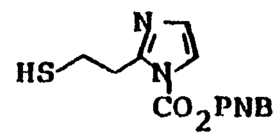 ; M=H |
| 56a-d.) | " | H | 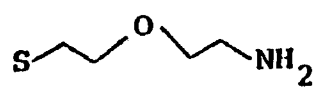 | X=$NH_2$<br>Y=H, $CH_3$, NH | 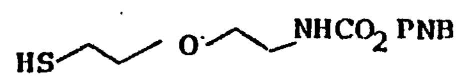<br>M=H |
| 57a-d.) | " | H | 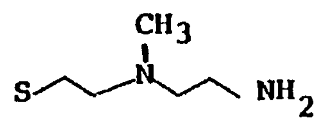 | X=$NH_2$<br>Y=H, $CH_3$, $NH_2$ | 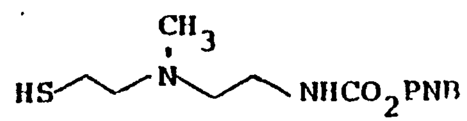<br>M=H |

| Compounds | |
|---|---|
| 58-89 | Compounds 58-89 correspond sequentially to compounds 26-57 (including listed amidine and guanidine derivatives, above), except that the value for $R^6$ is taken as $CH_3CH_2$ rather than the $CH_3C(OH)H$ of Compounds 26-57. |
| 90-121 | Compounds 90-121 correspond sequentially to compounds 26-57 (including listed amidine and guanidine derivatives, above), except that the value for $R^6$ is taken as $Cl_2CH\overset{OH}{C}H$ rather than the $CH_3C(OH)H$ of Compounds 26-57. |
| 122-153 | Compounds 122-153 correspond sequentially to compounds 26-57 (including listed amidine and guanidine derivatives, above), except that the value for $R^6$ is taken as $CF_3\overset{OH}{C}H$ rather than the $CH_3C(OH)H$ of Compounds 26-57. |
| 154-185 | Compounds 154-185 correspond sequentially to compounds 26-57 (including listed amidine and guanidine derivatives, above), except that the value for $R^6$ is taken as $HOCH_2\overset{OH}{C}H$ rather than the $CH_3C(OH)H$ of Compounds 26-57. |

| Compounds | |
|---|---|
| 186-217 | Compounds 186-217 correspond sequentially to compounds 26-57 (including listed amidine and guanidine derivatives, above), except that the value for $R^6$ is taken as $ClCH_2\overset{OH}{C}H$ rather than the $CH_3C(OH)H$ of Compounds 26-57. |
| 218-249 | Compounds 218-249 correspond sequentially to compounds 26-57 (including listed amidine and guanidine derivatives, above), except that the value for $R^6$ is taken as $CH_3CH_2\overset{OH}{C}H$ rather than the $CH_3C(OH)H$ of Compounds 26-57. |
| 250-281 | Compounds 250-281 correspond sequentially to compounds 26-57 (including listed amidine and guanidine derivatives, above), except that the value for $R^6$ is taken as 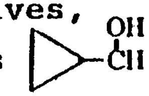 rather than the $CH_3C(OH)H$ of Compounds 26-57. |
| 282-313 | Compounds 282-313 correspond sequentially to compounds 26-57 (including listed amidine and guanidine derivatives, above), except that the value for $R^6$ is taken as $H_2NCH_2\overset{OH}{C}H$ rather than the $CH_3C(OH)H$ of Compounds 26-57. |

| Compounds | |
|---|---|
| 314-345 | Compounds 314-345 correspond sequentially to compounds 26-57 (including listed amidine and guanidine derivatives, above), except that the value for $R^6$ is taken as $CF_2H\overset{OH}{C}H$ rather than the $CH_3C(OH)H$ of Compounds 26-57. |
| 346-377 | Compounds 346-377 correspond sequentially to compounds 26-57 (including listed amidine and guanidine derivatives, above), except that the value for $R^6$ is taken as $HOCH_2$ rather than the $CH_3C(OH)H$ of Compounds 26-57. |
| 378-409 | Compounds 378-409 correspond sequentially to compounds 26-57 (including listed amidine and guanidine derivatives, above), except that the value for $R^6$ is taken as $HO_2CCH_2$ rather than the $CH_3C(OH)H$ of Compounds 26-57. |
| 410-441 | Compounds 410-441 correspond sequentially to compounds 26-57 (including listed amidine and guanidine derivatives, above), except that the value for $R^6$ is taken as $CH_3OCH_2\overset{OH}{C}H$ rather than the $CH_3C(OH)H$ of Compounds 26-57. |
| 442-473 | Compounds 442-473 correspond sequentially to compounds 26-57 (including listed amidine and guanidine derivatives, above), except that the value for $R^6$ is taken as $(CH_3)_3CCH_2\overset{OH}{C}H$ rather than the $CH_3C(OH)H$ of Compounds 26-57. |

## EXAMPLE 44

### Preparation of Pharmaceutical Compositions

One such unit dosage form is prepared by mixing 120 mg of 3-(2-aminoethylthio)-6-(2-hydroxyethyl)-7-oxo-1-azabicyclo-[3.2.0]hept-2-ene-2-carboxylic acid with 20 mg of lactose and 5 mg of magnesium stearate and placing the 145 mg mixture into a No. 3 gelatin capsule. Simiarly, by employing more of the active ingredient and less lactose, other dosage forms can be put up in No. 3 gelatin capsules, and, should it be necessary to mix more than 145 mg of ingredients together, larger capsules such as compressed tablets and pills can be prepared. The following examples are illustrative of the preparation of pharmaceutical formulations:

| TABLET | PER TABLET |
|---|---|
| 3-(2-aminoethylthio)-6-(2-hydroxyethyl)-7-oxo-1-azabicyclo[3·2·0]hept-2-ene-2-carboxylic acid | 125 mg. |
| Cornstarch, U.S.P. | 6 mg. |
| Dicalcium Phosphate | 192 mg. |
| Lactose, U.S.P. | 190 mg. |
| Magnesium Stearate | Balance/800 mg. |

The active ingredient is blended with the dicalcium phosphate, lactose and about half of the cornstarch. The mixture is then granulated with 15% cornstarch paste (6 mg) and rough-screened. It is dried at 45°C and screened again through No. 16 screens. The balance of the cornstarch and magnesium stearate is added and the mixture is compressed into tablets, approximately 0.5 inch in diameter each weighing 800 mg.

| PARENTERAL SOLUTION | PER TABLET |
|---|---|
| Ampoule: | |
| 3-(2-aminoethylthio)-6-(2-hydroxyethyl)-7-oxo-1-azabicyclo[3·2·0]hept-2-ene-2-carboxylic acid | 500 mg. |
| Diluent: Sterile Water for Injection | 2 cc. |

| OPTHALMIC SOLUTION | |
|---|---|
| 3-(2-aminoethylthio)-6-(2-hydroxyetnyl)-7-oxo-1-azabicyclo[3·2·0]hept-2-ene-2-carboxylic acid | 100 mg. |
| Hydropropylmethyl Cellulose | 5 mg. |
| Sterile Water | to 1 ml. |

| OTIC SOLUTION | |
|---|---|
| 3-(2-aminoethylthio)-6-2-hydroxyethyl)--7-oxo-1-azabicyclo[3·2·0]hept-2-ene-2-carboxylic acid | 100 mg. |
| Benzalkonium chloride | 0.1 mg. |
| Sterile Water | to 1 ml. |

| TOPICAL OINTMENT | |
|---|---|
| 3-(2-aminoethylthio)-6-(2-hydroxyethyl)--7-oxo-1-azabicyclo[3·2·0]hept-2-ene-2-carboxylic acid | 100 mg. |
| Polyethylene Glycol 4000 U.S.P. | 400 mg. |
| Polyethylene Glycol 400 U.S.P. | 1.0 gram |

WHAT IS CLAIMED IS:

1. A compound having the structural formula:

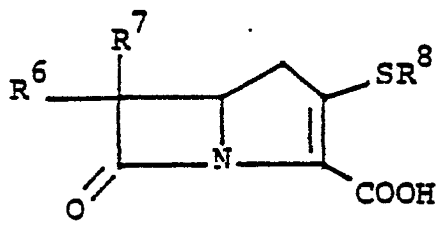

and pharmaceutically acceptable salt, ester and amide derivatives thereof; wherein $R^6$, $R^7$ and $R^8$ are independently selected from the group consisting of: hydrogen; substituted and unsubstituted: alkyl, alkenyl, and alkynyl, having from 1-10 carbon atoms; cycloalkyl, cycloalkylalkyl, and alkylcycloalkyl, having 3-6 carbon atoms in the cycloalkyl ring and 1-6 carbon atoms in the alkyl moieties; phenyl, aralkyl, aralkenyl, and aralkynyl wherein the aryl moiety is phenyl and the aliphatic portion has 1-6 carbon atoms; heteroaryl, heteroaralkyl, heterocyclyl and heterocyclylalkyl; wherein the substituent or substituents relative to the above-named radicals are selected from the group consisting of: chloro, bromo, fluoro, $R^1$,

$-OH$

$-OR^1$

$-O\overset{\overset{O}{\|}}{C}NR^1R^2$

$-\overset{\overset{O}{\|}}{C}NR^1R^2$

$-NR^1R^2$

$-NH_2$

$-NHR^1$

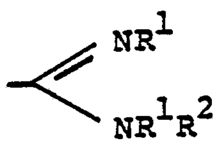

$-SO_2NR^1R^2$

$-NH\overset{\overset{\displaystyle O}{\|}}{C}NR^1R^2$

$-R^2N\overset{\overset{\displaystyle O}{\|}}{C}R^1$

$-CO_2H$

$-CO_2R^1$

$-\overset{\overset{\displaystyle O}{\|}}{C}R^1$

$-O\overset{\overset{\displaystyle O}{\|}}{C}R^1$

$-SH$

$-\overset{\overset{\displaystyle O}{\|}}{S}R^1$

$-\underset{\underset{\displaystyle O}{\|}}{\overset{\overset{\displaystyle O}{\|}}{S}}R^1$

$-CN$

$-N_3$

$-NO_2$

$-\overset{\oplus}{N}(R^1)_3$

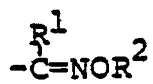

$-SR^1$ ;

wherein, relative to the above listed substituents on $R^6$, $R^7$ and $R^8$, the groups $R^1$ and $R^2$ are independently selected from: hydrogen, alkyl, alkenyl, and alkynyl, having from 1-10 carbon atoms; cycloalkyl, cycloalkylalkyl, and alkylcycloalkyl, having 3-6 carbon atoms in the cycloalkyl ring and 1-6 carbon atoms in the alkyl moieties; phenyl; aralkyl, aralkenyl, and aralkynyl wherein the aryl moiety is phenyl and the aliphatic portion has 1-6 carbon atoms; heteroaryl, heteroaralkyl, heterocyclyl and heterocyclylalkyl; and wherein the hetero atom or atoms in the above-named heterocyclic moieties are selected from the group consisting of 1-4 oxygen, nitrogen or sulphur atoms and wherein the alkyl moieties associated with said heterocyclic moieties have 1-6 carbon atoms;

$R^8$ is selected from:

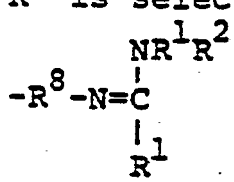

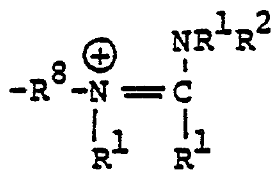

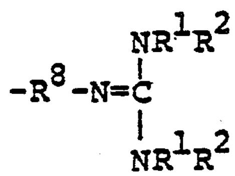

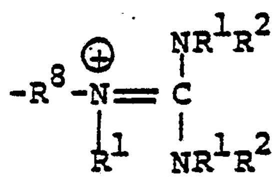

wherein: $R^8$, $R^1$ and $R^2$ are as defined; when $R^6/R^7$ is hydrogen $R^7/R^6$ is not: hydrogen; $CH_3CH_2$; or $CH_3\overset{O}{\overset{\|}{C}}-$ when $R^8$ is $-SCH_2CH_2NH_2$; or when $R^6/R^7$ is hydrogen $R^7/R^6$ is not $CH_3CH(OH)$, or O-derivative thereof, when $R^8$ is $-SCH_2CH_2NH_2$ or N-derivative thereof.

2. A compound according to Claim 1, wherein $R^7$ is selected from H, $OCH_3$ and $CH_3$.

3. A compound according to Claim 2 wherein $R^6$ is selected from the group consisting of: substituted and unsubstituted: alkyl, alkenyl and cycloalkylalkyl wherein the substituent or substituents are selected from hydroxyl, alkoxyl having from 1-6 carbon atoms, phenoxy, amino, and carboxy.

4. A compound according to Claims 2 or 3 wherein $R^6$ is selected from the group consisting of alkyl, cycloalkylalkyl, alkyl substituted by one or more hydroxyl groups, or cycloalkylalkyl substituted by one or more hydroxyl groups.

5. A compound according to Claims 2, 3 or 4 wherein $R^7$ is hydrogen.

6. A compound according to Claims 1, 2, 3, 4, or 5 wherein $R^6$ is selected from:

OH<br>|<br>$CH_3CH$

OH<br>|<br>$ØCH_2CH$ Ø = phenyl

OH<br>|<br>$ØCH_2CH_2CH$

OH<br>|<br>$CH_2$

$ØCH_2$

OH<br>|<br>$(CH_3)_2C$

$CH_2=CHCH_2$

$(CH_3)_2C=CHCH_2$

OH
|
$CH_3OCH_2CH$

$CH_3$

$CH_3CH_2$

$(CH_3)_2CH$

OH
|
$N_3CH_2CH$

OH
|
$(CH_3)_2NCH_2CH$

$HO_2CCH_2$

OH
|
$CF_3CF_2CH$

OH
|
$HO_2CCH_2CH$

OH
|
$CH_3CH(CH_3)CH$

OH
|
$(CH_3)_2CHCH_2CH$

OH
|
$CH_3CH_2CH$

$HOCH_2CH_2$

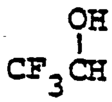

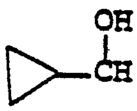

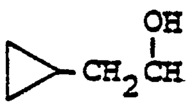

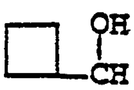

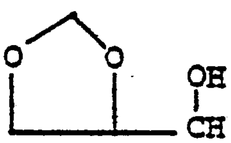

$HOCH_2CH_2CH_2$

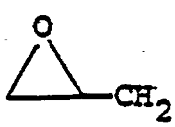

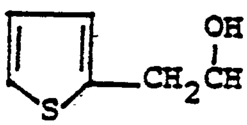

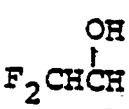

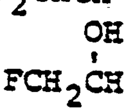

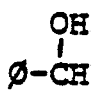

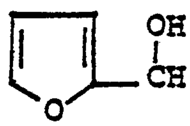

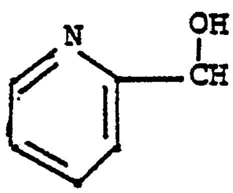

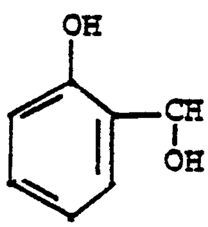

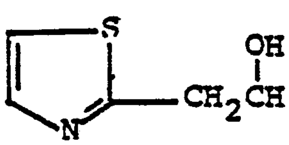

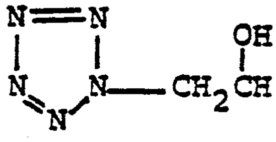

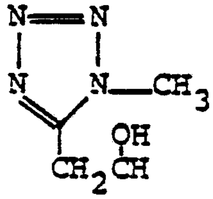

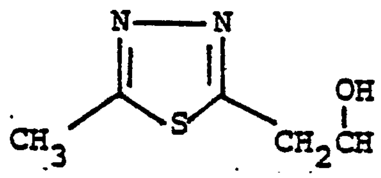

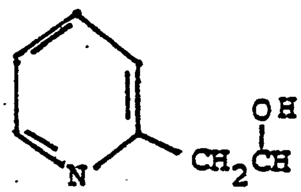

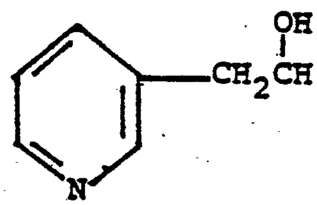

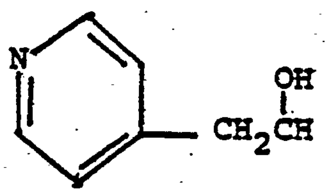

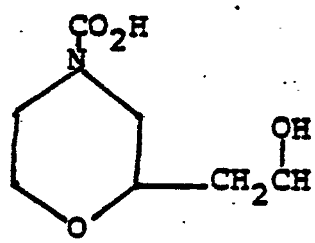

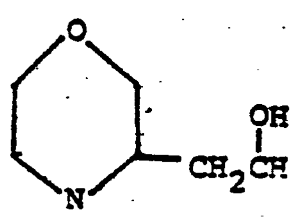

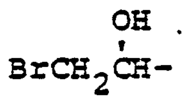

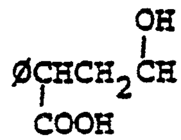

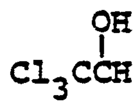

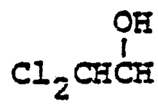

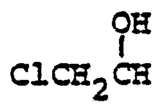

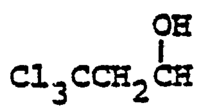

7. A compound according to Claims 1, 2, 3, 4, 5, or 6 wherein $R^6$ is:

$CH_3CH_2$

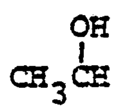

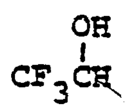

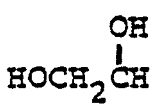

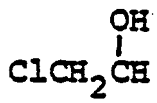

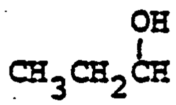

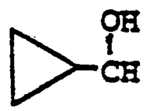

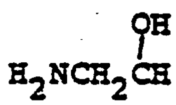

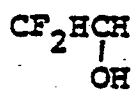

$HOCH_2$

$HO_2CCH_2$

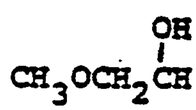

8. A compound according to Claims 1, 2, 3, 4, 5, 6 or 7 wherein $R^8$ is selected from:
1.) aliphatic (including carbocyclic) groups having 1-10 carbon atoms selected from: alkyl, cycloalkyl, alkenyl, cycloalkenyl and alkynyl;
2.) substituted aliphatic groups having 1-10 carbon atoms selected from: alkyl, cycloalkyl,

alkenyl, cycloalkenyl and alkynyl; wherein the substituents are selected from:

chloro, bromo, fluoro, $R^1$,

$-OH$

$-OR^1$

$-O\overset{O}{\overset{\|}{C}}NR^1R^2$

$-\overset{O}{\overset{\|}{C}}NR^1R^2$

$-NR^1R^2$

$-NH_2$

$-NHR^1$

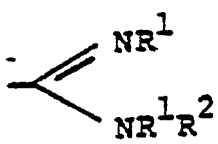

$-SO_2NR^1R^2$

$-NH\overset{O}{\overset{\|}{C}}NR^1R^2$

$-R^2N\overset{O}{\overset{\|}{C}}R^1$

$-CO_2H$

$-CO_2R^1$

$-\overset{O}{\overset{\|}{C}}R^1$

$-O\overset{O}{\overset{\|}{C}}R^1$

$-SH$

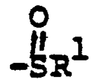

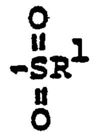

$-CN$

$-N_3$

$-NO_2$

$-\overset{\oplus}{N}(R^1)_3$

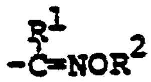

$-SR^1$

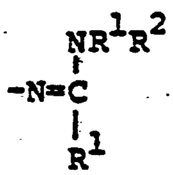

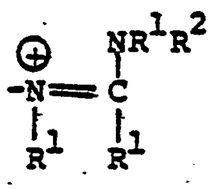

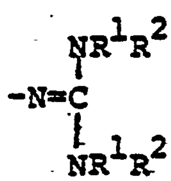

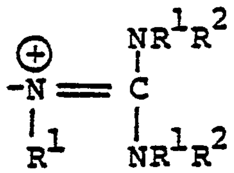 ;

3.) aryl and substituted aryl; wherein the aryl is phenyl and wherein the substituents are defined above under 2.);

4.) heteroaryl and substituted heteroaryl having 1-4 O, N or S atoms; and wherein the substituents are defined above under 2.);

5.) arylaliphatic, wherein aryl is phenyl, which are selected from the aliphatic groups defined under 1.) which are substituted by phenyl or substituted phenyl; wherein such substituents on phenyl are defined under 2.), above;

6.) heteroarylaliphatic and heterocyclylaliphatic; wherein the aliphatic moiety is defined under 1.), above; the substituted and unsubstituted heteroaryl and heterocyclic moieties have 1-4 O, N or S atoms; wherein such substituents are defined under 2.), above;

7.) substituted and unsubstituted alkyl-heteroatom-alkyl having 4-12 carbon atoms; wherein the heteroatom is selected from: O, S, NR° (R° is H, substituted or unsubstituted alkyl); wherein such substituents are defined under 2.), above.

9. A compound according to Claim 8, sub-paragraph 2.); wherein:

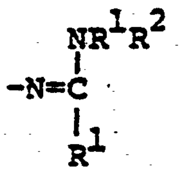 and 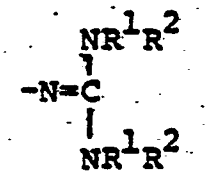

are selected from:

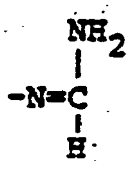 , 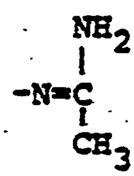 , and 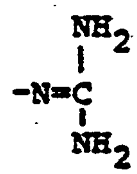 .

10. A compound according to Claim 8, sub-paragraph 3.); wherein:

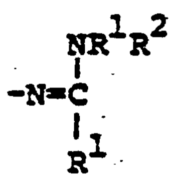 and 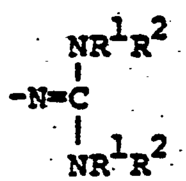

are selected from:

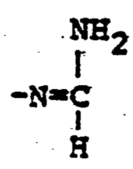 , 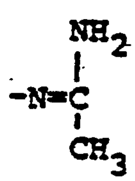 , and 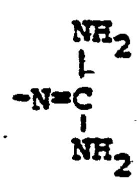 .

11. A compound according to Claim 8, sub-paragraph 4.); wherein:

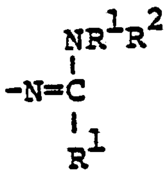

and

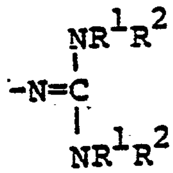

are selected from:

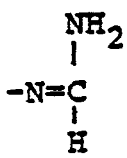

,

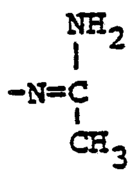

, and

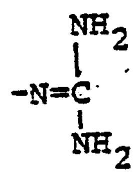

.

12. A compound according to Claim 8, sub-paragraph 5.); wherein:

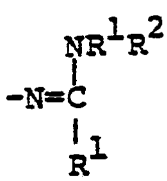

and

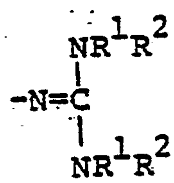

are selected from:

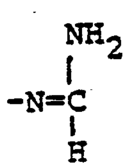

,

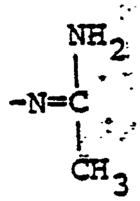

, and

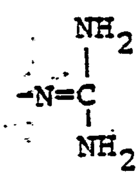

.

13. A compound according to Claim 8, subparagraph 6.); wherein:

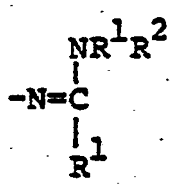 and 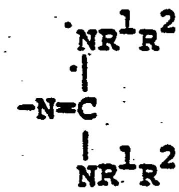

are selected from:

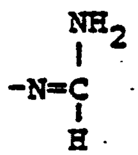 , 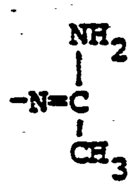 , and 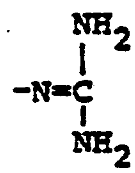 .

14. A compound according to Claim 8, subparagraph 7.); wherein:

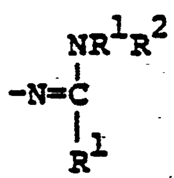 and 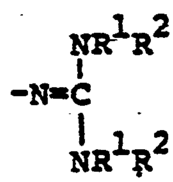

are selected from:

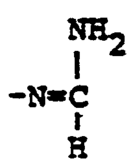 , 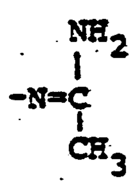 , and 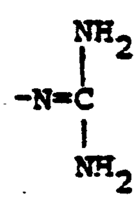 .

15. A compound according to Claim 8, sub-paragraph l.); wherein $R^8$ is selected from:

$-CH_3$

$-CH_2CH_3$

$-CH_2CH_2CH_3$

$-CH(CH_3)_2$

$-(CH_2)_3CH_3$

$-CH-CH_2CH_3$
$\;\;|$
$\;CH_3$

$-CH_2CH(CH_3)_2$

$\;\;\;\;CH_3$
$\;\;\;\;\;|$
$-\,C-CH_3$
$\;\;\;\;\;|$
$\;\;\;\;CH_3$

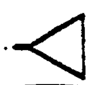

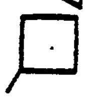

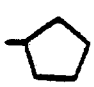

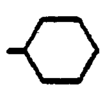

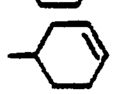

$-CH_2$ 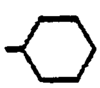

$-CH_2-CH=CH_2$

$-CH_2-CH=C(CH_3)_2$

$-CH_2-C\equiv CH$

$-CH_2-C\equiv C-CH_3$

16. A compound according to Claim 8, subparagraph 2.); wherein $R^8$ is selected from:

$-(CH_2)_nOR^1$ ; $n = 2-6$; $R^1 = H$, $\overset{O}{\overset{\|}{C}}CH_3$, $CH_3$

$-(CH_2)_n\overset{O}{\overset{\|}{C}}XR^1$; $n=1-6$; $X=O$, NH, $NR^1$, $R^1=H$, $CH_3$

$-(CH_2)_nNH_2$ ; $n=2-6$

$-(CH_2)_nNHR^1$ ; $n=2-6$; $R^1=CH_3$, $CH_2CH_3$, $CH_2CH_2CH_3$, $\overset{O}{\overset{\|}{C}}CH_3$

$-(CH_2)_nNR^1R^2$ ; $n=2-6$; $R^1/R^2=CH_3$, $CH_2CH_3$; $CH_3$,$CH_3$; $CH_2CH_3$,$CH_2CH_3$

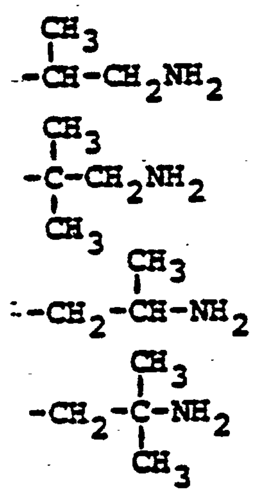

$-CH_2CH_2SCH_3$

$-CH_2CH_2NHC(CH_3)_3$

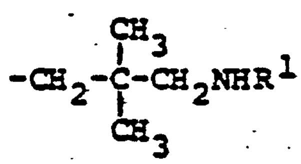 $R^1 = H$, $CH_3$, $\overset{O}{\overset{\|}{C}}CH_3$

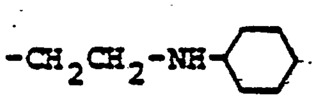

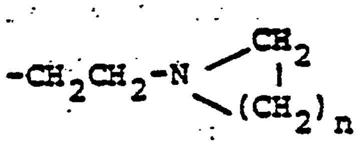

n=3-5

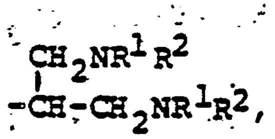

, $R^1$ and $R^2$ are independently chosen from H and $CH_3$

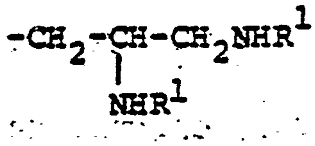

$R^1$=H, $CH_3$

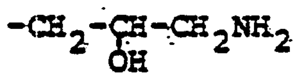

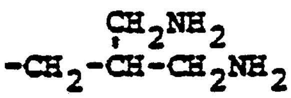

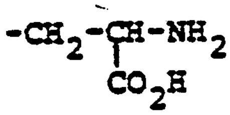

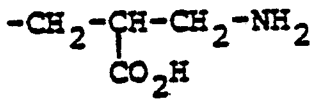

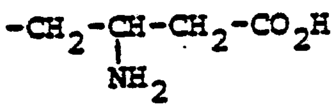

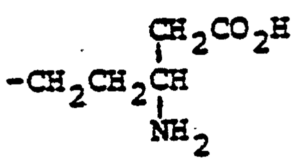

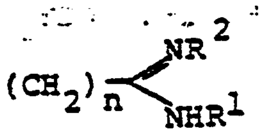

n=1, $R^2/R^1$=H,H; $CH_3$,H

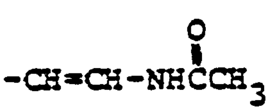

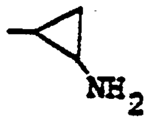

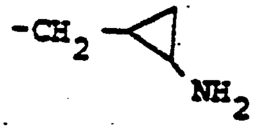

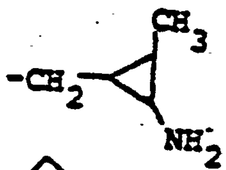

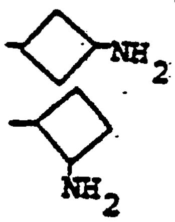

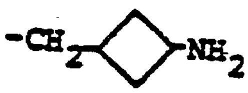

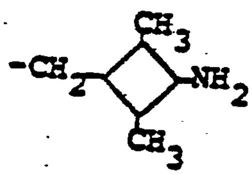

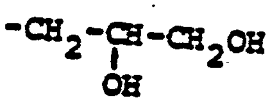

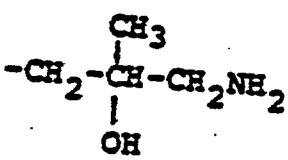

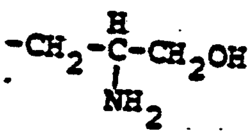

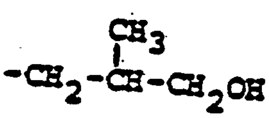

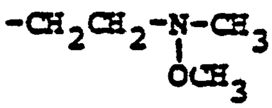

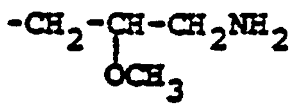

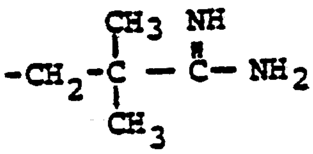

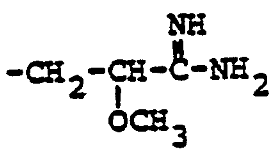

$-CH_2-CH=CH-CH_2NH_2$

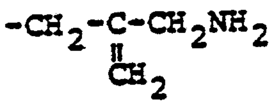

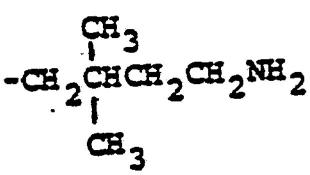

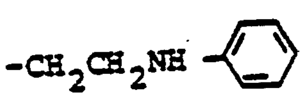

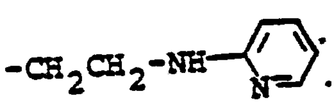

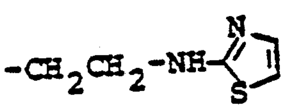

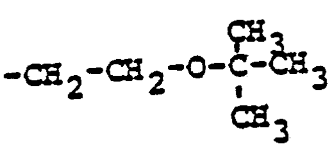

17. A compound according to Claim 8, subparagraph 2.); wherein $R^8$ is selected from:

$-(CH_2)_n-N=A$ n= 1-6

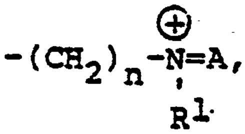 $R^1=CH_3$, $CH_2CH_3$, $C(CH_3)_3$, $(CH_2)_2CH_3$, 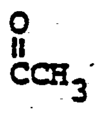 ; n= 1-6

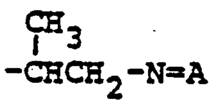

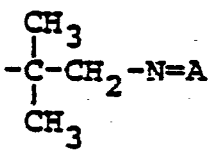

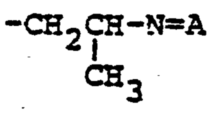

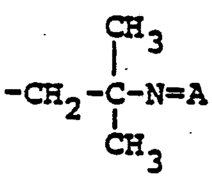

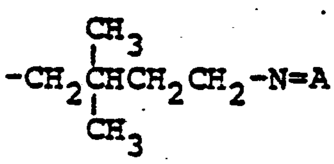

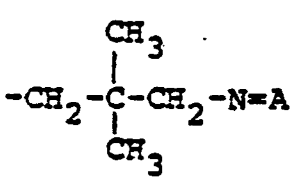

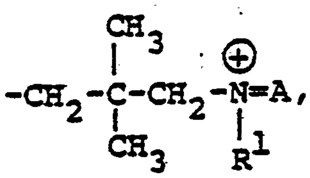, $R^1=CH_3$, $CH_2CH_3$, $C(CH_3)_3$, $(CH_2)_2CH_3$, 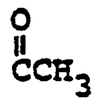

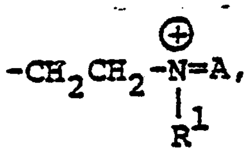, $R^1$ = 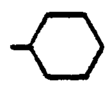, 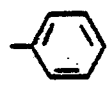, 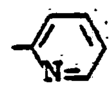, 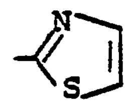

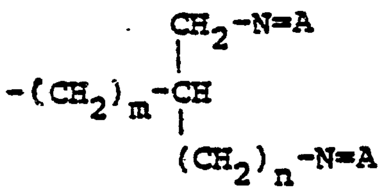, n=0, 1, 2; m=0, 1, 2

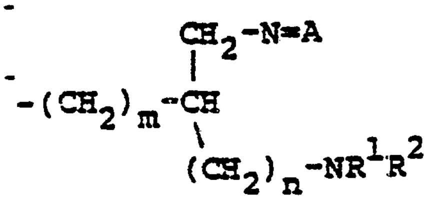, n=0, 1, 2; m=0, 1, 2
$R^1$ and $R^2$ = H,H; H,$CH_3$

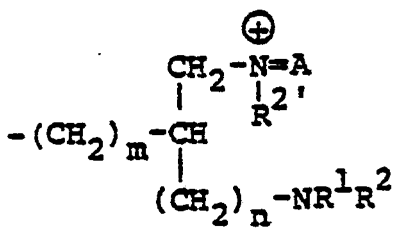, n=0, 1, 2; m=0, 1, 2
$R^1$ and $R^2$=H,H; H,$CH_3$;
$R^{2'}=CH_3$

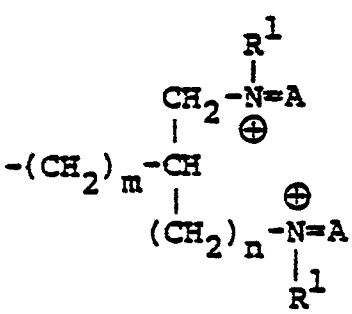, n=0, 1, 2; m=0, 1, 2
$R^1=CH_3$

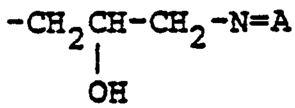

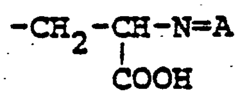

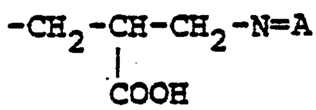

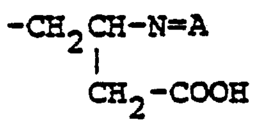

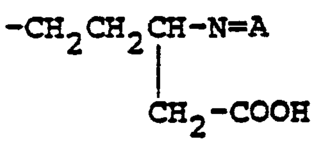

$-CH=CH-N=A$

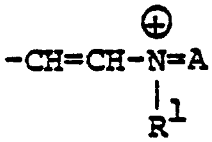 , $R^1=CH_3$, $CH_2CH_3$, $C(CH_3)_3$, $(CH_2)_3$, 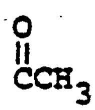

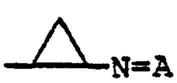

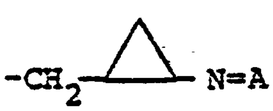

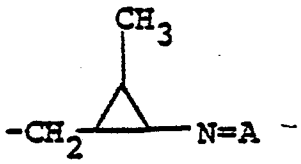

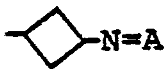

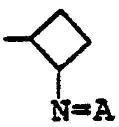

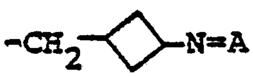

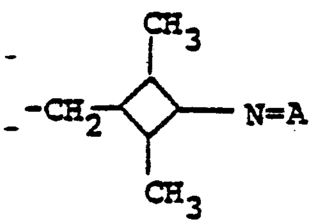

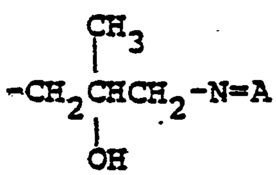

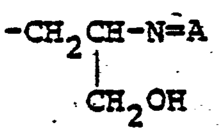

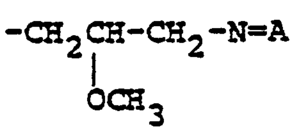

$-CH_2-CH=CH-CH_2-N=A$

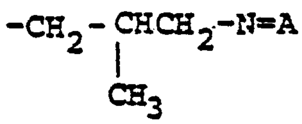

wherein A is selected from:

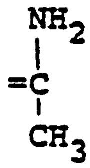 , 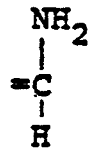 , and 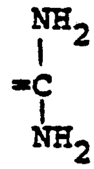 .

18. A compound according to Claim 8, subparagraph 3.); wherein $R^8$ is selected from:

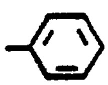

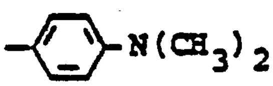

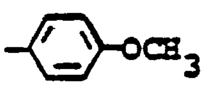

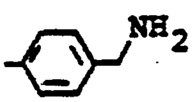

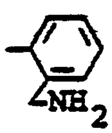

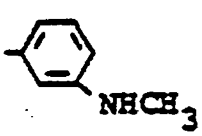

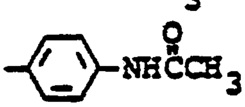

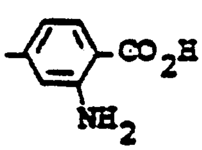

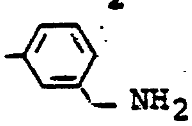

19. A compound according to Claim 8, subparagraph 3.); wherein $R^8$ is selected from:

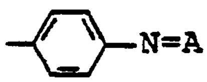

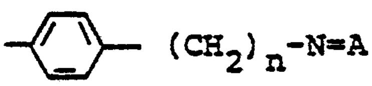 , $n = 1-4$

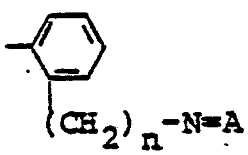 , $n = 1-4$

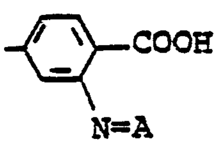

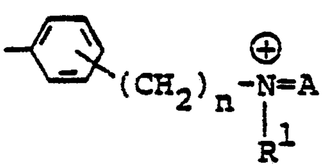 , $R^1 = CH_3$, $CH_2CH_3$, $CH_2CH_2CH_3$;

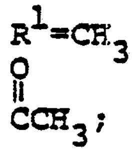 ; $n = 1-4$

wherein A is selected from:

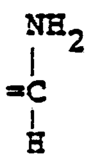 , 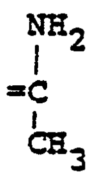 , 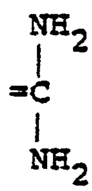 .

20. A compound according to Claim 8, subparagraph 4.), wherein $R^8$ is:

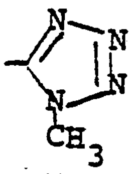

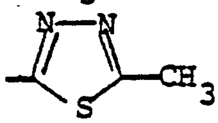

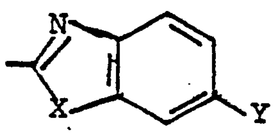

X=N,O Y=H
X=S Y=H, Cl, $OCH_2CH_3$

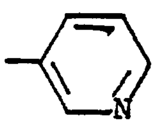

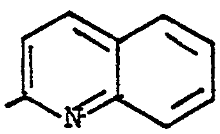

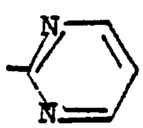

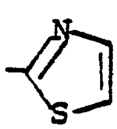

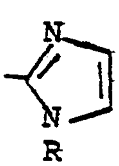

R=H, $CH_3$

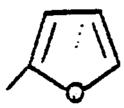

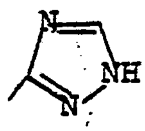

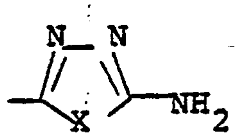

X=NH, S

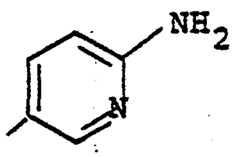

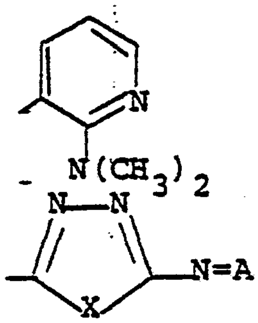

, X=NH, S

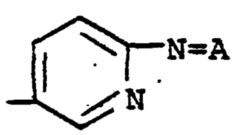

wherein A is selected from:

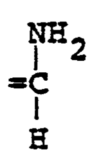

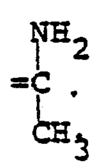

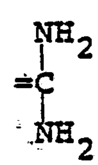

21. A compound according to Claim 8, subparagraph 5.); wherein $R^8$ is:

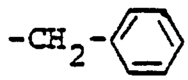

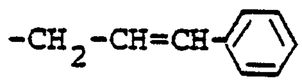

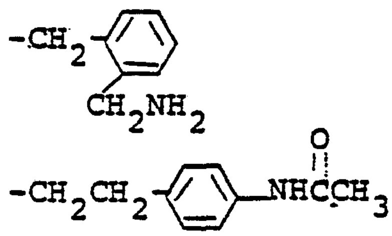

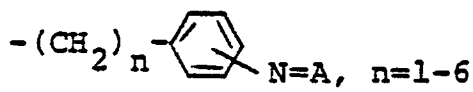

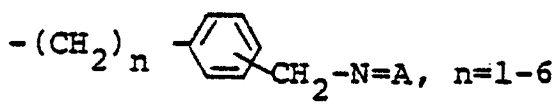

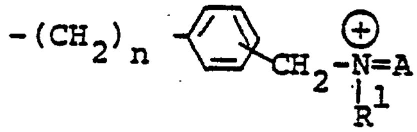

, $R^1=CH_3$, $CH_2CH_3$

$CH_2CH_3CH_3$, $C(CH_3)_3$,

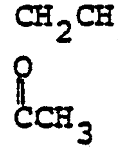

wherein A is selected from:

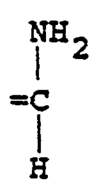

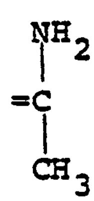

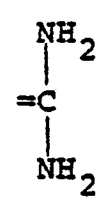

22. A compound according to Claim 8, subparagraph 6.); wherein $R^8$ is:

$-(CH_2)_n-$ 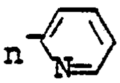

$-(CH_2)_n-$ 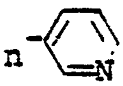

$-(CH_2)_n-$ 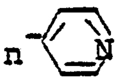

$-(CH_2)_n-$ 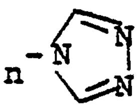

$-(CH_2)_n-$ 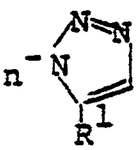 $R^1 = OCH_2CH_3$

$-(CH_2)_n-$ 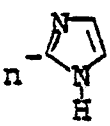

$-(CH_2)_n-$ 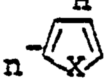

$-(CH_2)_n-$ 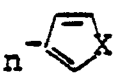 X = O, S, NH

$-(CH_2)_n-$ 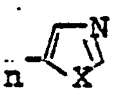 X = O, S, NH

$-(CH_2)_n-$ 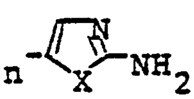 X = O, S, NH

$-(CH_2)_n-$ 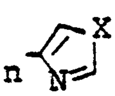 X = O, S, NH

$-(CH_2)_n-$ 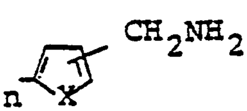 X = O, S, NH

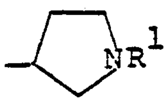 $R^1$=H, $CH_3$

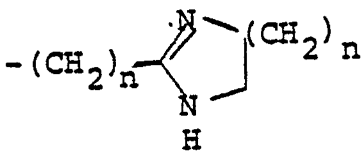 m = 1-3
n = 1-3

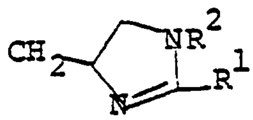 $R^2$=H, $CH_3$, $R^1$=H, $CH_3$, $NH_2$

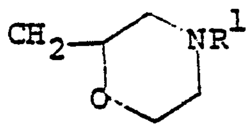 $R^1$= H, $CH_3$

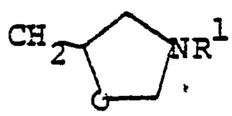 $R^1$=H, $CH_3$

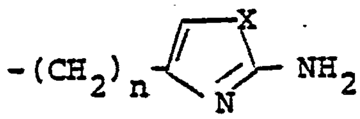

X = O, S, NH

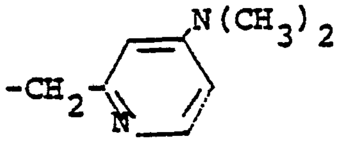

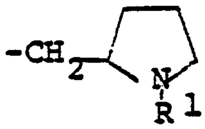

$R^1$ = H, $CH_3$

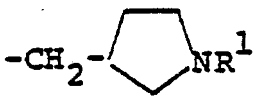

$R^1$ = H, $CH_3$

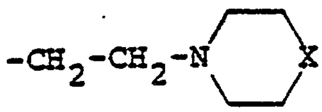

X = O, NH, $NCH_3$

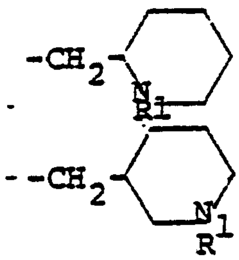

$R^1$ = H, $CH_3$

$R^1$ = H, $CH_3$

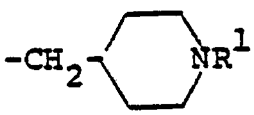

$R^1$ = H, $CH_3$

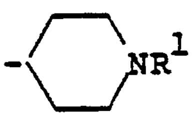

$R^1$ = H, $CH_3$

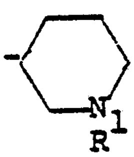

$R^1$ = H, $CH_3$

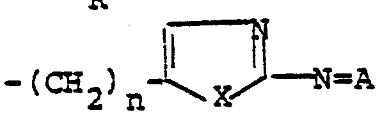 ,

X = O, S, NH

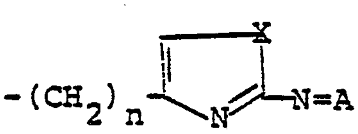 ,

X = O, S, NH

wherein: n = 1,2,3; and A is selected from:

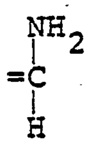

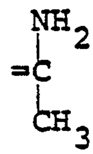

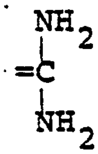

.

23. A compound according to Claim 8, subparagraph 7.); wherein $R^8$ is:

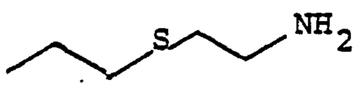

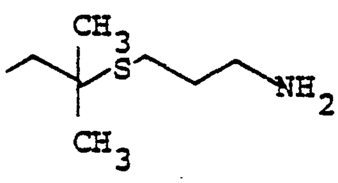

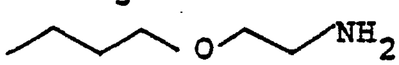

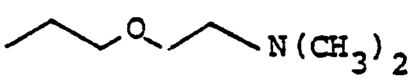

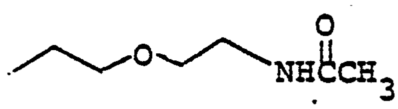

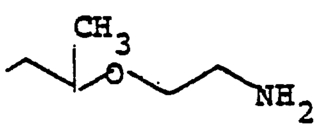

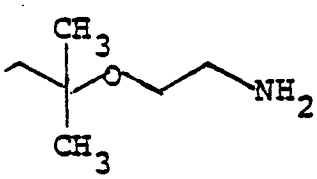

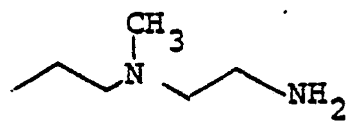

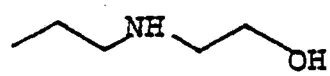

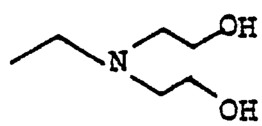

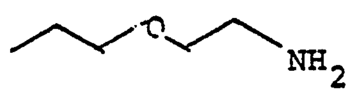

24. A compound according to Claim 8, subparagraph 7.); wherein $R^8$ is:

$CH_2CH_2SCH_2CH_2-N=A$

$CH_2C(CH_3)_2SCH_2CH_2-N=A$

$CH_2CH_2CH_2OCH_2CH_2-N=A$

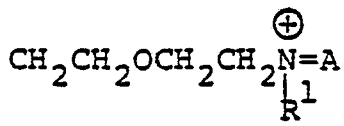 , $R^1 = CH_3$, $CH_2CH_3$, 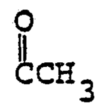

$CH_2CH(CH_3)OCH_2CH_2N=A$

$CH_2C(CH_3)_2OCH_2CH_2N=A$

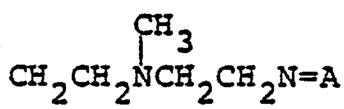

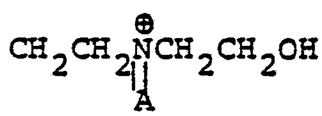

$CH_2CH_2OCH_2CH_2N=A$

wherein A is selected from:

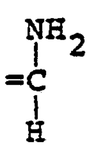 ; 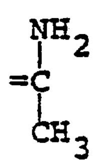 , 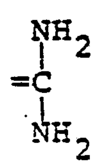 .

25. A compound according to Claims 15, 16, 17, 18, 19, 20, 21, 22, 23, or 24 wherein $R^7$ is hydrogen and $R^6$ is selected from the group consisting of $CH_3CH_2$,

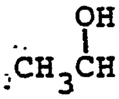 ,

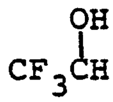 ,

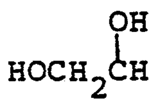 ,

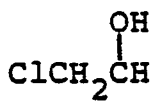 ,

$HOCH_2CH_2$ ,

$BrH_2CCH(OH)$ , $(CH_3)_3C-CH(OH)$ ,

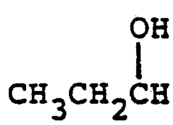 ,

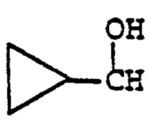 ,

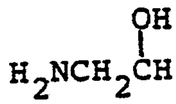 ,

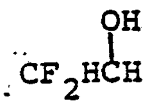 ,

$HOCH_2$ ,

$HO_2CCH_2$ and

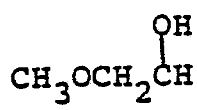 .

26. A compound according to Claim 1 selected from the group:

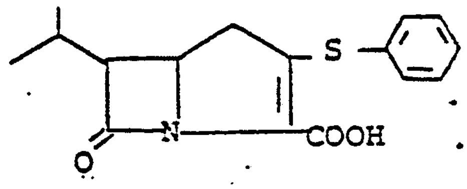

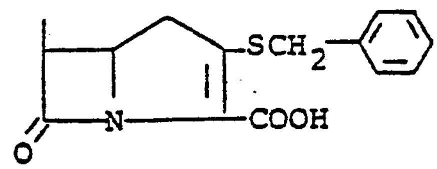

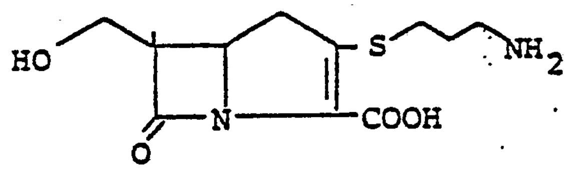

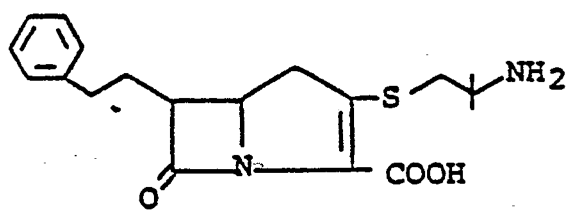

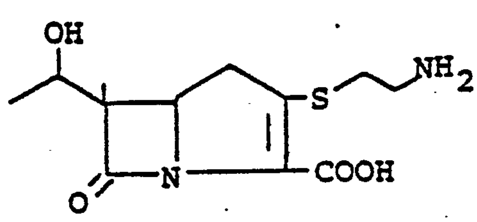

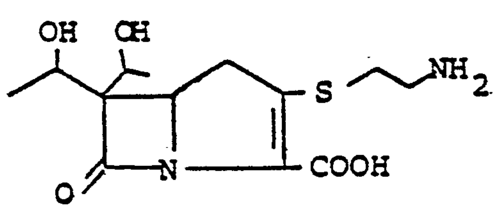

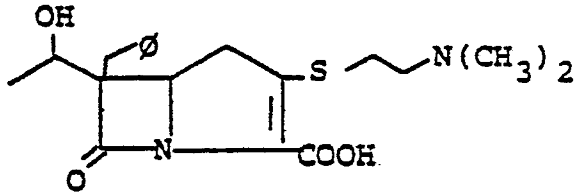

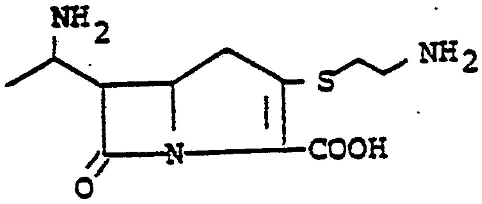

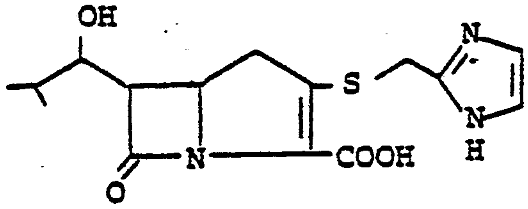

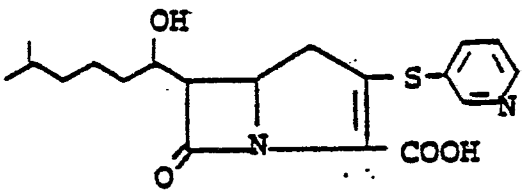

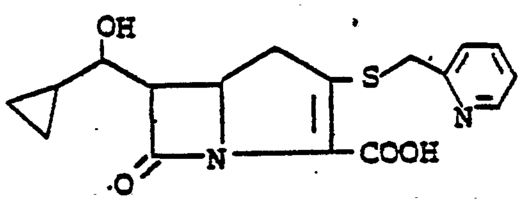

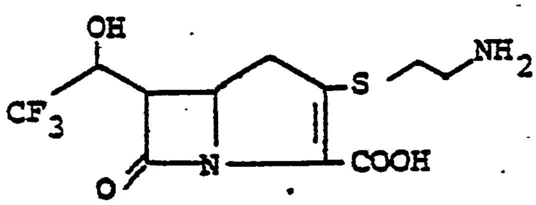

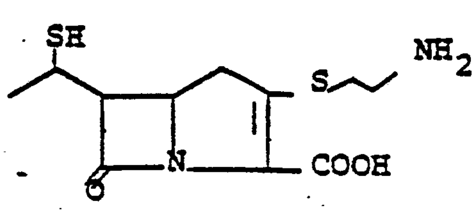

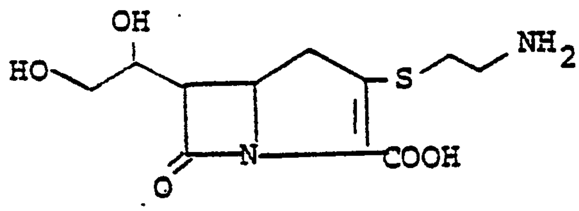

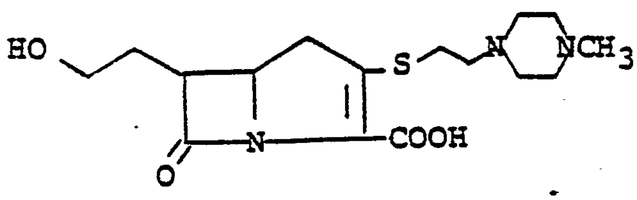

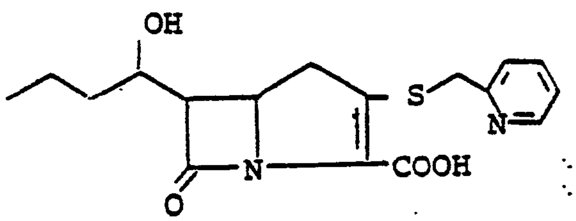

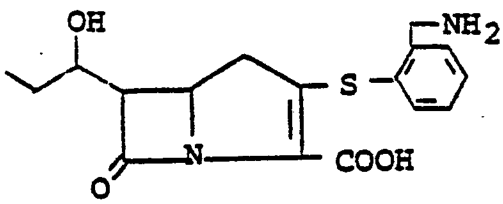

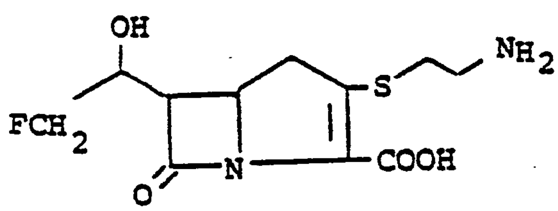

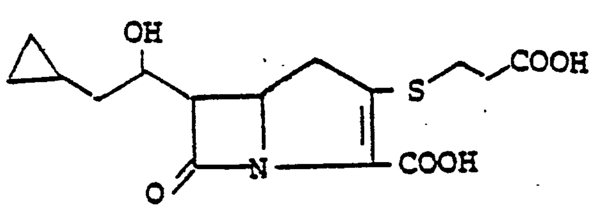

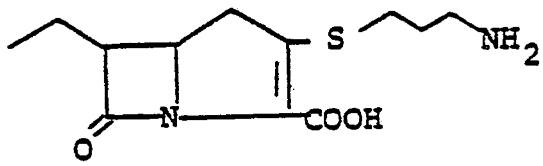

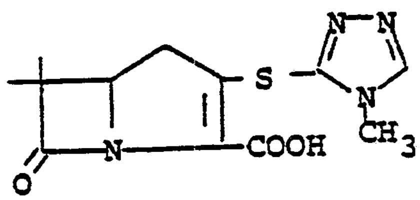

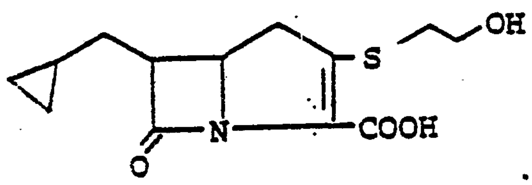

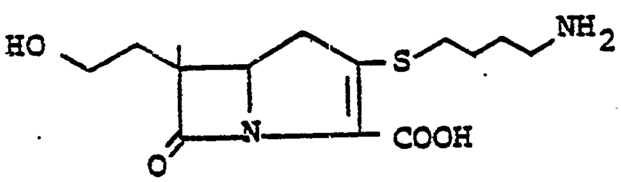

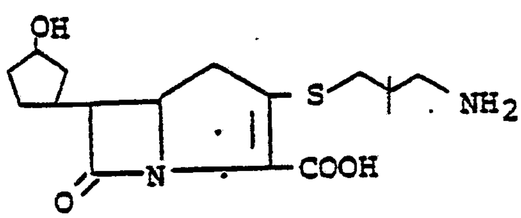

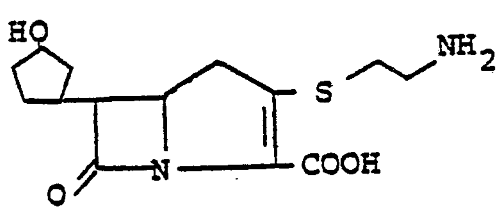

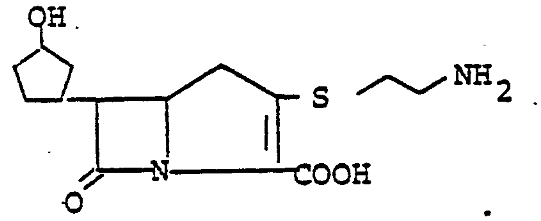

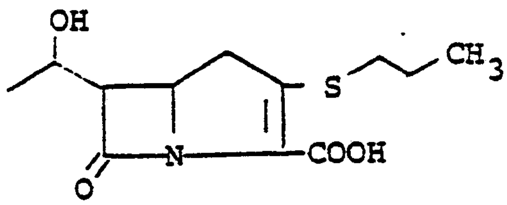

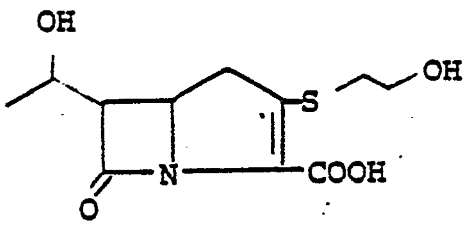

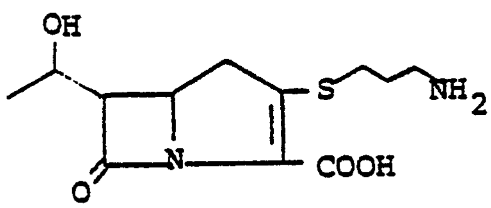

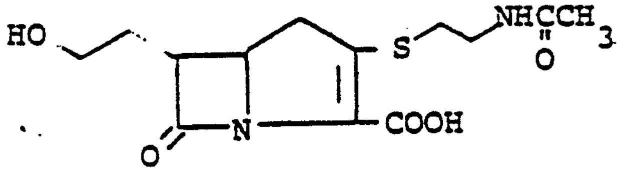

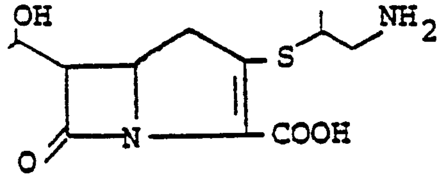

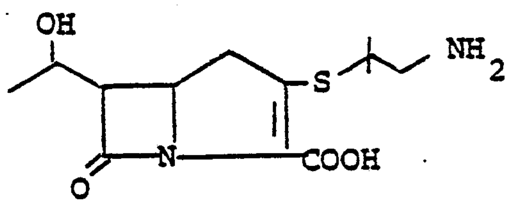

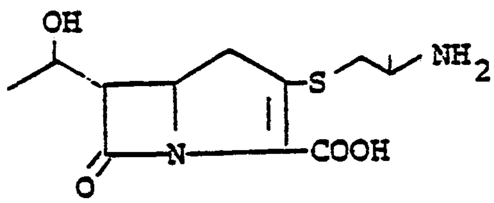

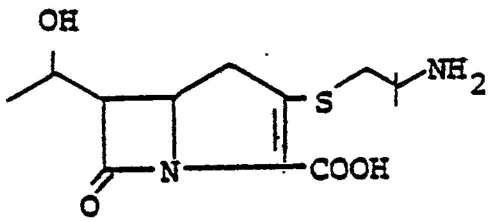

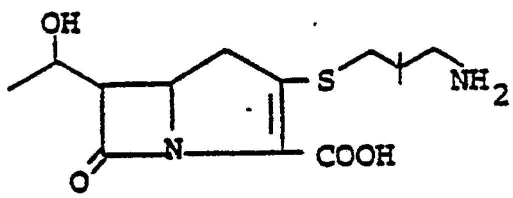

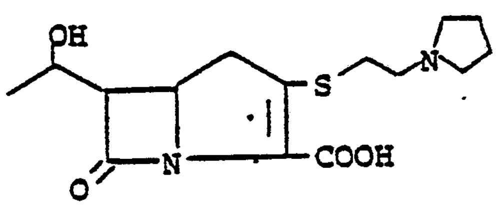

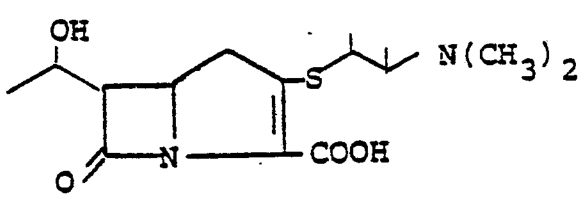

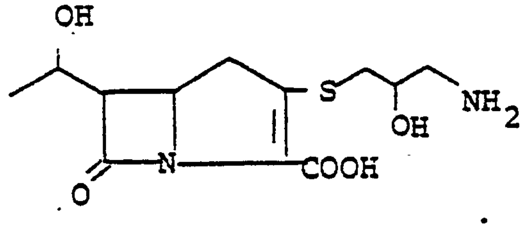

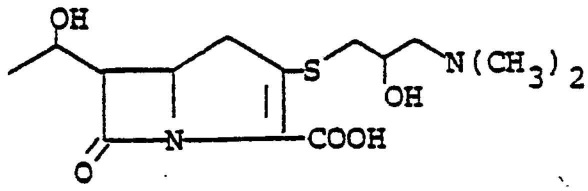

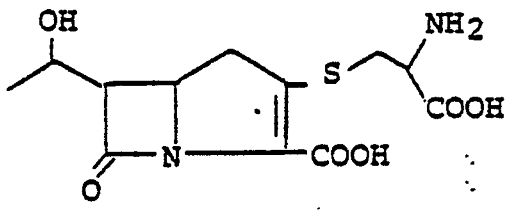

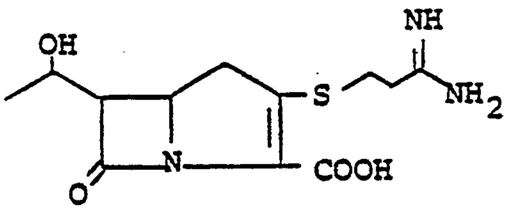

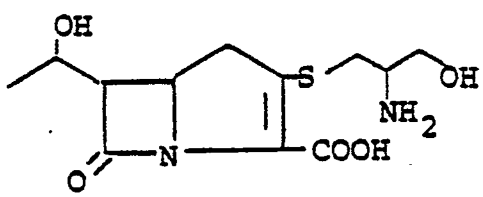

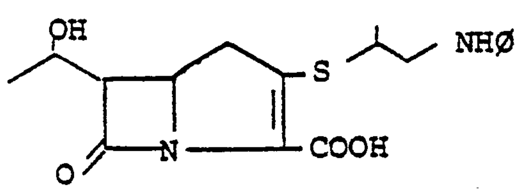

Ø = phenyl

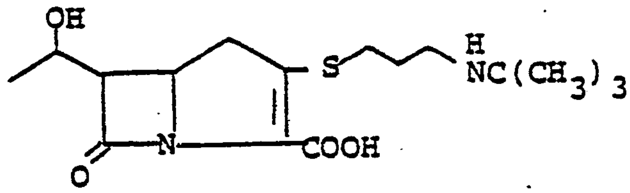

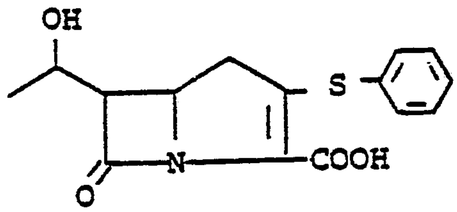

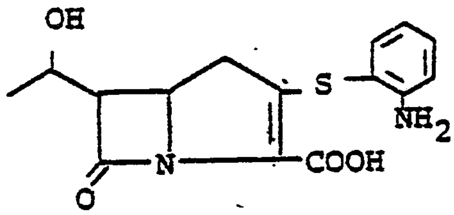

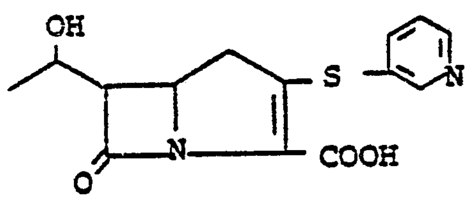

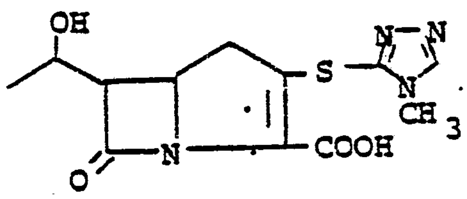

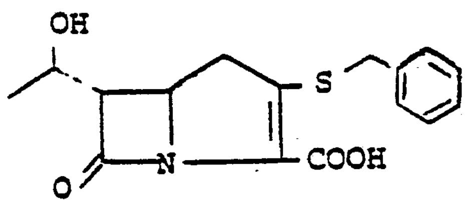

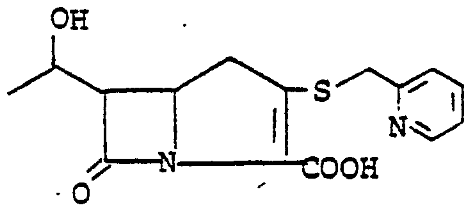

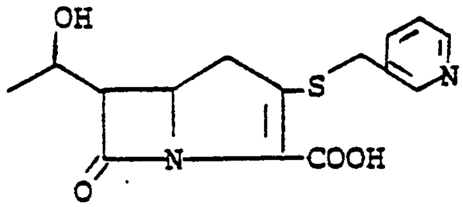

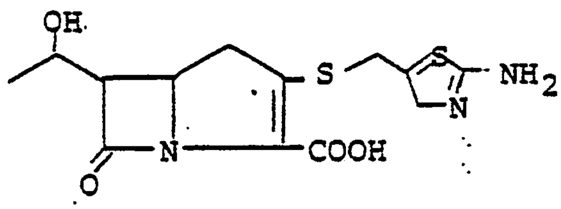

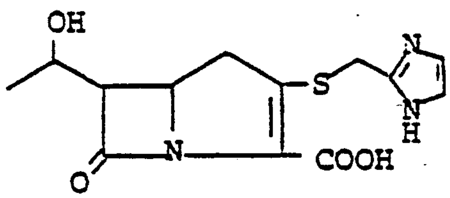

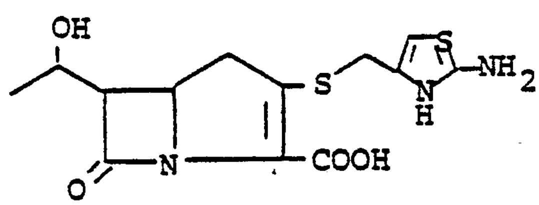

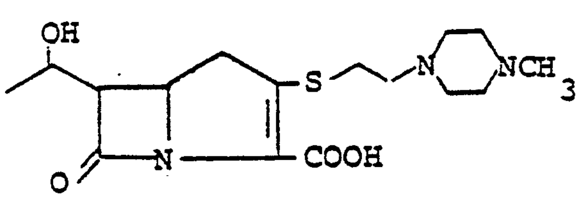

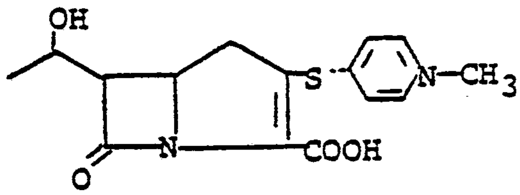

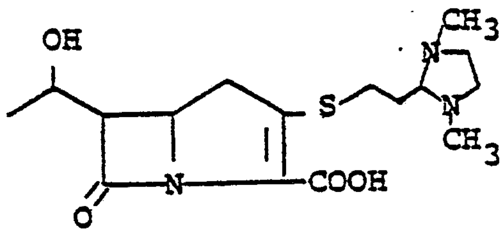

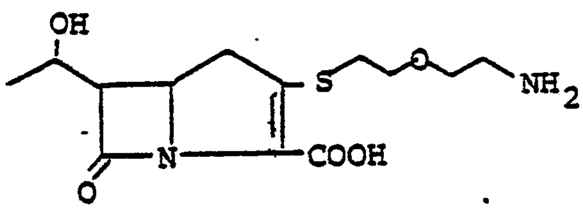

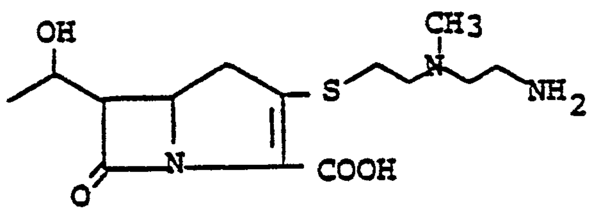

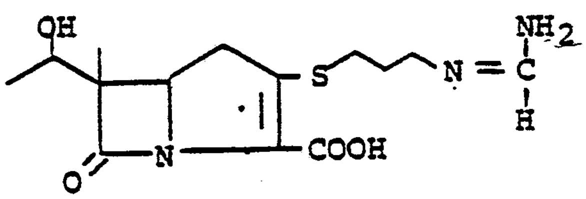

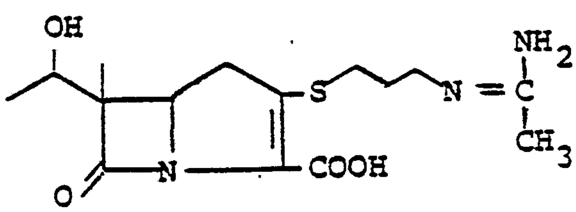

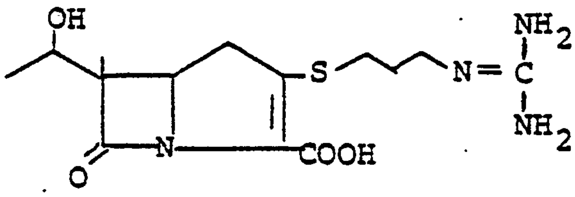

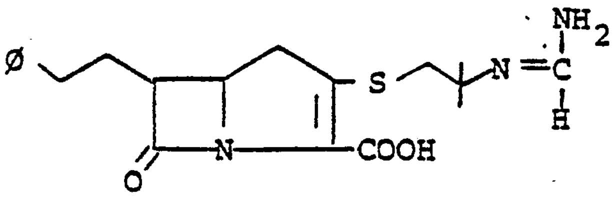

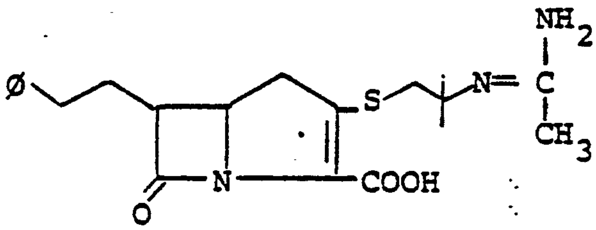

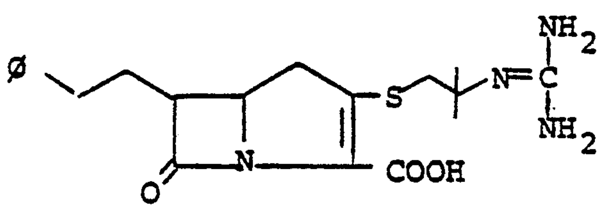

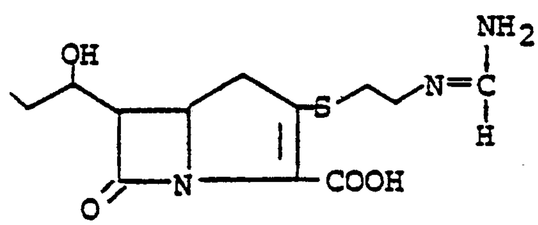

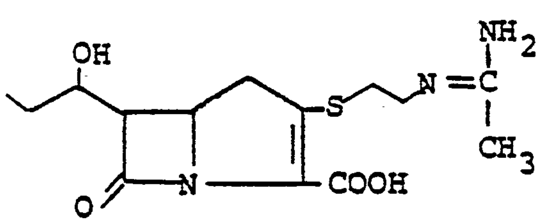

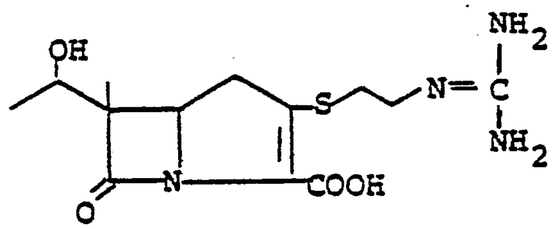

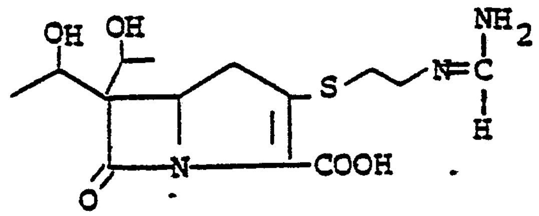

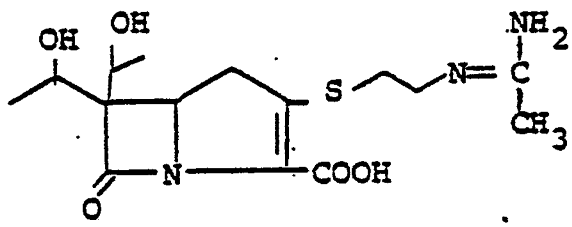

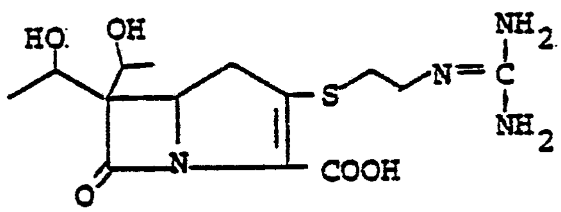

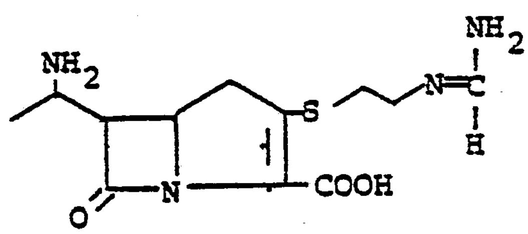

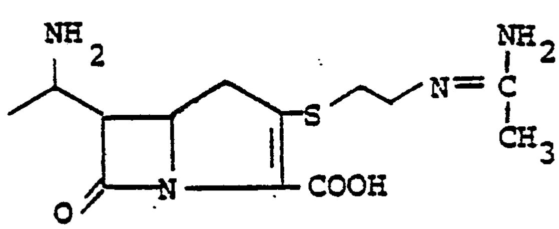

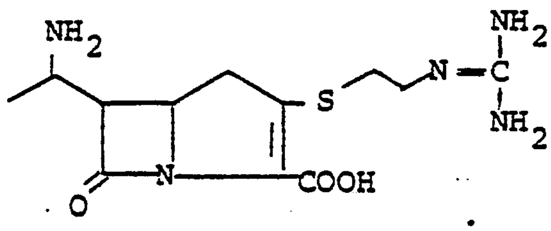

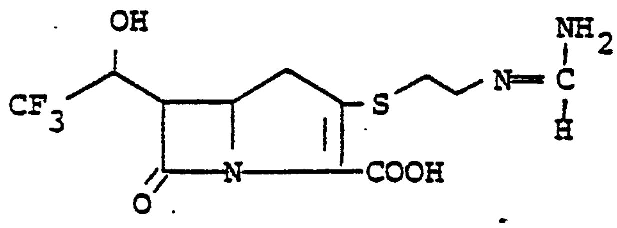

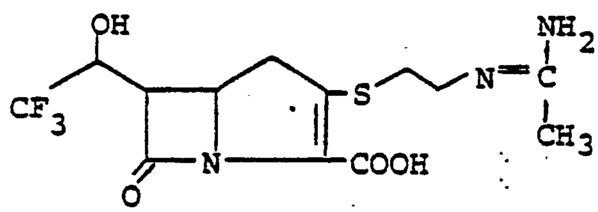

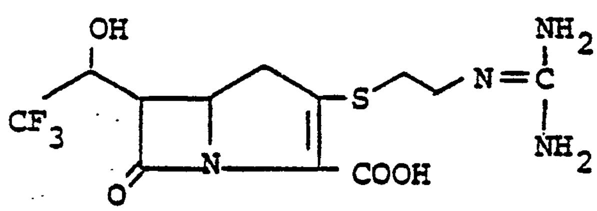

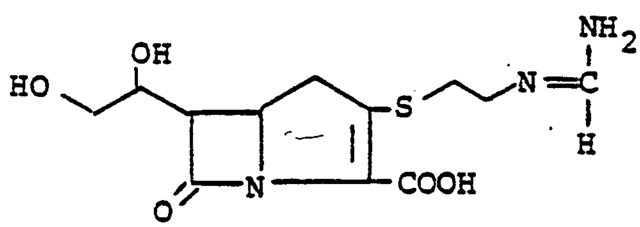

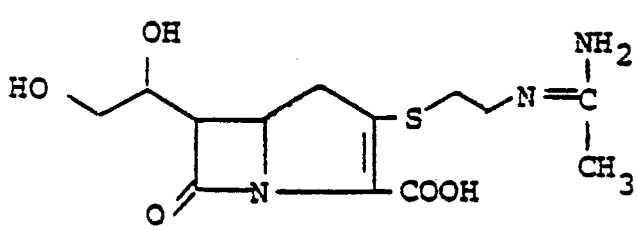

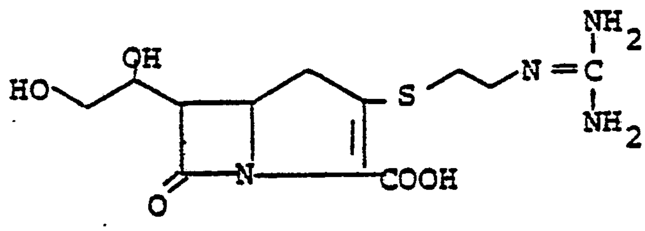

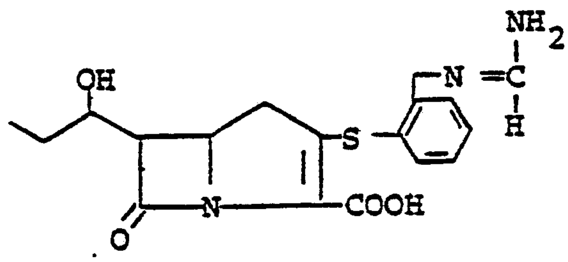

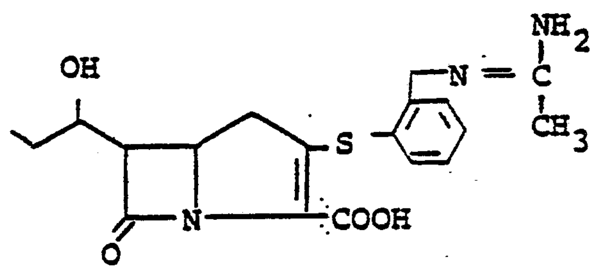

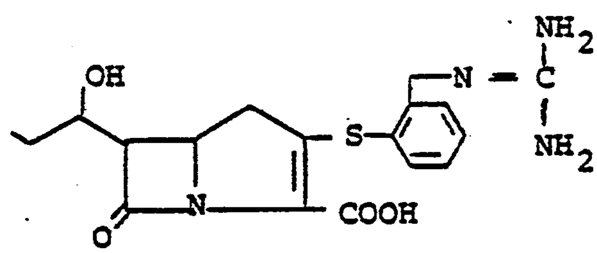

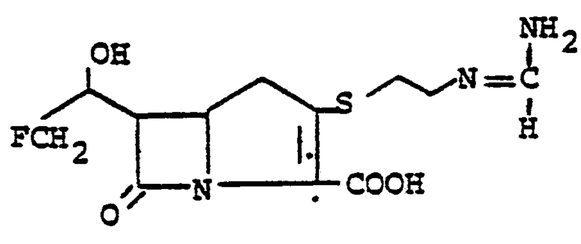

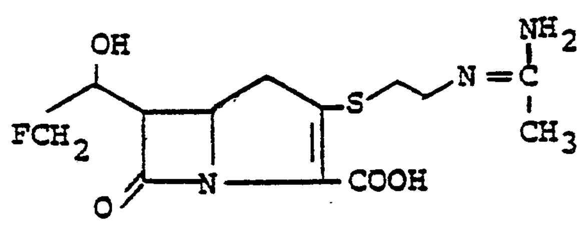

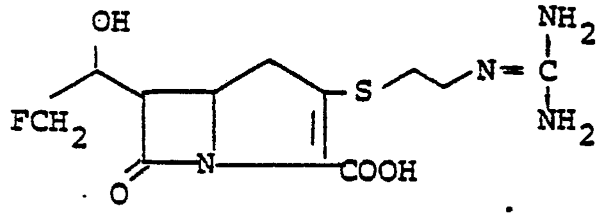

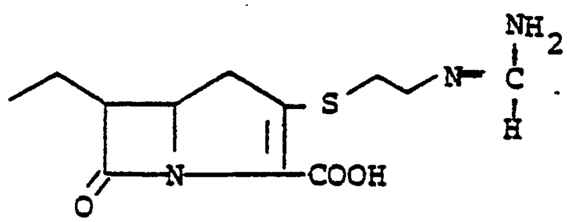

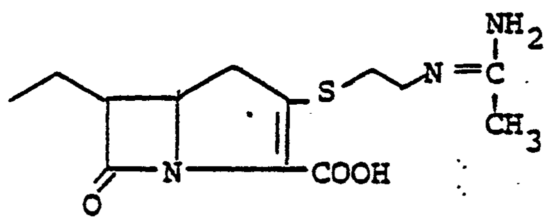

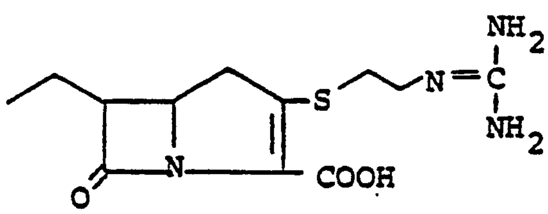

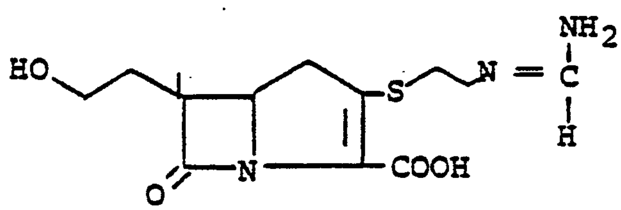

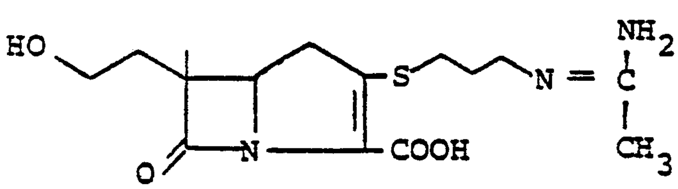

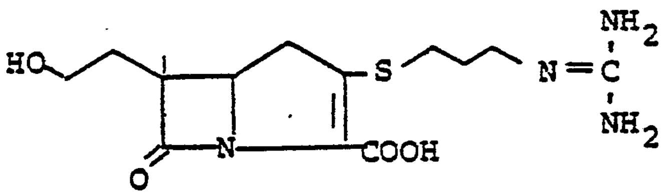

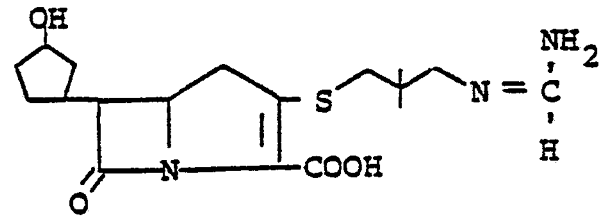

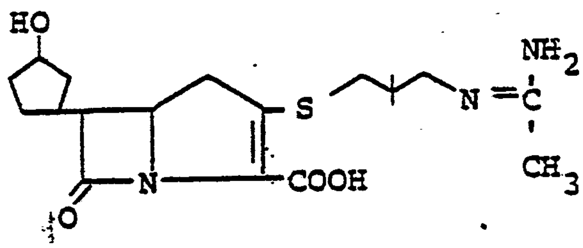

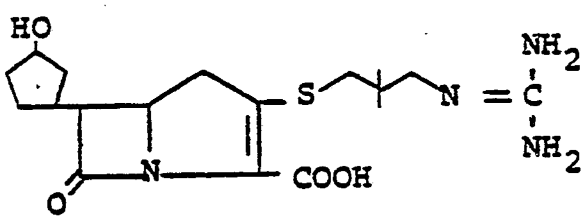

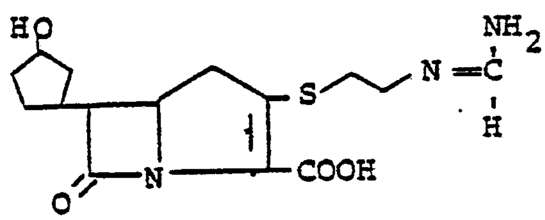

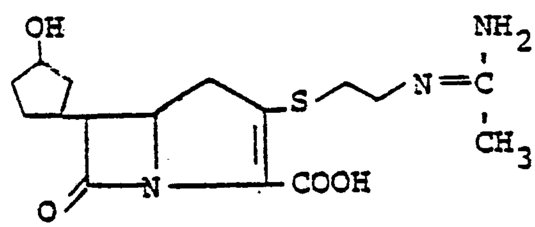

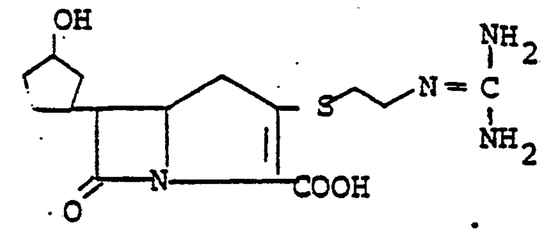

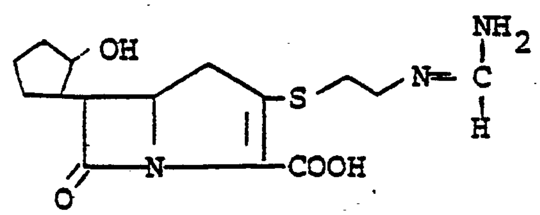

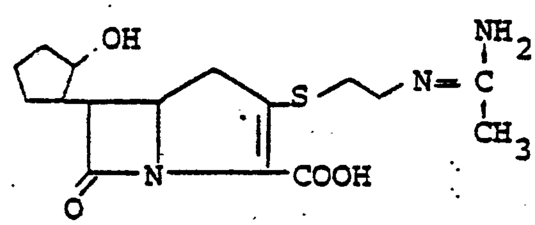

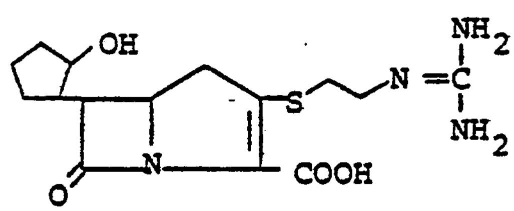

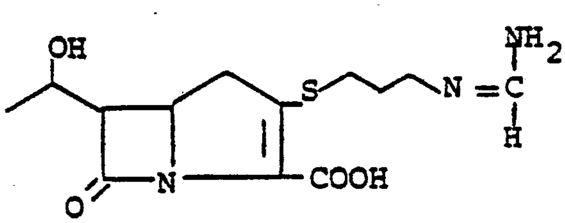

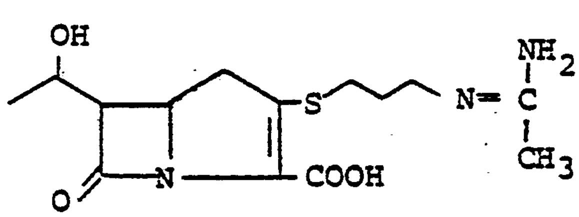

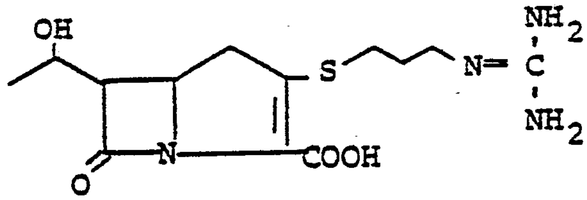

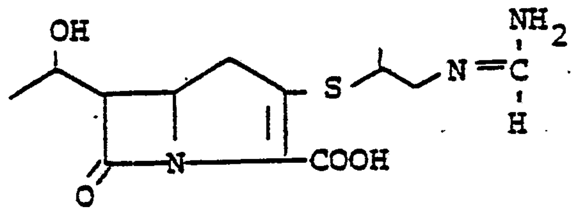

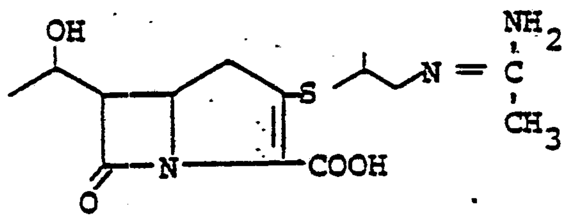

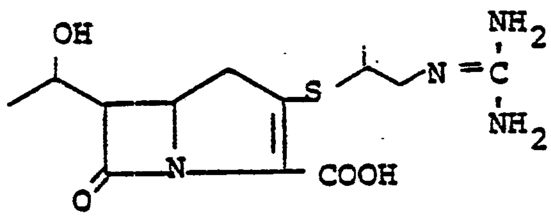

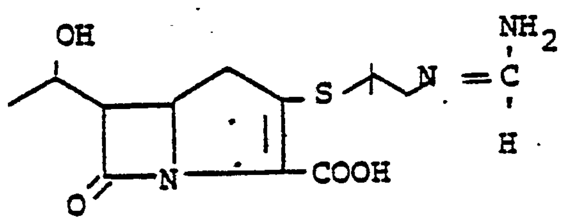

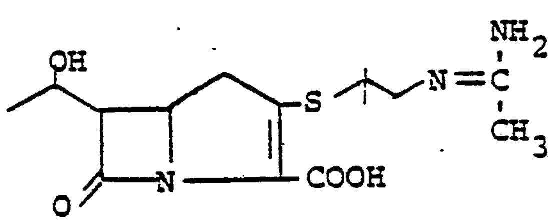

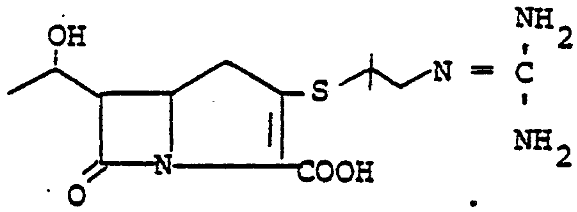

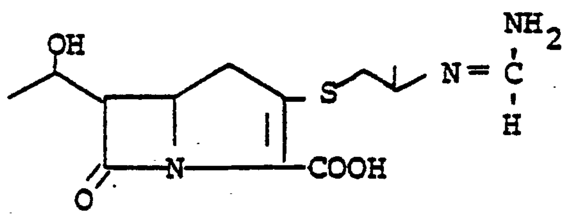

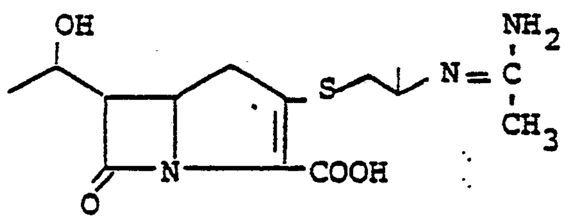

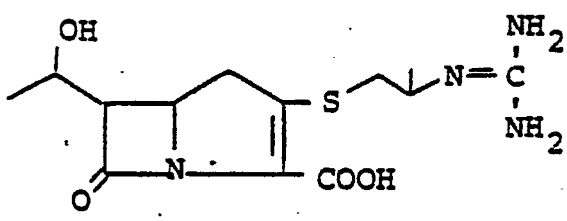

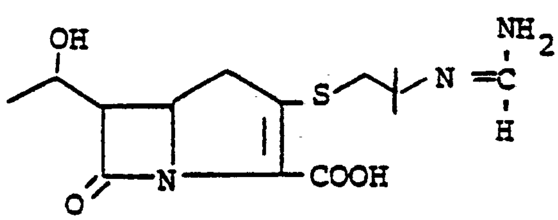

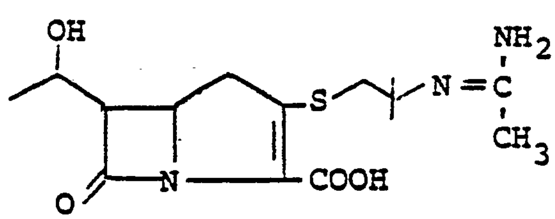

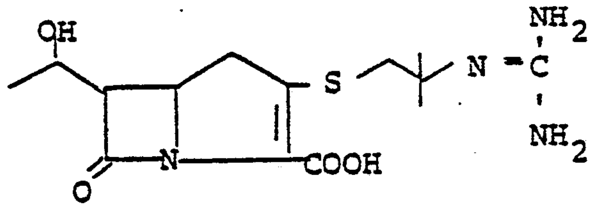

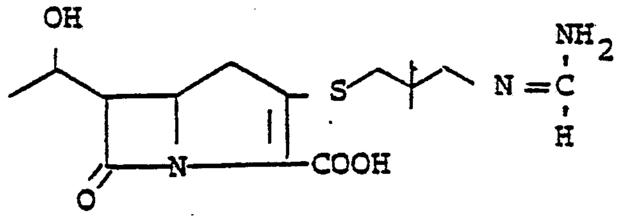

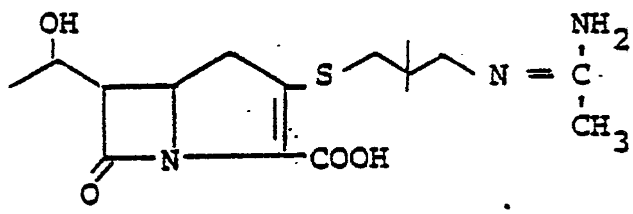

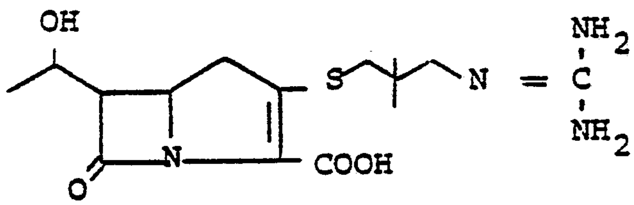

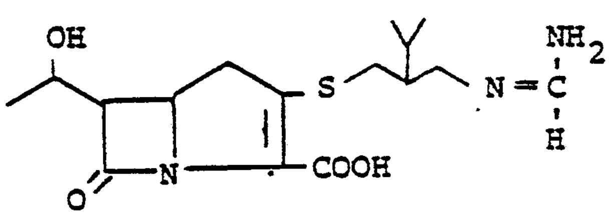

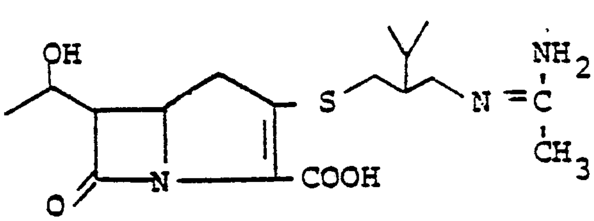

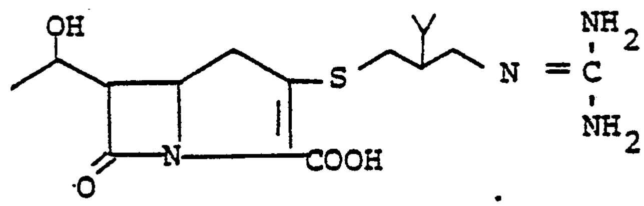

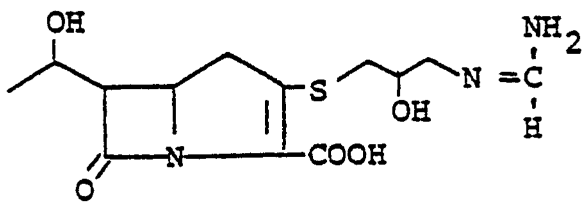

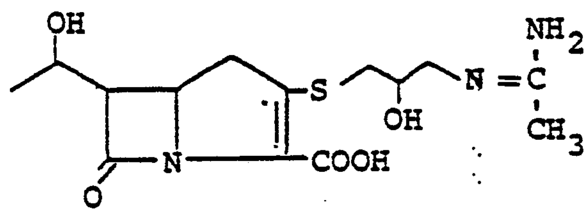

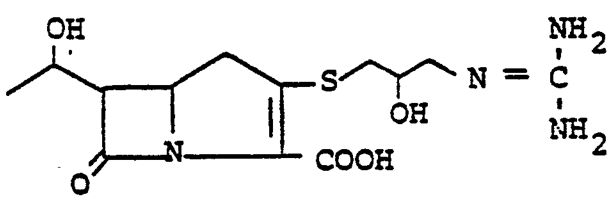

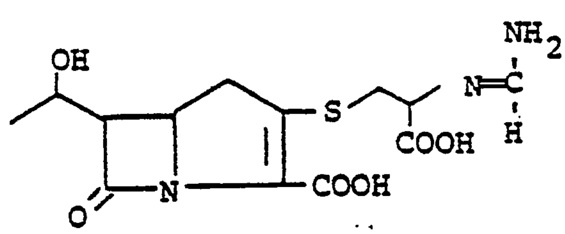

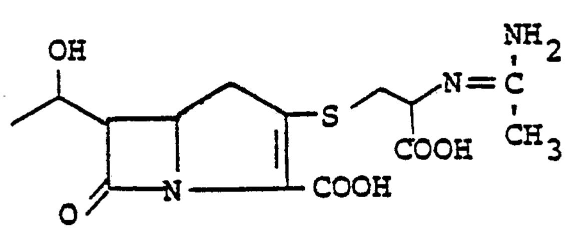

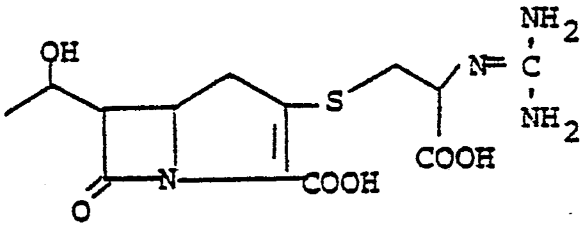

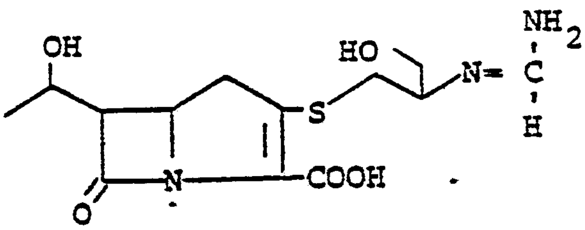

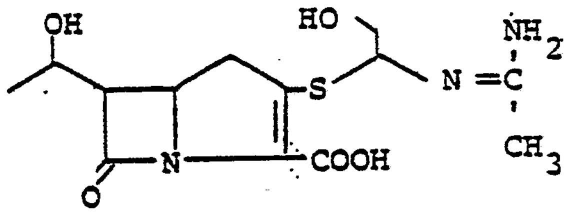

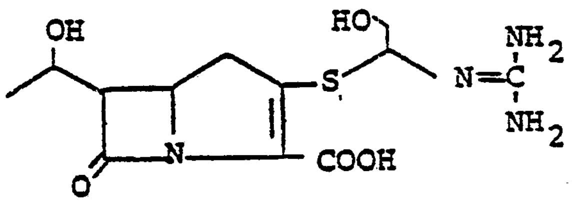

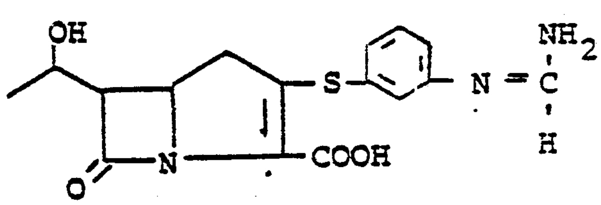

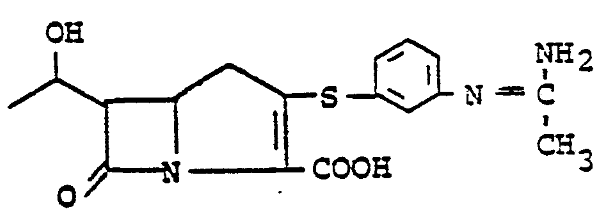

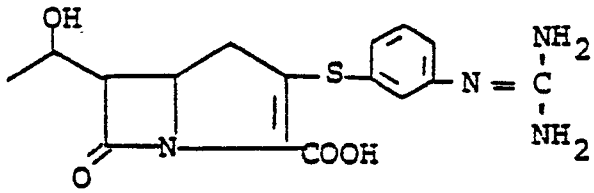

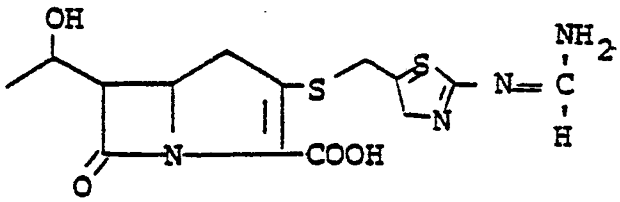

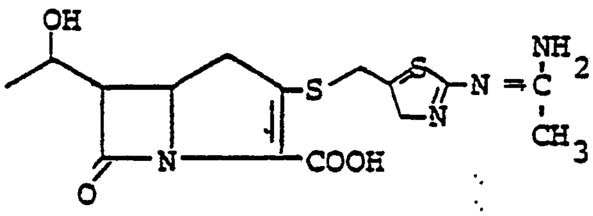

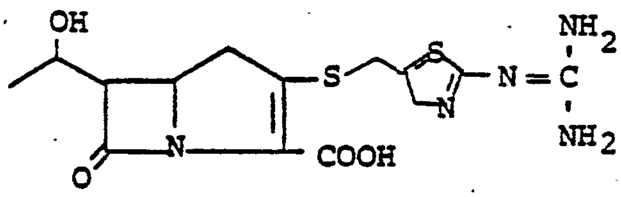

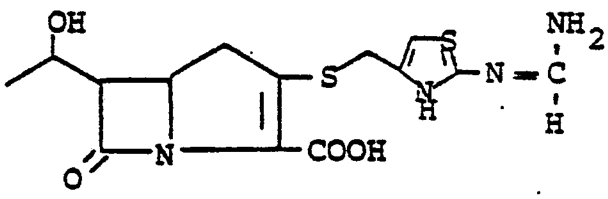

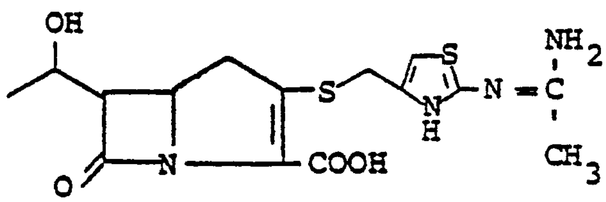

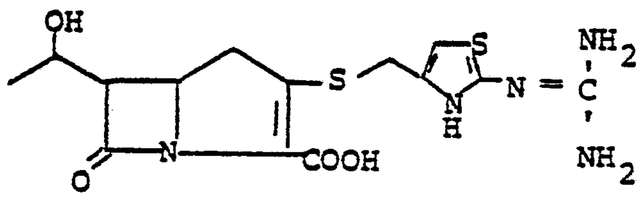

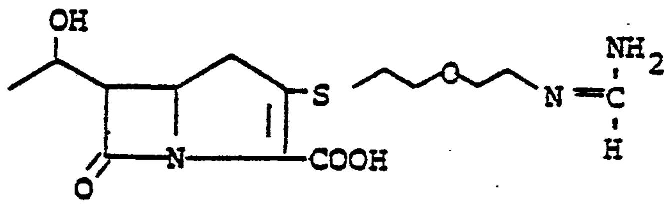

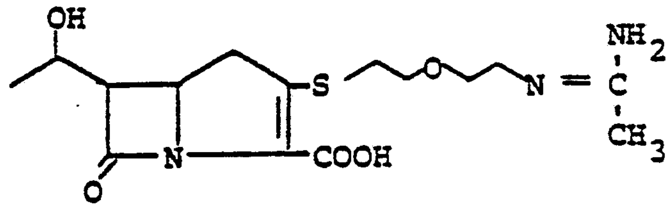

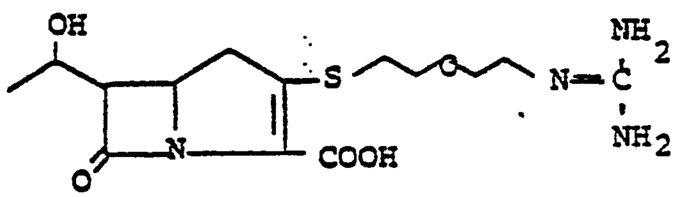

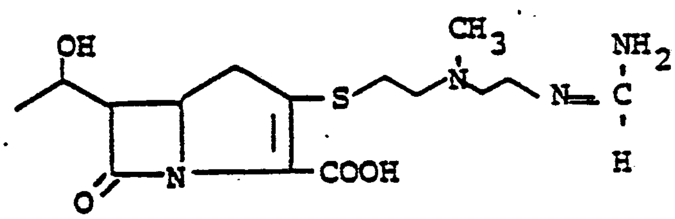

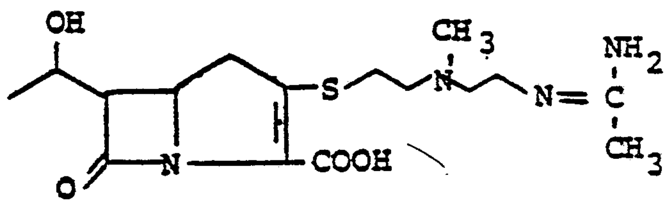

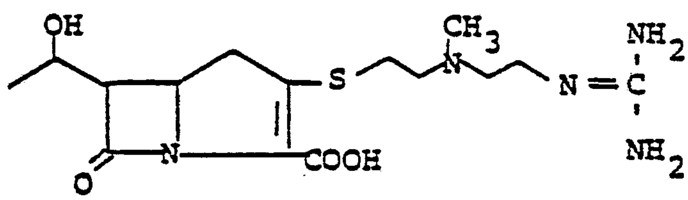

27. A compound according to Claim 1 having the structure:

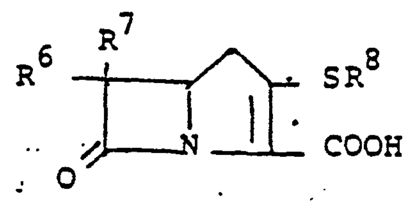

and the pharmaceutically acceptable salt and ester derivatives thereof; wherein $R^6$ and $R^7$ are independently selected from the group consisting of mercapto, hydroxyl, chloro, bromo, fluoro, hydrogen; substituted and unsubstituted: alkyl, alkoxyl, alkylthio, alkenyl, and alkynyl having from 1-6 carbon atoms; cycloalkyl, cycloalkylalkyl, and alkylcycloalkyl having 3-6 carbon atoms in the cycloalkyl ring and 1-6 carbon atoms in the alkyl moiety; aryl, aralkyl, aralkenyl, and aralkynyl wherein the aryl moiety is phenyl and the aliphatic moiety has 1-6 carbon atoms; heteroaryl, heteroaralkyl, heterocyclyl and heterocyclylalkyl wherein the alkyl moiety has 1-6 carbon atoms and wherein the heterocyclic structure comprises 4-6 atoms, the hetero atom or atoms being selected from the group consisting of 1-4 oxygen, nitrogen, or sulfur atoms; wherein the substituent or substituents on $R^6$ and $R^7$ are selected from the group consisting of: amino, mono- di- and trialkylamino, hydroxyl, alkoxyl, mercapto, alkylthio, chloro, bromo, fluoro, carboxy, oximino, ureido, alkoximino, and N-substituted ureido wherein the N-substituent or N-substituents are selected from alkyl, phenyl and phenylalkyl, and wherein the alkyl moieties of the aforementioned substituents have 1-6 carbon atoms; $R^8$ is selected from the group consisting of: hydrogen; substituted and unsubstituted:

alkyl, alkenyl, and alkynyl, having from 1-10 carbon atoms; cycloalkyl, cycloalkylalkyl, and alkylcycloalkyl, having 3-6 carbon atoms in the cycloalkyl ring and 1-6 carbon atoms in the alkyl moiety; aryl, aralkyl, aralkenyl, and aralkynyl wherein the aryl moiety is phenyl and the aliphatic moiety has 1-6 carbon atoms; heteroaryl, heteroaralkyl, heterocyclyl and heterocyclylalkyl having 4-6 ring atoms; wherein the substituent or substituents relative to $R^8$ are selected from the group consisting of: amino, mono-, di- and trialkylamino, hydroxyl, alkoxyl, mercapto, alkylthio, phenylthio, sulfamoyl, amidino, guanidino, nitro, chloro, bromo, fluoro, cyano and carboxy; and wherein the hetero atom or atoms in the above-named heterocyclic moieties are selected from the group consisting of 1-4 oxygen, nitrogen or sulphur atoms; and wherein the alkyl moieties of the above-recited substituents have 1-6 carbon atoms.

28. A compound according to Claim 27 wherein $R^8$ is selected from the group consisting of:

H,

$CH_3$,

$(CH_2)_2NH_2$,

$C(CH_3)_2CH_2NH_2$,

$C(CH_3)_2CH_2NH-\overset{NH}{\overset{\|}{C}}-H$ ,

$C(CH_3)_2CH_2NH-\overset{NH}{\overset{\|}{C}}-CH_3$ ,

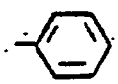 , 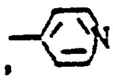 ,

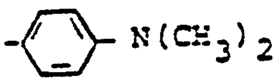 ,

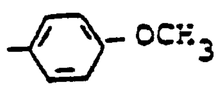 ,

$CH_2CH_2CH_2NH_2$ ,

$CH_2CH(CH_3)NH_2$ ,

$CH_2CH_2CH_2NH-\overset{NH}{\overset{\|}{C}}-H$ ,

$CH_2CH_2CH_2NH-\overset{NH}{\overset{\|}{C}}-CH_3$ ,

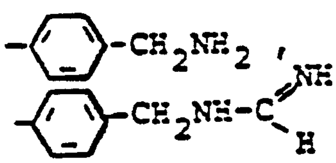 , ,

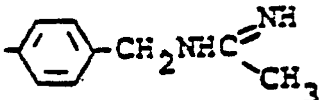 ,

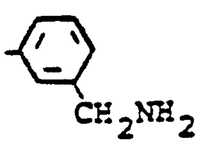 ,

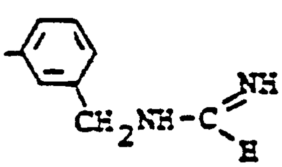 ,

$(CH_2)_2NH-\overset{NH}{\overset{\|}{C}}-H$ ,

$(CH_2)_2NH-\overset{NH}{\overset{\|}{C}}-CH_3$ ,

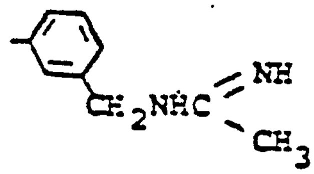

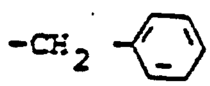

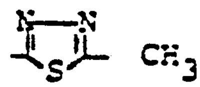 ,

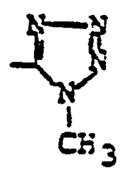 ,

$CH(CH_3)CH_2NH_2$,

$CH(CH_3)CH_2NH-\overset{NH}{\overset{\|}{C}}-H$ , or

$CH(CH_3)CH_2NH-\overset{NH}{\overset{\|}{C}}-CH_3$ .

$-CF_3$

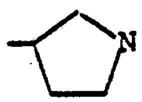

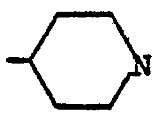

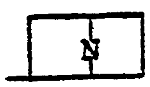

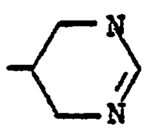

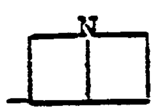

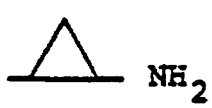

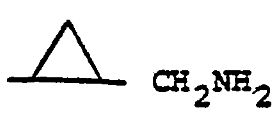

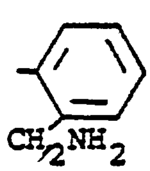

29. A compound according to Claim 28 wherein: $R^7$ is hydrogen, hydroxyl, alkoxyl, alkylthio, mercapto, chloro, fluoro, bromo, alkyl and substituted alkyl wherein the substituent or substituents are: alkoxyl or hydroxyl.

30. A compound according to Claim 29 wherein $R^7$ is hydrogen, hydroxyl, $OCH_3$, $CH_3$, hydroxyl- and polyhydroxyl-substituted alkyl.

31. A compound according to Claim 30 wherein $R^7$ is hydrogen, $CH_3$ or $OCH_3$.

32. A compound according to Claim 31 wherein $R^6$ is: hydrogen; substituted and unsubstituted: alkyl, cycloalkyl, cycloalkylalkyl, phenylalkyl; wherein the substituent or substituents are selected from: hydroxyl, chloro, fluoro, bromo, carboxyl, oximino, alkoximino, ureido, amino, alkoxyl, or alkylthio.

33. A compound according to Claim 32 wherein the substituent or substituents on $R^6$ are hydroxyl.

34. A compound according to Claim 27 wherein $R^7$ is hydrogen, hydroxyl, alkoxyl, alkylthio, chloro, fluoro, bromo, alkyl and substituted alkyl wherein the substituent or substituents are: alkoxyl, or hydroxyl; and $R^6$ is: hydrogen; substituted and unsubstituted: alkyl, cycloalkyl, cycloalkylalkyl, phenylalkyl; wherein the substituent or substituents are selected from: hydroxyl, chloro, fluoro, bromo, oximino, alkoximino, ureido, amino, alkoxyl or alkylthio.

35. A compound according to Claim 28 wherein $R^7$ is hydrogen, hydroxyl, alkoxyl, alkylthio, mercapto, chloro, fluoro, bromo, alkyl and substituted alkyl wherein the substituent or substituents are: alkoxyl, or hydroxyl; and $R^6$ is hydrogen; substituted and unsubstituted: alkyl, cycloalkyl, cycloalkylalkyl, phenylalkyl; wherein the substituent or substituents are selected from: hydroxyl, chloro, fluoro, bromo, oximino, alkoximino, ureido, amino, alkoxyl or alkylthio.

36. A compound according to Claim 35 wherein $R^7$ is hydrogen, $CH_3$ or $OCH_3$.

37. A compound according to Claim 36 wherein $R^6$ is selected from: H, 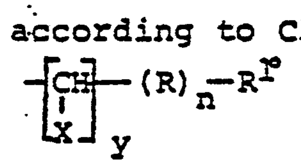

wherein: y = 0 or 1;
X = OH, $NH_2$; SH;
n = 0 or 1;
R = substituted or unsubstituted: alkyl, alkenyl or alkynyl having 1-6 carbon atoms wherein the substituent is $R^1$;
$R^1$ = alkoxyl, carboxyl, $CF_3$, OH, H, linear or branched alkyl bearing 1 or more hydroxyl groups, amino, aminoalkyl, Cl, F, Br, alkylthio, amidino, guanidino, oximino, phenyloxy, phenylthio,

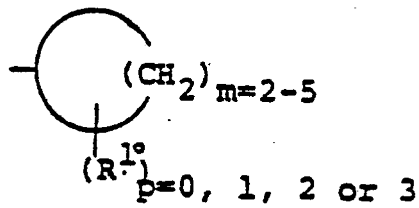

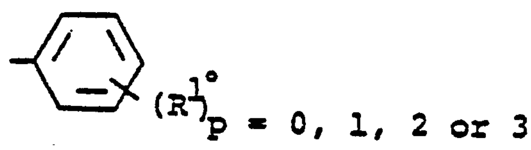

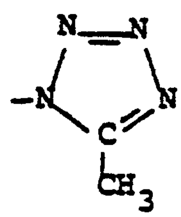

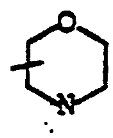

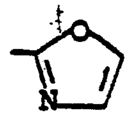

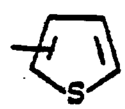

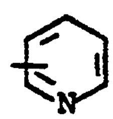

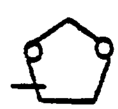

38. A compound according to Claim 36 wherein $R^6$ is selected from:

$-H$

$-CH_2OH$

$-CH(OH)CH_3$

$-CH_2CH_2OH$

$-CH(OH)CH(CH_3)_2$

$-CH(OH)CH_2CH(CH_3)_2$

$-CH_2CH_2CH_2OH$

$-CH_2CH_2CH_2CH_2OH$

$-CH(OH)CH_2CH_2OH$

$-CH(OH)CH_2-$ 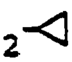

$-CH(OH)CH_2-$ 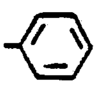

$-CH(OH)CH_2CH_2-$ 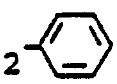

$-CH(OH)CH_2$ 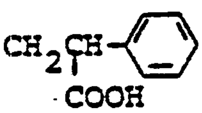

$-CH(OH)CH_2CH_2$ 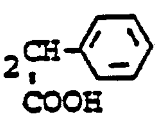

$-CH(OH)CH_2-$ 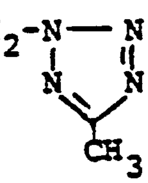

$-CH(OH)CH_2-$ 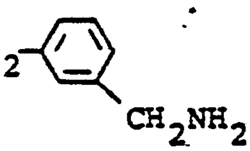

$-CH(OH)$ 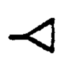

$-CH(OH)CH_2CH_3$

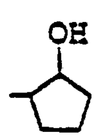

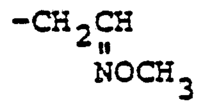

$-CH(OH)CF_3$

$-CH(OH)-$ 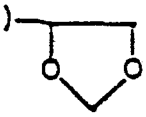

$-CH(NH_2)CH_3$

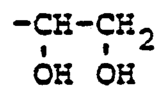

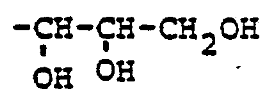

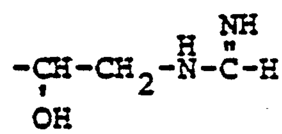

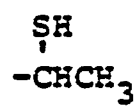

$-CH(OH)CH_2CH_3$

$-CH(OH)CH_2CH_2$ 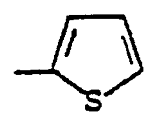

$-CH(OH)CH_2CH_2-O$ 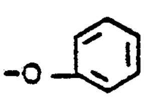

$-CH(OH)CH_2Cl$

$-CH(OH)CH_2F$

39. A compound according to Claim 38 wherein $R^8$ is:

$-CH_2CH_2NH_2$

$$\begin{array}{l} \qquad\qquad\quad NH_2 \\ -CH_2CH_2N{=}\overset{|}{C}{-}H \end{array}$$

$$\begin{array}{l} \qquad\qquad\quad NH_2 \\ -CH_2CH_2N{=}\overset{|}{\underset{|}{C}} \\ \qquad\qquad\quad NH_2 \end{array}$$

$$\begin{array}{l} \qquad\qquad\quad NH_2 \\ -CH_2CH_2N{=}\overset{|}{\underset{|}{C}} \\ \qquad\qquad\quad CH_3 \end{array}$$

40. A compound according to Claim 27 selected from the group consisting of:

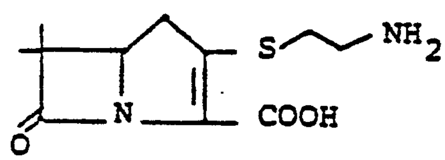

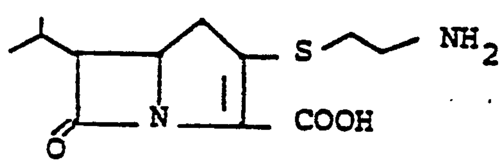

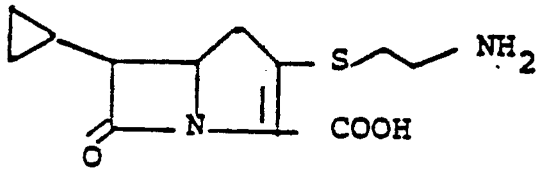

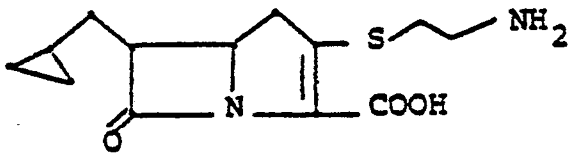

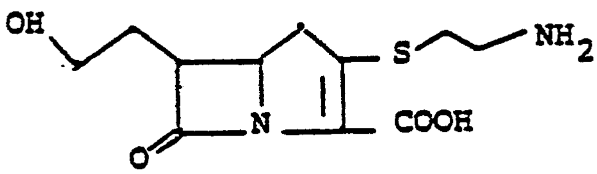

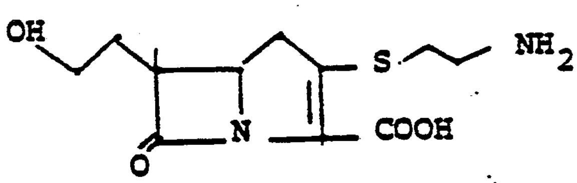

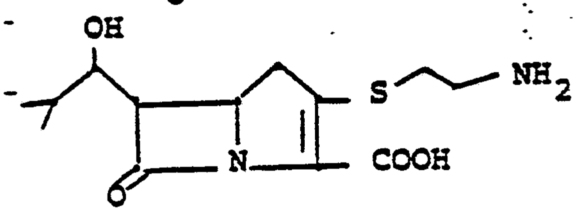

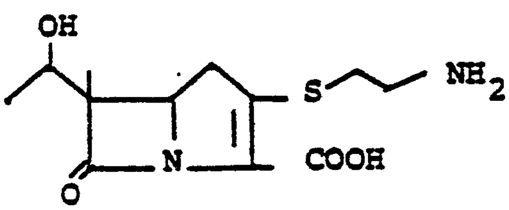

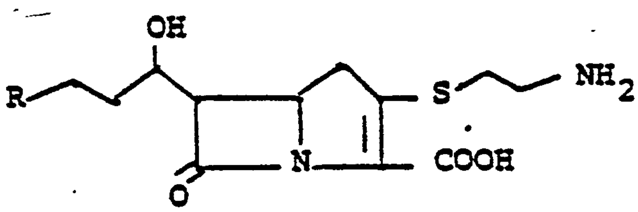

(R = phenyl, m-aminomethylphenyl, o-, p-, m-hydroxyphenyl)

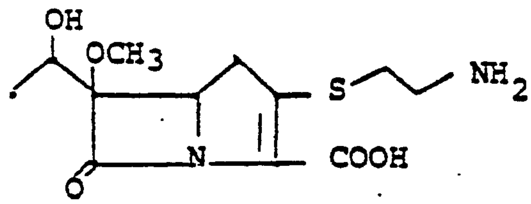

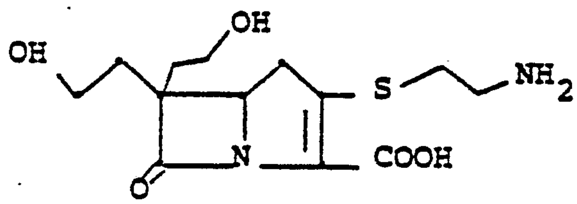

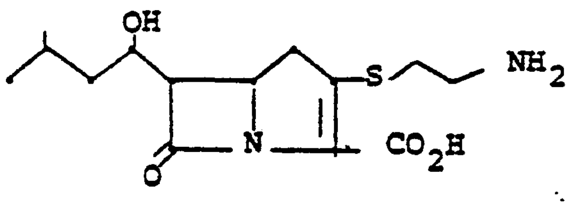

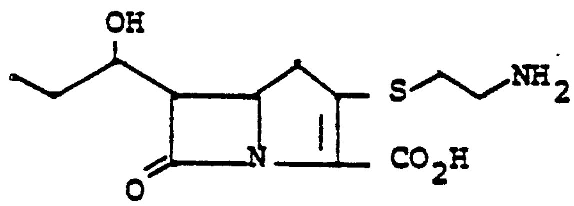

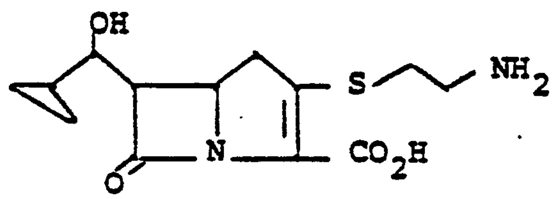

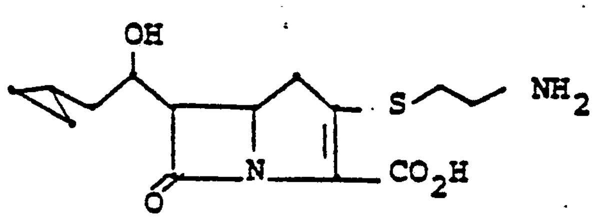

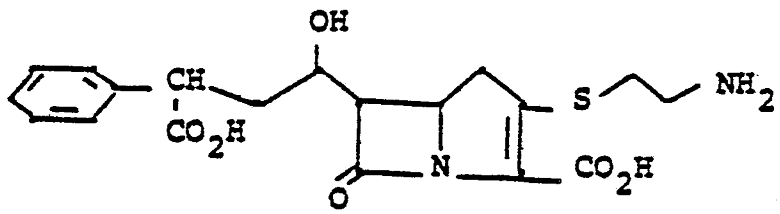

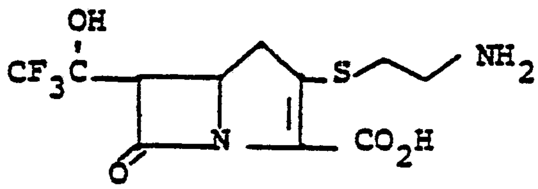

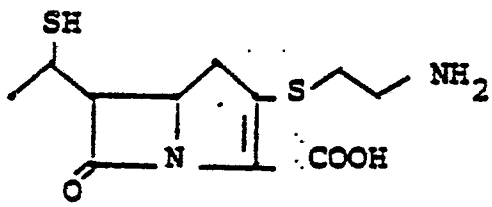

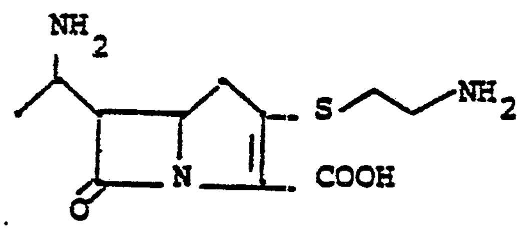

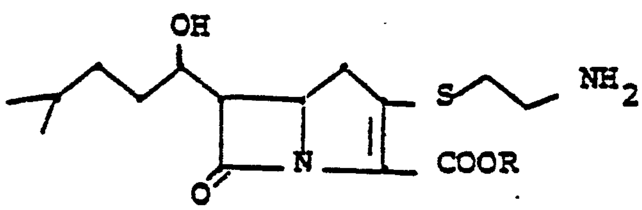

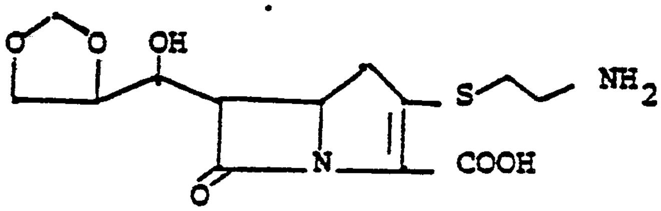

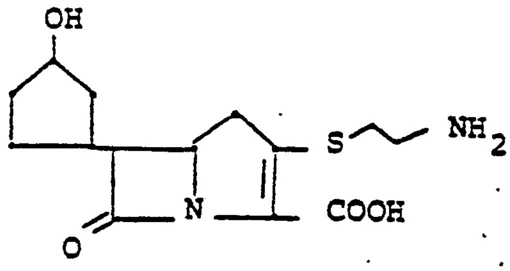

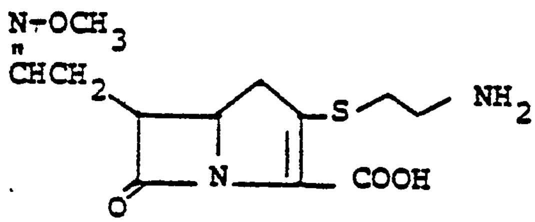

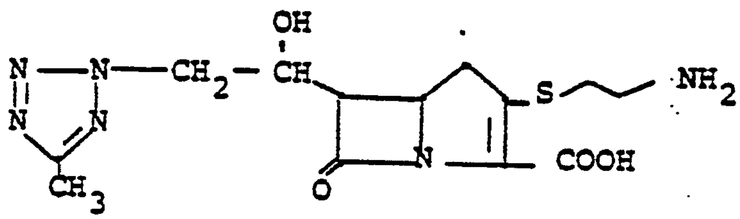

41. A compound according to Claim 40 wherein the aminoethylthio side chain, 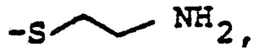, is replaced by a member of the group consisting of:

$-SCF_3$,

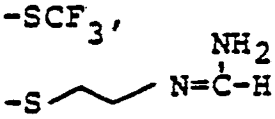 ,

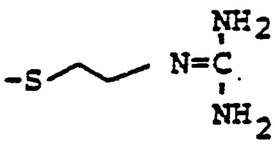 ,

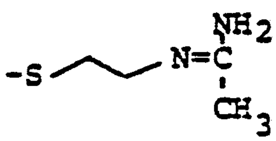 ,

$-S-(CH_2)_n-NH_2$ ,

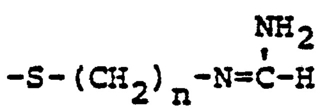 ,

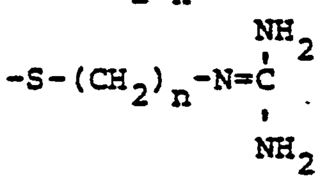 ,

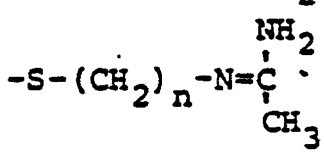 ,

n = 1, 3, 4, 5 or 6.

42. A process for preparing a compound according to Claims 1-40 or 41 comprising: halogenating followed by cyclizing in the presence of base:

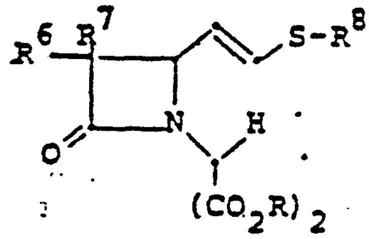

to form:

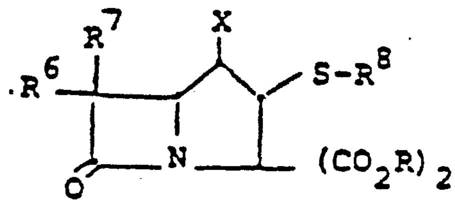

dehydrohalogenating the resulting intermediate in the presence of base to form:

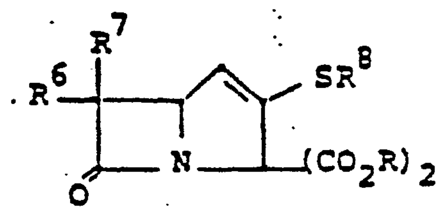

heating the resulting intermediate in the presence of a displacing agent to form:

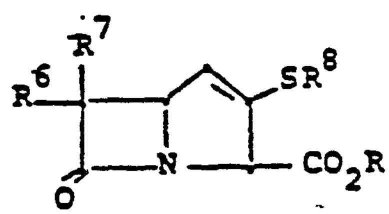

isomerizing the position of the double bond of the resulting intermediate to form:

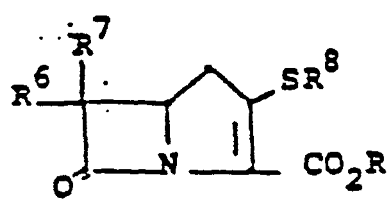

wherein X is halo.

43. A process for preparing a compound according to Claims 1-40 or 41 comprising treating:

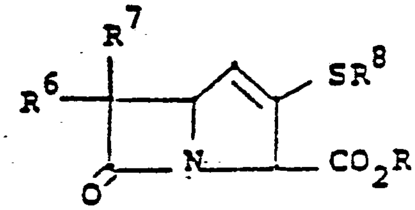

with a base capable of isomerizing the double bond to form:

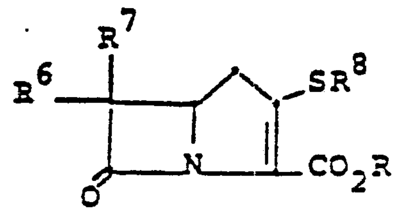

44. A process for preparing a compound of the formula:

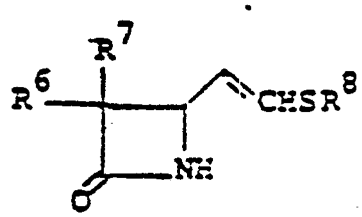

comprising reacting:

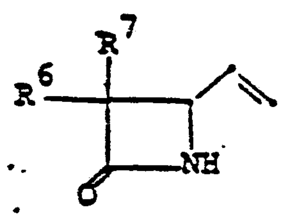

with $XSR^8$ to yield

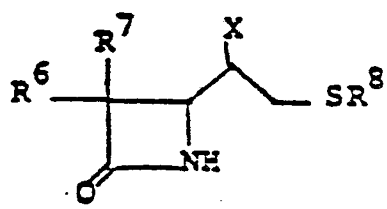

followed by elimination of HX; wherein X is halo.

45. A process for preparing a compound according to Claims 1-40 or 41 comprising reacting:

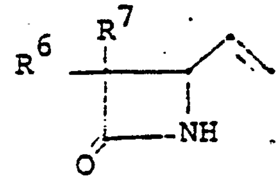

with $XSR^8$ wherein X is halo.

46. An antibiotic pharmaceutical composition comprising a therapeutically effective amount of a compound according to Claims 1-40 or 41 and a pharmaceutical carrier therefor.

47. A compound according to Claim 1 wherein $R^7$ is hydrogen and $R^6$ is $CH_3\underset{OH}{CH}-$ .

European Patent Office

# EUROPEAN SEARCH REPORT

0017992

Application number EP 80 10 2076

## DOCUMENTS CONSIDERED TO BE RELEVANT

| Category | Citation of document with indication, where appropriate, of relevant passages | Relevant to claim |
|---|---|---|
| | DE - A - 2 751 597 (MERCK)<br>* Claims 7-11 *<br>-- | 42-45 |
| P | EP - A - 0 001 628 (MERCK)<br>* Claims *<br>-- | 1-25, 42-47 |
| P | EP - A - 0 001 627 (MERCK)<br>* Claims *<br>-- | 1-25, 42-47 |
| P | EP - A - 0 008 497 (BEECHAM)<br>* Claims *<br>-- | 1,46 |
| P | EP - A - 0 008 514 (BEECHAM)<br>* Claims *<br>-- | 1,46 |
| P | EP - A - 0 008 888 (BEECHAM)<br>* Claims *<br>-- | 1,46 |
| E | EP - A - 0 011 173 (SANRAKU-OCEAN)<br>* Claims 1-11,13 *<br>---- | 1,46 |

CLASSIFICATION OF THE APPLICATION (Int. Cl. 3)

C 07 D 487/04
205/08
A 61 K 31/40//
C 07 D 498/04
405/12
C 07 C 125/06
69/66
C 07 F 7/08
(C 07 D 487/04
209/00
205/00)

TECHNICAL FIELDS SEARCHED (Int.Cl. 3)

C 07 D 487/04
205/08
A 61 K 31/40

CATEGORY OF CITED DOCUMENTS

X: particularly relevant
A: technological background
O: non-written disclosure
P: intermediate document
T: theory or principle underlying the invention
E: conflicting application
D: document cited in the application
L: citation for other reasons

&: member of the same patent family, corresponding document

The present search report has been drawn up for all claims

| Place of search | Date of completion of the search | Examiner |
|---|---|---|
| The Hague | 24-06-1980 | CHOULY |